# EUROPEAN PATENT SPECIFICATION

(11) **EP 4 110 276 B1**
(45) Date of publication and mention of the grant of the patent: **28.01.2026**
(21) Application number: 21830680.1
(22) Date of filing: 09.12.2021
(51) Int. Cl.: A61K 8/34, A61K 31/573, A61K 31/425, A61K 31/23, A61K 31/045, A61K 9/00, C11D 3/20, A61L 9/14, A61K 47/10, A61Q 19/00, A61Q 17/04, A61Q 17/00, A61Q 15/00, A61Q 13/00, A61Q 11/00, A61Q 5/12, A61Q 5/06, A61Q 5/02, C11D 3/34, C11D 3/00

(54) **COMPOSITIONS COMPRISING ALKANEDIOLS**
ZUSAMMENSETZUNGEN MIT ALKANDIOLEN
COMPOSITIONS COMPRENANT ALCANEDIOLS

(30) Priority: 09.12.2020 WO PCT/EP2020/085179
(43) Date of publication of application: 04.01.2023
(60) Divisional application: 24208974.6
(73) Proprietor: Symrise AG, 37603 Holzminden (DE)
(72) Inventor: LANGE, Sabine, 37603 Holzminden (DE); BUGDAHN, Nikolas, 37603 Holzminden (DE); KRAELING, Ricarda, 37603 Holzminden (DE)
(74) Representative: Global IP Europe Patentanwaltskanzlei
(86) International application number: PCT/EP2021/085023
(87) International publication number: WO 2022/122935

(56) References cited:
- WO-A1-2005/123028
- WO-A1-2018/099570
- WO-A2-2018/154145
- AU-A4- 2010 100 972
- JP-A- H11 322 591
- US-A1- 2012 129 937
- US-A1- 2012 213 717
- US-A1- 2013 136 709
- US-A1- 2015 250 722

## Description

### Technical field

The present invention relates to a cosmetic, pharmaceutical, preferably dermatological, composition or homecare product comprising or consisting of a specific lipophilic active component and an effective amount of a mixture of two or more different specific alkanediols and the use of said compositions as a cosmetic, for personal care, as a pharmaceutical, for animal care or for homecare. Additionally, the present invention relates to the use of a mixture of two or more different specific alkanediols for improving the solubility of a specific lipophilic active component in a cosmetic or pharmaceutical composition. Finally, the present invention relates to the use of a mixture of two or more \different specific alkanediols as solubilizer replacer in a cosmetic or pharmaceutical composition or homecare product.

### Background Art

Cosmetic, personal care, dermatological pharmaceutical preparations or homecare products are complex mixtures intended to be applied to the external parts of the human body or for laundring, washing and cleaning purposes. In the long list of ingredients are included lipophilic active components. A broad spectrum of lipophilic active components are extensively used in a wide range of cosmetic, personal care, or pharmaceutical preparations to serve a variety of functions, such as anti-inflammatory, anti-itching, anti-redness, anti-dandruff, anti-microbial, anti-perspirant, anti-cellulite, anti-viral, anti-oxidative, anti-wrinkle, skin-whitening, skin tanning, pain-reducing, swelling reduction, skin barrier strengthening, anti-mycotic, anti-sagging, deodorant and skin soothing properties, etc.

The drawback of these lipophilic active components is their poor water solubility. Their poor water solubility makes it difficult, to incorporate these components in aqueous, aqueous/alcoholic or aqueous/glycolic formulations such as gels, liquid soaps, shampoos, gels, etc. and even in emulsions. The incorporation of these components becomes more difficult with increased water content of the aqueous, aqueous/alcoholic or aqueous/glycolic formulations. Due to their poor water solubility, it is in particular difficult, to prepare clear and non-turbid formulations such as solutions for wet wipe, micellar water, mouth wash, after shave, cleansing solution, hair tonic, hair and body mist, facial serum, facial toner, deos, etc.

In order to improve the solubility of lipophilic active components or ingredients in cosmetic and pharmaceutical compositions or homecare products, solubilizers are widely used. A solubilizer is an ingredient that helps solubilize, i.e. make soluble, an ingredient which is otherwise insoluble in a medium. The best and most widely application of a solubilizer is to solubilize and to uniformly incorporate, i.e. dissolve, a lipophilic component, such as an essential oil, into a water-based formulation.

A solubilizer is neither a dispersant nor an emulsifier although all three solubilizer, dispersant or emulsifier belong to the surfactant family. An emulsifier helps two immiscible ingredients blend to an emulsion, for example to make an O/W or a W/O emulsion. The created emulsion usually has a particle size in micrometer range and appears milky. With an emulsifier, the dispersed phase has a concentration (depending on the emulsifier) between 1 to 80 %. The two immiscible ingredients are usually water and plant oils, fatty esters, waxes or fatty acids.

Solubilizers are similar to emulsifiers in that they have both hydrophilic and lipophilic traits, but solubilizers tend to be completely water soluble and only a little oil soluble. In practice this means they can suspend smaller amounts of oils. With a solubilizer, the dispersed phase has a particle size in nano-meter range and because they are solubilizing only small amounts of lipophilic active substances, the overall solution can still appear clear or lightly hazy.

Usually essential oils, fragrance oils or preservatives with oil soluble components (such as benzyl alcohol) or other lipophilic active substances in water (or hydrosols) are solubilized. The concentration of the to-be solubilized ingredients is usually between 0.01 to 5.0 %.

Dispersants are somewhere between solubilizers and emulsifiers. They can disperse very low concentrations of oils (such as vitamin E or even a triglyceride) in water. They don't completely solubilize the oil though; the product is not completely transparent. They disperse the oil in a translucent to milky solution which remains stable for a reasonable period of time.

Apart from the appearance, there is a safety and functional problem with such formulations. In a uniformly solubilized product, the lipophilic active ingredients are uniformly dispersed in the product as very small droplets and their concentration is exactly the same through the whole solution from the very first splash to the very last ml till the bottle is completely empty. Without the use of a solubilizer the distribution of the lipophilic active ingredient, would be quite haphazardly in the solution and would vary from application to application.

Solubilizers are commonly used in water-based formulas such as aqueous, aqueous alcoholic, aqueous glycolic preparations, gels, liquid soaps, shampoo but also used in products such as bath oils, tonics, and room sprays. They are also used to stabilize suspensions and prepare colloids and gels.

However, solubilizers have different negative side effects like increased foam formation. This behaviour is undesired in non-surfactant based formulations. Often, they are based on ethoxylated fatty alcohols which have negative side effects on the skin since they increase the skin permeation.

In addition, the current interest in "natural" cosmetics has turned the attention of the cosmetic chemist back to improved versions of the classical raw materials or natural and renewable raw materials, i.e. which are sustainable.

Thus, there is still an ongoing need to provide further substances which can improve the solubility of lipophilic active ingredients in topical compositions, and thus, increase their availability in the composition, and which do not have the above-described negative aspects of commonly used solubilizers.

When searching for agents improving solubility it must be taken into account that the substances used in the cosmetic or pharmaceutical sector must be toxicologically acceptable, well tolerated by the skin, stable, especially in the customary cosmetic and/or pharmaceutical formulations, substantially and preferably odourless and able to be preferred inexpensively, i.e. using standard methods.

However, there is no clear dependency between the chemical structure of a substance, on the one hand, and its capability in improving solubility, on the other hand, which makes the search for suitable substances more difficult. Furthermore, there is no predictable relationship between solubility improvement, toxicological acceptability, tolerability and the stability of a substance.

**It** is therefore an object of the present invention to provide a cosmetic or pharmaceutical composition, in particular dermatological, or homecare product, comprising or consisting of a lipophilic active component which has an improved solubility in the cosmetic or pharmaceutical composition or homecare product.

A further object of the present invention is to provide a cosmetic or pharmaceutical composition or homecare product, which is stable, i.e. in which the lipophilic active component remains solubilized and in which undesired crystallizing-out or precipitation of the lipophilic active component or undesired turbidity of the composition is prevented even when stored over long time.

**It** is another object of the present invention to provide a substance, which improves the solubility of a lipophilic active component in a cosmetic or pharmaceutical composition or homecare product.

Furthermore, it is an object of the present invention to provide a substance by which the amount of applied solubilizer in a cosmetic or pharmaceutical composition or homecare product can be reduced, i.e. a substance which is suited as a solubilizer replacer.

Document WO 2018/099570 A1 discloses various compositions comprising (a) 1,2-alkanediols and (b) at least one lipophilic active compound.

Document JP H11 322591 A discloses the use of various alkanediols including 1,2-pentanediol, 1,2-hexanediol and 1,2-octanediol to increase the solubility of a known antimicrobial substance that thas low solubility in water and alcohol.

Document WO 2005/123028 A1 relates to cosmetic compositions comprising (a) 1,2-hexanediol or 1,2-heptanediol and (b) various fragrance materials.

Document US 2015/250722 A1 discloses compositions for cosmetic or pharmaceutical use, comprising (a) alkanediols including 1,2-hexanediol, 1,2-heptanediol or 1,2-octanediol as cosurfactant B and (b) one or more oils.

Document US 2012/129937 A1 discloses compositions for cosmetic or pharmaceutical use comprising (a) 1,2-alkanediols with 5 to 8 carbon atoms, to increase the solubility of (b) organic compounds.

Document US 2013/136709 A1 teaches the use of (a) at least two 1,2-alkanediols selected from 1,2-decanediol, 1,2-octanediol, 1,2-hexanediol and 1,2-pentanediol and (b) phenoxyethanol.

Surprisingly it has been found that the addition of an effective amount of a mixture of two or more different specific alkanediols as defined herein to a topical composition comprising a lipophilic active component significantly improves the solubility of said lipophilic active component.

It has been observed that the overall amount of solubilizer in a topical composition comprising a lipophilic active component can be significantly reduced by the addition of an effective amount of a mixture of two or more different specific alkanediols as defined herein.

Additionally, it has been found that the addition of an effective amount of a mixture of two or more different specific alkanediols as defined herein to a topical composition comprising a lipophilic active component reduces foam formation.

### Summary of the invention

In a first aspect, the present invention provides a cosmetic or pharmaceutical composition or homecare product, comprising or consisting of
(a) a mixture comprising at least one first linear alkanediol having a carbon chain of 5 to 14 carbon atoms and one or more second linear alkanediol having a carbon chain of 5 to 14 carbon atoms which is different from the first linear alkanediol;
(b) at least one lipophilic active component; and optionally at least one active substance for a cosmetic or pharmaceutical composition or homecare product and/or additive, which is/are different from the lipophilic active component (b),
as defined in claim 1.

In a further aspect, the present invention provides for the use of the cosmetic or pharmaceutical composition as a cosmetic, for personal care, in particular for skin, hair, scalp and nail care, as a pharmaceutical, for animal care or for homecare.

In a further aspect, the present invention provides for the use of a mixture comprising at least one first linear alkanediol and one or more second linear alkanediol for improving the solubility of a lipophilic active component as defined herein in a cosmetic or pharmaceutical composition or homecare product, and/or for increasing the availability of a lipophilic active component as defined herein, and/or for reducing the foam formation of the cosmetic of pharmaceutical composition or homecare product comprising a solubilizer,or as solubilizing agent replacer in a cosmetic or pharmaceutical composition or homecare product, as defined in claim 10.

### Description of Figures

Figure 1 are photographs showing the solubility of samples of Bisabolol in combination with different 1,2-alkanediols.
Figure 2 is a diagram of the nephelometric turbidity units of samples of Bisabolol in combination with different 1,2-alkanediols.
Figure 3 are photographs showing the solubility of samples of Hydrocortisone in combination with different 1,2-alkanediols.
Figure 4 are photographs showing the solubility of samples of Ethylhexylglycerin in combination with different 1,2-alkanediols.
Figure 5 is a diagram of the nephelometric turbidity units of samples of Ethylhexylglycerin in combination with different 1,2-alkanediols.
Figure 6 are photographs showing the solubility of samples of o-Cymen-5-ol in combination with different 1,2-alkanediols.
Figure 7 is a diagram of the nephelometric turbidity units of samples of o-Cymen-5-ol in combination with different 1,2-alkanediols.
Figure 8 are photographs showing the solubility of samples of Propanediol Caprylate in combination with different 1,2-alkanediols.
Figure 9 is a diagram of the nephelometric turbidity units of samples of Propanediol Caprylate in combination with different 1,2-alkanediols.
Figure 10 are photographs showing the solubility of samples of Retinyl Palmitate in combination with different 1,2-alkanediols.
Figure 11 is a diagram of the nephelometric turbidity units of samples of Retinyl Palmitate in combination with different 1,2-alkanediols.
Figure 12 are photographs showing the solubility of samples of Glyceryl Caprylate in combination with different 1,2-alkanediols.
Figure 13 is a diagram of the nephelometric turbidity units of samples of Glycerly Caprylate in combination with different 1,2-alkanediols.
Figure 14 are photographs showing the solubility of samples of Hydroxyacetophenone in combination with different 1,2-alkanediols.
Figure 15 is a diagram of the nephelometric turbidity units of samples containing Bisabolol and 1,2-heptanediol, 2,3-heptanediol and a mixture including 1,2-heptanediol and 2,3-heptanediol.
Figure 16 is a diagram of the nephelometric turbidity units of samples containing Ethylhexylglycerin and 1,2-heptanediol, 2,3-heptanediol and a mixture including 1,2-heptanediol and 2,3-heptanediol.
Figure 17 is a diagram of the nephelometric turbidity units of samples containing Ethylhexylglycerin and 1,2-octanediol, 2,3-octanediol and a mixture including 1,2-octanediol and 2,3-octanediol.
Figure 18 is a diagram of the nephelometric turbidity units of samples containing Propanediol Caprylate and 1,2-heptanediol, 2,3-heptanediol and a mixture including 1,2-heptanediol and 2,3-heptanediol.
Figure 19 is a diagram of the nephelometric turbidity units of samples containing Propanediol Caprylate and 1,2-heptanediol, 2,3-heptanediol and a mixture including 1,2-heptanediol and 2,3-heptanediol.
Figure 20 are photographs showing the solubility of samples containing O-Cymene-5-ol in combination with 1,2-heptanediol, 2,3-heptanediol and a mixture including 1,2-heptanediol and 2,3-heptanediol.

### Detailed description of the invention

The present invention is specified in the appended claims. The invention itself, and its preferred variants, other objects and advantages, are however also apparent from the following detailed description in conjunction with the accompanying examples and figures.

In a first aspect, the present invention relates to a cosmetic or pharmaceutical composition or homecare product, comprising or consisting of:
(a) a mixture comprising at least one first linear alkanediol having a carbon chain of 5 to 14 carbon atoms with one or more second linear alkanediol having a carbon chain of 5 to 14 carbon atoms which is different from the first linear alkanediol;
(b) at least one lipophilic active component, having a log P_{O/W} value of ≥ 0 to 25.00; and
(c) optionally at least one active substance for a cosmetic or pharmaceutical composition or homecare product and/or additive, which is different from the lipophilic active component (b),
   wherein the mixture of at least one first linear alkanediol with one or more second linear alkanediol is selected from the group consisting of
   a mixture comprising 1,2-pentanediol and 2,3-pentanediol;
   a mixture comprising 1,2-hexanediol and 2,3-hexanediol;
   a mixture comprising 1,2-heptanediol and 2,3-heptanediol;
   a mixture comprising 1,2-octanediol and 2,3-octanediol;
   a mixture comprising 1,2-nonanediol and 2,3-nonanediol;
   a mixture comprising 1,2-decanediol and 2,3-decanediol;
   a mixture comprisjng 1,2-undecanediol and 2,3-undecanediol;
   a mixture comprising 1,2-dodecanediol and 2,3-dodecanediol; and
   a mixture comprising 1,2-tridecanediol and 2,3-tridecanediol;
      or
   wherein the mixture comprising at least one first linear alkanediol and one or more second linear alkanediol is selected from the group consisting of a mixture comprising 1,2-hexanediol and 2,3-octanediol;
   a mixture comprising 1,2-octanediol and 2,3-hexanediol; and
   a mixture comprising 1,2-octanediol and 2,3-heptanediol;
      or
   wherein the mixture comprising at least one first linear alkanediol and one or more second linear alkanediol is selected from the group consisting of a mixture comprising 1,2-pentanediol and 2,3-hexanediol;
   a mixture comprising 1,2-pentanediol and 2,3-heptanediol;
   a mixture comprising 1,2-pentanediol and 2,3-octanediol;
   a mixture comprising 1,2-pentanediol and 1,2-nonanediol; and
   a mixture comprising1,2-pentanediol and 2,3-nonanediol;
      or
   wherein the mixture comprising at least one first linear alkanediol and one or more second linear alkanediol is selected from the group consisting of a mixture comprising 1,2-heptanediol and 1,2-octanediol;
   a mixture comprising 1,2-heptanediol and 2,3-octanediol;
      a mixture comprising 1,2-heptanediol and 1,2-nonanediol; and a mixture comprising 1,2-heptanediol and 2,3-nonanediol.

In a preferred variant of the first aspect of the present invention, the component(s) (b) is/are different to component (a).

In a more preferred variant of the first aspect,
in the mixture comprising at least one first linear alkanediol having a carbon chain of 5 to 14 carbon atoms and one or more second linear alkanediol having a carbon chain of 5 to 14 carbon atoms, the number of the carbon atoms of the first and the second alkanediol is either same or different.

The term "comprising" means that the named components are essential, but other components may be added and is still embraced by the present invention.

**The** term "consisting of" as used according to the present invention means that the total amount of components (a) to (c) adds up to 100 % by weight, based on the total weight of the cosmetic or pharmaceutical composition or homecare product, and signifies that the subject matter is closed-ended and can only include the limitations that are expressly recited.

Whenever reference is made to "comprising" it is intended to cover both meanings as alternatives, that is the meaning can be either "comprising" or "consisting of", unless the context dictates otherwise.

The term "at least one ..." means that the cosmetic or pharmaceutical composition or homecare product according to the present invention can comprise either one or a mixture of two, three, four, five, six or even more different of the respective components following said term.

The term "optionally" means that the subsequently described compound may but need not to be present in the composition, and that the description includes variants, in which the compound is included or variants, in which the compound is absent.

Alkanediols are glycols, i.e. any of a class or organic ompounds belonging to the alcohol family; in the molecule of a glycol, two hydroxyl (-OH) groups are attached to different carbon atoms or a carbon chain.

Straight chain 1,2-alkanediols have been used for more than 15 years as multifunctional actives. Short chain 1,2-alkanediols are amphiphilic compounds and thus, like 1,2-pentanediol and 1,2-hexanediol, are soluble both in water and cosmetic oils. In contrast, 1,2-octanediol tends to precipitate or recrystallize in oily solutions. On the other hand, 1,2-decanediol is a solid and soluble only in cosmetic oils. Apart from moisturizing, some 1,2-alkanediols are used as viscosity modifiers.

According to the present invention, the component (a) includes at least one first linear alkanediol having a carbon chain of 5 to 14 carbon atoms and at least one second linear alkanediol having a carbon chain of 5 to 14 carbon atoms. Therefore, the invention entails a mixture of a first and a second alkanediol as described herein.

In mixtures of first and second linear alkanediols, where these are specifically different, the specific terms "first" and "second" will be used to distinguish the two alkanediol components.

As used in this document, the phrase "at least one first linear alkanediol" means that the composition can comprise one first linear alkanediol having a carbon chain of 5 to 14 carbon atoms or can comprise more than one first linear alkanediol having a carbon chain of 5 to 14 carbon atoms, i.e. two, three, four or more different first linear alkanediols having a carbon chain of 5 to 14 carbon atoms.

The first linear alkanediol consists of a chain of 5 to 14 carbon atoms joined to each other by single covalent bonds with two OH functional groups attached to two different carbon atoms in the chain.

The at least one first linear alkanediol having a carbon chain of 5 to 14 carbon atoms in the cosmetic or pharmaceutical composition or homecare product according to the present invention is selected from the group consisting of pentanediol, hexanediol heptanediol, octanediol, nonanediol, decanediol, undecanediol, dodecanediol, tridecanediol and tetradecanediol.

**In** a preferred variant, the at least one first linear alkanediol is selected from the group consisting of pentanediol, hexanediol, heptanediol, octanediol, nonanediol, decanediol, undecanediol, dodecanediol and tridecanediol.

**In** a more preferred variant, the at least one first linear alkanediol is selected from the group consisting of alkanediols having an uneven number of carbon atoms of 5 to 13, i.e. pentanediol, heptanediol, nonanediol, undecanediol, tridecanediol and mixtures thereof.

**In** a still more preferred variant, the at least one first linear alkanediol is selected from the group consisting of alkanediols having an uneven number of carbon atoms of 7, 9, 11 and 13, i.e. heptanediol, nonanediol, undecanediol and tridecanediol, or is selected from the group consisting of alkanediols having an uneven number of carbon atoms of 9 and 11, i.e. nonanediol and undecanediol, or is selected from the group consisting of alkanediols having an uneven number of carbon atoms of 9 and 13, i.e. nonanediol and tridecanediol, or is selected from the group consisting of alkanediols having an uneven number of carbon atoms of 11 and 13, i.e. undecanediol and tridecanediol.

**In** a still more preferred variant, the at least one first linear alkanediol is either pentanediol, hexanediol, heptanediol or octanediol.

In a most preferred variant, the at least one first linear alkanediol is heptanediol.

The cosmetic or pharmaceutical composition or homecare product according to the present invention comprises a mixture comprising at least one first linear alkanediol having a carbon chain of 5 to 14 carbon atoms and one or more second linear alkanediol having a carbon chain of 5 to 14 carbon atoms which is different from the first linear alkanediol.

This means that the alkanediol mixture is a mixture comprising at least one first linear alkanediol having a carbon chain of 5 to 14 carbon atoms and one or more, i.e. two, three, four or more second linear alkanediol having a carbon chain of 5 to 14 carbon atoms.

For example, the mixture can include one first linear alkanediol and one, two, three or more second linear alkanediols; or the mixture can include two first linear alkanediols with one, two, three or more second linear alkanediols, etc. with the proviso, that in each mixture, the first linear alkanediols and the second linear alkanediols are different from each other.

The phrase "different from each other" means that the first linear alkanediol and the second linear alkanediols in the mixture are either different with regard to the length of their carbon chain, i.e. number of the carbon atoms, and/or with regard to their constitutional isomerism or with regard to their stereoisomerism.

Likewise, the number of the carbon atoms of the first linear alkanediol and the second linear alkanediol in the mixture can also be same. For example, the first linear alkanediol and the second linear alkanediol have a carbon chain of 7 carbon atoms, but the first linear alkanediol and the second linear alkanediol are different with regard to their constitutional isomerism or with regard to their stereoisomerism.

The second linear alkanediol consists of a chain of 5 to 14 carbon atoms joined to each other by single covalent bonds with two OH functional groups attached to two different carbon atoms in the chain.

The at least one second linear alkanediol having a carbon chain of 5 to 14 carbon atoms in the cosmetic or pharmaceutical composition or homecare product according to the present invention is selected from the group consisting of pentanediol, hexanediol, heptanediol, octanediol, nonanediol, decanediol, undecanediol, dodecanediol, tridecanediol and tetradecanediol.

In a preferred variant, the at least one second linear alkanediol is selected from the group consisting of pentanediol, hexanediol, heptanediol, octanediol, nonanediol, decanediol, undecanediol, dodecanediol and tridecanediol. If both the first and the second alkanediol is for example heptanediol, then the second linear alkanediol heptanediol is a different constitutional isomer or stereoisomer from the first linear alkanediol.

In a more preferred variant, the at least one second linear alkanediol is selected from the group consisting of pentanediol, hexanediol, heptanediol, octanediol and nonanediol, most preferably heptanediol, octanediol and nonanediol.

The term "alkanediol" within the context of the present invention also includes its constitutional isomers or position isomers. Constitutional isomers are compounds that have the same molecular formula and different connectivity. Position isomers, a particular form of constitutional isomerism, are structural isomers that can be viewed as differing only on the position of a functional group on a parent structure, which in this case is the position of the two alcohol functions.

Depending on the number of the carbon atoms of the carbon chain of the alkanediol, there are various position isomers of the alkanediol: (x,x+1) constitutional isomers; (x,x+2) constitutional isomers; (x,x+3) constitutional isomers; etc. and alpha,omega constitutional isomers when the alcohol functions are at the terminal ends of the carbon chain, wherein x stands for the number of the carbon atom in the alkanediol chain, to which the OH groups of the alkanediol are chemically bonded. For example: if x is 1, the two OH groups of the alkanediol are chemically bonded to the C1 and C2 carbon atoms in the alkanediol chain; if x is 2, the two OH groups of the alkanediol are chemically bonded to the C2 and C3 carbon atoms in the alkanediol chain, etc.

In the (x,x+1) constitutional isomers, the two OH functional groups are vicinal attached to two different adjacent carbon atoms in the chain. In the (x,x+2) constitutional isomers, the two OH functional groups are attached to two different carbon atoms in the chain where the two carbon atoms are separated by one C atom. In the (x,x+3) constitutional isomers, the two OH functional groups are attached to two different carbon atoms in the chain where the two carbon atoms are separated by two C atoms. In the alpha,omega constitutional isomers, the two functional groups are attached to the first C atom and to the terminal C atom.

In a preferred variant of the cosmetic or pharmaceutical composition or homecare product according to the present invention, the first linear alkanediol and/or the second linear alkanediol having a carbon chain of 5 to 14 carbon atoms is a vicinal (x,x+1) diol, selected from the group consisting of a 1,2-diol, 2,3-diol, and mixtures thereof, preferably an alpha,beta 1,2 constitutional isomer.

According to the present invention, vicinal (x,x+1) diols are most preferred, such as alpha,beta or beta,gamma.

In a still preferred variant of the cosmetic or pharmaceutical composition or homecare product according to the present invention, the first linear alkanediol and/or the second linear alkanediol, is a 1,2-alkanediol, a 2,3-alkanediol, or mixtures thereof, more preferred 2,3-alkanediol.

The 1,2-alkanediols of the first linear alkanediol having a carbon chain of 5 to 14 carbon atoms and/or the second linear alkanediol having a carbon chain of 5 to 14 carbon atoms, can preferably be those as represented by the following formulae:
1,2-pentanediol 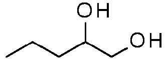
1,2-hexanediol 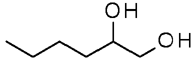
1,2-heptanediol 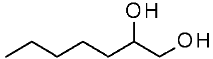
1,2-octanediol 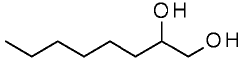
1,2-nonaediol 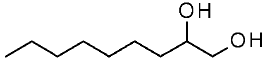
1,2-decanediol 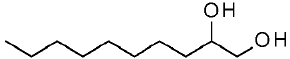
1,2-undecanediol 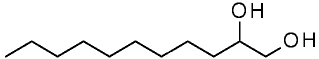
1,2-dodecanediol 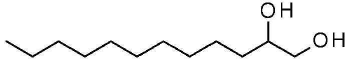
1,2-tridecanediol 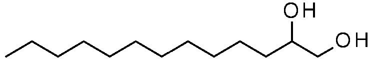
1,2-tetradecanediol

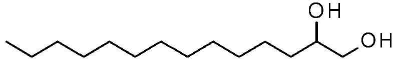

The 2,3-alkanediols of the first linear alkanediol having a carbon chain of 5 to 14 carbon atoms and/or the second linear alkanediol having a carbon chain of 5 to 14 carbon atoms, can preferably be those as represented by the following formulae:
2,3-pentanediol 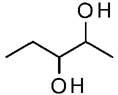
2,3-hexanediol 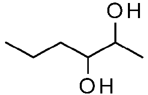
2,3-heptanediol 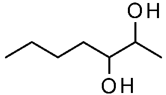
2,3-octanediol 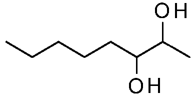
2,3-nonaediol 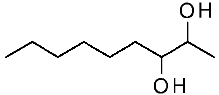
2,3-decanediol 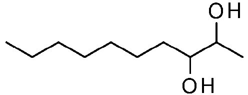
2,3-undecanediol 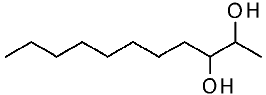
2,3-dodecanediol 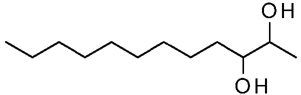
2,3-tridecanediol 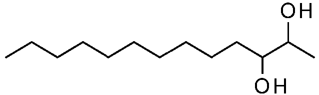
2,3-tetradecanediol

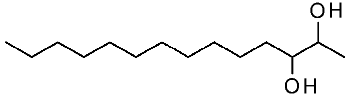

In a preferred variant, the cosmetic or pharmaceutical composition according to the present invention comprises a mixture comprising at least one first linear alkanediol having a carbon chain of 5 to 14 carbon atoms and one or more second linear alkanediol having a carbon chain of 5 to 14 carbon atoms wherein the number of the carbon atoms of the first and the second alkanediol is either same or different.

**If** both, the first and the second alkanediol have the same number of carbon atoms, such an alkanediol combination is herein also referred to as "homo alkanediol mixture" or "homo combination". For example, the first linear alkanediol and the second linear alkanediol have a carbon chain of 7 carbon atoms, but the first linear alkanediol and the second linear alkanediol are different with regard to their constitutional isomerism or with regard to their stereoisomerism.

**If** the first and the second alkanediol have a different number of carbon atoms, such an alkanediol combination is herein also referred to as "hetero alkanediol mixture" or "hetero combination". For example, the first linear alkanediol has a carbon chain of 7 carbon atoms and the second linear alkanediol has a carbon chain of 8 carbon atoms. However, beyond that, the first linear alkanediol and the second linear alkanediol can be different with regard to their constitutional isomerism or with regard to their stereoisomerism.

In a preferred variant of the cosmetic or pharmaceutical composition according to the present invention, the first linear alkanediol is selected from the group consisting of:
1,2-pentanediol, 2,3-pentanediol,
1,2-hexanediol, 2,3-hexanediol,
1,2-heptanediol, 2,3-heptanediol,
1,2-octanediol, 2,3-octanediol,
1,2-nonanediol, 2,3-nonanediol,
1,2-decanediol, 2,3-decanediol,
1,2-undecanediol, 2,3-undecanediol,
1,2-dodecanediol, 2,3-dodecanediol,
1,2-tridecanediol, 2,3-tridecanediol, and mixtures thereof.

Of the aforesaid first linear alkanediols, the alpha,beta or beta,gamma constitutional isomers are preferred: 1,2-pentanediol, 2,3-pentanediol, 1,2-hexanediol, 2,3-hexanediol, 1,2-heptanediol, 2,3-heptanediol, 2,3-octanediol, 2,3-nonanediol, 2,3-decanediol, 2,3-undecanediol, 2,3-dodecanediol, 2,3-tridecanediol or mixtures thereof. Said alkanediols are liquid at a purity of 90 to 99 %.

Of the aforesaid liquid alkanediols 1,2-pentanediol, 2,3-pentanediol, 1,2-hexanediol, 2,3-hexanediol, 1,2-heptanediol, 2,3-heptanediol, 2,3-octanediol, 2,3-nonanediol or mixtures of said liquid alkanediols are particularly preferred. Said alkanediols can be easier incorporated into semi-finished products or final products.

In a more preferred variant, the at least one first linear alkanediol is selected from the group consisting of 1,2-alkanediols having an uneven number of carbon atoms of 5 to 13, i.e. 1,2-pentanediol, 1,2-heptanediol, 1,2-nonanediol, 1,2-undecanediol, 1,2-tridecanediol and mixtures thereof.

In a more preferred variant, the first linear alkanediol is selected from the group consisting of 1,2-alkanediols having an uneven number of carbon atoms of 7, 9, 11 and 13, i.e. 1,2-heptanediol, 1,2-nonanediol, 1,2-undecanediol or 1,2-tridecanediol, or is selected from the group consisting of 1,2-alkanediols having an uneven number of carbon atoms of 9 and 11, i.e. 1,2-nonanediol and 1,2-undecanediol, or is selected from the group consisting of 1,2-alkanediols having an uneven number of carbon atoms of 9 and 13, i.e. 1,2-nonanediol and 1,2-tridecanediol, or is selected from the group consisting of 1,2-alkanediols having an uneven number of carbon atoms of 11 and 13, i.e. 1,2-undecanediol and 1,2-tridecanediol.

In a more preferred variant, the at least one first linear alkanediol is selected from the group consisting of 2,3-alkanediols having an uneven number of carbon atoms of 5 to 13, i.e. 2,3-pentanediol, 2,3-heptanediol, 2,3-nonanediol, 2,3-undecanediol, 2,3-tridecanediol and mixtures thereof.

In a still more preferred variant, the first linear alkanediol is selected from the group consisting of 2,3-alkanediols having an uneven number of carbon atoms of 7, 9, 11 and 13, i.e. 2,3-heptanediol, 2,3-nonanediol, 2,3-undecanediol or 2,3-tridecanediol, or is selected from the group consisting of 2,3-alkanediols having an uneven number of carbon atoms of 9 and 11, i.e. 2,3-nonanediol and 2,3-undecanediol, or is selected from the group consisting of 2,3-alkanediols having an uneven number of carbon atoms of 9 and 13, i.e. 2,3-nonanediol and 2,3-tridecanediol, or is selected from the group consisting of 2,3-alkanediols having an uneven number of carbon atoms of 11 and 13, i.e. 2,3-undecanediol and 2,3-tridecanediol.

In a further preferred variant of the cosmetic or pharmaceutical composition or homecare product according to the present invention, the first linear alkanediol is selected from the group consisting of:
1,2-pentanediol, 2,3-pentanediol,
1,2-hexanediol, 2,3-hexanediol,
1,2-heptanediol, 2,3-heptanediol,
1,2-octanediol, 2,3-octanediol,
1,2-nonanediol, 2,3-nonanediol, and mixtures thereof.

More preferred, in the cosmetic or pharmaceutical composition or homecare product according to the present invention, the first linear alkanediol is selected from the group consisting of 1,2-pentanediol, 2,3-pentanediol, and mixtures thereof or is selected from the group consisting of 1,2-heptanediol, 2,3-heptanediol, and mixtures thereof. However, the first linear alkanediol can also preferably be selected from the group consisting of 1,2-octanediol, 2,3-octanediol, and mixtures thereof.

Most preferred, the first linear alkanediol is 1,2-heptanediol or 1,2-octanediol.

Likewise, in a further preferred variant of the cosmetic or pharmaceutical composition or homecare product according to the present invention, the first linear alkanediol is selected from the group consisting of:
1,2-nonanediol, 2,3-nonanediol,
1,2-decanediol, 2,3-decanediol,
1,2-undecanediol, 2,3-undecanediol, and mixtures thereof.

More preferred, in the cosmetic or pharmaceutical composition or homecare product according to the present invention, the first linear alkanediol is selected from the group consisting of 1,2-nonanediol, 2,3-nonanediol, and mixtures thereof, or can also be preferably selected from the group consisting of 1,2-decanediol, 2,3-decanediol, and mixtures thereof, or can also be preferably selected from the group consisting of 1,2-undecanediol, 2,3-undecanediol, and mixtures thereof.

Additionally, the first linear alkanediol can also preferably be 1,2-undecanediol, 2,3-undecanediol, or a mixture thereof. A mixture of 1,2-nonanediol and/or 2,3-decanediol and/or 2,3-undecanediol or a mixture of 1,2-decanediol and/or 2,3-nonanediol and/or 2,3-undecanediol or a mixture of 1,2-undecanediol and/or 2,3-nonanediol and 2,3-decanediol is also possible.

In a preferred variant of the cosmetic or pharmaceutical composition or homecare product according to the present invention, the second linear alkanediol is selected from the group consisting of:
1,2-pentanediol, 2,3-pentanediol,
1,2-hexanediol, 2,3-hexanediol,
1,2-heptanediol, 2,3-heptanediol,
1,2-octanediol, 2,3-octanediol,
1,2-nonanediol, 2,3-nonanediol,
1,2-decanediol, 2,3-decanediol,
1,2-undecanediol, 2,3-undecanediol,
1,2-dodecanediol, 2,3-dodecanediol,
1,2-tridecanediol, 2,3-tridecanediol, and mixtures thereof.

Of the aforesaid second linear alkanediols, the following (x,x+1) constitutional isomers are preferred: 1,2-pentanediol, 2,3-pentanediol, 1,2-hexanediol, 2,3-hexanediol, 1,2-heptanediol, 2,3-heptanediol, 2,3-octanediol, 2,3-nonanediol, 2,3-decanediol, 2,3-undecanediol, 2,3-dodedcanediol, or 2,3-tridecanediol. Said alkanediols are liquid at a purity of 90 to 99 %

Of the aforesaid liquid alkanediols 1,2-pentanediol, 2,3-pentanediol, 1,2-hexanediol, 2,3-hexanediol, 1,2-heptanediol, 2,3-heptanediol, 2,3-octanediol, 2,3-nonanediol or mixtures of said liquid alkanediols are particularly preferred. Said alkanediols can be easier incorporated into semi-finished products or final products.

**In** a more preferred variant, the second linear alkanediol is selected from the group consisting of 1,2-alkanediols having an uneven number of carbon atoms of 5 to 13, i.e. 1,2-pentanediol, 1,2-heptanediol, 1,2-nonanediol, 1,2-undecanediol, 1,2-tridecanediol and mixtures thereof.

In a more preferred variant, the second linear alkanediol is selected from the group consisting of 1,2-alkanediols having an uneven number of carbon atoms of 7, 9, 11 and 13, i.e. 1,2-heptanediol, 1,2-nonanediol, 1,2-undecanediol and 1,2-tridecanediol, or is selected from the group consisting of 1,2-alkanediols having an uneven number of carbon atoms of 9 and 11, i.e. 1,2-nonanediol and 1,2-undecanediol, or is selected from the group consisting of 1,2-alkanediols having an uneven number of carbon atoms of 9 and 13, i.e. 1,2-nonanediol and 1,2-tridecanediol, or is selected from the group consisting of 1,2-alkanediols having an uneven number of carbon atoms of 11 and 13, i.e. 1,2-undecanediol and 1,2-tridecanediol.

In a more preferred variant, the second linear alkanediol is selected from the group consisting of 2,3-alkanediols having an uneven number of carbon atoms of 5 to 13, i.e. 2,3-pentanediol, 2,3-heptanediol, 2,3-nonanediol, 2,3-undecanediol, 2,3-tridecanediol and mixtures thereof.

In a still more preferred variant, the second linear alkanediol is selected from the group consisting of 2,3-alkanediols having an uneven number of carbon atoms of 7, 9, 11 and 13, i.e. 2,3-heptanediol, 2,3-nonanediol, 2,3-undecanediol and 2,3-tridecanediol, or is selected from the group consisting of 2,3-alkanediols having an uneven number of carbon atoms of 9 and 11, i.e. 2,3-nonanediol and 2,3-undecanediol, or is selected from the group consisting of 2,3-alkanediols having an uneven number of carbon atoms of 9 and 13, i.e. 2,3-nonanediol and 2,3-tridecanediol, or is selected from the group consisting of 2,3-alkanediols having an uneven number of carbon atoms of 11 and 13, i.e. 2,3-undecanediol and 2,3-tridecanediol.

**In** a more preferred variant of the cosmetic or pharmaceutical composition or homecare product according to the present invention, the second linear alkanediol is selected from the group consisting of:
1,2-pentanediol, 2,3-pentanediol,
1,2-hexanediol, 2,3-hexanediol,
1,2-heptanediol, 2,3-heptanediol,
1,2-octanediol, 2,3-octanediol,
1,2-nonanediol, 2,3-nonanediol, and mixtures thereof.

More preferred, in the cosmetic or pharmaceutical composition or the homecare product according to the present invention, the second linear alkanediol is selected from the group consisting of 1,2-pentanediol, 2,3-pentanediol, and mixtures thereof. However, the second linear alkanediol can also preferably be selected from the group consisting of 1,2-hexanediol, 2,3-hexanediol, and mixtures thereof or can also preferably be selected from the group consisting of 1,2-heptanediol, 2,3-heptanediol, and mixtures thereof.

Most preferred, the second linear alkanediol is 1,2-pentanediol or 2,3-pentanediol or 1,2-hexanediol or 2,3-hexanediol or 1,2-heptanediol or 2,3-heptanediol or 2,3-octanediol or 2,3-octanediol or 1,2-nonanediol or 2,3-nonanediol.

The cosmetic or pharmaceutical composition or homecare product according to the present invention which comprises a mixture or a combination comprising at least one first linear alkanediol with and or more second linear alkanediol includes any one of the following mixtures/combinations:
▪ a mixture comprising 1,2-pentanediol and 2,3-pentanediol;
▪ a mixture comprising 1,2-hexanediol and 2,3-hexanediol;
▪ a mixture comprising 1,2-heptanediol and 2,3-heptanediol;
▪ a mixture comprising 1,2-octanediol and 2,3-octanediol;
▪ a mixture comprising 1,2-nonanediol and 2,3-nonanediol;
▪ a mixture comprising 1,2-decanediol and 2,3-decanediol;
▪ a mixture comprising 1,2-undecanediol and 2,3-undecanediol;
▪ a mixture comprising 1,2-dodecanediol and 2,3-dodecanediol; or
▪ a mixture comprising 1,2-tridecanediol and 2,3-tridecanediol.

In said homo alkanediol mixtures the first linear alkanediol and the second linear alkanediol have the same number of carbon atoms.

Particularly favorable is a mixture including 1,2-heptanediol in combination with 2,3-heptanediol or a mixture including 1,2-octanediol in combination with 2,3-octanediol.

The above specified alkanediol mixtures according to the present invention, comprising a 1,2-alkanediol and a 2,3-alkanediol either having the same number of carbon atoms, are characterized in that they significantly improve the solubility of a lipophilic active component compared to the respective single 1,2-alkanediol or 2,3-alkanediol substances which have only a poor solubility for lipophilic active components. This effect is observed for aqueous/glycolic formulations even with a high water content as well as for emulsions. The lipophilic active component remains solubilized and does not crystallize out or does not precipitate out during storage and, thus, results in a stable cosmetic or pharmaceutical formulation or homecare product.

Said effect or action of improving the solubility of a lipophilic active component is found for the homo mixture including 1,2-pentanediol and 2,3-pentanediol. The mixture of said alkanediol mixture and a lipophilic active component results in a clear solution and the lipophilic component does not crystallize our or does not precipitate out during storage.

A solubility improving effect can also be observed for the homo mixture including 1,2-hexanediol and 2,3-hexanediol. The mixture of said alkanediol mixture and a lipophilic active component shows high clarity and the lipophilic active component does not crystallize our or does not precipitate out during storage.

The homo mixturre including 1,2-heptanediol and 2,3-heptanediol is beneficial since it has a particular solubility improving effect of a lipophilic active component. The resulting solution of said alkanediol mixture and a liphophilic active component is clear and the lipophilic active component does not crystallize out or does not precipitate out during storage.

The alkanediol mixture comprising 1,2-octanediol and 2,3-octanediol or an alkanediol mixture comprising 1,2-nonanediol and 2,3-nonanediol are particularly advantageous, since they improve solubility of a lipophilic active component. A solution of said alkanediol mixture and a lipophilic active component shows no turbidity and the lipophilic active substance does not crystallize our or does not precipitate out during storage.

Also an alkanediol mixture comprising 1,2-decanediol and 2,3-decanediol or the alkanediol mixture comprising 1,2-undecanediol and 2,3-undecanediol significantly improves solubility of lipophilic active substances. The mixtures of said alkanediol mixtures and a lipophilic active component result in clear solutions which are stable during storage, i.e. the lipophilic active component remains solubilized in the formulation.

A solubility enhancing effect is also true for an alkanediol mixture including 1,2-dodecanediol and 2,3-dodecanediol or an alkanediol mixture including 1,2-tridecanediol and 2,3-tridecanediol. The mixtures of said alkanediol mixture and a lipophilic active component result in clear, non turbid solutions with not precipitation of the lipophilic active component during storage.

The afore specified homo alkane diol mixtures including a 1,2-alkanediol and the corresponding 2,3-alkanediol display a significant solubility improvement for lipholic active substandes and are clearly superior to the individually corresponding 1,2-alkanediols or 2,3-alkanediols, having the same concentration, but which show a poorer solubility. All solutions of said 1,2-alkanediol and 2,3-alkanediol mixtures with a lipophilic active component are clear and stable and the lipophilic active substance does not precipitate out even during storage.

Additionally, with the admixture of the corresponding liquid 2,3-alkanediol, even in small amounts, to a solid 1,2-alkanediol, the solid 1,2-alkanedioles, such as 1,2-octanediol, 1,2-nonanediol etc., can be solved, resulting in a liquid alkanediol mixture. The 2,3-alkanediol serves as solvent for the solid 1,2-alkanediols. Such liquid mixtures have the benefit that firstly the availability of the solid 1,2-alkanediol in the mixture is improved and secondly the incorporation of the solid 1,2-alkanediol in semi-finished products or final products is facilitated. This effect is particularly favorably for emulsions, in which lipophilic 1,2-alkanediols having a carbon chain of 8 or more carbon atoms, when used alone, tend to migrate into the oil phase or tend to precipitate or recrystallize.

With the solution of the solid 1,2-octanediol in the liquid 2,3-octanediol or of the solid 1,2-nonanediol in the liquid 2,3-nonanediol, the availability of the 1,2-octanediol or 1,2-nonanediol can be likewise improved in the end use.

Due to its better solubility, the availability of the lipophilic active substance or component in the cosmetic or pharmaceutical composition is increased by the above alkanediol mixtures including a 1,2-alkanediol and a 2,3-alkanediol. Additionally, due to its better solubility, the lipophilic active substance can be furnished in a higher concentration in the final formulation or can be furnished in a lower concentration in the final formulation compared to a composition including a lipophilic active component with low solubility and without the additional of an alkanediol.

Due to its better solubility, a uniformly solubilized product, in which the lipophilic active component is uniformly dissolved in the product can be obtained. Thus, the concentration of the lipophilic active component is exactly the same through the whole formulation from the very first splash to the very last ml, untill the bottle is completely empty. Without the use of an alkanediol as specified herein, the distribution of the lipophilic active component would be quite haphazardly in the formulation and would vary from application to application.

In addition, the above alkanediol mixtures comprising a 1,2-alkanediol and the corresponding 2,3-alkanediol render possible the cold preparation of cosmetic or pharmaceutical formulations or homecare products, i.e. no heating during preparation is necessary.

Thus, with the afore-mentioned properties of the specified alkanediol mixtures including a 1,2-alkanediol and the respective 2,3-alkanediol the processability in formulations can be improved.

Hence, a good compromise can be achieved when the mixture is combined in such a way as to maintain the solublity improving effect, while improving processability in formulations. The above specified 1,2-alkanediol and corresponding 2,3-alkanediol combinations solve this balancing problem over the 1,2-alkanediol substances or 2,3-alkanediol substances alone. This is well achieved by combinations such as comprising 1,2-pentanediol and 2,3-pentanediol but also for 1,2-hexanediol and 2,3-hexandiol. Also, combinations such as comprising 1,2-heptanediol and 2,3-heptanediol, but also 1,2-octanediol and 2,3-octanediol show this effect. The same also counts for 1,2-nonanediol in combination with 2,3-nonanediol, but also for 1,2-decanediol in combination with 2,3-decanediol.

The afore specified homo alkanediol mixtures including a 1,2-alkanediol and the corresponding 2,3-alkanediol display a remarkably synergistic activity and are clearly superior to the individually corresponding 1,2-alkanediols or 2,3-alkanediols and having the same concentration.

In a particularly preferred variant, the cosmetic or pharmaceutical composition or homecare product according to the present invention which comprises a mixture or a combination comprising at least one first linear alkanediol and one or more second linear alkanediol thus includes any one of the following mixtures/combinations:
▪ 1,2-pentanediol in combination with 1,2-nonanediol;
▪ 1,2-heptanediol in combination with one of 1,2-octanediol or , 1,2-nonanediol;

In a particular preferred variant, the cosmetic or pharmaceutical composition or homecare product according to the present invention which comprises a mixture or a combination comprising at least one first linear alkanediol and one or more second linear alkanediol thus may include any of the following mixtures/combinations:
▪ a mixture comprising 1,2-heptanediol and 1,2-octanediol; or
▪ a mixture comprising 1,2-heptanediol and 1,2-nonanediol.

In a particular preferred variant, the cosmetic or pharmaceutical composition or homecare product according to the present invention which comprises a mixture or a combination comprising at least one first linear alkanediol and one or more second linear alkanediol thus may include any of the following mixtures/combinations:
▪ a mixture comprising 1,2-heptanediol and 2,3-heptanediol; or
▪ a mixture comprising 1,2-heptanediol and 2,3-octanediol; or
▪ a mixture comprising 1,2-heptanediol and 2,3-nonanediol.

In a particular preferred variant, the cosmetic or pharmaceutical composition or homecare product according to the present invention which comprises a mixture or a combination comprising at least one first linear alkanediol and one or more second linear alkanediol thus may include any of the following mixtures/combinations:
▪ a mixture comprising 1,2-pentanediol and 2,3-pentanediol;
▪ a mixture comprising 1,2-pentanediol and 2,3-hexanediol;
▪ a mixture comprising 1,2-pentanediol and 2,3-heptanediol;
▪ a mixture comprising 1,2-pentanediol and 2,3-octanediol;
▪ a mixture comprising 1,2-pentanediol and 2,3-nonanediol
▪ a mixture comprising 1,2-hexanediol and 2,3-hexanediol;
▪ a mixture comprising 1,2-hexanediol and 2,3-octanediol;
▪ a mixture comprising 1,2-heptanediol and 2,3-heptanediol;
▪ a mixture comprising 1,2-heptanediol and 2,3-octanediol;
▪ a mixture comprising 1,2-heptanediol and 2,3-nonanediol;
▪ a mixture comprising 1,2-octanediol and 2,3-hexanediol;
▪ a mixture comprising 1,2-octanediol and 2,3-heptanediol;
▪ a mixture comprising 1,2-octanediol and 2,3-octanediol;
▪ a mixture comprising 1,2-nonanediol and 2,3-nonanediol;
▪ a mixture comprising 1,2-decanediol and 2,3-decanediol;
▪ a mixture comprising 1,2-undecanediol and 2,3-undecanediol;
▪ a mixture comprising 1,2-dodecanediol and 2,3-dodecanediol

In the hetero alkanediol mixtures described before the first linear alkanediol and the second linear alkanediol have a different number of carbon atoms.

The above specified alkanediol mixtures according to the present invention, comprising a 1,2-alkanediol and a 2,3-alkanediol, are characterized in that they significantly improve the solubility of a lipophilic active component compared to the respective individual 1,2-alkanediol or 2,3-alkanediol substances which have only a poor solubility for lipophililc active components. This effect is observed for aqueous/glycolic formulations even with a high water content as well as for emulsions. The lipophilic active components remain solubilized and does not crystalliz out or does not precipitate out during storage and, thus, results in a stable cosmetic or pharmaceutical formulation or homecare product.

Of the above specified alkanediol mixtures or combinations comprising at least one first linear alkanediol and one or more second linear alkanediol according to the present invention, particularly favorable is a hetero mixture or a combination of alkanediols according to the present invention comprising 1,2-hexanediol and 2,3-octanediol or a mixture or combination of alkanediols comprising 1,2-octanediol and 2,3-hexanediol or a mixture or combination of alkanediols comprising 1,2-octanediol and 2,3-heptanediol.

These specified hetero alkanediol mixtures according to the present invention, show a synergistically intensified action, i.e. these mixtures improve solublitly for a lipophilic active component in a cosmetic or pharmaceutical preparation or homecare product resulting in a clear, non turbid formulation compared to their corresponding single 1,2-alkanediol or 2,3-alkanediol substances, namely 1,2-hexanediol or 1,2-octanediol or 2,3-octanediol or 2,3-hexanediol or 2,3-heptanediol.

The alkanediol mixture comprising 1,2-hexanediol and 2,3-octanediol improves the solubility of a lipophilic active substance. The mixture of said alkanediol mixture and a lipophilic active substance shows high clearness, and the lipophilic active component does not crystallize out or does not precipitate out even during storage.

A solubility improving effect can also be observed for the hetero mixtures including 1,2-octanediol and 2,3-hexanediol. The resulting mixture of said alkanediol mixture and a lipophilic active substance is clear without turbidity and the lipophilic active component does not crystallize out or does not precipitate out even during storage.

A solubility improving effect toward a lipophilic active substance can also be observed for the hetero alkanediol mixture comprising 1,2-octanediol and 2,3-heptanediol.

The afore-specified mixtures are distinguished in an aqueous/glycolic formulation even with a high water content as well as an emulsion.

Additionally, with the admixture of 2,3-hexanediol or 2,3-heptanediol to the solid 1,2-octanediol, the solid 1,2-octanediol can be solved, resulting in a liquid alkanediol mixture. The 2,3-hexanediol or the 2,3-heptanediol serves as solvent for the solid 1,2-octanediol. Such liquid mixtures have the benefit that firstly the availability of the 1,2-octanediol in the mixture is improved and secondly the incorporation of the 1,2-octanediol in semi-finished products or final products is facilitated. This effect is particularly favorably for emulsions, in which lipophilic 1,2-alkanediols having a carbon chain of 8 or more carbon atoms, when used alone, tend to migrate into the oil phase or tend to recrystallize or to precipitate.

Furthermore, a distinct solubility improvement can be observed for the following specified alkanediol mixtures or combinations comprising at least one first linear alkanediol and one or more second linear alkanediol according to the present invention, namely a mixture of alkanediols including 1,2-pentanediol and 2,3-hexanediol; or a mixture including 1,2-pentanediol and 2,3-heptanediol; or a mixture including 1,2-pentanediol and 2,3-octanediol; or a mixture including 1,2-pentanediol and 1,2-nonanediol; or a mixture including 1,2-pentanediol and 2,3-nonanediol.

The afore specified hetero alkanediol mixtures including a 1,2-alkanediol and a 2,3-alkanediol considerably improve the solubility of a lipophilic active substance resulting in a clear solution; said effect is clearly superior to the corresponding individual 1,2-alkanediols or 2,3-alkanediols.

The alkanediol mixture including 1,2-pentanediol and 2,3-hexanediol clearly improves solubility of a lipopohilic active component. The resulting solution of said alkanediol mixture and a lipophilic active component is clear and the lipophilic active component does not crystallize out or does not precipitate out during storage.

A solubility improving effect can also be observed for the alkanediol mixture comprising 1,2-pentanediol and 2,3-heptanediol. The same counts for the alkanediol mixture comprising 1,2-pentanediol and 2,3-octanediol. The resulting solutions of said alkanediol mixtures and a lipophilic active component are clear without crystallization or precipitation even during storage.

Also, an alkanediol mixture comprising 1,2-pentanediol and 1,2-nonanediol or an alkanediol mixture including 1,2-pentanediol and 2,3-nonanediol is effective in improving the solubility of a lipophilic active component. The mixture result in a stable formulation, i.e. in wich the lipohilic active component remains solbulized in which undesired crystallizing-out or precipitation of the lipophilic active component does not occur even when the mixture is stored.

With the solubility improvement of the liphilic active component, the shelf life of the cosmetic or pharmaceutical preparation or homecare product comprising such an alkanediol/lipophilic active component mixture can considerably be improved.

Furthermore, a significant improvement in the solubility of a lipophilic active component can also be observed for the hetero alkanediol mixtures, namely a mixture of alkanediols including 1,2-heptanediol and 1,2-octanediol or a mixture of alkanediols including 1,2-heptanediol and 2,3-octanediol or a mixture of alkanediols including 1,2-heptanediol and 1,2-nonanediol or a mixture or a combination of alkanediols including 1,2-heptanediol and 2,3-nonanediol.

In particular, the alkanediol mixture including 1,2-heptanediol and 1,2-octanediol or an alkanediol mixture including 1,2-heptanediol and 2,3-octanediol remarkably has a solubility improving effect towards a lipophilic active component; no or less recrystallization or precipitation of the lipophilic active component can be observed, even during storage.

A good solubility of the lipohilic active compouent can also be observed for the alkanediol mixture including 1,2-heptanediol and 1,2-nonanediol as well as for the alkanediol mixture including 1,2-heptanediol and 2,3-nonanediol. Both alkanediol mixtures are effective in better solubilize a lipophilic active component, resulting in a clear, non turbid formulations in which undesired crystallizing-out or precipitation of the liqpohilic active component remains undone, even during storage.

The afore specified hetero alkanediol mixtures show a significant solubility improvement for lipholic active substandes and are clearly superior to the individually corresponding 1,2-alkanediols or 2,3-alkanediols, having the same concentration, but which show a poorer solubility. All solutions of said hetero alkanediol mixtures with a lipophilic active component are clear and stable and the lipophilic active substance does not precipitate out even during storage.

Consequently, with the improvement of the solubility of a lipophilic active substance the storage stability and, thus, shelf life of the cosmetic or pharmaceutical preparation or homecare product can be considerably prolonged.

Due to its better solubility, the availability of the lipophilic active substance or component in the cosmetic or pharmaceutical composition is increased. Additionally, due to its better solubility, the lipophilic active substance can be furnished in a higher concentration in the final formulation or can be furnished in a lower concentration in the final formulation compared to a composition including a lipophilic active component with low solubility and without the additional of an alkanediol.

Due to its better solubility, a uniformly solubilized product, were the lipophilic active component is uniformly dissolved in the product can be obtained. Thus, the concentration of the lipophilic active component is exactly the same through the whole formulation from the very first splash to the very last ml, untill the bottle is completely empty. Without the use of an alkanediol as specified herein, the distribution of the lipophilic active component would be quite haphazardly in the formulation and would vary from application to application.

In addition, the above described hetero alkanediol mixtures comprising a 2,3-alkanediol render possible the cold preparation of cosmetic or pharmaceutical formulations or homecare products, i.e. no heating during preparation is necessary. Thus, processability in formulations can be improved.

Hence, a good compromise can be achieved when the active mixture is combined in such a way as to improve solubility for a liphophilic active component, while improving processability in formulations. The above specified alkanediol combinations comprising a first linear alkanediol and a second linear alkanediol solve this balancing problem over the the respective 1,2-alkanediol substances or 2,3-alkanediol substances alone.

Consequently, with the improvement of the solublitiy of a lipophilic active substance the storage stability and shelf life of the cosmetic or pharmaceutical preparation or homecare product can be considerably improved.

In a particularly advantageous variant according to the present invention, the first alkanediol or the second alkanediol is a liquid alkanediol.

The term "liquid alkanediol" within the context of the present invention means an alkanediol component which is liquid at room or ambient temperature and under normal pressure, i.e. standard RTP conditions.

In a very preferred variant according to the present invention, the first alkanediol or the second alkanediol is a liquid alkanediol selected from the group consisting of 1,2-pentanediol, 2,3-pentanediol, 1,2-hexanediol, 2,3-hexanediol, 1,2-heptanediol, 2,3-heptanediol, 2,3-octanediol, 2,3-nonanediol, 2,3-decanediol, 2,3-undecanediol, and mixtures thereof.

Of the aforesaid alkanediols 1,2-pentanediol, 2,3-pentanediol, 1,2-hexanediol, 2,3-hexanediol, 1,2-heptanediol, 2,3-heptanediol, 2,3-octanediol, 2,3-nonanediol or mixtures thereof are most preferred.

In a most particularly advantageous variant according to the present invention, the first alkanediol or the second alkanediol are characterized by a maximum water solubility of less than or equal to 10 % by weight. In a preferred variant, the maximum water solubility of the first alkanediol or the second alkanediol is less than or equal to 10 % by weight and more than or equal to 1.2 % by weight. More preferred, the maximum water solubility of the first linear alkanediol or the second linear alkanediol is less than or equal to 5 % by weight and more than or equal to 1.4 % by weight.

Preferably, the alkanediol with said water solubility is selected from the group consisting of 1,2-heptanediol, 2,3-heptanediol, 2,3-octanediol, and mixtures thereof.

The water solubility of a substance is the saturation mass concentration of the substance in water at a given temperature. The determination of the solubility in water relates to essentially pure substances which are stable in water and not volatile. The determination of the water solubility of the alkanediols is further described in the following Example 2.

The term "alkanediol" within the context of the present invention also includes its stereoisomers. Stereoisomers are molecules that have the same molecular formula and differ only in how their atoms are arranged in three-dimensional space. In accordance with said definition, the first alkanediol having a carbon chain of 5 to 14 carbon atoms or the second linear alkanediol having a carbon chain of 5 to 14 carbon atoms as described above in detail encompass the following stereoisomers:
1,2-alkanediol stereoisomers:
   (2S)-pentane-1,2-diol,
   (2S)-hexane-1,2-diol,
   (2S)-heptane-1,2-diol,
   (2S)-octane-1,2-diol,
   (2S)-nonane-1,2-diol,
   (2S)-decane-1,2-diol,
   (2S)-undecane-1,2-diol
   (2S)-dodecane-1,2-diol,
   (2S)-tridecane-1,2-diol,
   (2S)-tetradecane-1,2-diol,
   (2R)-pentane-1,2-diol,
   (2R)-hexane-1,2-diol,
   (2R)-heptane-1,2-diol,
   (2R)-octane-1,2-diol,
   (2R)-nonane-1,2-diol,
   (2R)-decane-1,2-diol,
   (2R)-undecane-1,2-diol,
   (2R)-dodecane-1,2-diol,
   (2R)-tridecane-1,2-diol,
   (2R)-tetradecane-1,2-diol,
2,3-alkanediol stereoisomers:
   (2S,3S)-pentane-2,3-diol,
   (2S,3S)-hexane-2,3-diol,
   (2S,3S)-heptane-2,3-diol,
   (2S,3S)-octane-2,3-diol,
   (2S,3S)-nonane-2,3-diol,
   (2S,3S)-decane-2,3-diol,
   (2S,3S)-undecane-2,3-diol
   (2S,3S)-dodecane-2,3-diol,
   (2S,3S)-tridecane-2,3-diol,
   (2S,3S)-tetradecane-2,3-diol,
   (2R,3R)-pentane-2,3-diol,
   (2R,3R)-hexane-2,3-diol,
   (2R,3R)-heptane-2,3-diol,
   (2R,3R)-octane-2,3-diol,
   (2R,3R)-nonane-2,3-diol,
   (2R,3R)-decane-2,3-diol,
   (2R,3R)-undecane-2,3-diol,
   (2R,3R)-dodecane-2,3-diol,
   (2R,3R)-tridecane-2,3-diol,
   (2R,3R)-tetradecane-2,3-diol,
   (2S,3R)-pentane-2,3-diol,
   (2S,3R)-hexane-2,3-diol,
   (2S,3R)-heptane-2,3-diol,
   (2S,3R)-octane-2,3-diol,
   (2S,3R)-nonane-2,3-diol,
   (2S,3R)-decane-2,3-diol,
   (2S,3R)-undecane-2,3-diol,
   (2S,3R)-dodecane-23-diol,
   (2S,3R)-tridecane-2,3-diol,
   (2S,3R)-tetradecane-2,3-diol,
   (2R,3S)-pentane-2,3-diol,
   (2R,3S)-hexane-2,3-diol,
   (2R,3S)-heptane-2,3-diol,
   (2R,3S)-octane-2,3-diol,
   (2R,3S)-nonane-2,3-diol,
   (2R,3S)-decane-2,3-diol,
   (2R,3S)-undecane-2,3-diol,
   (2R,3S)-dodecane-2,3-diol,
   (2R,3S)-tridecane-2,3-diol, and
   (2R,3S)-tetradecane-2,3-diol.

In the context of the present text, the terms "1,2-diol", or "2,3-diol" includes both the corresponding S-configured enantiomers and also the R-enantiomeras well as arbitrary mixutres of these S- and R-configured enantiomers, i.e. mixtures of racemates of the respective diols.

The first linear alkanediol and the second linear alkanediol as defined in detail above may be present in the alkanediol mixture according to the present invention in a ratio in a range of 50 : 50 to 99.9 : 0.1, preferably in a ratio in a range of 75 : 25 to 99 : 1, more preferred in a ratio in a range of 80 : 20 to 98 : 2, still more preferred in a ratio in a range of 90 : 10 to 95 : 5.

In the cosmetic or pharmaceutical preparation according to the present invention, the first linear alkanediol and the second linear alkanediol are comprised in the mixture preferably in a ratio in a range of 98 : 2 to 99.9 : 0.1

In the homecare products according to the present invention, the first linear alkanediol and the second alkanediol are comprised in the alkanediol mixture preferably in a ratio in a range of 95 : 5 to 99.9 : 0.1.

Preferably, the first linear alkanediol and the second alkanediol are comprised in the mixture of the cosmetic or pharmaceutical composition or homecare product according to the s present invention in a ratio in a range of ≥ 95 : ≤ 5, more preferred in a ratio of ≥ 96 : ≤ 4; still more preferred in a ratio of ≥ 97 : ≤ 3, and most preferred in a ratio of ≥ 98 : ≤ 2.

Most of all the mixture of the cosmetic or pharmaceutical composition or homecare product according to the present invention comprises the first linear alkanediol and the second linear alkanediol in a ratio of ≥ 95 : ≤ 5, including the ratios ≥ 95.5 : ≤ 4.5; ≥ 96 : ≤ 4; ≥ 96.5 : ≤ 3.5; ≥ 97 : ≤ 3; ≥ 97.5 : 2,5 and ≥ 98.0 : ≤ 2.0.

Even more preferred the alkanediol mixture of the cosmetic or pharmaceutical composition or homecare product according to the present invention comprises the first linear alkanediol and the second alkanediol in a ratio in a range of ≥ 98 : ≤ 2, including the ratios of ≥ 98.1 : ≤ 1.9; ≥ 98.2 : ≤ 1.8; ≥ 98.3 : ≤ 1.7; ≥ 98.4 : ≤ 1.6; ≥ 98.5 : ≤ 1.5; ≥ 98.6 : ≤ 1.4; ≥ 98.7 : ≤ 1.3; ≥ 98.8 : ≤ 1.2; ≥ 98.9 : ≤ 1.1; ≥ 99 : ≤ 1.0; ≥ 99.1 : ≤ 0.9; ≥ 99.2 : ≤ 0.8; ≥ 99.3 : ≤ 0.7; ≥ 99.4 : ≤ 0.6; ≥ 99.5 : ≤ 0.5; ≥ 99.6 : ≤ 0.4; ≥ 99.7 : ≤ 0.3; ≥ 99.8 : ≤ 0.2 and ≥ 99.9 : ≤ 0.1.

Alternatively, said ratio ranges for the first and second alkanediol are switched, such that the second alkanediol is the main component and the first alkanedio is the secondary component.

**In** an advantageous variant according to the present invention, the mixture comprises as first linear alkanediol an 1,2-alkanediol, such as 1,2-pentanediol, 1,2-hexanediol, 1,2-heptanediol, 1,2-octanediol, 1,2-nonanediol, 1,2-decanediol, 1,2-undecanediol, 1,2-dodecanediol, or 1,2-tridecanediol and as second linear alkanediol the corresponding 2,3-alkanediol, such as 2,3,-pentanediol, 2,3-hexanediol, 2,3-heptanediol, 2,3-octanediol, 2,3-nonanediol, 2,3-decanediol, 2,3-undecanediol, 2,3-dodecanediol, or 2,3-tridecanediol in a ratio in a range of 50 : 50 to 99.9 : 0.1, preferably in a ratio in a range of 75 : 25 to 99 : 1, more preferred in a ratio in a range of 80 : 20 to 98: 2, still more preferred in a ratio in a range of 90 : 10 to 95 : 5.

In a still further variant, according to the present invention, the mixture comprises as first linear alkanediol an 2,3-alkanediol, such as 2,3-pentanediol, 2,3-hexanediol, 2,3-heptanediol, 2,3-octanediol, 2,3-nonanediol, 2,3-decanediol, 2,3-undecanediol, 2,3-dodecanediol, or 2,3-tridecanediol and as second linear alkanediol the corresponding 1,2-alkanediol, such as 1,2-pentanediol, 1,2-hexanediol, 1,2-heptanediol, 1,2- octanediol, 1,2-nonanediol, 1,2-decanediol, 1,2-undecanediol, 1,2-dodecanediol, or 1,2-tridecanediol in a ratio in a range of 50 : 50 to 99.9 : 0.1, preferably in a ratio in a range of 75 : 25 to 99 : 1, more preferred in a ratio in a range of 80 : 20 to 98 : 2, still more preferred in a ratio in a range of 90 : 10 to 95 : 5.

Likewise, for said mixtures, the ratios of first linear alkanediol : second alkanediol or ranges of ratios as described above are also applicable.

In a more advantageous variant according to the present invention, in the mixtures comprising a combination of a 1,2-alkanediol as first linear alkanediol and the corresponding 2,3-alkanediol as second linear alkanediol, such as
1,2-pentanediol and 2,3-pentanediol, or
1,2-hexanediol and 2,3-hexanediol, or
1,2-heptanediol and 2,3-heptanediol, or
1,2-octanediol and 2,3-octanediol, or
1,2-nonanediol and 2,3-nonanediol, or
1,2-decanediol and 2,2-decanediol, or
1,2-undecanediol and 2,3-undecenaediol, or
1,2-dodecanediol and 2,3-dodecanediol, or
1,2-tridecanediol and 2,3-tridecanediol,
the first linear alkanediol and the second linear alkanediol are present in a ratio in a range of 50 : 50 to 99.9 : 0.1, preferably in a ratio in a range of 75 : 25 to 99 : 1, more preferred in a ratio in a range of 80 : 20 to 98 : 2, still more preferred in ratio in a range of 90 : 10 to 95 : 5.

Likewise, for said mixtures, the ratios of first linear alkanediol : second alkanediol or ranges of ratios as described above are also applicable.

By these mixing ratios the afore mentioned mixtures show an improved solubility for a lipophilic active substance in direct comparison with the corresponding individual substances as it is described and demonstrated in the experimental part.

In a more advantageous variant according to the present invention, in the mixtures comprising as first linear alkanediol a 2,3-alkanediol and as second linear alkanediol the corresponding 1,2-alkanediols, such as mixtures including
2,3-pentanediol and 1,2-pentanediol, or
2,3-hexanediol and 1,2-hexanediol, or
2,3-heptanediol and 1,2-heptanediol, or
2,3-octanediol and 1,2-octanediol, or
2,3-nonanediol and 1,2-nonanediol, or
2,3-decanediol and 1,2-decanediol, or
2,3-undecanediol and 1,2-undecenaediol, or
2,3-dodecanediol and 1,2-dodecanediol, or
2,3-tridecanediol and 1,2-tridecanediol,
the first linear alkanediol and the second linear alkanediol are present in a ratio in a range of 50 : 50 to 99.9 : 0.1, preferably in a ratio in a range of 75 : 25 to 99 : 1, more preferred in a ratio in a range of 80 : 20 to 98 : 2, still more preferred in a ratio in a range of 90 : 10 to 95 : 5.

Likewise, for said mixtures, the ratios of first linear alkanediol : second alkanediol or ranges of ratios as described above are also applicable.

By these mixing ratios the afore mentioned mixtures show an improved solubility for a lipophilic active substance in direct comparison with the corresponding individual substances as it is described and demonstrated in the experimental part.

A preferred variant according to the present invention also encompasses a mixture including as first linear alkanediol 1,2-hexanediol and as second linear alkanediol 2,3-octanediol or a mixture including as first linear alkanediol 1,2-octanediol and as second linear alkanediol 2,3-hexanediol or a mixture including as first linear alkanediol 1,2-octanediol and and as second linear alkanediol 2,3-heptanediol either in a ratio in a range of 50 : 50 to 99.9 : 0.1, preferably in a ratio in a range of 75 : 25 to 99 : 1, more preferred in a ratio in a range of 80 : 20 to 98 : 2, still more preferred in a ratio in a range of 90 : 10 to 95 : 5.

Likewise, for said mixtures, the ratios of first linear alkanediol : second alkanediol or ranges of ratios as described above are also applicable.

By these mixing ratios the afore mentioned mixtures show an improved solubility for a lipophilic active substance in direct comparison with the corresponding individual substances as it is described and demonstrated in the experimental part.

In a further beneficial variant according to the present invention, the mixtures comprise a first linear alkanediol and a second linear alkanediol, such as mixtures including
1,2-pentanediol and 2,3-hexanediol; or
1,2-pentanediol and 2,3-heptanediol; or
1,2-pentanediol and 2,3-octanediol; or
1,2-pentanediol and 1,2-nonanediol; or
1,2-pentanediol and 2,3-nonanediol,
wherein the first linear alkanediol and the second linear alkanediol are present in a ratio in a range of 50 : 50 to 99.9 : 0.1, preferably in a ratio in a range of 75 : 25 to 99 : 1, more preferred in a ratio in a range of 80 : 20 to 98 : 2, still more preferred in a ratio in a range of 90 : 10 to 95 : 5.

Likewise, for said mixtures, the ratios of first linear alkanediol : second alkanediol or ranges of ratios as described above are also applicable.

By these mixing ratios the afore mentioned mixtures show an improved solubility for a lipophilic active substance in direct comparison with the corresponding individual substances as it is described and demonstrated in the experimental part.

A particularly preferred variant according to the present invention also encompasses a mixture including as first linear alkanediol 1,2-heptanediol and as second linear alkanediol 1,2-octanediol, or a mixture including as first liear alkanediol 1,2-heptanediol and as second linear alkanediol 2,3-octanediol or a mixture including as first linear alkanediol 1,2-heptanediol and as second linear alkanediol 1,2-nonanediol or a mixture including as first linear alkanediol 1,2-heptanediol and as second linear alkanediol 2,3-nonanediol wherein the first linear alkanediol and the second linear alkanediol are present in a ratio in a range of 50 : 50 to 99.9 : 0.1, preferably in a ratio in a range of 75 : 25 to 99 : 1, more preferred in a ratio in a range of 80 : 20 to 98 : 2 or still more preferred in a ratio in a range of 90 : 10 to 95 : 5.

Likewise, for said mixtures, the ratios of first linear alkanediol : second alkanediol or ranges of ratios as described above are also applicable.

By these mixing ratios the afore mentioned mixtures show an improved solubility for a lipophilic active substance in direct comparison with the corresponding individual substances as it is described and demonstrated in the experimental part.

The alkanediols are obtained either by synthesis from petrochemical or other fossil fuel sources by known methods such as olefin bishydroxylation, hydrolysis from epoxide or various chemical transformations or from bioderived feedstock by fermentation or from bio-based natural and renewable feedstock such as biomass by catalytic synthesis as it is described in US 2019/0241491 A1 and US 2020/0189995 A1. The alkanediols used according to the present invention comprise either petrochemically derived and biobased natural and renewable feedstock derived alkanediols. Preferably, the alkanediols are from bio-based sources and are thus bioalkanediols.

The compound (b) of the cosmetic or pharmaceutical composition or homecare product according to the present invention relates to a lipophilic active component, which is used in many cosmetic compositions, in particular skincare, haircare or body care, or for pharmaceutical compositions.

The term "lipophilic" or "lipophilicity" refers to the ability of a chemical compound to dissolve in fats, oils, lipids and non-polar solvents such as hexane or toluene. Such non-polar solvents are themselves lipophilic. Thus, lipophilic substances tend to dissolve in other lipophilic substances, whereas hydrophilic substances tend to dissolve in water and other hydrophilic substances. The "lipophilic" substances according to the present invention may be not soluble in water at RTP in a concentration of more than 0.01 % by weight. The water solubility of a substance is the saturation mass concentration of the substance in water at ambient temperature.

The term "active component" within the context of the present invention means a component or a substance which imparts one or more than one benefit, functions or operate via more than one mode of action, such as anti-inflammatory, anti-itching, anti-redness, anti-dandruff, anti-microbial, anti-perspirant, deodorant, hair growth strengthening, anti-dark-spot, anti-cellulite, anti-viral, anti-oxidative, anti-wrinkle, skin-whitening, skin tanning, pain-reducing, swelling reduction, skin barrier strengthening, anti-mycotic, anti-sagging, and skin soothing properties.

The lipophilic active component according to the present invention is characterized by a log P_{O/W} value range of ≥ 0 to 25.00. Preferably, the lipophilic active component has a log P_{O/W} value range of ≥ 0 to 20.00, more preferred a log P_{O/W} value range of ≥ 0 to 16.00, particularly preferred a log P_{O/W} value range of ≥ 0 to 5.00, calculated by EPISuite v4.40 (U.S.Environmental Protection Agency; calculated with KOWWIN v1.68) . The higher the log P_{O/W} value the poorer the solubility of the lipophilic active component in an aqueous system, such as an aqueous, aqueous/alcoholic or aqueous/glycolic based solution. More preferred is a log P_{O/W} value range for the lipophilic active component from greater or equal to 1.00 to less than or equal to 4.00.

In general, the log P value is a constant defined in the following manner: log P = log10 (partition coefficient). Partition Coefficient, P = [organic]/[aqueous], where [ ] indicates the concentration of solute in the organic and aqueous partition, in the present case in the 1-octane and water partition. A negative value for log P means the compound has a higher affinity for the aqueous phase (it is more hydrophilic). When log P = 0 the compound is equally partitioned between the lipid and aqueous phases. A positive value for log P denotes a higher concentration in the lipid phase (i.e. the compound is more lipophilic). Log P = 1 means there is a 10 : 1 partitioning in organic : aqueous phases. log P values are important to many industries and areas of research in determining how to deliver chemical substances. Log P is used in the pharmaceutical/biotech industries to understand the behaviour of drug molecules in the body. Drug candidates are often screened according to log P, among other criteria, to help guide drug selection and analogue optimization. This is because lipophilicity is a major determining factor in a compound's absorption, distribution in the body, penetration across vital membranes and biological barriers, metabolism and excretion (ADME properties). For topical applications, the log P_{O/W} value of a compound should be preferably ≥ 0, more preferably in a range of ≥ 0.50 to 16.00.

The lipophilic active component in the cosmetic or pharmaceutical composition or homecare product according to the present invention is either a lipophilic cosmetically active component or a lipophilic pharmaceutically active component.

The lipophilic active component in the cosmetic or pharmaceutical composition or homecare product according to the present invention encompasses at least one lipophilic cosmetically active component selected from the group consisting of Tocopherol (log P_{O/W}: 12.18), Bisabolol (log P_{O/W}: 5.63), Ethylhexylglycerin (log P_{O/W}: 2.22), o-Cymen-5-ol (log P_{O/W}: 3.52), Propanediol Caprylate (log P_{O/W}: 2.84), Glyceryl Caprylate (log P_{O/W}: 1.71), Retinyl Palmitate (log P_{O/W}: 15.51), Retinol (log P_{O/W}: 7.62 ), Farnesol (log P_{O/W}: 5.77), Glyceryl Laurate (log P_{O/W}: 3.67), Polyglyceryl-2 caprylate (log P_{O/W}: > 1), Hydroxyacetophenone (log P_{O/W}: 1.19), Caffeine (log P_{O/W}: 0.16), Undecylenoyl Phenylalanine (log P_{O/W}: 5.31), Phenylethyl Resorcinol (log P_{O/W}: 3.19), Hydroxymethoxyphenyl decanone (log P_{O/W}: 4.62), Zingiber Officinale (Ginger) Root Extract, Myristoyl Nonapeptide-3 (log P_{O/W}: > 1), Palmitoyl Tripeptide-1 (log P_{O/W}: > 1), Palmitoyl Tetrapeptide-7 (log P_{O/W}: > 1), Palmitoyl Pentapeptide-4 (log P_{O/W}: > 1), Hydroxypinacolone Retinoate (log P_{O/W}: 8.93), Isochrysis Galbana Extract, Sclareolide (log P_{O/W}: 3.86), Vanillyl Butylether (log P_{O/W}: 2.59), Hydroxyphenyl Propamidobenzoic Acid (log P_{O/W}: 3.71), 4-t-Butylcyclohexanol (log P_{O/W}: 3.42), Ubiquinone-10 (log P_{O/W}: 23.40), Climbazole (log P_{O/W}: 3.72), Decylene Glycol (log P_{O/W}: 2.66), and mixtures of two or more of the aforesaid lipophilic cosmetically active components. Said lipophilic cosmetically active components are characterized by a log P_{O/W} value in a range of ≥ 0 to 24.00.

The lipophilic active component in the cosmetic or pharmaceutical composition or homecare product according to the present invention preferably encompasses at least one lipophilic cosmetically active component selected from the group consisting of Tocopherol (log P_{O/W}: 12.18), Ethylhexylglycerin (log P_{O/W}: 2.22), o-Cymen-5-ol (log P_{O/W}: 3.52), Propanediol Caprylate (log P_{O/W}: 2.84), Glyceryl Caprylate (log P_{O/W}: 1.71), Retinyl Palmitate (log P_{O/W}: 15.51), Retinol (log P_{O/W}: 7.62 ), Farnesol (log P_{O/W}: 5.77), Glyceryl Laurate (log P_{O/W}: 3.67), Polyglyceryl-2 caprylate (log P_{O/W}: > 1), Hydroxyacetophenone (log P_{O/W}: 1.19), Caffeine (log P_{O/W}: 0.16), Undecylenoyl Phenylalanine (log P_{O/W}: 5.31), Phenylethyl Resorcinol (log P_{O/W}: 3.19), Hydroxymethoxyphenyl decanone (log P_{O/W}: 4.62), Zingiber Officinale (Ginger) Root Extract, Myristoyl Nonapeptide-3 (log P_{O/W}: > 1), Palmitoyl Tripeptide-1 (log P_{O/W}: > 1), Palmitoyl Tetrapeptide-7 (log P_{O/W}: > 1), Palmitoyl Pentapeptide-4 (log P_{O/W}: > 1), Hydroxypinacolone Retinoate (log P_{O/W}: 8.93), Isochrysis Galbana Extract, Sclareolide (log P_{O/W}: 3.86), Vanillyl Butylether (log P_{O/W}: 2.59), Hydroxyphenyl Propamidobenzoic Acid (log P_{O/W}: 3.71), 4-t-Butylcyclohexanol (log P_{O/W}: 3.42), Ubiquinone-10 (log P_{O/W}: 23.40), Climbazole (log P_{O/W}: 3.72), Decylene Glycol (log P_{O/W}: 2.66),and mixtures of two or more of the aforesaid lipophilic cosmetically active components. Said lipophilic cosmetically active components are characterized by a log P_{O/W} value in a range of ≥ 0 to 24.00.

In an alternative variant, the lipophilic active component of the cosmetic or pharmaceutical composition or homecare product of the present invention is a lipophilic pharmaceutically active component selected from the group consisting of Erythromycin (log P_{O/W}: 2.48), Betamethasone (log P_{O/W}: 1.72), Ibuprofen (log P_{O/W}: 3.79), Ketoprofen (log P_{O/W}: 3.00), Diclofenac (log P_{O/W}: 0.57), Metronidazole (log P_{O/W}: 0.00), Imiquimod (log P_{O/W}: 3.06), Celecoxib (log P_{O/W}: 3.47), Indomethacin (log P_{O/W}: 4.23), Caffeine (log P_{O/W}: 0.16), Minoxidil (log P_{O/W}: 0.27), Hydrocortisone (log P_{O/W}: 1.62), Dimetindene (log P_{O/W}: 4.98), and mixtures of two or more of the aforesaid lipophilic pharmaceutical active components. Said pharmaceutical active components are characterized by a log P_{O/W} value in the range of ≥ 0 to 4.50.

**In** a particularly preferred variant, the lipophilic pharmaceutically active component of the cosmetic or pharmaceutical composition of the present invention is selected from the group consisting of Minoxidil, Dimetindene, Caffeine, Hydrocortisone, and mixtures of two or more of the aforesaid lipophilic pharmaceutically active components.

**The** aforesaid lipophilic active components can be used either as a single component or in combination with one or more further lipophilic active components as specified above.

**In** a preferred variant the component (b) is different to the component (a) of the cosmetic or pharmaceutical composition or homecare product according to the present invention.

**In** a preferred advantageously variant, the cosmetic or pharmaceutical composition or homecare product according to the present invention comprises one of the following combinations of components (a) and (b):
▪ 1,2-heptanediol in combination with 2,3-heptanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-heptanediol in combination with one or more of 1,2-octanediol or 1,2-nonanediol, plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-heptanediol in combination with one or more of 2,3-octanediol or 2,3-nonanediol, plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-pentanediol in combination with 2,3-pentanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-hexanediol in combination with 2,3-hexanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-octanediol in combination with 2,3-octanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-nonanediol in combination with 2,3-nonanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-decanediol in combination with 2,3-decanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-undecanediol in combination with 2,3-undecanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-dodecanediol in combination with 2,3-dodecanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-tridecanediol in combination with 2,3-tridecanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-hexanediol in combination with 2,3-octanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-octanediol in combination with 2,3-hexanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-octanediol in combination with 2,3-heptanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-pentanediol in combination with 2,3-hexanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-pentanediol in combination with 2,3-heptanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-pentanediol in combination with 2,3-octanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-pentanediol in combination with 1,2-nonanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-pentanediol in combination with 2,3-nonanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-heptanediol in combination with 1,2-octanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-heptanediol in combination with 2,3-octanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-heptanediol in combination with 1,2-nonanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-heptanediol in combination with 2,3-nonanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole.

More preferred combinations of component (a) and (b) in the cosmetic or pharmaceutical composition or homecare product according to the present invention are:
▪ 1,2-heptanediol in combination with 2,3-heptanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-heptanediol in combination with one or more of 1,2-octanediol or 1,2-nonanediol, plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-heptanediol in combination with one or more of 2,3-octanediol or 2,3-nonanediol, plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-pentanediol in combination with 2,3-pentanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-hexanediol in combination with 2,3-hexanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-octanediol in combination with 2,3-octanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-nonanediol in combination with 2,3-nonanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-decanediol in combination with 2,3-decanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-undecanediol in combination with 2,3-undecanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-dodecanediol in combination with 2,3-dodecanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole; or
▪ 1,2-tridecanediol in combination with 2,3-tridecanediol plus one or more lipophilic active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, and Climbazole.

**In** a further particular advantageously variant, the cosmetic or pharmaceutical composition or homecare product according to the present invention comprises one of the following combinations of components (a) and (b):
▪ 1,2-heptanediol in combination with 2,3-heptanediol plus one or more lipophilic active component selected from the group consisting of Erythromycin, Betamethasone, Ibuprofen, Ketoprofen, Diclofenac, Metronidazole, Imiquimod, Celecoxib, Indomethacin, Caffeine, Minoxidil, and Hydrocortisone; or
▪ 1,2-heptanediol in combination with one or more of 1,2-octanediol or 1,2-nonanediol, plus one or more lipophilic active component selected from the group consisting of Erythromycin, Betamethasone, Ibuprofen, Ketoprofen, Diclofenac, Metronidazole, Imiquimod, Celecoxib, Indomethacin, Caffeine, Minoxidil, and Hydrocortisone; or
▪ 1,2-heptanediol in combination with one or more of 2,3-octanediol or 2,3-nonanediol, plus one or more lipophilic active component selected from the group consisting of Erythromycin, Betamethasone, Ibuprofen, Ketoprofen, Diclofenac, Metronidazole, Imiquimod, Celecoxib, Indomethacin, Caffeine, Minoxidil, and Hydrocortisone; or
▪ 1,2-pentanediol in combination with 2,3-pentanediol plus one or more lipophilic active component selected from the group consisting of Erythromycin, Betamethasone, Ibuprofen, Ketoprofen, Diclofenac, Metronidazole, Imiquimod, Celecoxib, Indomethacin, Caffeine, Minoxidil, and Hydrocortisone; or
▪ 1,2-hexanediol in combination with 2,3-hexanediol plus one or more lipophilic active component selected from the group consisting of Erythromycin, Betamethasone, Ibuprofen, Ketoprofen, Diclofenac, Metronidazole, Imiquimod, Celecoxib, Indomethacin, Caffeine, Minoxidil, and Hydrocortisone; or
▪ 1,2-octanediol in combination with 2,3-octanediol plus one or more lipophilic active component selected from the group consisting of Erythromycin, Betamethasone, Ibuprofen, Ketoprofen, Diclofenac, Metronidazole, Imiquimod, Celecoxib, Indomethacin, Caffeine, Minoxidil, and Hydrocortisone; or
▪ 1,2-nonanediol in combination with 2,3-nonanediol plus one or more lipophilic active component selected from the group consisting of Erythromycin, Betamethasone, Ibuprofen, Ketoprofen, Diclofenac, Metronidazole, Imiquimod, Celecoxib, Indomethacin, Caffeine, Minoxidil, and Hydrocortisone; or
▪ 1,2-decanediol in combination with 2,3-decanediol plus one or more lipophilic active component selected from the group consisting of Erythromycin, Betamethasone, Ibuprofen, Ketoprofen, Diclofenac, Metronidazole, Imiquimod, Celecoxib, Indomethacin, Caffeine, Minoxidil, and Hydrocortisone; or
▪ 1,2-undecanediol in combination with 2,3-undecanediol plus one or more lipophilic active component selected from the group consisting of Erythromycin, Betamethasone, Ibuprofen, Ketoprofen, Diclofenac, Metronidazole, Imiquimod, Celecoxib, Indomethacin, Caffeine, Minoxidil, and Hydrocortisone; or
▪ 1,2-dodecanediol in combination with 2,3-dodecanediol plus one or more lipophilic active component selected from the group consisting of Erythromycin, Betamethasone, Ibuprofen, Ketoprofen, Diclofenac, Metronidazole, Imiquimod, Celecoxib, Indomethacin, Caffeine, Minoxidil, and Hydrocortisone; or
▪ 1,2-tridecanediol in combination with 2,3-tridecanediol plus one or more lipophilic active component selected from the group consisting of Erythromycin, Betamethasone, Ibuprofen, Ketoprofen, Diclofenac, Metronidazole, Imiquimod, Celecoxib, Indomethacin, Caffeine, Minoxidil, and Hydrocortisone; or
▪ 1,2-hexanediol in combination with 2,3-octanediol plus one or more lipophilic active component selected from the group consisting of Erythromycin, Betamethasone, Ibuprofen, Ketoprofen, Diclofenac, Metronidazole, Imiquimod, Celecoxib, Indomethacin, Caffeine, Minoxidil, and Hydrocortisone; or
▪ 1,2-octanediol in combination with 2,3-hexanediol plus one or more lipophilic active component selected from the group consisting of Erythromycin, Betamethasone, Ibuprofen, Ketoprofen, Diclofenac, Metronidazole, Imiquimod, Celecoxib, Indomethacin, Caffeine, Minoxidil, and Hydrocortisone; or
▪ 1,2-octanediol in combination with 2,3-heptanediol plus one or more lipophilic active component selected from the group consisting of Erythromycin, Betamethasone, Ibuprofen, Ketoprofen, Diclofenac, Metronidazole, Imiquimod, Celecoxib, Indomethacin, Caffeine, Minoxidil, and Hydrocortisone; or
▪ 1,2-pentanediol in combination with 2,3-hexanediol plus one or more lipophilic active component selected from the group consisting of Erythromycin, Betamethasone, Ibuprofen, Ketoprofen, Diclofenac, Metronidazole, Imiquimod, Celecoxib, Indomethacin, Caffeine, Minoxidil, and Hydrocortisone; or
▪ 1,2-pentanediol in combination with 2,3-heptanediol plus one or more lipophilic active component selected from the group consisting of Erythromycin, Betamethasone, Ibuprofen, Ketoprofen, Diclofenac, Metronidazole, Imiquimod, Celecoxib, Indomethacin, Caffeine, Minoxidil, and Hydrocortisone; or
▪ 1,2-pentanediol in combination with 2,3-octanediol plus one or more lipophilic active component selected from the group consisting of Erythromycin, Betamethasone, Ibuprofen, Ketoprofen, Diclofenac, Metronidazole, Imiquimod, Celecoxib, Indomethacin, Caffeine, Minoxidil, and Hydrocortisone; or
▪ 1,2-pentanediol in combination with 1,2-nonanediol plus one or more lipophilic active component selected from the group consisting of Erythromycin, Betamethasone, Ibuprofen, Ketoprofen, Diclofenac, Metronidazole, Imiquimod, Celecoxib, Indomethacin, Caffeine, Minoxidil, and Hydrocortisone; or
▪ 1,2-pentanediol in combination with 2,3-nonanediol plus one or more lipophilic active component selected from the group consisting of Erythromycin, Betamethasone, Ibuprofen, Ketoprofen, Diclofenac, Metronidazole, Imiquimod, Celecoxib, Indomethacin, Caffeine, Minoxidil, and Hydrocortisone; or
▪ 1,2-heptanediol in combination with 1,2-octanediol plus one or more lipophilic active component selected from the group consisting of Erythromycin, Betamethasone, Ibuprofen, Ketoprofen, Diclofenac, Metronidazole, Imiquimod, Celecoxib, Indomethacin, Caffeine, Minoxidil, and Hydrocortisone; or
▪ 1,2-heptanediol in combination with 2,3-octanediol plus one or more lipophilic active component selected from the group consisting of Erythromycin, Betamethasone, Ibuprofen, Ketoprofen, Diclofenac, Metronidazole, Imiquimod, Celecoxib, Indomethacin, Caffeine, Minoxidil, and Hydrocortisone; or
▪ 1,2-heptanediol in combination with 1,2-nonanediol plus one or more lipophilic active component selected from the group consisting of Erythromycin, Betamethasone, Ibuprofen, Ketoprofen, Diclofenac, Metronidazole, Imiquimod, Celecoxib, Indomethacin, Caffeine, Minoxidil, and Hydrocortisone; or
▪ 1,2-heptanediol in combination with 2,3-nonanediol plus one or more lipophilic active component selected from the group consisting of Erythromycin, Betamethasone, Ibuprofen, Ketoprofen, Diclofenac, Metronidazole, Imiquimod, Celecoxib, Indomethacin, Caffeine, Minoxidil, and Hydrocortisone.

In a very preferred and advantageously variant, the cosmetic or pharmaceutical composition or homecare product according to the present invention comprises one of the following combinations of components (a) and (b):
▪ 1,2-heptanediol in combination with 2,3-heptanediol plus one or more lipophilic active component selected from the group consisting of Minoxidil, Dimetindene, Caffeine, and Hydrocortisone; or
▪ 1,2-heptanediol in combination with one or more of 1,2-octanediol or 1,2-nonanediol, plus one or more lipophilic active component selected from the group consisting of Minoxidil, Dimetindene, Caffeine, and Hydrocortisone; or
▪ 1,2-heptanediol in combination with one or more of 2,3-octanediol or 2,3-nonanediol, plus one or more lipophilic active component selected from the group consisting of Minoxidil, Dimetindene, Caffeine, and Hydrocortisone; or
▪ 1,2-pentanediol in combination with 2,3-pentanediol plus one or more lipophilic active component selected from the group consisting of Minoxidil, Dimetindene, Caffeine, and Hydrocortisone; or
▪ 1,2-hexanediol in combination with 2,3-hexanediol plus one or more lipophilic active component selected from the group consisting of Minoxidil, Dimetindene, Caffeine, and Hydrocortisone; or
▪ 1,2-octanediol in combination with 2,3-octanediol plus one or more lipophilic active component selected from the group consisting of Minoxidil, Dimetindene, Caffeine, and Hydrocortisone; or
▪ 1,2-nonanediol in combination with 2,3-nonanediol plus one or more lipophilic active component selected from the group consisting of Minoxidil, Dimetindene, Caffeine, and Hydrocortisone; or
▪ 1,2-decanediol in combination with 2,3-decanediol plus one or more lipophilic active component selected from the group consisting of Minoxidil, Dimetindene, Caffeine, and Hydrocortisone; or
▪ 1,2-undecanediol in combination with 2,3-undecanediol plus one or more lipophilic active component selected from the group consisting of Minoxidil, Dimetindene, Caffeine, and Hydrocortisone; or
▪ 1,2-dodecanediol in combination with 2,3-dodecanediol plus one or more lipophilic active component selected from the group consisting of Minoxidil, Dimetindene, Caffeine, and Hydrocortisone; or
▪ 1,2-tridecanediol in combination with 2,3-tridecanediol plus one or more lipophilic active component selected from the group consisting of Minoxidil, Dimetindene, Caffeine, and Hydrocortisone; or
▪ 1,2-hexanediol in combination with 2,3-octanediol plus one or more lipophilic active component selected from the group consisting of Minoxidil, Dimetindene, Caffeine, and Hydrocortisone; or
▪ 1,2-octanediol in combination with 2,3-hexanediol plus one or more lipophilic active component selected from the group consisting of Minoxidil, Dimetindene, Caffeine, and Hydrocortisone; or
▪ 1,2-octanediol in combination with 2,3-heptanediol plus one or more lipophilic active component selected from the group consisting of Minoxidil, Dimetindene, Caffeine, and Hydrocortisone; or
▪ 1,2-pentanediol in combination with 2,3-hexanediol plus one or more lipophilic active component selected from the group consisting of Minoxidil, Dimetindene, Caffeine, and Hydrocortisone; or
▪ 1,2-pentanediol in combination with 2,3-heptanediol plus one or more lipophilic active component selected from the group consisting of Minoxidil, Dimetindene, Caffeine, and Hydrocortisone; or
▪ 1,2-pentanediol in combination with 2,3-octanediol plus one or more lipophilic active component selected from the group consisting of Minoxidil, Dimetindene, Caffeine, and Hydrocortisone; or
▪ 1,2-pentanediol in combination with 1,2-nonanediol plus one or more lipophilic active component selected from the group consisting of Minoxidil, Dimetindene, Caffeine, and Hydrocortisone; or
▪ 1,2-pentanediol in combination with 2,3-nonanediol plus one or more lipophilic active component selected from the group consisting of Minoxidil, Dimetindene, Caffeine, and Hydrocortisone; or
▪ 1,2-heptanediol in combination with 1,2-octanediol plus one or more lipophilic active component selected from the group consisting of Minoxidil, Dimetindene, Caffeine, and Hydrocortisone; or
▪ 1,2-heptanediol in combination with 2,3-octanediol plus one or more lipophilic active component selected from the group consisting of Minoxidil, Dimetindene, Caffeine, and Hydrocortisone; or
▪ 1,2-heptanediol in combination with 1,2-nonanediol plus one or more lipophilic active component selected from the group consisting of Minoxidil, Dimetindene, Caffeine, and Hydrocortisone; or
▪ 1,2-heptanediol in combination with 2,3-nonanediol plus one or more lipophilic active component selected from the group consisting of Minoxidil, Dimetindene, Caffeine, and Hydrocortisone.

**In** a most preferred variant, the cosmetic or pharmaceutical composition or homecare product according to the present invention comprises one of the following combinations of components (a) and (b):
▪ 1,2-heptanediol in combination with 2,3-heptanediol plus caffeine; or
▪ 1,2-heptanediol in combination with one or more of 1,2-octanediol or 1,2-nonanediol, plus caffeine; or
▪ 1,2-heptanediol in combination with one or more of 2,3-octanediol or 2,3-nonanediol, plus caffeine; or
▪ 1,2-pentanediol in combination with 2,3-pentanediol plus caffeine; or
▪ 1,2-hexanediol in combination with 2,3-hexanediol plus caffeine; or
▪ 1,2-octanediol in combination with 2,3-octanediol plus caffeine; or
▪ 1,2-nonanediol in combination with 2,3-nonanediol plus caffeine; or
▪ 1,2-decanediol in combination with 2,3-decanediol plus caffeine; or
▪ 1,2-undecanediol in combination with 2,3-undecanediol plus caffeine; or
▪ 1,2-dodecanediol in combination with 2,3-dodecanediol plus caffeine; or
▪ 1,2-tridecanediol in combination with 2,3-tridecanediol plus caffeine; or
▪ 1,2-hexanediol in combination with 2,3-octanediol plus caffeine; or
▪ 1,2-octanediol in combination with 2,3-hexanediol plus caffeine; or
▪ 1,2-octanediol in combination with 2,3-heptanediol plus caffeine; or
▪ 1,2-pentanediol in combination with 2,3-hexanediol plus caffeine; or
▪ 1,2-pentanediol in combination with 2,3-heptanediol plus caffeine; or
▪ 1,2-pentanediol in combination with 2,3-octanediol plus caffeine; or
▪ 1,2-pentanediol in combination with 1,2-nonanediol plus caffeine; or
▪ 1,2-pentanediol in combination with 2,3-nonanediol plus caffeine; or
▪ 1,2-heptanediol in combination with 1,2-octanediol plus caffeine; or
▪ 1,2-heptanediol in combination with 2,3-octanediol plus caffeine; or
▪ 1,2-heptanediol in combination with 1,2-nonanediol plus caffeine; or
▪ 1,2-heptanediol in combination with 2,3-nonanediol.

Of the above combinations, the alkanediol mixtures comprising a 1,2-alkanediol and a 2,3-alkanediol, result in distinguished solubility properties, i.e. the lipophilic active component(s) can be solubilized in the ready-to-use composition or formulation, in particular in an aqueous, aqueous/alcoholic or aqueous/glycolic based solution or an emulsion, and does/do not crystallize out or precipitate out of the composition or formulation. This effect is demonstrated by the following examples.

The above-defined specific combinations of components (a) and (b) can be combined again with one or more further component(s) as described later on.

In order to further increase the solubility of the lipophilic active substance(s), the cosmetic or pharmaceutical composition or homecare product as defined herein, is advantageously combined with at least one solubilizing agent and/or at least one hydrophilic solvent.

Solubilizing agents or solubilizers in the context of the present invention are substances which aid in the solubilization of an ingredient (solute) in a medium in which it is otherwise insoluble. This definition is specific and excludes co-solvency, i.e., the use of a mixed solvent such as alcohols and water in a clear fragrance product. It also excludes changes in solubility effected by pH modification, such as the dissolution of lauric acid in aqueous ammonium hydroxide. The process of solubilization generally requires that the solute becomes part of the micelle formed by the solubilizing agent.

The solubilizers which can be used in the cosmetic or pharmaceutical composition or homecare product according to the present invention are preferred but naturally are not limiting: PEG-40 Hydrogenated Castor Oil, Polysorbate-20, 60 and 80, PEG-8, PEG-7 Glyceryl Cocoate, Decyl Glucoside, Ceteareth-20, Cetearyl Glucoside, PEG-60 Hydrogenated Castor Oil, Steareth-2, Steareth-20, PPG-26-buteth-26, PPG-5-Laureth-5, Steareth-21, Trideceth-9, PEG-32, Glycereth-26, Polyglyceryl-3 Distearate, Methyl Gluceth-20, PEG-12 Laurate, Polyglyceryl-4 Caprylate, Oleth-20, Oleth-2, Polyglyceryl-4 Caprate, PPG-1-PEG-9 Lauryl Glycol Ether, Polyglyceryl-2 Caprate, Isoceteth-20, PPG-13-decyltetradeceth-24, 1,2-pentanediol, 1,2-hexanediol, 1,2-heptanediol, 2,3-pentanediol, 2,3-hexanediol, 2,3-heptanediol, 2,3-octanediol, 2,3-nonanediol, 2,3-decanediol, or mixtures of two or more of the aforementioned solubilizing agents.

In a more preferred variant, the cosmetic or pharmaceutical composition or homecare product according to the present invention comprises a combination with one or more solubilizing agents which are selected from the group consisting of PEG-40 Hydrogenated Castor Oil, Polysorbate-20, 60 and 80, PEG-8, PEG-7 Glyceryl Cocoate, Decyl Glucoside, Ceteareth-20, Cetearyl Glucoside, PEG-60 Hydrogenated Castor Oil, Steareth-2, Steareth-20, PPG-26-buteth-26, PPG-5-Laureth-5, Steareth-21, Trideceth-9, and mixtures of two or more of the aforementioned solubilizing agents.

The hydrophilic solvents which can be used in the cosmetic or pharmaceutical composition or homecare product according to the present invention are preferred but naturally are not limiting: Glycerin, Propylene Glycol, Butylene Glycol, Isopropyl Alcohol, Porpanediol, Dipropylene Glycol, Propylene Carbonate, Ethanol, Hexylene Glycol, Glycerin Carbonate, Methylpropanediol, Butylene Carbonate, Dimethyl Isosorbide, or mixtures of two or more of the aforementioned hydrophilic solvents.

The solubilizing agent and/or the hydrophilic solvent is/are different to the component(s) (a) of the cosmetic or pharmaceutical composition or homecare product according to the present invention.

The cosmetic or pharmaceutical, in particular dermatological, composition or homecare product as defined herein, are preferably based on a carrier, which comprises at least one oil phase. However, preparations solely based on water or water/alcohol or water/glycol are likewise possible or even preferred.

The oil phase or oil component in the cosmetic or pharmaceutical composition or homecare product according to the present invention which may be suitable are for example plant oils, hydrocarbon, fatty alcohols, fatty acid esters, liquid UV filters, or mixtures of two or more of the aforesaid liquid oil components.

The oil phase or oil component in the cosmetic or pharmaceutical composition or homecare product according to the present invention is preferably a plant oil and even more preferably a liquid plant oil. It can also advantageously be a mixture of two or more plant oils components, especially liquid plant oil mixtures.

Plant oils or vegetable oils are oils extracted from seeds, or less often, from other parts of fruits. Like animal fats, plant oils are mixtures of triglycerides. Soybean oil, rapeseed oil and cocoa butter are examples of plant oils from seeds. Olive oil, palm oil and rice bran oil are examples of oils from other parts of fruits. In common usage, plant oil or vegetable oil may refer exclusively to vegetable fats which are liquid at room temperature or at 35 to 37 °C skin temperature. Vegetable oils are usually edible.

The term "plant oils" also includes unsaturated plant oils. Unsaturated oils or vegetable oils can be transformed through partial or complete "hydrogenation" into oils of higher melting point. The hydrogenation process involves "sparging" the oil at high temperature and pressure with hydrogen in the presence of a catalyst, typically a powdered nickel compound. As each carbon-carbon double-bond is chemically reduced to a single bond, two hydrogen atoms each form single bonds with the two carbon atoms. The elimination of double bonds by adding hydrogen atoms is called saturation; as the degree of saturation increases, the oil progresses toward being fully hydrogenated. An oil may be hydrogenated to increase resistance to rancidity (oxidation) or to change its physical characteristics. As the degree of saturation increases, the oil's viscosity and melting point increase.

In a preferred variant, the plant oil is selected from the group consisting of Persea Gratissima (Avocado Oil), Abies Alba Seed Oil, Acacia Victoriae Seed Oil, Actinidia Chinensis (Kiwi) Seed Oil, Amaranthus Hypochondriacus Seed Oil, Arachis Hypogaea (Peanut) Oil, Astrocaryum Murumuru Seed Butter, Astrocaryum Tucuma Seed Butter, Astrocaryum Tucuma Seed Oil, Astrocaryum Vulgare Fruit Oil, Astrocaryum Vulgare Kernel Oil, Avena Sativa (Oat) Kernel Oil, Brassica Alba Seed Oil, Brassica Campestris (Rapeseed) Seed Oil, Butyrospermum Parkii (Shea) Butter, Butyrospermum Parkii (Shea) Oil, Calendula Officinalis Seed Oil, Calophyllum Inophyllum Seed Oil, Calophyllum Tacamahaca Seed Oil, Camellia Oleifera Seed Oil, Camellia Reticulata Seed Oil, Camellia Sinensis Seed Oil, Cannabis Sativa Seed Oil, Cannabis Sativa Seed/Stem Oil, Canola Oil, Carthamus Tinctorius (Safflower) Seed Oil, Chlorella Vulgaris Oil, Citrullus Lanatus (Watermelon) Seed Oil, Citrus Aurantifolia (Lime) Seed Oil, Citrus Aurantium Dulcis (Orange) Seed Oil, Citrus Grandis (Grapefruit) Seed Oil, Cocos Nucifera (Coconut) Oil, Cocos Nucifera (Coconut) Seed Butter, Coffea Arabica (Coffee) Seed Oil, Chlorella Oil (biotech), Corylus Americana (Hazelnut) Seed Oil, Corylus Avellana (Hazelnut) Seed Oil, Cucumis Melo (Melon) Seed Oil, Cucumis Sativus (Cucumber) Seed Oil, Cucurbita Pepo (Pumpkin) Seed Oil, Elaeis Guineensis (Palm) Oil, Elaeis (Palm) Fruit Oil, Glycine Soja (Soybean) Oil, Gossypium Herbaceum (Cotton) Seed Oil, Gossypium Hirsutum (Cotton) Seed Oil, Helianthus Annuus (Sunflower) Seed Oil, Macadamia Integrifolia Seed Oil, Macadamia Ternifolia Seed Oil, Mangifera Indica (Mango) Seed Butter, Mangifera Indica (Mango) Seed Oil, Melissa Officinalis Seed Oil, Microalgae Oil, Moringa Oleifera Seed Oil, Moringa Peregrina Seed Oil, Oenothera Biennis (Evening Primrose) Oil, Olea Europaea (Olive) Fruit Oil, Olus Oil, Orbignya Oleifera Seed Oil, Orbignya Speciosa Kernel Oil, Oryza Sativa (Rice) Bran/Germ Oil, Oryza Sativa (Rice) Bran Oil, Oryza Sativa (Rice) Germ Oil, Oryza Sativa (Rice) Lipids, Oryza Sativa (Rice) Seed Oil, Papaver Somniferum Seed Oil, Passiflora Edulis Seed Oil, Persea Gratissima (Avocado) Butter, Persea Gratissima (Avocado) Oil, Prunus Amygdalus Dulcis (Sweet Almond) Oil, Prunus Armeniaca (Apricot) Kernel Oil, Prunus Persica (Peach) Kernel Oil, Punica Granatum Seed Oil, Pyrus Malus (Apple) Seed Oil, Ricinoleic/Caproic/Caprylic/Capric Triglyceride(s), Ricinus Communis (Castor) Seed Oil, Rosa Canina Fruit Oil, Rosa Moschata Seed Oil, Rubus Idaeus (Raspberry) Seed Oil, Sesamum Indicum (Sesame) Seed Butter, Soybean Glycerides, Theobroma Cacao (Cocoa) Seed Butter, Theobroma Grandiflorum Seed Butter, Triticum Vulgare (Wheat) Bran Lipids, Triticum Vulgare (Wheat) Germ Oil, Vitis Vinifera (Grape) Seed Oil, Zea Mays (Corn) Germ Oil, and Zea Mays (Corn) Oil.

**In** a particular preferred variant, the cosmetic or pharmaceutical composition or homecare product according to the present invention comprises as oil component a plant oil, selected from the group consisting of Caprylic Capric Triglycerides, Helianthus Annuus (Sunflower) Seed Oil, Simmondsia Chinensis ((Jojoba) Seed Oil, Olea Europaea (Olive) Fruit Oil, Argania spinosa kernel oil (Argan oil), Prunus Amygdalus Dulcis (Sweet Almond) Oil, Persea Gratissima (Avocado Oil), Butyrospermum Parkii (Shea) Butter, Cocos Nucifera (Coconut) Oil, Theobroma Cacao (Cocoa) Seed Butter, and mixtures of two or more of the aforesaid plant oils. The plant oil can be used either as a single component or in mixture with one or more further different plant oil(s) as specified above.

Hydrocarbons (mineral oils) are in general organic compounds consisting entirely of hydrogen and carbon. As defined by IUPAC nomenclature or organic chemistry, the classifications for hydrocarbons are:
1. Saturated hydrocarbons are the simplest of the hydrocarbon species. They are composed entirely of single bonds and are saturated with hydrogen. The formula for acyclic saturated hydrocarbons (i.e., alkanes) is C*ₙ*H_{2*n*+2}.The most general form of saturated hydrocarbons is C*ₙ*H_{2*n*+2(1-*r*)}, where r is the number of rings. Those with exactly one ring are the cycloalkanes. Saturated hydrocarbons are the basis of petroleum fuels and are found as either linear or branched species.
2. Unsaturated hydrocarbons have one or more double or triple bonds between carbon atoms. Those with double bond are called alkenes. Those with one double bond have the formula C*ₙ*H_{2*n*} (assuming non-cyclic structures). Those containing triple bonds are called alkynes. Those with one triple bond have the formula C*ₙ*H_{2*n*-2}.
3. Aromatic hydrocarbons, also known as arenes, are hydrocarbons that have at least one aromatic ring.

Hydrocarbons can be inter alia liquids (e.g. hexane and benzene), waxes or low melting solids (e.g. paraffin wax and naphthalene). The term 'aliphatic' refers to non-aromatic hydrocarbons. Saturated aliphatic hydrocarbons are sometimes referred to as "paraffins". Mineral oils and waxes are mixtures of predominantly saturated hydrocarbons consisting of straight-chain, branched and ring structures with carbon chain lengths greater than C14. Mineral oils and waxes are chemical substances prepared from naturally occurring crude petroleum oil. They mainly consist of mineral oil saturated hydrocarbons (MOSH) and mineral oil aromatic hydrocarbons (MOAH). Hydrocarbons have been used for many decades in skin and lip care cosmetic products due to their excellent skin tolerance as well as their high protecting and cleansing performance and broad viscosity options. In contrast to vegetable oils, mineral oils are non-allergenic since they are highly stable and not susceptible to oxidation or rancidity.

In a preferred variant, the hydrocarbon as the oil component of the cosmetic or pharmaceutical composition or homecare product of the present invention is selected from the group consisting of undecane, tridecane, mineral oil, petrolatum, squalane, isohexadecane, C7 - C8 isoparaffin, C8 - C9 isoparaffin, C9 - C11 isoparaffin, C9 - C12 isoparaffin, C9 - C13 isoparaffin, C9 - C14 isoparaffin, C9 - C16 isoparaffin, C10 - C11 isoparaffin, C10 - C12 isoparaffin, C10 - C13 isoparaffin, C11 - C12 isoparaffin, C11 - C13 isoparaffin, C11 - C14 isoparaffin, C12 - C14 isoparaffin, C12 - C15 isoparaffin, C12 - C20 isoparaffin, C13 - C14 isoparaffin, C13 - C16 isoparaffin, C14 - C16 isoparaffin, C15 - C19 isoparaffin, and C18 - C70 isoparaffin. The hydrocarbon can be used either as a single component or in mixture with one or more further different hydrocarbon(s) as specified above.

In a particular preferred variant, the oil component of the cosmetic or pharmaceutical composition or homecare product of the present invention is a hydrocarbon selected from the group consisting of hydrocarbons, petrolatum, squalane, isohexadecane, C13 - C14 isoparaffin, and mixtures of two or more of the aforesaid hydrocarbons.

A fatty alcohol (or long-chain alcohol) is usually a high-molecular-weight, straight-chain primary alcohol, but can also range from as few as 4 to 6 carbons to as many as 22 to 26, derived from natural fats and oils. The precise chain length varies with the source. Some commercially important fatty alcohols are lauryl, stearyl and oleyl alcohols. They are colourless oily liquids (for smaller carbon numbers) or waxy solids, although impure samples may appear yellow. Fatty alcohols usually have an even number of carbon atoms and a single alcohol group (-OH) attached to the terminal carbon. Some are unsaturated and some are branched. Most fatty alcohols in nature are found as waxes which are esters with fatty acids and fatty alcohols. The traditional sources of fatty alcohols have largely been various vegetable oils and these remain a large-scale feedstock. The alcohols are obtained from the triglycerides (fatty acid triesters), which form the bulk of the oil. The process involves the transesterification of the triglycerides to give methyl esters which are then hydrogenated to give the fatty alcohols. Fatty alcohols are also prepared from petrochemical sources. In the Ziegler process, ethylene is oligomerized using triethylaluminium followed by air oxidation. Alternatively, ethylene can be oligomerized to give mixtures of alkenes, which are subjected to hydroformylation, this process affording odd-numbered aldehyde, which is subsequently hydrogenated. Fatty alcohols are mainly used in the production of detergents and surfactants. They are components also of cosmetic solvents. They find use as co-emulsifiers, emollients and thickeners in cosmetics.

In a preferred variant, the fatty alcohol as the oil component of the cosmetic or pharmaceutical composition or homecare product of the present invention is selected from the group consisting of phenyl propanol, dimethyl phenylbutanol, hexyldecanol, octyldodecanol, octyldecanol, tridecylalcohol, isostearyl alcohol, phenylisohexanol, phenylpropanol, trimethylbenzenepropanol, isoamylalcohol, isostearyl alcohol, and isotridecyl alcohol. The fatty alcohol can be used either as a single component or in mixture with one or more further different fatty alcohol(s) as specified above.

A fatty acid ester is a type of ester that results from the combination of a fatty acid with an alcohol. When the alcohol component is glycerol, the fatty acid esters produced can be monoglycerides, diglycerides or triglycerides. Fatty acid esters have a conditioning effect of softening the skin to create a smoothing sensation. They are also added to cosmetics to dissolve high-polarity active ingredients and UV absorbers. Esters of straight-chain fatty acids and lower alcohols are effective for dissolving slightly soluble ingredients for oils with a light touch during application. Isostearic acids and other liquid oils with branched fatty acids and unsaturated fatty acids are commonly used as emollients. Higher fatty acid esters and esters of higher alcohols with relatively high melting points are added to skin creams to adjust the application touch.

In a preferred variant, the fatty acid ester as the oil component of the cosmetic or pharmaceutical composition or homecare product of the present invention is selected from the group consisting of C12 - C15 Alkyl Benzoate, Capric/Lauric/Myristic/Oleic Triglyceride, Caprylic/Capric Triglyceride, Caprylic/Capric/Lauric Triglyceride, Caprylic/Capric/Linoleic Triglyceride, Caprylic/Capric/Myristic/Stearic Triglyceride, Caprylic/Capric/Palmitic/Stearic Triglyceride, Caprylic/Capric/Stearic Triglyceride, Caprylic/Capric/Succinic Triglyceride, Caprylyl Caprylate, Cetearyl Ethylhexanoate, Cetearyl Isononanoate, Cetearyl Nonanoate, Coco-Caprylate, Decyl Cocoate, Decyl Oleate, Dicaprylyl Carbonate, Diethyl Succinate, Diethylhexyl 2,6-Naphthalate, Diethylhexyl Carbonate, Dibutyl Adipate, Diisopropyl Adipate, Dipropylheptyl Carbonate, Ethyl Laurate, Ethylhexyl Isononanoate, Ethylhexyl Palmitate, Ethylhexyl Stearate, Glyceryl Caprylate Caprate, Glyceryl Caprylate, Glyceryl Laurate, Glyceryl Triacetyl Hydroxystearate, Glyceryl Triacetyl Ricinoleate, Hexyl Laurate, Isoamyl acetate, Isoamyl Cocoate, Isopropyl Palmitate, Isosorbide Dicaprylate, Isopropyl Myristate, Myristyl Myristate, Oleic/Linoleic Triglyceride, Oleic/Palmitic Triglyceride, Oleic/Palmitic/Lauric/Myristic/Linoleic Triglyceride, Propanediol Caprylate, Ricinoleic/Caproic/Caprylic/ Capric Triglyceride, Oleostearine, Oleyl Erucate, Palmitic/Stearic Triglyceride, Propanediol Dicaprylate Caprate, Propylheptyl Caprylate, Stearyl Heptanoate/Stearyl Caprylate, Triheptanoin, Trihydroxystearin, Triisononanoin, Triisopalmitin, Triisostearin, Trilaurin, Trilinolein, Trilinolenin, Trimyristin, Triolein, Tripalmitin, Tripalmitolein, Tripelargonin, Triricinolein, and Tristearin.

**In** a particular preferred variant, the oil component of the cosmetic or pharmaceutical composition or homecare product of the present invention is a fatty acid ester selected from the group consisting of C12 - C15 Alkyl Benzoate, Caprylic/Capric Triglyceride, Caprylyl Caprylate, Cetearyl Ethylhexanoate, Cetearyl Isononanoate, Cetearyl Nonanoate, Coco-Caprylate, Decyl Cocoate, Decyl Oleate, Dicaprylyl Carbonate, Diethylhexyl 2,6-Naphthalate, Dibutyl Adipate, Diisopropyl Adipate, Ethyl Laurate, Ethylhexyl Isononanoate, Ethylhexyl Palmitate, Ethylhexyl Stearate, Glyceryl Caprylate, Glyceryl Laurate, Hexyl Laurate, Isoamyl Cocoate, Isopropyl Palmitate, Isopropyl Myristate, Myristyl Myristate, Propanediol Caprylate, Oleyl Erucate, Palmitic/Stearic Triglyceride, Propanediol Dicaprylate Caprate, Trihydroxystearin, Triisostearin, Triisononanoin, Tristearin, and mixtures of two or more of the aforesaid fatty acid esters. The fatty acid ester can be used either as a single component or in mixture with one or more further different fatty acid ester(s) as specified above.

An UV filter is a compound or a mixture of compounds that block or absorb ultraviolet (UV) light. Since excessive UV radiation can cause sunburn, photoaging, and skin cancer, care products such as sunscreen usually include a classification for the specific wavelengths they filter. UV classifications include UVA (320 - 400 nm), UVB (290 - 320 nm) and UVC (200 - 280 nm). UV-absorbing compounds are used not only in sunscreen, but also in other personal care products, such as lipstick, shampoo, hair spray, body wash, toilet soap, and insect repellent. Chemical filters protect against UV radiation by absorbing, reflecting, or scattering. Reflection and scattering are accomplished by inorganic physical UV filters, such as titanium dioxide (TiO₂) and zinc oxide (ZnO). Absorption, mainly of UVB, is done by organic UV filters, which are known as chemical UV filters.

In a preferred variant, the liquid UV filter as the oil component of the cosmetic or pharmaceutical composition or homecare product of the present invention is selected from the group consisting of Octocrylene, Ethylhexyl Salicylate, Homosalate, Ethylhexyl Methoxycinnamate, Isoamyl p-Methoxycinnamate, Camphor Benzalkonium Methosulfate, Polyarylamidomethyl Benzylidene Camphor, Isooctyl Methoxycionnamate, Ethylhexyl Triazone, Drometrizole Trisiloxane, Diethylhexyl Butamido Triazone, 3-Benzylidene Camphor, Octyl Salicylate, Ethylhexyl Dimethyl PABA, Bis-Ethylhexyloxyphenol Methoxyphenyl Triazine, Polysilicone-15, Diethylamino Hydroxybenzoyl Hexyl benzoate, N,N,N-Trimethyl-4-(2-oxoborn-3-ylidenemethyl) anilinium methyl sulfate, Benzoic acid-2-hydroxy-3,3,5-trimethylcyclohexyl ester/Homosalate, 2-Cyano-3,3-diphenyl acrylic acid, 2- Ethylhexyl ester/Octocrilene, 2-Ethylhexyl-4-methoxycinnamate/Octinoxate, Ethoxylated Ethyl-4-aminobenzoate, 2-Ethylhexyl salicylate/Octisalate, 2-Ethylhexyl-4-(dimethylamino)benzoate/Padimate O (USAN:BAN), 2,2'-Methylene-bis(6-(2H-benzotriazol-2-yl)-4-(1,1,3,3-tetramethyl-butyl)phenol)/Bisoctrizole, and Dimethicodiethylbenzalmalonate. The UV filter can be used either as a single component or in mixture with one or more further different **UV** filter (s) as specified above.

**In** a particular preferred variant the oil component of the cosmetic or pharmaceutical composition or homecare product of the present invention is a liquid **UV** filter selected from the group consisting of Octocrylene, Ethylhexyl Salicylate, Homosalate, Ethylhexyl Methoxycinnamate, Isoamyl p-Methoxycinnamate, **N,N,N-**Trimethyl-4-(2-oxoborn-3-ylidenemethyl) anilinium methyl sulfate, Benzoic acid-2-hydroxy-3,3,5-trimethylcyclohexyl ester/Homosalate, 2-Cyano-3,3-diphenyl acrylic acid, 2- Ethylhexyl ester/Octocrilene, 2-Ethylhexyl-4-methoxycinnamate/Octinoxate, Ethoxylated Ethyl-4-aminobenzoate, 2-Ethylhexyl salicylate/Octisalate, 2-Ethylhexyl-4-(dimethylamino)benzoate/Padimate O (USAN:BAN), 2,2'-Methylene-bis(6-(2H-benzotriazol-2-yl)-4-(1,1,3,3-tetramethyl-butyl)phenol)/Bisoctrizole, Dimethicodiethylbenzalmalonate, and mixtures of two or more of the aforesaid UV filters.

In addition to the oil phase or oil component as defined before, the cosmetic or pharmaceutical composition or homecare product according to the present invention preferably includes one or more further oil bodies. Suitable oil bodies, which form constituents of the O/W emulsions, are, for example, Guerbet alcohols based on fatty alcohols having 6 to 18, preferably 8 to 10, carbon atoms, esters of linear C5 - C22 fatty acids with linear or branched C5 - C22 fatty alcohols or esters of branched C1 - C13 carboxylic acids with linear or branched C6 - C22 fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C6 - C22 fatty acids with branched alcohols, in particular 2-ethylhexanol, esters of C18 - C38 alkylhydroxy carboxylic acids with linear or branched C6 - C22 fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C6 - C10 fatty acids, liquid mono-/di-/triglyceride mixtures based on C6 - C18 fatty acids, esters of C6 - C22 fatty alcohols and/or Guerbet alcohols with aromatic carboxylic acids, in particular benzoic acid, esters of C2 - C12 dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched CC - C22 fatty alcohol carbonates, such as, for example, Dicaprylyl Carbonate (Cetiol^{®} CC), Guerbet carbonates, based on fatty alcohols having 6 to 18, preferably 8 to 10, carbon atoms, esters of benzoic acid with linear and/or branched C₆-C₂₂-alcohols (e.g. Finsolv^{®} TN), linear or branched, symmetrical or asymmetrical dialkyl ethers having 6 to 22 carbon atoms per alkyl group, such as, for example, dicaprylyl ether (Cetiol^{®} OE), ring-opening products of epoxidized fatty acid esters with polyols, silicone oils (cyclomethicones, silicone methicone grades, etc.) and/or aliphatic or naphthenic hydrocarbons, such as, for example, squalane, squalene or dialkylcyclohexanes.

Within the context of the present invention, it is also possible and in some cases advantageous to combine the cosmetic or pharmaceutical, in particular dermatological, composition or homecare product according to the present invention with other active substances, adjuvants or additives which are different from the lipophilic active component (b) of the cosmetic or pharmaceutical composition according to the present invention.

The active agents and/or adjuvants and/or additives which are usually used in cosmetic or pharmaceuticyl compositions or homecare products are different from the lipophilic active component (b); they provide in some instances one or more than one benefit or operate via more than one mode of action.

Thus, optionally, other conventionally active substances, adjuvants or additives for cosmetic or pharmaceutical compositions or homecare products, as further described below, may be added as component (c), i.e. in order to obtain a ready-for-use cosmetic or pharmaceutical composition or formulation or homecare product.

The cosmetic or pharmaceutical composition according to the present invention can advantageously be combined with other cosmetically or pharmaceutically active agents and/or adjuvants and/or additives or auxiliaries, such as are customarily used in such compositions, such as for example abrasives, antiacne agents, agents against ageing of the skin, anti-cellulitis agents, anti-dandruff agents, anti-inflammatory agents, anti-microbial agents, irritation-preventing agents, irritation-inhibiting agents, antioxidants, astringents, odor absorbers, perspirationinhibiting agents, antiseptic agents, anti-statics, binders, buffers, carrier materials, chelating agents, cell stimulants, cleansing agents, depilatory agents, surface-active substances, deodorizing agents, antiperspirants, softeners, emulsifiers, enzymes, enzyme inhibitors, essential oils, fibers, film-forming agents, fixatives, foam-forming agents, foam stabilizers, substances for preventing foaming, foam boosters, gelling agents, gel-forming agents, hair care agents, hair-setting agents, hair-straightening agents, moisture-donating agents, moisturizing substances, moisture-retaining substances, bleaching agents, strengthening agents, stain-removing agents, optically brightening agents, impregnating agents, dirt-repellent agents, dyes, friction-reducing agents, lubricants, moisturizing creams, ointments, opacifying agents, plasticizing agents, covering agents, polish, preservatives, gloss agents, green and synthetic polymers, powders, proteins, re-oiling agents, abrading agents, silicones, skinsoothing agents, skin-cleansing agents, skin care agents, skin-healing agents, skinlightening agents, skin-protecting agents, skin-softening agents, hair promotion agents, cooling agents, skin-cooling agents, warming agents, skin-warming agents, stabilizers, surfactants, UV-absorbing agents, UV filters, primary sun protection factors, secondary sun protection factors, detergents, fabric conditioning agents, suspending agents, skin-tanning agents, actives modulating skin or hair pigmentation, matrix-metalloproteinase inhibitors, skin moisturizing agents, glycosaminoglycan stimulators, TRPV1 antagonists, desquamating agents, anti-cellulite agents or fat enhancing agents, hair growth activators or inhibitors, thickeners, rheology additives, vitamins, oils, waxes, pearlizing waxes, fats, phospholipids, saturated fatty acids, mono- or polyunsaturated fatty acids, α-hydroxy acids, polyhydroxyfatty acids, liquefiers, dyestuffs, colour-protecting agents, pigments, anti-corrosives, fragrances or perfume oils, aromas, flavouring substances, odoriferous substances, polyols, electrolytes, organic solvents, and mixtures of two or more of the aforementioned substances, as further described below.

Of the above cosmetically or pharmaceutically active agents and/or adjuvants and/or additives or auxiliaries, agents against ageing of the skin, anti-microbial agents, antioxidants, chelating agents, emulsifiers, surfactants, preservatives, green and synthetic polymers, skin-cooling agents, rheology additives, oils, fragrances or perfume oils, and polyols are particularly preferred in the preparation of cosmetic and pharmaceutical composition.

Since dermatological conditions or diseases are often associated with dry skin, scratched skin, skin lesions or even inflammation, the cosmetic or pharmaceutical composition according to the present invention advantageously contains preferably anti-inflammatories, antibacterial or antimycotic substances, substances having a reddening-alleviating or itch-alleviating action, lenitive substances, moisturisers and/or cooling agents, osmolytes, keratolytic substances, nurturing substances, anti-inflammatory, antibacterial or antimycotic substances, substances having a reddening-alleviating or itch-alleviating action, lenitive substances, anti-dandruff substances, or other active compounds such as solvents, fragrances antioxidants, preservatives, (metal) chelating agents, penetration enhancers, or mixtures of two or more of afore specified agents, as further described below.

**Anti-ageing actives:** The cosmetic or pharmaceutical composition according to the present invention preferably contains one or more anti-ageing actives. In the context of the invention, anti-ageing or biogenic agents are, for example antioxidants, matrix-metalloproteinase inhibitors (MMPI), skin moisturizing agents, glycosaminglycan stimulators, anti-inflammatory agents, TRPV1 antagonists and plant extracts.

**Antioxidants:** A preferred cosmetic or pharmaceutical composition according to the present invention comprises one or more antioxidants. Suitable antioxidants encompass amino acids (preferably glycine, histidine, tyrosine, tryptophan) and derivatives thereof, imidazoles (preferably urocanic acid) and derivatives thereof, peptides, preferably D,L-carnosine, D-carnosine, L-carnosine and derivatives thereof (preferably anserine), carnitine, creatine, matrikine peptides (preferably lysyl-threonyl-threonyl-lysyl-serine) and palmitoylated pentapeptides, carotenoids, carotenes (preferably alpha-carotene, beta-carotene, lycopene) and derivatives thereof, lipoic acid and derivatives thereof (preferably dihydrolipoic acid), aurothioglucose, propyl thiouracil and other thiols (preferably thioredoxine, glutathione, cysteine, cystine, cystamine and glycosyl, N-acetyl, methyl, ethyl, propyl, amyl, butyl and lauryl, palmitoyl, oleyl, gamma-linoleyl, cholesteryl, glyceryl and oligoglyceryl esters thereof) and salts thereof, dilauryl thiodipropionate, distearyl thiodipropionate, thiodipropionic acid and derivatives thereof (preferably esters, ethers, peptides, lipids, nucleotides, nucleosides and salts) and sulfoximine compounds (preferably buthionine sulfoximines, homocysteine sulfoximine, buthionine sulfones, penta-, hexa-, heptathionine sulfoximine) in very small tolerated doses (e.g. pmol to µmol/kg), also (metal) chelators (preferably alpha-hydroxy fatty acids, palmitic acid, phytic acid, lactoferrin, alpha-hydroxy acids (preferably citric acid, lactic acid, malic acid), humic acid, bile acid, bile extracts, tannins, bilirubin, biliverdin, EDTA, EGTA and derivatives thereof), unsaturated fatty acids and derivatives thereof (preferably gamma-linolenic acid, linoleic acid, oleic acid), folic acid and derivatives thereof, ubiquinone and derivatives thereof, ubiquinol and derivatives thereof, vitamin C and derivatives (preferably ascorbyl palmitate, Mg ascorbyl phosphate, ascorbyl acetate, ascorbyl glucoside), tocopherols and derivatives (preferably vitamin E acetate), vitamin A and derivatives (vitamin A palmitate) and coniferyl benzoate of benzoic resin, rutinic acid and derivatives thereof, flavonoids and glycosylated precursors thereof, in particular quercetin and derivatives thereof, preferably alpha-glucosyl rutin, rosmarinic acid, carnosol, carnosolic acid, resveratrol, caffeic acid and derivatives thereof, sinapic acid and derivatives thereof, ferulic acid and derivatives thereof, curcuminoids, chlorogenic acid and derivatives thereof, retinoids, preferably retinyl palmitate, retinol or tretinoin, ursolic acid, levulinic acid, butyl hydroxytoluene, butyl hydroxyanisole, nordihydroguaiac acid, nordihydroguaiaretic acid, trihydroxybutyrophenone, uric acid and derivatives thereof, mannose and derivatives thereof, zinc and derivatives thereof (preferably ZnO, ZnSO₄), selenium and derivatives thereof (preferably selenium methionine), superoxide dismutase, stilbenes and derivatives thereof (preferably stilbene oxide, trans-stilbene oxide) and the derivatives (salts, esters, ethers, sugars, nucleotides, nucleosides, peptides and lipids) of these cited active ingredients which are suitable according to the invention or extracts or fractions of plants having an antioxidant effect, preferably green tea, rooibos, honeybush, grape, rosemary, sage, melissa, thyme, lavender, olive, oats, cocoa, ginkgo, ginseng, liquorice, honeysuckle, sophora, pueraria, pinus, citrus, Phyllanthus emblica or St. John's wort, grape seeds, wheat germ, Phyllanthus emblica, coenzymes, preferably coenzyme Q10, plastoquinone and menaquinone. Preferred antioxidants are selected from the group consisting of vitamin A and derivatives, vitamin C and derivatives, tocopherol and derivatives, preferably tocopheryl acetate, and ubiquinone. If vitamin E and/or derivatives thereof are used as the antioxidant(s), it is advantageous to choose their concentrations from the range from about 0.001 to about 10 % by weight, based on the total weight of the composition. If vitamin A or vitamin A derivatives or carotenes or derivatives thereof are used as the antioxidant(s), it is advantageous to choose their concentrations from the range from about 0.001 to about 10 % by weight based on the total weight of the composition.

**Matrix-Metalloproteinase inhibitors (MMPI):** A preferred cosmetic or pharmaceutical composition according to the present invention comprises one or more matrix-metalloproteinase inhibitors, especially those inhibiting matrix-metalloproteinases enzymatically cleaving collagen, selected from the group consisting of ursolic acid, retinyl palmitate, propyl gallate, precocenes, 6-hydroxy-7-methoxy-2,2-dimethyl-1(2H)-benzopyran, 3,4-dihydro-6-hydroxy-7-methoxy-2,2-dimethyl-1(2H)-benzopyran, benzamidine hydrochloride, the cysteine proteinase inhibitors N-ethylmalemide and epsilon-amino-n-caproic acid of the serinprotease inhibitors: phenylmethylsufonylfluoride, collhibin (company Pentapharm; INCI: hydrolysed rice protein), oenotherol (company Soliance; INCI: propylene glycol, aqua, Oenothera biennis root extract, ellagic acid and ellagitannins, for example from pomegranate), phosphoramidone hinokitiol, EDTA, galardin, EquiStat (company Collaborative Group; apple fruit extract, soya seed extract, ursolic acid, soya isoflavones and soya proteins), sage extracts, MDI (company Atrium; INCI: glycosaminoglycans), fermiskin (company Silab/Mawi; INCI: water and lentinus edodes extract), actimp 1.9.3 (company Expanscience/Rahn; INCI: hydrolysed lupine protein), lipobelle soyaglycone (company Mibelle; INCI: alcohol, polysorbate 80, lecithin and soy isoflavones), extracts from green and black tea and further plant extracts, proteins or glycoproteins from soya, hydrolysed proteins from rice, pea or lupine, plant extracts which inhibit MMPs, preferably extracts from shitake mushrooms, extracts from the leaves of the Rosaceae family, sub-family Rosoideae, quite particularly extracts of blackberry leaf, as e.g. SymMatrix (company Symrise, INCI: Maltodextrin, Rubus Fruticosus (Blackberry) Leaf Extract). Preferred actives of are selected from the group consisting of retinyl palmitate, ursolic acid, extracts from the leaves of the Rosaceae family, sub-family Rosoideae, genistein and daidzein.

**Skin-moisturizing agents:** A preferred cosmetic or pharmaceutical composition according to the present invention comprises one or more skin-moisturizing agents. Preferred skin moisturizing agents are selected from the group consisting of alkane diols or alkane triols comprising 3 to 12 carbon atoms, preferably C₃-C₁₀-alkane diols and C₃-C₁₀-alkane triols. More preferably the skin moisturizing agents are selected from the group consisting of glycerol, 1,2-propylene glycol, 1,2-butylene glycol, 1,3-butylene glycol, 1,2-pentanediol, 1,2-hexanediol, 1,2-octanediol and 1,2-decanediol.

**Glycosaminoglycan stimulators:** A preferred cosmetic or pharmaceutical composition according to the present invention comprises one or more substances stimulating the synthesis of glycosaminoglycans which are selected from the group consisting of hyaluronic acid and derivatives or salts, Subliskin (Sederma, INCI: Sinorhizobium Meliloti Ferment Filtrate, Cetyl Hydroxyethylcellulose, Lecithin), Hyalufix (BASF, INCI: Water, Butylene Glycol, Alpinia galanga leaf extract, Xanthan Gum, Caprylic/Capric Triglyceride), Stimulhyal (Soliance, INCI: Calcium ketogluconate), Syn-Glycan (DSM, INCI: Tetradecyl Aminobutyroylvalylaminobutyric Urea Trifluoroacetate, Glycerin, Magnesium chloride), Kalpariane (Biotech Marine), DC Upregulex (Distinctive Cosmetic Ingredients, INCI: Water, Butylene Glycol, Phospholipids, Hydrolyzed Sericin), glucosamine, N-acetyl glucosamine, retinoids, preferably retinol and vitamin A, Arctium lappa fruit extract, Eriobotrya japonica extract, Genkwanin, N-Methyl-L-serine, (-)-alpha-bisabolol or synthetic alpha-bisabolol such as e.g. Dragosantol and Dragosantol 100 from Symrise, oat glucan, Echinacea purpurea extract and soy protein hydrolysate. Preferred actives are selected from the group consisting of hyaluronic acid and derivatives or salts, retinol and derivatives, (-)-alpha-bisabolol or synthetic alpha-bisabolol such as e.g. Dragosantol and Dragosantol 100 from Symrise, oat glucan, Echinacea purpurea extract, Sinorhizobium Meliloti Ferment Filtrate, Calcium ketogluconate, Alpinia galanga leaf extract and tetradecyl aminobutyroylvalylaminobutyric urea trifluoroacetate.

**TRPV1 antagonists:** A preferred cosmetic or pharmaceutical composition according to the present invention comprises one or more TRPV1 antagonsits. Suitable compounds which reduce the hypersensitivity of skin nerves based on their action as TRPV1 antagonists, encompass e.g. trans-4-tert-butyl cyclohexanol, or indirect modulators of TRPV1 by an activation of the µ-receptor, e.g. acetyl tetrapeptide-15, are preferred.

**Plant extracts:** A preferred cosmetic or pharmaceutical composition according to the present invention comprises one or more plant extracts. Plant extracts, special highly active plant extract fractions and also highly pure active substances isolated from plant extracts can also be used in the cosmetic or pharmaceutical composition according to the present invention. Extracts, fractions and active substances from camomile, *aloe vera, Commiphora* species, *Rubia* species, willows, willow-herb, ginger, marigold, arnica, Glycyrrhiza species, Echinacea species, Rubus species and pure substances such as *inter alia* bisabolol, apigenin, apigenin-7-glucoside, gingerols such as [6]-gingerol, paradols such as [6]-paradol, boswellic acid, phytosterols, glycyrrhizine, glabridin or licochalcone A are particularly preferred.

**Anti-inflammatory agents:** The cosmetic or pharmaceutical composition according to the present invention preferably also contains anti-inflammatory and/or redness and/or itch ameliorating ingredients, in particular steroidal substances of the corticosteroid type selected from the group consisting of hydrocortisone, dexamethasone, dexamethasone phosphate, methyl prednisolone or cortisone, are advantageously used as anti-inflammatory active ingredients or active ingredients to relieve reddening and itching, the list of which can be extended by the addition of other steroidal anti-inflammatories. Non-steroidal anti-inflammatories can also be used. More particularly:
(i) steroidal anti-inflammatory substances of the corticosteroid type, in particular hydrocortisone, hydrocortisone derivatives such as hydrocortisone 17-butyrate, dexamethasone, dexamethasone phosphate, methylprednisolone or cortisone,
(ii) non-steroidal anti-inflammatory substances, in particular oxicams such as piroxicam or tenoxicam, salicylates such as aspirin, disalcid, solprin or fendosal, acetic acid derivatives such as diclofenac, fenclofenac, indomethacin, sulindac, tolmetin or clindanac, fenamates such as mefenamic, meclofenamic, flufenamic or niflumic, propionic acid derivatives such as ibuprofen, naproxen or benoxaprofen, pyrazoles such as phenylbutazone, oxyphenylbutazone, febrazone or azapropazone,
(iii) natural or naturally occuring anti-inflammatory substances or substances that alleviate reddening and/or itching, in particular extracts or fractions from camomile, Aloe vera, Commiphora species, Rubia species, willow, willow-herb, oats, calendula, arnica, St John's wort, honeysuckle, rosemary, Passiflora incarnata, witch hazel, ginger or Echinacea, or single active compounds thereof,
(iv) histamine receptor antagonists, serine protease inhibitors (e.g. of Soy extracts), TRPV1 antagonists (e.g. 4-t-Butylcyclohexanol), NK1 antagonists (e.g. Aprepitant, Hydroxyphenyl Propamidobenzoic Acid), cannabinoid receptor agonists (e.g. Palmitoyl Ethanolamine) and TRPV3 antagonists.

Examples which can be cited here are oxicams such as piroxicam or tenoxicam; salicylates such as aspirin, disalcid, solprin or fendosal; acetic acid derivatives such as diclofenac, fenclofenac, indomethacin, sulindac, tolmetin or clindanac; fenamates such as mefenamic, meclofenamic, flufenamic or niflumic; propionic acid derivatives such as ibuprofen, naproxen, benoxaprofen or pyrazoles such as phenylbutazone, oxyphenylbutazone, febrazone or azapropazone. Anthranilic acid derivatives are preferred anti-itch ingredients in a composition according to the present invention.

Also useful are natural or naturally occurring anti-inflammatory mixtures of substances or mixtures of substances that alleviate reddening and/or itching, in particular extracts or fractions from camomile, Aloe vera, Commiphora species, Rubia species, willow, willow-herb, oats, calendula, arnica, St John's wort, honeysuckle, rosemary, Passiflora incarnata, witch hazel, ginger or Echinacea; preferably selected from the group consisting of extracts or fractions from camomile, Aloe vera, oats, calendula, arnica, honeysuckle, rosemary, witch hazel, ginger or Echinacea, and/or pure substances, preferably alpha-bisabolol, apigenin, apigenin-7-glucoside, gingerols, shogaols, gingerdiols, dehydrogingerdiones, paradols, natural or naturally occuring avenanthramides, preferably tranilast, avenanthramide A, avenanthramide B, avenanthramide C, non-natural or non-naturally occuring avenanthramides, preferably dihydroavenanthramide D, dihydroavenanthramide E, avenanthramide **D,** avenanthramide E, avenanthramide F, boswellic acid, phytosterols, glycyrrhizin, glabridin and licochalcone A; preferably selected from the group consisting of alpha-bisabolol, natural avenanthramides, non-natural avenanthramides, preferably dihydroavenanthramide D (as described in WO 2004 047833 A1), boswellic acid, phytosterols, glycyrrhizin, and licochalcone A, and/or allantoin, panthenol, lanolin, (pseudo-)ceramides [preferably Ceramide 2, hydroxypropyl bispalmitamide MEA, cetyloxypropyl glyceryl methoxypropyl myristamide, N-(1-hexadecanoyl)-4-hydroxy-L-proline (1-hexadecyl) ester, hydroxyethyl palmityl oxyhydroxypropyl palmitamide], glycosphingolipids, phytosterols, chitosan, mannose, lactose and β-glucans, in particular 1,3->1,4-β-glucan from oats.

When bisabolol is used in the context of the present invention it can be of natural or synthetic origin and is preferably "alpha-bisabolol". Preferably, the bisabolol used is synthetically prepared or natural (-)-alpha-bisabolol and/or synthetic mixedisomer alpha-bisabolol. If natural (-)-alpha-bisabolol is used, this can also be employed as a constituent of an essential oil or of a plant extract or of a fraction thereof, for example as a constituent of (fractions of) oil or extracts of camomile or of Vanillosmopsis (in particular Vanillosmopsis erythropappa or Vanillosmopsis arborea). Synthetic alpha-bisabolol is obtainable, for example, under the name "Dragosantol" from Symrise.

In case ginger extract is used in the context of the present invention, preferably extracts of the fresh or dried ginger root are used which are prepared by extraction with methanol, ethanol, iso-propanol, acetone, ethyl acetate, carbon dioxide (CO₂), hexane, methylene chloride, chloroform or other solvents or solvent mixtures of comparable polarity. The extracts are characterized by the presence of active skin irritationreducing amounts of constituents such as e.g. gingerols, shogaols, gingerdiols, dehydrogingerdiones and/or paradols.

**Physiological cooling agents:** The cosmetic or pharmaceutical composition according to the present invention can be particularly advantageously combined with one or more physiological cooling agent(s). The use of cooling agents can alleviate itching. Preferred individual cooling agents for use within the framework of the present invention are listed below. The person skilled in the art can add many other cooling agents to this list; the cooling agents listed can also be used in combination with one another: which are preferably selected here from the following list: menthol and menthol derivatives (for example L-menthol, D-menthol, racemic menthol, isomenthol, neoisomenthol, neomenthol) menthylethers (for example (I-menthoxy)-1,2-propanediol, (I-menthoxy)-2-methyl-1,2-propanediol, I-menthyl-methylether), menthone glyceryl acetal, menthone glyceryl ketal or mixtures of both, menthylesters (for example menthylformiate, menthylacetate, menthylisobutyrate, menthyhydroxyisobutyrat, menthyllactates, L-menthyl-L-lactate, L-menthyl-D-lactate, menthyl-(2-methoxy)acetate, menthyl-(2-methoxyethoxy)acetate, menthylpyroglutamate), menthylcarbonates (for example menthylpropyleneglycolcarbonate, menthylethyleneglycolcarbonate, menthylglycerolcarbonate or mixtures thereof), the semi-esters of menthols with a dicarboxylic acid or derivatives thereof (for example mono-menthylsuccinate, monomenthylglutarate, mono-menthylmalonate, O-menthyl succinic acid ester-N,N-(dimethyl)amide, O-menthyl succinic acid ester amide), menthanecarboxylic acid amides (in this case preferably menthanecarboxylic acid-N-ethylamide [WS3] or N^{α}-(menthanecarbonyl)glycinethylester [WS5], menthanecarboxylic acid-N-(4-cyanophenyl)amide or menthanecarboxylic acid-N-(4-cyanomethylphenyl)amide, menthanecarboxylic acid-N-(alkoxyalkyl)amides), menthone and menthone derivatives (for example L-menthone glycerol ketal), 2,3-dimethyl-2-(2-propyl)-butyric acid derivatives (for example 2,3-dimethyl-2-(2-propyl)-butyric acid-N-methylamide [WS23]), isopulegol or its esters (I-(-)-isopulegol, I-(-)-isopulegolacetate), menthane derivatives (for example p-menthane-3,8-diol), cubebol or synthetic or natural mixtures, containing cubebol, pyrrolidone derivatives of cycloalkyldione derivatives (for example 3-methyl-2(1-pyrrolidinyl)-2-cyclopentene-1-one) or tetrahydropyrimidine-2-one (for example iciline or related compounds, as described in WO 2004/026840), further carboxamides (for example N-(2-(pyridin-2-yl)ethyl)-3-p-menthanecarboxamide or related compounds), (1R,2S,5R)-N-(4-Methoxyphenyl)-5-methyl-2-(1-isopropyl)cyclohexane-carboxamide [WS12], oxamates and [(1R,2S,5R)-2-isopropyl-5-methyl-cyclohexyl] 2-(ethylamino)-2-oxo-acetate (X Cool). Cooling agents which are preferred due to their particular synergistic effect are I-menthol, d-menthol, racemic menthol, menthone glycerol acetal (trade name: Frescolat^{®} MGA), menthyl lactate (preferably I-menthyl lactate, in particular I-menthyl I-lactate (trade name: Frescolat^{®} ML)), substituted menthyl-3-carboxamides (such as menthyl-3-carboxylic acid N-ethyl amide), 2-isopropyl-N-2,3-trimethyl butanamide, substituted cyclohexane carboxamides, 3-menthoxypropane-1,2-diol, 2-hydroxyethyl menthyl carbonate, 2-hydroxypropyl menthyl carbonate and isopulegol. Particularly preferred cooling agents are I-menthol, racemic menthol, menthone glycerol acetal (trade name: Frescolat^{®} MGA), menthyl lactate (preferably I-menthyl lactate, in particular I-menthyl I-lactate (trade name: Frescolat^{®} ML)), 3-menthoxypropane-1,2-diol, 2-hydroxyethyl menthyl carbonate and 2-hydroxypropyl menthyl carbonate. Very particularly preferred cooling agents are I-menthol, menthone glycerol acetal (trade name: Frescolat^{®} MGA) and menthyl lactate (preferably I-menthyl lactate, in particular I-menthyl I-lactate (trade name: Frescolat^{®} ML).

**Moisturising and/or moisture-retaining substances:** Itching occurs with particular intensity when the skin is dry. The use of skin-moisturising and/or moisture-retaining substances can significantly alleviate itching. The cosmetic or pharmaceutical composition according to the present invention can therefore advantageously also contain one or more of the following moisturising and/or moisture-retaining substances: sodium lactate, urea, urea derivatives, alcohols, glycerol, diols such as propylene glycol, hexylene glycol, collagen, elastin or hyaluronic acid, diacyl adipates, petrolatum, urocanic acid, lecithin, panthenol, phytantriol, lycopene, (pseudo-)ceramides, glycosphingolipids, cholesterol, phytosterols, chitosan, chondroitin sulphate, lanolin, lanolin esters, amino acids, alpha-hydroxy acids (such as citric acid, lactic acid, malic acid) and their derivatives, mono-, di- and oligosaccharides such as glucose, galactose, fructose, mannose, fructose and lactose, polysugars such as R-glucans, in particular 1,3-1,4-β-glucan from oats, alpha-hydroxy fatty acids, triterpene acids such as betulinic acid or ursolic acid, and algae extracts.

**Lenitive substances:** The cosmetic or pharmaceutical composition according to the present invention can also contain advantageously one or more lenitive substances, wherein any lenitive substances can be used which are suitable or customary in cosmetic or pharmaceutical applications such as alpha-bisabolol, azulene, guaiazulene, 18-beta-glycyrrhetinic acid, allantoin, Aloe vera juice or gel, extracts of Hamamelis virginiana (witch hazel), Echinacea species, Centella asiatica, chamomile, Arnica monatana, Glycyrrhiza species, algae, seaweed and Calendula officinalis, and vegetable oils such as sweet almond oil, baobab oil, olive oil and panthenol, Laureth-9, Trideceth-9 and 4-t-butylcyclohexanol.

**Antibacterial or antimycotic active substances:** Antibacterial or antimycotic active substances can also particularly advantageously be used in the cosmetic or pharmaceutical composition according to the present invention, wherein any antibacterial or antimycotic active substances can be used which are suitable or customary in cosmetic or pharmaceutical, in particular dermatological, applications. In addition to the large group of conventional antibiotics, other products which are advantageous here include those relevant to cosmetics such as in particular triclosan, climbazole, octoxyglycerin, Octopirox^{®} (1-hydroxy-4-methyl-6-(2,4,4-trimethylpentyl)-2(1H)-pyridone 2-aminoethanol salt), chitosan, farnesol, glycerol monolaurate or combinations of said substances, which are used *inter alia* against underarm odour, foot odour or dandruff.

**Anti-microbial agents:** The cosmetic or pharmaceutical composition according to the present invention preferably contains one or more anti-microbioal agents. Suitable anti-microbial agents are, in principle, all substances effective against Gram-positive bacteria, such as, for example, 4- hydroxybenzoic acid and its salts and esters, N-(4-chlorophenyl)-N'-(3,4- dichlorophenyl)urea, 2,4,4'-trichloro-2'-hydroxydiphenyl ether (triclosan), 4-chloro-3,5-dimethyl-phenol, 2,2'-methylenebis(6-bromo-4-chlorophenol), 3-methyl-4-(1-methylethyl)phenol, 2-benzyl-4-chloro-phenol, 3-(4-chlorophenoxy)-1,2-propanediol, 3-iodo-2-propynyl butylcarbamate, chlorhexidine, 3,4,4'-trichlorocarbanilide (TTC), antibacterial fragrances, thymol, thyme oil, eugenol, oil of cloves, menthol, mint oil, farnesol, phenoxyethanol, glycerol monocaprate, glycerol monocaprylate, glycerol monolaurate (GML), diglycerol monocaprate (DMC), salicylic acid N-alkylamides, such as, for example, n-octylsalicylamide or n-decylsalicylamide.

**Desquamating agents:** The cosmetic or pharmaceutical composition according to the present invention preferably contains one or more desquamating agents. The expression "desquamating agent" is understood to mean any compound capable of acting:
- either directly on desquamation by promoting exfoliation, such as β-hydroxy acids, in particular salicylic acid and its derivatives (including 5-n-octanoylsalicylic acid); α-hydroxy acids, such as glycolic, citric, lactic, tartaric, malic or mandelic acids; urea; gentisic acid; oligofucoses; cinnamic acid; extract of Sophora japonica; resveratrol and some derivatives of jasmonic acid;
- or on the enzymes involved in the desquamation or the degradation of the corneodesmosomes, glycosidases, stratum corneum chymotryptic enzyme (SCCE) or other proteases (trypsin, chymotrypsin-like). There may be mentioned agents chelating inorganic salts: EDTA; N-acyl-N,N',N'-ethylenediaminetriacetic acid; aminosulphonic compounds and in particular (N-2-hydroxyethylpiperazine-N-2-ethane)sulphonic acid (HEPES); derivatives of 2-oxothiazolidine-4-carboxylic acid (procysteine); derivatives of alpha-amino acids of the glycine type (as described in EP-0 852 949, and sodium methylglycine diacetate marketed by BASF under the trade name TRILON M); honey; sugar derivatives such as O-octanoyl-6-D-maltose and N-acetylglucosamine; chestnut extracts such as those marketed by the company SILAB under the name Recoverine^{®}, prickly pear extracts such as those marketed under the name Exfolactive^{®} by the company SILAB, or Phytosphingosine SLC^{®} (phytosphingosine grafted with a salicylic acid) marketed by the company Degussa.

Desquamating agents suitable for the invention may be chosen in particular from the group comprising sulphonic acids, calcium chelators, α-hydroxy acids such as glycolic, citric, lactic, tartaric, malic or mandelic acids; ascorbic acid and its derivatives such as ascorbyl glucoside and magnesium ascorbyl phosphate; nicotinamide; urea; (N-2-hydroxyethylpiperazine-N-2-ethane)sulphonic acid (HEPES), β-hydroxy acids such as salicylic acid and its derivatives, retinoids such as retinol and its esters, retinal, retinoic acid and its derivatives, chestnut or prickly pear extracts, in particular marketed by SILAB; reducing compounds such as cysteine or cysteine precursors.

Desquamating agents which can be used are also nicotinic acid and its esters and nicotinamide, also called vitamin B3 or vitamin PP, and ascorbic acid and its precursors.

**Anti-dandruff substances:** In addition, the cosmetic or pharmaceutical composition according to the present invention can also advantageously be used in combination with one or more anti-dandruff substances, including triclosan, climbazole, octoxyglycerin, Octopirox^{®} (1-hydroxy-4-methyl-6-(2,4,4-trimethylpentyl)-2(1H)-pyridone 2-aminoethanol salt), chitosan, farnesol, glycerol monolaurate, Propanediol Monocaprylate or combinations of said substances, which are used inter alia against dandruff.

Further suitable anti-dandruff agents are Pirocton Olamin (1-hydroxy-4-methyl-6-(2,4,4-trimethylpentyl)-2-(1H)-pyridinone monoethanolamine salt), Baypival^{®} (Climbazole), Ketoconazol^{®} (4-acetyl-1-{4-[2-(2,4-dichlorophenyl) r-2-(1H-imidazol-1-ylmethyl)-1,3-dioxylan-c-4-ylmethoxyphenyl}-piperazine, ketoconazole, elubiol, selenium disulfide, colloidal sulfur, sulfur polyethylene glycol sorbitan monooleate, sulfur ricinol polyethoxylate, sulfur tar distillate, salicylic acid (or in combination with hexachlorophene), undecylenic acid, monoethanolamide sulfosuccinate Na salt, Lamepon^{®} UD (protein/undecylenic acid condensate), zinc pyrithione, aluminium pyrithione and magnesium pyrithione/dipyrithione magnesium sulfate.

**(Metal) chelating agents:** A combination with one or more (metal) chelating agents can also be advantageously used in the cosmetic or pharmaceutical composition according to the present invention, wherein any metal chelating agents can be used which are suitable or customary in cosmetic or pharmaceutical applications. Preferred (metal) chelating agents include α-hydroxy fatty acids, phytic acid, lactoferrin, α-hydroxy acids, such as *inter alia* gluconic acid, glyceric acid, glycolic acid, isocitric acid, citric acid, lactic acid, malic acid, mandelic acid, tartaric acid, as well as humic acids, bile acids, bile extracts, bilirubin, biliverdin or EDTA, EGTA and their derivatives. The use of one or more chelating agent(s) improves the stability of the composition according to the present invention.

**Emulsifiers:** In addition, the cosmetic or pharmaceutical composition according to the present invention can also advantageously contain one or more emulsifiers, including for example:
- products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
- C_{12/18} fatty acid monoesters and diesters of addition products of 1 to 30 mol ethylene oxide onto glycerol;
- glycerol mono- and diesters and sorbitan mono- and diesters of saturated and unsaturated fatty acids containing 6 to 22 carbon atoms and ethylene oxide addition products thereof;
- addition products of 15 to 60 mol ethylene oxide onto castor oil and/or hydrogenated castor oil;
- polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
- addition products of 2 to 15 mol ethylene oxide onto castor oil and/or hydrogenated castor oil;
- partial esters based on linear, branched, unsaturated or saturated C_{6/22} fatty acids, ricinoleic acid and 12-hydroxystearic acid and glycerol, polyglycerol, pentaerythritol, dipentaerythritol, sugar alcohols (for example sorbitol), alkyl glucosides (for example methyl glucoside, butyl glucoside, lauryl glucoside) and polyglucosides (for example cellulose);
- mono-, di and trialkyl phosphates and mono-, di- and/or tri-PEG-alkyl phosphates and salts thereof;
- wool wax alcohols;
- polysiloxane/polyalkyl polyether copolymers and corresponding derivatives;
- mixed esters of pentaerythritol, fatty acids, citric acid and fatty alcohol and/or mixed esters of C₆₋₂₂ fatty acids, methyl glucose and polyols, preferably glycerol or polyglycerol,
- polyalkylene glycols and
- glycerol carbonate.

The addition products of ethylene oxide and/or propylene oxide onto fatty alcohols, fatty acids, alkylphenols, glycerol mono- and diesters and sorbitan mono- and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. C_{12/18} fatty acid monoesters and diesters of addition products of ethylene oxide onto glycerol are known as lipid layer enhancers for cosmetic formulations. The preferred emulsifiers are described in more detail as follows:

Partial glycerides: Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride and technical mixtures thereof which may still contain small quantities of triglyceride from the production process. Addition products of 1 to 30 and preferably 5 to 10 mol ethylene oxide onto the partial glycerides mentioned are also suitable.

Sorbitan esters: Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 and preferably 5 to 10 mol ethylene oxide onto the sorbitan esters mentioned are also suitable.

Polyglycerol esters: Typical examples of suitable polyglycerol esters are Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} GI 34), Polyglyceryl-3 Oleate, Diisostearoyl Polyglyceryl-3 Diisostearate (Isolan^{®} PDI), Polyglyceryl-3 Methylglucose Distearate (Tego Care^{®} 450), Polyglyceryl-3 Beeswax (Cera Bellina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor^{®} GS 32), Polyglyceryl Polyricinoleate (Admul^{®} WOL 1403), Polyglyceryl Dimerate Isostearate, and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.

Anionic emulsifiers: Typical anionic emulsifiers are aliphatic C12 to C 22 fatty acids, such as palmitic acid, stearic acid or behenic acid for example, and C12 to C22 dicarboxylic acids, such as azelaic acid or sebacic acid for example.

Amphoteric emulsifiers: Other suitable emulsifiers are amphoteric or zwitterionic surfactants. Zwitterionic surfactants are surface-active compounds which contain at least one quaternary ammonium group and at least one carboxylate and one sulfonate group in the molecule. Particularly suitable zwitterionic surfactants are the so-called betaines, such as the N-alkyl-N,N-dimethyl ammonium glycinates, for example cocoalkyl dimethyl ammonium glycinate, N-acylaminopropyl-N,N-dimethyl ammonium glycinates, for example cocoacylaminopropyl dimethyl ammonium glycinate, and 2-alkyl-3-carboxymethyl-3-hydroxyethyl imidazolines containing 8 to 18 carbon atoms in the alkyl or acyl group and cocoacylaminoethyl hydroxyethyl carboxymethyl glycinate. The fatty acid amide derivative known under the CTFA name of *Cocamidopropyl Betaine* is particularly preferred. Ampholytic surfactants are also suitable emulsifiers. Ampholytic surfactants are surface-active compounds which, in addition to a C_{8/18} alkyl or acyl group, contain at least one free amino group and at least one -COOH- or -SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-coco-alkylaminopropionate, cocoacylaminoethyl aminopropionate and C_{12/18} acyl sarcosine.

**Surfactants:** The cosmetic or pharmaceutical composition or homecare product according to the present invention preferably includes one or more anionic and/or amphoteric or zwitterionic surfactants. Typical examples encompass: Almondamidopropylamine Oxide, Almondamidopropyl Betaine, Aminopropyl Laurylglutamine, Ammonium C12-15 Alkyl Sulfate, Ammonium C12-16 Alkyl Sulfate, Ammonium Capryleth Sulfate, Ammonium Cocomonoglyceride Sulfate, Ammonium Coco-Sulfate, Ammonium Cocoyl Isethionate, Ammonium Cocoyl Sarcosinate, Ammonium C12-15 Pareth Sulfate, Ammonium C9-10 Perfluoroalkylsulfonate, Ammonium Dinonyl Sulfosuccinate, Ammonium Dodecylbenzenesulfonate, Ammonium Isostearate, Ammonium Laureth-6 Carboxylate, Ammonium Laureth-8 Carboxylate, Ammonium Laureth Sulfate, Ammonium Laureth-5 Sulfate, Ammonium Laureth-7 Sulfate, Ammonium Laureth-9 Sulfate, Ammonium Laureth-12 Sulfate, Ammonium Lauroyl Sarcosinate, Ammonium Lauryl Sulfate, Ammonium Lauryl Sulfosuccinate, Ammonium Myreth Sulfate, Ammonium Myristyl Sulfate, Ammonium Nonoxynol-4 Sulfate, Ammonium Nonoxynol-30 Sulfate, Ammonium Oleate, Ammonium Palm Kernel Sulfate, Ammonium Stearate, Ammonium Tallate, AMPD-Isostearoyl Hydrolyzed Collagen, AMPD-Rosin Hydrolyzed Collagen, AMP-Isostearoyl Hydrolyzed Collagen, AMP-Isostearoyl Hydrolyzed Keratin, AMP-Isostearoyl Hydrolyzed Soy Protein, AMP-Isostearoyl Hydrolyzed Wheat Protein, Apricotamidopropyl Betaine, Arachidic Acid, Arginine Hexyldecyl Phosphate, Avocadamidopropyl Betaine, Avocado Oil Glycereth-8 Esters, Babassu Acid, Babassuamidopropylamine Oxide, Babassuamidopropyl Betaine, Beeswax Acid, Behenamidopropyl Betaine, Behenamine Oxide, Beheneth-25, Beheneth-30, Behenic Acid, Behenyl Betaine, Bis- Butyldimethicone Polyglyceryl-3, Butoxynol-5 Carboxylic Acid, Butoxynol-19 Carboxylic Acid, Butyldimoniumhydroxypropyl Butylglucosides Chloride, Butyldimoniumhydroxypropyl Laurylglucosides Chloride, Butyl Glucoside, Butylglucoside Caprate, Butylglucosides Hydroxypropyltrimonium Chloride, Butyloctanoic Acid, C18-36 Acid, C20-40 Acid, C30-50 Acid, C16-22 Acid Amide MEA, Calcium Dodecylbenzenesulfonate, Calcium Lauroyl Taurate, C9-16 Alkane/Cycloalkane, C10-14 Alkyl Benzenesulfonic Acid, C12-14 Alkyl Diaminoethylglycine HCL, C9-15 Alkyl Phosphate, Candida Bombicola/Glucose/Methyl Rapeseedate Ferment, Canolamidopropyl Betaine, Capric Acid, Caproic Acid, Caproyl Ethyl Glucoside, Capryl/Capramidopropyl Betaine, Capryleth-4 Carboxylic Acid, Capryleth-6 Carboxylic Acid, Capryleth-9 Carboxylic Acid, Caprylic Acid, Capryloyl Collagen Amino Acids, Capryloyl Glycine, Capryloyl Hydrolyzed Collagen, Capryloyl Hydrolyzed Keratin, Capryloyl Keratin Amino Acids, Capryloyl Silk Amino Acids, Caprylyl/Capryl Glucoside, Caprylyl/Capryl Wheat Bran/Straw Glycosides, Caprylyl Glucoside, Caprylyl Glyceryl Ether, Caprylyl Pyrrolidone, Carnitine, Ceteareth-20, Ceteareth-23, Ceteareth-24, Ceteareth-25, Ceteareth-27, Ceteareth-28, Ceteareth-29, Ceteareth-30, Ceteareth-33, Ceteareth-34, Ceteareth-40, Ceteareth-50, Ceteareth-55, Ceteareth-60, Ceteareth-80, Ceteareth-100, Ceteareth-25 Carboxylic Acid, Ceteareth-2 Phosphate, Ceteareth-4 Phosphate, Ceteareth-5 Phosphate, Ceteareth-10 Phosphate, Ceteth-20, Ceteth-23, Ceteth-24, Ceteth-25, Ceteth-30, Ceteth-40, Ceteth-45, Ceteth-150, Ceteth-8 Phosphate, Ceteth-10 Phosphate, Ceteth-20 Phosphate, Cetoleth-22, Cetoleth-24, Cetoleth-25, Cetoleth-30, Cetyl Betaine, Chrysanthemum Sinense Flower Extract, C12-14 Hydroxyalkyl Hydroxyethyl Beta-Alanine, C12-14 Hydroxyalkyl Hydroxyethyl Sarcosine, Cocamidoethyl Betaine, Cocamidopropylamine Oxide, Cocamidopropyl Betainamide MEA Chloride, Cocamidopropyl Betaine, Cocamidopropyl Hydroxysultaine, Cocamine Oxide, Cocaminobutyric Acid, Cocaminopropionic Acid, Coceth-7 Carboxylic Acid, Coceth-4 Glucoside, Cocoamphodipropionic Acid, Cocobetainamido Amphopropionate, Coco-Betaine, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Coco-Glucoside, Cocoglucosides Hydroxypropyltrimonium Chloride, Coco- Hydroxysultaine, Coco-Morpholine Oxide, Coconut Acid, Coconut Oil Glycereth-8 Esters, Coco/Oleamidopropyl Betaine, Coco-Sultaine, Coco/Sunfloweramidopropyl Betaine, Cocoylcholine Methosulfate, Cocoyl Glutamic Acid, Cocoyl Hydrolyzed Collagen, Cocoyl Hydrolyzed Keratin, Cocoyl Hydrolyzed Oat Protein, Cocoyl Hydrolyzed Rice Protein, Cocoyl Hydrolyzed Silk, Cocoyl Hydrolyzed Soy Protein, Cocoyl Hydrolyzed Wheat Protein, Cocoyl Sarcosine, Corn Acid, Cottonseed Acid, Cottonseed Oil Glycereth-8 Esters, C10-16 Pareth-1, C10-16 Pareth-2, C11-13 Pareth-6, C11-13 Pareth-9, C11-13 Pareth-10, C11-15 Pareth-30, C11-15 Pareth-40, C12-13 Pareth-1, C12-13 Pareth- 23, C12-14 Pareth-5, C12-14 Pareth-9, C13-15 Pareth-21, C14-15 Pareth-8, C20-22 Pareth-30, C20- 40 Pareth-40, C20-40 Pareth-95, C22-24 Pareth-33, C30-50 Pareth-40, C9-11 Pareth-6 Carboxylic Acid, C9-11 Pareth-8 Carboxylic Acid, C11-15 Pareth-7 Carboxylic Acid, C12-13 Pareth-5 Carboxylic Acid, C12-13 Pareth-7 Carboxylic Acid, C12-13 Pareth-8 Carboxylic Acid, C12-13 Pareth-12 Carboxylic Acid, C12-15 Pareth-7 Carboxylic Acid, C12-15 Pareth-8 Carboxylic Acid, C12-15 Pareth- 12 Carboxylic Acid, C14-15 Pareth-8 Carboxylic Acid, C6-10 Pareth-4 Phosphate, C12-13 Pareth-2 Phosphate, C12-13 Pareth-10 Phosphate, C12-15 Pareth-6 Phosphate, C12-15 Pareth-8 Phosphate, C12-15 Pareth-10 Phosphate, C12-16 Pareth-6 Phosphate, C4-18 Perfluoroalkylethyl Thiohydroxypropyltrimonium Chloride, Cupuassuamidopropyl Betaine, DEA-C12-13 Alkyl Sulfate, DEA-C12-15 Alkyl Sulfate, DEA-Ceteareth-2 Phosphate, DEA-Cetyl Sulfate, DEA- Cocoamphodipropionate, DEA-C12-13 Pareth-3 Sulfate, DEA-Cyclocarboxypropyloleate, DEA- Dodecylbenzenesulfonate, DEA-Isostearate, DEA-Laureth Sulfate, DEA-Lauryl Sulfate, DEA- Linoleate, DEA-Methyl Myristate Sulfonate, DEA-Myreth Sulfate, DEA-Myristate, DEA-Myristyl Sulfate, DEA-Oleth-5 Phosphate, DEA-Oleth-20 Phosphate, DEA PG-Oleate, Deceth-7 Carboxylic Acid, Deceth-7 Glucoside, Deceth-9 Phosphate, Decylamine Oxide, Decyl Betaine, Decyl Glucoside, Decyltetradeceth-30, Decyltetradecylamine Oxide, Diammonium Lauramido-MEA Sulfosuccinate, Diammonium Lauryl Sulfosuccinate, Diammonium Oleamido PEG-2 Sulfosuccinate, Dibutoxymethane, Di-Cl 2-15 Pareth-2 Phosphate, Di-Cl 2-15 Pareth-4 Phosphate, Di-Cl 2-15 Pareth-6 Phosphate, Di- C12-15 Pareth-8 Phosphate, Di-Cl 2-15 Pareth-10 Phosphate, Didodecyl Butanetetracarboxylate, Diethylamine Laureth Sulfate, Diethylhexyl Sodium Sulfosuccinate, Dihydroxyethyl C8-10 Alkoxypropylamine Oxide, Dihydroxyethyl C9-11 Alkoxypropylamine Oxide, Dihydroxyethyl C12-15 Alkoxypropylamine Oxide, Dihydroxyethyl Cocamine Oxide, Dihydroxyethyl Lauramine Oxide, Dihydroxyethyl Stearamine Oxide, Dihydroxyethyl Tallowamine Oxide, Dimethicone PEG-7 Phosphate, Dimethicone PEG-10 Phosphate, Dimethicone PEG/PPG-7/4 Phosphate, Dimethicone PEG/PPG-12/4 Phosphate, Dimethicone/Polyglycerin-3 Crosspolymer, Dimethicone Propyl PG- Betaine, Dimyristyl Phosphate, Dioleoylamidoethyl Hydroxyethylmonium Methosulfate, DIPA-Hydrogenated Cocoate, DIPA-Lanolate, DIPA-Myristate, Dipotassium Capryloyl Glutamate, Dipotassium Lauryl Sulfosuccinate, Dipotassium Undecylenoyl Glutamate, Disodium Babassuamido MEA-Sulfosuccinate, Disodium Caproamphodiacetate, Disodium Caproamphodipropionate, Disodium Capryloamphodiacetate, Disodium Capryloamphodipropionate, Disodium Capryloyl Glutamate, Disodium Cetearyl Sulfosuccinate, Disodium Cetyl Phenyl Ether Disulfonate, Disodium Cetyl Sulfosuccinate, Disodium Cocamido MEA-Sulfosuccinate, Disodium Cocamido MIPA PEG-4 Sulfosuccinate, Disodium Cocamido MIPA-Sulfosuccinate, Disodium Cocamido PEG-3 Sulfosuccinate, Disodium Coceth-3 Sulfosuccinate, Disodium Cocoamphocarboxyethylhydroxypropylsulfonate, Disodium Cocoamphodiacetate, Disodium Cocoamphodipropionate, Disodium Coco-Glucoside Sulfosuccinate, Disodium Coco-Sulfosuccinate, Disodium Cocoyl Butyl Gluceth-10 Sulfosuccinate, Disodium Cocoyl Glutamate, Disodium C12-14 Pareth-1 Sulfosuccinate, Disodium C12-14 Pareth-2 Sulfosuccinate, Disodium C12-15 Pareth Sulfosuccinate, Disodium C12-14 Sec-Pareth-3 Sulfosuccinate, Disodium C12-14 Sec-Pareth-5 Sulfosuccinate, Disodium C12-14 Sec-Pareth-7 Sulfosuccinate, Disodium C12-14 Sec-Pareth-9 Sulfosuccinate, Disodium C12-14 Sec-Pareth-12 Sulfosuccinate, Disodium Deceth-5 Sulfosuccinate, Disodium Deceth-6 Sulfosuccinate, Disodium Decyl Phenyl Ether Disulfonate, Disodium Dihydroxyethyl Sulfosuccinylundecylenate, Disodium Ethylene Dicocamide PEG-15 Disulfate, Disodium Hydrogenated Cottonseed Glyceride Sulfosuccinate, Disodium Hydrogenated Tallow Glutamate, Disodium Hydroxydecyl Sorbitol Citrate, Disodium Isodecyl Sulfosuccinate, Disodium Isostearamido MEA-Sulfosuccinate, Disodium Isostearamido MIPA-Sulfosuccinate, Disodium Isostearoamphodiacetate, Disodium Isostearoamphodipropionate, Disodium Isostearyl Sulfosuccinate, Disodium Laneth-5 Sulfosuccinate, Disodium Lauramido MEA-Sulfosuccinate, Disodium Lauramido MIPA Glycol Sulfosuccinate, Disodium Lauramido PEG-2 Sulfosuccinate, Disodium Lauramido PEG-5 Sulfosuccinate, Disodium Laureth-5 Carboxyamphodiacetate, Disodium Laureth-7 Citrate, Disodium Laureth Sulfosuccinate, Disodium Laureth-6 Sulfosuccinate, Disodium Laureth-9 Sulfosuccinate, Disodium Laureth-12 Sulfosuccinate, Disodium Lauriminobishydroxypropylsulfonate, Disodium Lauriminodiacetate, Disodium Lauriminodipropionate, Disodium Lauriminodipropionate Tocopheryl Phosphates, Disodium Lauroamphodiacetate, Disodium Lauroamphodipropionate, Disodium N-Lauroyl Aspartate, Disodium Lauroyl Glutamate, Disodium Lauryl Phenyl Ether Disulfonate, Disodium Lauryl Sulfosuccinate, Disodium Myristamido MEA-Sulfosuccinate, Disodium Nonoxynol-10 Sulfosuccinate, Disodium Oleamido MEA-Sulfosuccinate, Disodium Oleamido MIPA-Sulfosuccinate, Disodium Oleamido PEG-2 Sulfosuccinate, Disodium Oleoamphodipropionate, Disodium Oleth-3 Sulfosuccinate, Disodium Oleyl Phosphate, Disodium Oleyl Sulfosuccinate, Disodium Palmitamido PEG-2 Sulfosuccinate, Disodium Palmitoleamido PEG-2 Sulfosuccinate, Disodium PEG-4 Cocamido MIPA-Sulfosuccinate, Disodium PEG-12 Dimethicone Sulfosuccinate, Disodium PEG-8 Palm Glycerides Sulfosuccinate, Disodium PPG-2-Isodeceth-7 Carboxyamphodiacetate, Disodium Ricinoleamido MEA-Sulfosuccinate, Disodium Sitostereth-14 Sulfosuccinate, Disodium Soyamphodiacetate, Disodium Stearamido MEA-Sulfosuccinate, Disodium Steariminodipropionate, Disodium Stearoamphodiacetate, Disodium Stearoyl Glutamate, Disodium Stearyl Sulfosuccinamate, Disodium Stearyl Sulfosuccinate, Disodium 2-Sulfolaurate, Disodium 2-Sulfopalmitate, Disodium Tallamido MEA-Sulfosuccinate, Disodium Tallowamido MEA-Sulfosuccinate, Disodium Tallowamphodiacetate, Disodium Tallowiminodipropionate, Disodium Tallow Sulfosuccinamate, Disodium Tridecylsulfosuccinate, Disodium Undecylenamido MEA-Sulfosuccinate, Disodium Undecylenamido PEG-2 Sulfosuccinate, Disodium Undecylenoyl Glutamate, Disodium Wheat Germamido MEA-Sulfosuccinate, Disodium Wheat Germamido PEG-2 Sulfosuccinate, Disodium Wheatgermamphodiacetate, Di-TEA-Cocamide Diacetate, Di-TEA-Oleamido PEG-2 Sulfosuccinate, Di-TEA-Palmitoyl Aspartate, Ditridecyl Sodium Sulfosuccinate, Dodecylbenzene Sulfonic Acid, Erucamidopropyl Hydroxysultaine, Ethylhexeth-3 Carboxylic Acid, Ethyl PEG-15 Cocamine Sulfate, Glyceryl Capryl Ether, Hexyldecanoic Acid, Hydrogenated Coconut Acid, Hydrogenated Laneth-25, Hydrogenated Menhaden Acid, Hydrogenated Palm Acid, Hydrogenated Palm Kernel Amine Oxide, Hydrogenated Tallow Acid, Hydrogenated Tallowamine Oxide, Hydrogenated Tallow Betaine, Hydrogenated Talloweth-25, Hydrogenated Tallowoyl Glutamic Acid, Hydrolyzed Candida Bombicola Extract, Hydroxyceteth-60, Hydroxyethyl Acetomonium PG-Dimethicone, Hydroxyethylbutylamine Laureth Sulfate, Hydroxyethyl Carboxymethyl Cocamidopropylamine, Hydroxyethyl Hydroxypropyl C12-15 Alkoxypropylamine Oxide, Hydroxylauryl/Hydroxymyristyl Betaine, Hydroxystearic Acid, Hydroxysuccinimidyl C10-40 Isoalkyl Acidate, Hydroxysuccinimidyl C21-22 Isoalkyl Acidate, Hydroxysultaines, IPDI/PEG-15 Soyamine Oxide Copolymer, IPDI/PEG-15 Soyethonium Ethosulfate Copolymer, IPDI/PEG-15 Soy Glycinate Copolymer, Isoceteth-30, Isolaureth-4 Phosphate, Isopolyglyceryl-3 Dimethicone, Isopolyglyceryl-3 Dimethiconol, Isopropanolamine Lanolate, Isopropylamine Dodecylbenzenesulfonate, Isostearamidopropylamine Oxide, Isostearamidopropyl Betaine, Isostearamidopropyl Morpholine Oxide, Isosteareth-8, Isosteareth-16, Isosteareth-22, Isosteareth-25, Isosteareth-50, Isostearic Acid, Isostearoyl Hydrolyzed Collagen, Jojoba Oil PEG-150 Esters, Jojoba Wax PEG-80 Esters, Jojoba Wax PEG-120 Esters, Laneth-20, Laneth-25, Laneth-40, Laneth-50, Laneth-60, Laneth-75, Lanolin Acid, Lauramidopropylamine Oxide, Lauramidopropyl Betaine, Lauramidopropyl Hydroxysultaine, Lauramine Oxide, Lauraminopropionic Acid, Laurdimoniumhydroxypropyl Decylglucosides Chloride, Laurdimoniumhydroxypropyl Laurylglucosides Chloride, Laureth-16, Laureth-20, Laureth-21, Laureth-23, Laureth-25, Laureth-30, Laureth-38, Laureth-40, Laureth-3 Carboxylic Acid, Laureth-4 Carboxylic Acid, Laureth-5 Carboxylic Acid, Laureth- 6 Carboxylic Acid, Laureth-8 Carboxylic Acid, Laureth-10 Carboxylic Acid, Laureth-11 Carboxylic Acid, Laureth-12 Carboxylic Acid, Laureth-13 Carboxylic Acid, Laureth-14 Carboxylic Acid, Laureth-17 Carboxylic Acid, Laureth-6 Citrate, Laureth-7 Citrate, Laureth-1 Phosphate, Laureth-2 Phosphate, Laureth-3 Phosphate, Laureth-4 Phosphate, Laureth-7 Phosphate, Laureth-8 Phosphate, Laureth-7 Tartrate, Laurie Acid, Laurimino Bispropanediol, Lauriminodipropionic Acid, Lauroamphodipropionic Acid, Lauroyl Beta-Alanine, Lauroyl Collagen Amino Acids, Lauroyl Ethyltrimonium Methosulfate, Lauroyl Hydrolyzed Collagen, Lauroyl Hydrolyzed Elastin, Lauroyl Methyl Glucamide, Lauroyl Sarcosine, Lauroyl Silk Amino Acids, Lauryl Betaine, Lauryl Dimethicone/Polyglycerin-3 Crosspolymer, Lauryldimoniumhydroxypropyl Cocoglucosides Chloride, Lauryl Glucoside, Laurylglucosides Hydroxypropyltrimonium Chloride, Lauryl Glycol Hydroxypropyl Ether, Lauryl Hydroxysultaine, Lauryl Malamide, Lauryl Methylglucamide, Lauryl/Myristyl Glycol Hydroxypropyl Ether, Lauryl/Myristyl Wheat Bran/Straw Glycosides, Lauryl Polyglyceryl-3 Polydimethylsiloxyethyl Dimethicone, Lauryl Pyrrolidone, Lauryl Sultaine, Linoleic Acid, Linolenic Acid, Linseed Acid, Lysine Cocoate, Macadamia Seed Oil Glycereth-8 Esters, Magnesium Coceth Sulfate, Magnesium Coco-Sulfate, Magnesium Isododecylbenzenesulfonate, Magnesium Laureth-11 Carboxylate, Magnesium Laureth Sulfate, Magnesium Laureth-5 Sulfate, Magnesium Laureth-8 Sulfate, Magnesium Laureth-16 Sulfate, Magnesium Laureth-3 Sulfosuccinate, Magnesium Lauryl Hydroxypropyl Sulfonate, Magnesium Lauryl Sulfate, Magnesium Methyl Cocoyl Taurate, Magnesium Myreth Sulfate, Magnesium Oleth Sulfate, Magnesium/TEA-Coco-Sulfate, Manicouagan Clay, MEA-Cocoate, MEA-Laureth-6 Carboxylate, MEA- Laureth Sulfate, MEA-Lauryl Sulfate, MEA PPG-6 Laureth-7 Carboxylate, MEA-PPG-8-Steareth-7 Carboxylate, MEA-Undecylenate, Meroxapol 108, Meroxapol 174, Meroxapol 178, Meroxapol 254, Meroxapol 255, Meroxapol 258, Meroxapol 314, Methoxy PEG-450 Amidoglutaroyl Succinimide, Methoxy PEG-450 Amido Hydroxysuccinimidyl Succinamate, Methoxy PEG-450 Maleimide, Methyl Morpholine Oxide, Milkamidopropyl Amine Oxide, Milkamidopropyl Betaine, Minkamidopropylamine Oxide, Minkamidopropyl Betaine, MIPA C12-15 Pareth Sulfate, MIPA-Dodecylbenzenesulfonate, MIPA-Laureth Sulfate, MIPA-Lauryl Sulfate, Mixed Isopropanolamines Lanolate, Mixed Isopropanolamines Lauryl Sulfate, Mixed Isopropanolamines Myristate, Morpholine Oleate, Morpholine Stearate, Myreth-3 Carboxylic Acid, Myreth-5 Carboxylic Acid, Myristalkonium Chloride, Myristamidopropylamine Oxide, Myristamidopropyl Betaine, Myristamidopropyl Dimethylamine Phosphate, Myristamidopropyl Hydroxysultaine, Myristamidopropyl PG-Dimonium Chloride Phosphate, Myristamine Oxide, Myristaminopropionic Acid, Myristic Acid, Myristoyl Ethyltrimonium Methosulfate, Myristoyl Glutamic Acid, Myristoyl Hydrolyzed Collagen, Myristoyl Sarcosine, Myristyl Betaine, Myristyl/Cetyl Amine Oxide, Myristyldimoniumhydroxypropyl Cocoglucosides Chloride, Myristyl Glucoside, Myristyl Phosphate, Nonoxynol-20, Nonoxynol-23, Nonoxynol-25, Nonoxynol-30, Nonoxynol-35, Nonoxynol-40, Nonoxynol-44, Nonoxynol-50, Nonoxynol-100, Nonoxynol-120, Nonoxynol-5 Carboxylic Acid, Nonoxynol-8 Carboxylic Acid, Nonoxynol-10 Carboxylic Acid, Nonoxynol-3 Phosphate, Nonoxynol-4 Phosphate, Nonoxynol-6 Phosphate, Nonoxynol-9 Phosphate, Nonoxynol-10 Phosphate, Nonyl Nonoxynol-30, Nonyl Nonoxynol-49, Nonyl Nonoxynol-100, Nonyl Nonoxynol-150, Nonyl Nonoxynol-7 Phosphate, Nonyl Nonoxynol-8 Phosphate, Nonyl Nonoxynol-9 Phosphate, Nonyl Nonoxynol-10 Phosphate, Nonyl Nonoxynol-11 Phosphate, Nonyl Nonoxynol-15 Phosphate, Nonyl Nonoxynol-24 Phosphate, Oatamidopropyl Betaine, Octoxynol-16, Octoxynol-25, Octoxynol-30, Octoxynol-33, Octoxynol-40, Octoxynol-70, Octoxynol-20 Carboxylic Acid, Octyldodeceth-20, Octyldodeceth-25, Octyldodeceth-30, Oleamidopropylamine Oxide, Oleamidopropyl Betaine, Oleamidopropyl Hydroxysultaine, Oleamine Oxide, Oleic Acid, Oleoyl Hydrolyzed Collagen, Oleoyl Sarcosine, Oleth-20, Oleth-23, Oleth-24, Oleth-25, Oleth-30, Oleth-35, Oleth-40, Oleth-44, Oleth-50, Oleth-3 Carboxylic Acid, Oleth-6 Carboxylic Acid, Oleth-10 Carboxylic Acid, Oleyl Betaine, Olivamidopropylamine Oxide, Olivamidopropyl Betaine, Olive Acid, Olivoyl Hydrolyzed Wheat Protein, Ophiopogon Extract Stearate, Ozonized Oleth-10, Ozonized PEG-10 Oleate, Ozonized PEG-14 Oleate, Ozonized Polysorbate 80, Palm Acid, Palmamidopropyl Betaine, Palmeth-2 Phosphate, Palmitamidopropylamine Oxide, Palmitamidopropyl Betaine, Palmitamine Oxide, Palmitic Acid, Palmitoyl Collagen Amino Acids, Palmitoyl Glycine, Palmitoyl Hydrolyzed Collagen, Palmitoyl Hydrolyzed Milk Protein, Palmitoyl Hydrolyzed Wheat Protein, Palmitoyl Keratin Amino Acids, Palmitoyl Oligopeptide, Palmitoyl Silk Amino Acids, Palm Kernel Acid, Palm Kernelamidopropyl Betaine, Peach Kernel Oil Glycereth-8 Esters, Peanut Acid, PEG-10 Castor Oil, PEG-40 Castor Oil, PEG-44 Castor Oil, PEG-50 Castor Oil, PEG-54 Castor Oil, PEG-55 Castor Oil, PEG-60 Castor Oil, PEG-80 Castor Oil, PEG-100 Castor Oil, PEG-200 Castor Oil, PEG-11 Cocamide, PEG-6 Cocamide Phosphate, PEG-4 Cocamine, PEG-8 Cocamine, PEG-12 Cocamine, PEG-150 Dibehenate, PEG-90 Diisostearate, PEG-75 Dilaurate, PEG-150 Dilaurate, PEG-75 Dioleate, PEG-150 Dioleate, PEG-75 Distearate, PEG-120 Distearate, PEG-150 Distearate, PEG-175 Distearate, PEG-190 Distearate, PEG-250 Distearate, PEG-30 Glyceryl Cocoate, PEG-40 Glyceryl Cocoate, PEG-78 Glyceryl Cocoate, PEG-80 Glyceryl Cocoate, PEG-30 Glyceryl Isostearate, PEG-40 Glyceryl Isostearate, PEG-50 Glyceryl Isostearate, PEG-60 Glyceryl Isostearate, PEG-90 Glyceryl Isostearate, PEG-23 Glyceryl Laurate, PEG-30 Glyceryl Laurate, PEG-25 Glyceryl Oleate, PEG-30 Glyceryl Oleate, PEG-30 Glyceryl Soyate, PEG-25 Glyceryl Stearate, PEG-30 Glyceryl Stearate, PEG-40 Glyceryl Stearate, PEG-120 Glyceryl Stearate, PEG-200 Glyceryl Stearate, PEG-28 Glyceryl Tallowate, PEG-80 Glyceryl Tallowate, PEG-82 Glyceryl Tallowate, PEG-130 Glyceryl Tallowate, PEG-200 Glyceryl Tallowate, PEG-45 Hydrogenated Castor Oil, PEG-50 Hydrogenated Castor Oil, PEG-54 Hydrogenated Castor Oil, PEG-55 Hydrogenated Castor Oil, PEG-60 Hydrogenated Castor Oil, PEG-80 Hydrogenated Castor Oil, PEG-100 Hydrogenated Castor Oil, PEG-200 Hydrogenated Castor Oil, PEG-30 Hydrogenated Lanolin, PEG-70 Hydrogenated Lanolin, PEG-50 Hydrogenated Palmamide, PEG-2 Isostearate, PEG-3 Isostearate, PEG-4 Isostearate, PEG-6 Isostearate, PEG-8 Isostearate, PEG-10 Isostearate, PEG-12 Isostearate, PEG-20 Isostearate, PEG-30 Isostearate, PEG-40 Isostearate, PEG- 26 Jojoba Acid, PEG-40 Jojoba Acid, PEG-15 Jojoba Alcohol, PEG-26 Jojoba Alcohol, PEG-40 Jojoba Alcohol, PEG-35 Lanolin, PEG-40 Lanolin, PEG-50 Lanolin, PEG-55 Lanolin, PEG-60 Lanolin, PEG- 70 Lanolin, PEG-75 Lanolin, PEG-85 Lanolin, PEG-100 Lanolin, PEG-150 Lanolin, PEG-75 Lanolin Oil, PEG-2 Lauramide, PEG-3 Lauramine Oxide, PEG-20 Laurate, PEG-32 Laurate, PEG-75 Laurate, PEG-150 Laurate, PEG-70 Mango Glycerides, PEG-20 Mannitan Laurate, PEG-8 Methyl Ether Dimethicone, PEG-120 Methyl Glucose Dioleate, PEG-80 Methyl Glucose Laurate, PEG-120 Methyl Glucose Trioleate, PEG-4 Montanate, PEG-30 Oleamine, PEG-20 Oleate, PEG-23 Oleate, PEG-32 Oleate, PEG-36 Oleate, PEG-75 Oleate, PEG-150 Oleate, PEG-20 Palmitate, PEG-150 Polyglyceryl-2 Tristearate, PEG/PPG-28/21 Acetate Dimethicone, PEG/PPG-24/18 Butyl Ether Dimethicone, PEG/PPG-3/17 Copolymer, PEG/PPG-5/35 Copolymer, PEG/PPG-8/55 Copolymer, PEG/PPG-10/30 Copolymer, PEG/PPG-10/65 Copolymer, PEG/PPG-12/35 Copolymer, PEG/PPG-16/17 Copolymer, PEG/PPG-20/9 Copolymer, PEG/PPG-20/20 Copolymer, PEG/PPG-20/60 Copolymer, PEG/PPG- 20/65 Copolymer, PEG/PPG-22/25 Copolymer, PEG/PPG-28/30 Copolymer, PEG/PPG-30-35 Copolymer, PEG/PPG-30/55 Copolymer, PEG/PPG-35/40 Copolymer, PEG/PPG-50/40 Copolymer, PEG/PPG-150/35 Copolymer, PEG/PPG-160/30 Copolymer, PEG/PPG-190/60 Copolymer, PEG/PPG-200/40 Copolymer, PEG/PPG-300/55 Copolymer, PEG/PPG-20/22 Methyl Ether Dimethicone, PEG-26-PPG-30 Phosphate, PEG/PPG-4/2 Propylheptyl Ether, PEG/PPG-6/2 Propylheptyl Ether, PEG-7/PPG-2 Propylheptyl Ether, PEG/PPG-8/2 Propylheptyl Ether, PEG/PPG- 10/2 Propylheptyl Ether, PEG/PPG-14/2 Propylheptyl Ether, PEG/PPG-40/2 Propylheptyl Ether, PEG/PPG-10/2 Ricinoleate, PEG/PPG-32/3 Ricinoleate, PEG-55 Propylene Glycol Oleate, PEG-25 Propylene Glycol Stearate, PEG-75 Propylene Glycol Stearate, PEG-120 Propylene Glycol Stearate, PEG-5 Rapeseed Sterol, PEG-10 Rapeseed Sterol, PEG-40 Ricinoleamide, PEG-75 Shea Butter Glycerides, PEG-75 Shorea Butter Glycerides, PEG-20 Sorbitan Cocoate, PEG-20 Sorbitan Isostearate, PEG-40 Sorbitan Lanolate, PEG-75 Sorbitan Lanolate, PEG-10 Sorbitan Laurate, PEG-40 Sorbitan Laurate, PEG-44 Sorbitan Laurate, PEG-75 Sorbitan Laurate, PEG-80 Sorbitan Laurate, PEG-20 Sorbitan Oleate, PEG-80 Sorbitan Palmitate, PEG-40 Sorbitan Stearate, PEG-60 Sorbitan Stearate, PEG-160 Sorbitan Triisostearate, PEG-40 Soy Sterol, PEG-2 Stearamide Carboxylic Acid, PEG-9 Stearamide Carboxylic Acid, PEG-20 Stearate, PEG-23 Stearate, PEG-25 Stearate, PEG-30 Stearate, PEG-32 Stearate, PEG-35 Stearate, PEG-36 Stearate, PEG-40 Stearate, PEG-45 Stearate, PEG-50 Stearate, PEG-55 Stearate, PEG-75 Stearate, PEG-90 Stearate, PEG-100 Stearate, PEG- 120 Stearate, PEG-150 Stearate, PEG-45 Stearate Phosphate, PEG-20 Tallate, PEG-50 Tallow Amide, PEG-2 Tallowamide DEA, PEG-20 Tallowate, PEG-66 Trihydroxystearin, PEG-200 Trihydroxystearin, PEG-60 Tsubakiate Glycerides, Pelargonic Acid, Pentadoxynol-200, Pheneth-6 Phosphate, Poloxamer 105, Poloxamer 108, Poloxamer 182, Poloxamer 183, Poloxamer 184, Poloxamer 188, Poloxamer 217, Poloxamer 234, Poloxamer 235, Poloxamer 237, Poloxamer 238, Poloxamer 288, Poloxamer 334, Poloxamer 335, Poloxamer 338, Poloxamine 908, Poloxamine 1508, Polydimethylsiloxy PEG/PPG-24/19 Butyl Ether Silsesquioxane, Polydimethylsiloxy PPG-13 Butyl Ether Silsesquioxane, Polyglyceryl-6 Caprate, Polyglyceryl-10 Dilaurate, Polyglyceryl-20 Heptacaprylate, Polyglyceryl-20 Hexacaprylate, Polyglyceryl-2 Lauryl Ether, Polyglyceryl-10 Lauryl Ether, Polyglyceryl-20 Octaisononanoate, Polyglyceryl-6 Pentacaprylate, Polyglyceryl-10 Pentacaprylate, Polyglyceryl-3 Polydimethylsiloxyethyl Dimethicone, Polyglyceryl-6 Tetracaprylate, Polyglyceryl-10 Tetralaurate, Polyglyceryl-6 Tricaprylate, Polyglyceryl-10 Trilaurate, Polyquaternium-77, Polyquaternium-78, Polyquaternium-79, Polyquaternium-80, Polyquaternium-81, Polyquaternium- 82, Pomaderris Kumerahou Flower/Leaf Extract, Poria Cocos Extract, Potassium Abietoyl Hydrolyzed Collagen, Potassium Babassuate, Potassium Behenate, Potassium C9-15 Alkyl Phosphate, Potassium C11-15 Alkyl Phosphate, Potassium C12-13 Alkyl Phosphate, Potassium C12-14 Alkyl Phosphate, Potassium Caprate, Potassium Capryloyl Glutamate, Potassium Capryloyl Hydrolyzed Rice Protein, Potassium Castorate, Potassium Cocoate, Potassium Cocoyl Glutamate, Potassium Cocoyl Glycinate, Potassium Cocoyl Hydrolyzed Casein, Potassium Cocoyl Hydrolyzed Collagen, Potassium Cocoyl Hydrolyzed Corn Protein, Potassium Cocoyl Hydrolyzed Keratin, Potassium Cocoyl Hydrolyzed Oat Protein, Potassium Cocoyl Hydrolyzed Potato Protein, Potassium Cocoyl Hydrolyzed Rice Bran Protein, Potassium Cocoyl Hydrolyzed Rice Protein, Potassium Cocoyl Hydrolyzed Silk, Potassium Cocoyl Hydrolyzed Soy Protein, Potassium Cocoyl Hydrolyzed Wheat Protein, Potassium Cocoyl Hydrolyzed Yeast Protein, Potassium Cocoyl PCA, Potassium Cocoyl Sarcosinate, Potassium Cocoyl Taurate, Potassium Cornate, Potassium Cyclocarboxypropyloleate, Potassium Dihydroxyethyl Cocamine Oxide Phosphate, Potassium Dimethicone PEG-7 Phosphate, Potassium Dodecylbenzenesulfonate, Potassium Hempseedate, Potassium Hydrogenated Cocoate, Potassium Hydrogenated Palmate, Potassium Hydrogenated Tallowate, Potassium Hydroxystearate, Potassium Isostearate, Potassium Lanolate, Potassium Laurate, Potassium Laureth-3 Carboxylate, Potassium Laureth-4 Carboxylate, Potassium Laureth-5 Carboxylate, Potassium Laureth-6 Carboxylate, Potassium Laureth-10 Carboxylate, Potassium Laureth Phosphate, Potassium Lauroyl Collagen Amino Acids, Potassium Lauroyl Glutamate, Potassium Lauroyl Hydrolyzed Collagen, Potassium Lauroyl Hydrolyzed Pea Protein, Potassium Lauroyl Hydrolyzed Soy Protein, Potassium Lauroyl PCA, Potassium Lauroyl Pea Amino Acids, Potassium Lauroyl Sarcosinate, Potassium Lauroyl Silk Amino Acids, Potassium Lauroyl Wheat Amino Acids, Potassium Lauryl Phosphate, Potassium Lauryl Sulfate, Potassium Linoleate, Potassium Metaphosphate, Potassium Methyl Cocoyl Taurate, Potassium Myristate, Potassium Myristoyl Glutamate, Potassium Myristoyl Hydrolyzed Collagen, Potassium Octoxynol-12 Phosphate, Potassium Oleate, Potassium Oleoyl Hydrolyzed Collagen, Potassium Olivate, Potassium Olivoyl Hydrolyzed Oat Protein, Potassium Olivoyl Hydrolyzed Wheat Protein, Potassium Olivoyl/Lauroyl Wheat Amino Acids, Potassium Olivoyl PCA, Potassium Palmate, Potassium Palmitate, Potassium Palmitoyl Hydrolyzed Corn Protein, Potassium Palmitoyl Hydrolyzed Oat Protein, Potassium Palmitoyl Hydrolyzed Rice Protein, Potassium Palmitoyl Hydrolyzed Sweet Almond Protein, Potassium Palmitoyl Hydrolyzed Wheat Protein, Potassium Palm Kernelate, Potassium Peanutate, Potassium Rapeseedate, Potassium Ricinoleate, Potassium Safflowerate, Potassium Soyate, Potassium Stearate, Potassium Stearoyl Hydrolyzed Collagen, Potassium Tallate, Potassium Tallowate, Potassium Taurate, Potassium Taurine Laurate, Potassium Trideceth-3 Carboxylate, Potassium Trideceth-4 Carboxylate, Potassium Trideceth-7 Carboxylate, Potassium Trideceth-15 Carboxylate, Potassium Trideceth-19 Carboxylate, Potassium Trideceth-6 Phosphate, Potassium Trideceth-7 Phosphate, Potassium Tsubakiate, Potassium Undecylenate, Potassium Undecylenoyl Hydrolyzed Collagen, Potassium Undecylenoyl Hydrolyzed Rice Protein, PPG-30- Buteth-30, PPG-36-Buteth-36, PPG-38-Buteth-37, PPG-30-Capryleth-4 Phosphate, PPG-10 Cetyl Ether Phosphate, PPG-2 C9-11 Pareth-8, PPG-1-Deceth-5, PPG-3-Deceth-2 Carboxylic Acid, PPG-30 Ethylhexeth-4 Phosphate, PPG-20-Glycereth-30, PPG-2 Hydroxyethyl Coco/Isostearamide, PPG-2- Isodeceth-8, PPG-2-Isodeceth-10, PPG-2-Isodeceth-18, PPG-2-Isodeceth-25, PPG-4-Isodeceth-10, Propyltrimonium Hydrolyzed Collagen, Quaternium-24, Quaternium-52, Quaternium-87, Rapeseed Acid, Rice Bran Acid, Rice Oil Glycereth-8 Esters, Ricinoleamidopropyl Betaine, Ricinoleic Acid, Ricinoleth-40, Safflower Acid, Sapindus Oahuensis Fruit Extract, Saponaria Officinalis Root Powder, Saponins, Sekken-K, Sekken-Na/K, Sekken Soji, Sekken Soji-K, Sesame Oil Glycereth-8 Esters, Sesamidopropylamine Oxide, Sesamidopropyl Betaine, Shea Butteramidopropyl Betaine, Shea Butter Glycereth-8 Esters, Sodium Arachidate, Sodium Arganampohoacetate, Sodium Astrocaryum Murumuruate, Sodium Avocadoate, Sodium Babassuamphoacetate, Sodium Babassuate, Sodium Babassu Sulfate, Sodium Behenate, Sodium Bisglycol Ricinosulfosuccinate, Sodium Bis-Hydroxyethylglycinate Coco-Glucosides Crosspolymer, Sodium Bis-Hydroxyethylglycinate Lauryl- Glucosides Crosspolymer, Sodium Borageamidopropyl PG-Dimonium Chloride Phosphate, Sodium Butoxynol-12 Sulfate, Sodium Butylglucosides Hydroxypropyl Phosphate, Sodium C13-17 Alkane Sulfonate, Sodium C14-18 Alkane Sulfonate, Sodium C12-15 Alkoxypropyl Iminodipropionate, Sodium C10-16 Alkyl Sulfate, Sodium C11-15 Alkyl Sulfate, Sodium C12-13 Alkyl Sulfate, Sodium C12-15 Alkyl Sulfate, Sodium C12-18 Alkyl Sulfate, Sodium C16-20 Alkyl Sulfate, Sodium C9-22 Alkyl Sec Sulfonate, Sodium C14-17 Alkyl Sec Sulfonate, Sodium Caprate, Sodium Caproamphoacetate, Sodium Caproamphohydroxypropylsulfonate, Sodium Caproamphopropionate, Sodium Caproyl Methyltaurate, Sodium Caprylate, Sodium Capryleth-2 Carboxylate, Sodium Capryleth-9 Carboxylate, Sodium Capryloamphoacetate, Sodium Capryloamphohydroxypropylsulfonate, Sodium Capryloamphopropionate, Sodium Capryloyl Glutamate, Sodium Capryloyl Hydrolyzed Wheat Protein, Sodium Caprylyl PG-Sulfonate, Sodium Caprylyl Sulfonate, Sodium Castorate, Sodium Ceteareth-13 Carboxylate, Sodium Cetearyl Sulfate, Sodium Ceteth-13 Carboxylate, Sodium Cetyl Sulfate, Sodium Cocamidopropyl PG-Dimonium Chloride Phosphate, Sodium Cocaminopropionate, Sodium Coceth Sulfate, Sodium Coceth-30 Sulfate, Sodium Cocoabutteramphoacetate, Sodium Cocoa Butterate, Sodium Cocoamphoacetate, Sodium Cocoamphohydroxypropylsulfonate, Sodium Cocoamphopropionate, Sodium Cocoate, Sodium Coco/Babassu/Andiroba Sulfate, Sodium Coco/Babassu Sulfate, Sodium Cocoglucosides Hydroxypropyl Phosphate, Sodium Cocoglucosides Hydroxypropylsulfonate, Sodium Coco-Glucoside Tartrate, Sodium Cocoglyceryl Ether Sulfonate, Sodium Coco/Hydrogenated Tallow Sulfate, Sodium Cocoiminodiacetate, Sodium Cocomonoglyceride Sulfate, Sodium Cocomonoglyceride Sulfonate, Sodium Coco PG-Dimonium Chloride Phosphate, Sodium Coco-Sulfate, Sodium Coco Sulfoacetate, Sodium Cocoyl Alaninate, Sodium Cocoyl Amino Acids, Sodium Cocoyl Collagen Amino Acids, Sodium Cocoyl Glutamate, Sodium Cocoyl Glutaminate, Sodium Cocoyl Glycinate, Sodium Cocoyl/Hydrogenated Tallow Glutamate, Sodium Cocoyl Hydrolyzed Collagen, Sodium Cocoyl Hydrolyzed Keratin, Sodium Cocoyl Hydrolyzed Rice Protein, Sodium Cocoyl Hydrolyzed Silk, Sodium Cocoyl Hydrolyzed Soy Protein, Sodium Cocoyl Hydrolyzed Sweet Almond Protein, Sodium Cocoyl Hydrolyzed Wheat Protein, Sodium Cocoyl Hydrolyzed Wheat Protein Glutamate, Sodium Cocoyl Isethionate, Sodium Cocoyl Methylaminopropionate, Sodium Cocoyl Oat Amino Acids, Sodium Cocoyl/Palmoyl/Sunfloweroyl Glutamate, Sodium Cocoyl Proline, Sodium Cocoyl Sarcosinate, Sodium Cocoyl Taurate, Sodium Cocoyl Threoninate, Sodium Cocoyl Wheat Amino Acids, Sodium C12-14 Olefin Sulfonate, Sodium C14-16 Olefin Sulfonate, Sodium C14- 18 Olefin Sulfonate, Sodium C16-18 Olefin Sulfonate, Sodium Cornamphopropionate, Sodium Cottonseedamphoacetate, Sodium C13-15 Pareth-8 Butyl Phosphate, Sodium C9-11 Pareth-6 Carboxylate, Sodium C11-15 Pareth-7 Carboxylate, Sodium C12-13 Pareth-5 Carboxylate, Sodium C12-13 Pareth-8 Carboxylate, Sodium C12-13 Pareth-12 Carboxylate, Sodium C12-15 Pareth-6 Carboxylate, Sodium C12-15 Pareth-7 Carboxylate, Sodium C12-15 Pareth-8 Carboxylate, Sodium C14-15 Pareth-8 Carboxylate, Sodium C12-14 Sec-Pareth-8 Carboxylate, Sodium C14-15 Pareth-PG Sulfonate, Sodium C12-13 Pareth-2 Phosphate, Sodium C13-15 Pareth-8 Phosphate, Sodium C9-15 Pareth-3 Sulfate, Sodium C10-15 Pareth Sulfate, Sodium C10-16 Pareth-2 Sulfate, Sodium C12-13 Pareth Sulfate, Sodium C12-15 Pareth Sulfate, Sodium C12-15 Pareth-3 Sulfate, Sodium C13-15 Pareth-3 Sulfate, Sodium C12-14 Sec-Pareth-3 Sulfate, Sodium C12-15 Pareth-3 Sulfonate, Sodium C12-15 Pareth-7 Sulfonate, Sodium C12-15 Pareth-15 Sulfonate, Sodium Deceth-2 Carboxylate, Sodium Deceth Sulfate, Sodium Decylbenzenesulfonate, Sodium Decylglucosides Hydroxypropyl Phosphate, Sodium Decylglucosides Hydroxypropylsulfonate, Sodium Dilaureth-7 Citrate, Sodium Dilaureth-10 Phosphate, Sodium Dilinoleamidopropyl PG-Dimonium Chloride Phosphate, Sodium Dilinoleate, Sodium Dioleth-8 Phosphate, Sodium Dodecylbenzenesulfonate, Sodium Ethyl 2-Sulfolaurate, Sodium Glyceryl Oleate Phosphate, Sodium Grapeseedamidopropyl PG-Dimonium Chloride Phosphate, Sodium Grapeseedamphoacetate, Sodium Grapeseedate, Sodium Hempseedamphoacetate, Sodium Hexeth-4 Carboxylate, Sodium Hydrogenated Cocoate, Sodium Hydrogenated Cocoyl Methyl Isethionate, Sodium Hydrogenated Palmate, Sodium Hydrogenated Tallowate, Sodium Hydrogenated Tallowoyl Glutamate, Sodium Hydroxylauryldimonium Ethyl Phosphate, Sodium Hydroxypropyl Palm Kernelate Sulfonate, Sodium Hydroxypropylphosphate Decylglucoside Crosspolymer, Sodium Hydroxypropylphosphate Laurylglucoside Crosspolymer, Sodium Hydroxypropylsulfonate Cocoglucoside Crosspolymer, Sodium Hydroxypropylsulfonate Decylglucoside Crosspolymer, Sodium Hydroxypropylsulfonate Laurylglucoside Crosspolymer, Sodium Hydroxystearate, Sodium Isostearate, Sodium Isosteareth-6 Carboxylate, Sodium Isosteareth-11 Carboxylate, Sodium Isostearoamphoacetate, Sodium Isostearoamphopropionate, Sodium N- Isostearoyl Methyltaurate, Sodium Laneth Sulfate, Sodium Lanolate, Sodium Lardate, Sodium Lauramido Diacetate, Sodium Lauraminopropionate, Sodium Laurate, Sodium Laureth-3 Carboxylate, Sodium Laureth-4 Carboxylate, Sodium Laureth-5 Carboxylate, Sodium Laureth-6 Carboxylate, Sodium Laureth-8 Carboxylate, Sodium Laureth-11 Carboxylate, Sodium Laureth-12 Carboxylate, Sodium Laureth-13 Carboxylate, Sodium Laureth-14 Carboxylate, Sodium Laureth-16 Carboxylate, Sodium Laureth-17 Carboxylate, Sodium Laureth Sulfate, Sodium Laureth-5 Sulfate, Sodium Laureth-7 Sulfate, Sodium Laureth-8 Sulfate, Sodium Laureth-12 Sulfate, Sodium Laureth-40 Sulfate, Sodium Laureth-7 Tartrate, Sodium Lauriminodipropionate, Sodium Lauroamphoacetate, Sodium Lauroamphohydroxypropylsulfonate, Sodium Lauroampho PG-Acetate Phosphate, Sodium Lauroamphopropionate, Sodium Lauroyl Aspartate, Sodium Lauroyl Collagen Amino Acids, Sodium Lauroyl Glycine Propionate, Sodium Lauroyl Hydrolyzed Collagen, Sodium Lauroyl Hydrolyzed Silk, Sodium Lauroyl Hydroxypropyl Sulfonate, Sodium Lauroyl Isethionate, Sodium Lauroyl Methylaminopropionate, Sodium Lauroyl Methyl Isethionate, Sodium Lauroyl Millet Amino Acids, Sodium Lauroyl/Myristoyl Aspartate, Sodium Lauroyl Oat Amino Acids, Sodium Lauroyl Sarcosinate, Sodium Lauroyl Silk Amino Acids, Sodium Lauroyl Taurate, Sodium Lauroyl Wheat Amino Acids, Sodium Lauryl Diethylenediaminoglycinate, Sodium Lauryl Glucose Carboxylate, Sodium Laurylglucosides Hydroxypropyl Phosphate, Sodium Laurylglucosides Hydroxypropylsulfonate, Sodium Lauryl Glycol Carboxylate, Sodium Lauryl Hydroxyacetamide Sulfate, Sodium Lauryl Phosphate, Sodium Lauryl Sulfate, Sodium Lauryl Sulfoacetate, Sodium Linoleate, Sodium Macadamiaseedate, Sodium Mangoamphoacetate, Sodium Mangoseedate, Sodium/MEA Laureth-2 Sulfosuccinate, Sodium Methoxy PPG-2 Acetate, Sodium Methyl Cocoyl Taurate, Sodium Methyl Lauroyl Taurate, Sodium Methyl Myristoyl Taurate, Sodium Methyl Oleoyl Taurate, Sodium Methyl Palmitoyl Taurate, Sodium Methyl Stearoyl Taurate, Sodium Methyl 2-Sulfolaurate, Sodium Methyl 2-Sulfopalmitate, Sodium Methyltaurate Isopalmitamide, Sodium Methyltaurine Cocoyl Methyltaurate, Sodium Myreth Sulfate, Sodium Myristate, Sodium Myristoamphoacetate, Sodium Myristoyl Glutamate, Sodium Myristoyl Hydrolyzed Collagen, Sodium Myristoyl Isethionate, Sodium Myristoyl Sarcosinate, Sodium Myristyl Sulfate, Sodium Nonoxynol-6 Phosphate, Sodium Nonoxynol-9 Phosphate, Sodium Nonoxynol-1 Sulfate, Sodium Nonoxynol-3 Sulfate, Sodium Nonoxynol-4 Sulfate, Sodium Nonoxynol-6 Sulfate, Sodium Nonoxynol-8 Sulfate, Sodium Nonoxynol-10 Sulfate, Sodium Nonoxynol-25 Sulfate, Sodium Octoxynol-2 Ethane Sulfonate, Sodium Octoxynol-2 Sulfate, Sodium Octoxynol-6 Sulfate, Sodium Octoxynol-9 Sulfate, Sodium Oleate, Sodium Oleoamphoacetate, Sodium Oleoamphohydroxypropylsulfonate, Sodium Oleoamphopropionate, Sodium Oleoyl Hydrolyzed Collagen, Sodium Oleoyl Isethionate, Sodium Oleth Sulfate, Sodium Oleyl Methyl Isethionate, Sodium Oleyl Sulfate, Sodium Olivamphoacetate, Sodium Olivate, Sodium Olivoyl Glutamate, Sodium Palmamphoacetate, Sodium Palmate, Sodium Palm Glyceride Sulfonate, Sodium Palmitate, Sodium Palmitoyl Hydrolyzed Collagen, Sodium Palmitoyl Hydrolyzed Wheat Protein, Sodium Palmitoyl Sarcosinate, Sodium Palm Kernelate, Sodium Palm Kerneloyl Isethionate, Sodium Palmoyl Glutamate, Sodium Passiflora Edulis Seedate, Sodium Peanutamphoacetate, Sodium Peanutate, Sodium PEG-6 Cocamide Carboxylate, Sodium PEG-8 Cocamide Carboxylate, Sodium PEG-4 Cocamide Sulfate, Sodium PEG-3 Lauramide Carboxylate, Sodium PEG-4 Lauramide Carboxylate, Sodium PEG-8 Palm Glycerides Carboxylate, Sodium Pentaerythrityl Hydroxypropyl Iminodiacetate Dendrimer, Sodium Propoxy PPG-2 Acetate, Sodium Rapeseedate, Sodium Ricebranamphoacetate, Sodium Ricinoleate, Sodium Ricinoleoamphoacetate, Sodium Rose Hipsamphoacetate, Sodium Rosinate, Sodium Safflowerate, Sodium Saffloweroyl Hydrolyzed Soy Protein, Sodium Sesameseedate, Sodium Sesamphoacetate, Sodium Sheabutteramphoacetate, Sodium Soyate, Sodium Soy Hydrolyzed Collagen, Sodium Stearate, Sodium Stearoamphoacetate, Sodium Stearoamphohydroxypropylsulfonate, Sodium Stearoamphopropionate, Sodium Stearoyl Casein, Sodium Stearoyl Glutamate, Sodium Stearoyl Hyaluronate, Sodium Stearoyl Hydrolyzed Collagen, Sodium Stearoyl Hydrolyzed Corn Protein, Sodium Stearoyl Hydrolyzed Silk, Sodium Stearoyl Hydrolyzed Soy Protein, Sodium Stearoyl Hydrolyzed Wheat Protein, Sodium Stearoyl Lactalbumin, Sodium Stearoyl Methyl Isethionate, Sodium Stearoyl Oat Protein, Sodium Stearoyl Pea Protein, Sodium Stearoyl Soy Protein, Sodium Stearyl Dimethyl Glycine, Sodium Stearyl Sulfate, Sodium Sunflowerseedamphoacetate, Sodium Surfactin, Sodium Sweetalmondamphoacetate, Sodium Sweet Almondate, Sodium Tallamphopropionate, Sodium Tallate, Sodium Tallowamphoacetate, Sodium Tallowate, Sodium Tallow Sulfate, Sodium Tamanuseedate, Sodium Taurate, Sodium Taurine Cocoyl Methyltaurate, Sodium Taurine Laurate, Sodium/TEA-Lauroyl Collagen Amino Acids, Sodium/TEA-Lauroyl Hydrolyzed Collagen, Sodium/TEA-Lauroyl Hydrolyzed Keratin, Sodium/TEA- Lauroyl Keratin Amino Acids, Sodium/TEA-Undecylenoyl Collagen Amino Acids, Sodium/TEA- Undecylenoyl Hydrolyzed Collagen, Sodium/TEA-Undecylenoyl Hydrolyzed Corn Protein, Sodium/TEA-Undecylenoyl Hydrolyzed Soy Protein, Sodium/TEA-Undecylenoyl Hydrolyzed Wheat Protein, Sodium Theobroma Grandiflorum Seedate, Sodium Trideceth-3 Carboxylate, Sodium Trideceth-4 Carboxylate, Sodium Trideceth-6 Carboxylate, Sodium Trideceth-7 Carboxylate, Sodium Trideceth-8 Carboxylate, Sodium Trideceth-12 Carboxylate, Sodium Trideceth-15 Carboxylate, Sodium Trideceth-19 Carboxylate, Sodium Trideceth Sulfate, Sodium Tridecylbenzenesulfonate, Sodium Tridecyl Sulfate, Sodium Trimethylolpropane Hydroxypropyl Iminodiacetate Dendrimer, Sodium Undeceth-5 Carboxylate, Sodium Undecylenate, Sodium Undecylenoamphoacetate, Sodium Undecylenoamphopropionate, Sodium Undecylenoyl Glutamate, Sodium Wheat Germamphoacetate, Sorbeth-160 Tristearate, Soy Acid, Soyamidopropylamine Oxide, Soyamidopropyl Betaine, Soybean Oil Glycereth-8 Esters, Stearamidopropylamine Oxide, Stearamidopropyl Betaine, Stearamine Oxide, Steareth-15, Steareth-16, Steareth-20, Steareth-21, Steareth-25, Steareth-27, Steareth-30, Steareth- 40, Steareth-50, Steareth-80, Steareth-100, Steareth-2 Phosphate, Steareth-3 Phosphate, Stearic Acid, Stearoxypropyltrimonium Chloride, Stearoyl Glutamic Acid, Stearoyl Sarcosine, Stearyl Betaine, Stearyldimoniumhydroxypropyl Butylglucosides Chloride, Stearyldimoniumhydroxy-propyl Decylglucosides Chloride, Stearyldimoniumhydroxypropyl Laurylglucosides Chloride, Sulfated Castor Oil, Sulfated Coconut Oil, Sulfated Glyceryl Oleate, Sulfated Olive Oil, Sulfated Peanut Oil, Sunfloweramide MEA, Sunflower Seed Acid, Sunflowerseedamidopropyl Hydroxyethyldimonium Chloride, Sunflower Seed Oil Glycereth-8 Esters, Tall Oil Acid, Tallow Acid, Tallowamidopropylamine Oxide, Tallowamidopropyl Betaine, Tallowamidopropyl Hydroxysultaine, Tallowamine Oxide, Tallow Betaine, Tallow Dihydroxyethyl Betaine, Tallowoyl Ethyl Glucoside, TEA-Abietoyl Hydrolyzed Collagen, TEA-C12-14 Alkyl Phosphate, TEA-C10-15 Alkyl Sulfate, TEA-C11-15 Alkyl Sulfate, TEA-C12-13 Alkyl Sulfate, TEA-C12-14 Alkyl Sulfate, TEA-C12-15 Alkyl Sulfate, TEA C14-17 Alkyl Sec Sulfonate, TEA-Canolate, TEA-Cocamide Diacetate, TEA-Cocoate, TEA-Coco-Sulfate, TEA-Cocoyl Alaninate, TEA-Cocoyl Glutamate, TEA-Cocoyl Glutaminate, TEA-Cocoyl Glycinate, TEA-Cocoyl Hydrolyzed Collagen, TEA-Cocoyl Hydrolyzed Soy Protein, TEA-Cocoyl Sarcosinate, TEA- Dimethicone PEG-7 Phosphate, TEA-Dodecylbenzenesulfonate, TEA-Hydrogenated Cocoate, TEA- Hydrogenated Tallowoyl Glutamate, TEA-Isostearate, TEA-Isostearoyl Hydrolyzed Collagen, TEA-Lauraminopropionate, TEA-Laurate, TEA-Laurate/Myristate, TEA-Laureth Sulfate, TEA-Lauroyl Collagen Amino Acids, TEA-Lauroyl Glutamate, TEA-Lauroyl Hydrolyzed Collagen, TEA-Lauroyl Keratin Amino Acids, TEA-Lauroyl Methylaminopropionate, TEA-Lauroyl/Myristoyl Aspartate, TEA-Lauroyl Sarcosinate, TEA-Lauryl Phosphate, TEA-Lauryl Sulfate, TEA-Myristaminopropionate, TEA- Myristate, TEA-Myristoyl Hydrolyzed Collagen, TEA-Oleate, TEA-Oleoyl Hydrolyzed Collagen, TEA- Oleoyl Sarcosinate, TEA-Oleyl Sulfate, TEA-Palmitate, TEA-Palm Kernel Sarcosinate, TEA-PEG-3 Cocamide Sulfate, TEA-Rosinate, TEA-Stearate, TEA-Tallate, TEA-T ridecylbenzenesulfonate, TEA- Undecylenate, TEA-Undecylenoyl Hydrolyzed Collagen, Tetramethyl Decynediol, Tetrasodium Dicarboxyethyl Stearyl Sulfosuccinamate, TIPA-Laureth Sulfate, TIPA-Lauryl Sulfate, TIPA-Myristate, TIPA-Stearate, Tocopheryl Phosphate, Trehalose Undecylenoate, TM-C12-15 Pareth-2 Phosphate, TM-C12-15 Pareth-6 Phosphate, TM-C12-15 Pareth-8 Phosphate, TM-C12-15 Pareth-10 Phosphate, Trideceth-20, Trideceth-50, Trideceth-3 Carboxylic Acid, Trideceth-4 Carboxylic Acid, Trideceth-7 Carboxylic Acid, Trideceth-8 Carboxylic Acid, Trideceth-15 Carboxylic Acid, Trideceth-19 Carboxylic Acid, Trideceth-10 Phosphate, Tridecylbenzenesulfonic Acid, Trilaureth-9 Citrate, Trimethylolpropane Hydroxypropyl Bis-Hydroxyethylamine Dendrimer, Trisodium Lauroampho PG-Acetate Chloride Phosphate, Undecanoic Acid, Undeceth-5 Carboxylic Acid, Undecylenamidopropylamine Oxide, Undecylenamidopropyl Betaine, Undecylenic Acid, Undecylenoyl Collagen Amino Acids, Undecylenoyl Glycine, Undecylenoyl Hydrolyzed Collagen, Undecylenoyl Wheat Amino Acids, Undecyl Glucoside, Wheat Germ Acid, Wheat Germamidopropylamine Oxide, Wheat Germamidopropyl Betaine, Yucca Schidigera Leaf/Root/Stem Extract, Yucca Schidigera Stem Extract, Zinc Coceth Sulfatea and Zinc Coco-Sulfate.

**Preservatives:** For preservative purposes, the cosmetic or pharmaceutical composition according to the present invention preferably includes one or more preservatives which are suitable or customary in cosmetic or pharmaceutical compositions. Suitable and advantageously preservatives are, for example, phenoxyethanol, formaldehyde solution, parabens, pentanediol or sorbic acid.

**Green and synthetic polymers:** The cosmetic or pharmaceutical composition according to the present invention preferably includes one or more polymers. Suitable cationic polymers are, for example, cationic cellulose derivatives such as, for example, the quaternized hydroxyethyl cellulose obtainable from Amerchol under the name of Polymer JR 400^{®}, cationic starch, copolymers of diallyl ammonium salts and acrylamides, quaternized vinyl pyrrolidone/vinyl imidazole polymers such as, for example, Luviquat^{®} (BASF), condensation products of polyglycols and amines, quaternized collagen polypeptides such as, for example, Lauryldimonium Hydroxypropyl Hydrolyzed Collagen (Lamequat^{®} L, Grünau), quaternized wheat polypeptides, polyethyleneimine, cationic silicone polymers such as, for example, amodimethicone, copolymers of adipic acid and dimethylaminohydroxypropyl diethylenetriamine (Cartaretine^{®}, Sandoz), copolymers of acrylic acid with dimethyl diallyl ammonium chloride (Merquat^{®} 550, Chemviron), polyaminopolyamides and crosslinked watersoluble polymers thereof, cationic chitin derivatives such as, for example, quaternized chitosan, optionally in microcrystalline distribution, condensation products of dihaloalkyls, for example dibromobutane, with bis-dialkylamines, for example bis-dimethylamino-1,3-propane, cationic guar gum such as, for example, Jaguar^{®}CBS, Jaguar^{®}C-17, Jaguar^{®}C-16 of Celanese, quaternized ammonium salt polymers such as, for example, Mirapol^{®} A-15, Mirapol^{®} AD-1, Mirapol^{®} AZ-1 of Miranol and the various polyquaternium types (for example 6, 7, 32 or 37) which can be found in the market under the tradenames Rheocare^{®} CC or Ultragel^{®} 300. Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized cellulose ethers and silicones. In a preferred variant, the cosmetic or pharmaceutical composition according to the present invention preferably includes an uncrosslinked or polyol-crosslinked polyacrylic acid as polymer component.

**Thickening agents and/or rheology additives:** The cosmetic or pharmaceutical composition according to the present invention preferably includes one or more thickening agents and/or rheology additives. Suitable thickeners are polymeric thickeners, such as Aerosil^{®} types (hydrophilic silicas), polysaccharides, more especially xanthan gum, guar-guar, agar-agar, alginates and tyloses, carboxymethyl cellulose and hydroxyethyl cellulose, also relatively high molecular weight polyethylene glycol monoesters and diesters of fatty acids, polyacrylates (for example Carbopols^{®} [Goodrich] or Synthalens^{®} [Sigma]), polyacrylamides, polyvinyl alcohol and polyvinyl pyrrolidone, surfactants such as, for example, ethoxylated fatty acid glycerides, esters of fatty acids with polyols, for example pentaerythritol or trimethylol propane, narrowrange fatty alcohol ethoxylates and electrolytes, such as sodium chloride and ammonium chloride.

**Perfume oils and/or fragrances:** The cosmetic or pharmaceutical composition according to the present invention preferably includes one or more perfume oils and/or fragrances. Suitable perfume oils are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert. butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, beta-isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

**Anti-cellulite agent:** The cosmetic or pharmaceutical composition according to the present invention preferably includes one or more anti-cellulite agents. Anti-cellulite agents and lipolytic agents are preferably selected from the group consisting of beta-adrenergic receptor agonists such as synephrine and its derivatives, and cyclohexyl carbamates. Agents enhancing or boosting the activity of anti-cellulite agents, in particular agents which stimulate and/or depolarise C nerve fibres, are preferably selected from the group consisting of capsaicin and derivatives thereof, vanillyl-nonylamid and derivatives thereof, L-carnitine, coenzym A, isoflavonoides, soy extracts, ananas extract and conjugated linoleic acid.

**Fat enhancing agents:** The cosmetic or pharmaceutical composition according to the present invention preferably includes one or more fat enhancing agents and/or adipogenic agents as well as agents enhancing or boosting the activity of fat enhancing agents. A fat enhancing agent is for example hydroxymethoxyphenyl propylmethylmethoxybenzofuran (trade name: Sym3D^{®}).

**Superfatting agents and/or consistency factors:** The cosmetic or pharmaceutical composition according to the present invention preferably includes one or more superfatting agents and/or consistency factors. Superfatting agents may be selected from such substances as, for example, lanolin and lecithin and also polyethoxylated or acylated lanolin and lecithin derivatives, polyol fatty acid esters, monoglycerides and fatty acid alkanolamides, the fatty acid alkanolamides also serving as foam stabilizers. The consistency factors mainly used are fatty alcohols or hydroxyfatty alcohols containing 12 to 22 and preferably 16 to 18 carbon atoms and also partial glycerides, fatty acids or hydroxyfatty acids. A combination of these substances with alkyl oligoglucosides and/or fatty acid N-methyl glucamides of the same chain length and/or polyglycerol poly-12-hydroxystearates is preferably used.

**Pearlizing waxes:** The cosmetic or pharmaceutical composition according to the present invention preferably includes one or more pearlizing waxes. Suitable pearlising waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxysubstituted carboxylic acids with fatty alcohols containing 6 to 22 carbon atoms, especially long-chain esters of tartaric acid; fatty compounds, such as for example fatty alcohols, fatty ketones, fatty aldehydes, fatty ethers and fatty carbonates which contain in all at least 24 carbon atoms, especially laurone and distearylether; fatty acids, such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups, and mixtures thereof.

**Silicones:** In order to impart a silky, spreadable, and luxurious texture and to make skin look and feel smoother, and additionally to improve processability (antifoaming) the cosmetic or pharmaceutical composition according to the present invention preferably includes one or more silicone(s) or silicone derative(s). Suitable silicones can be chosen from the group consisting of Acefylline Methylsilanol Mannuronate, Acetylmethionyl Methylsilanol Elastinate Acrylates/Behenyl, Acrylate/Dimethicone Methacrylate Copolymer, Acrylates/Behenyl Methacrylate/Dimethicone Methacrylate Copolymer, Acrylates/Bis-Hydroxypropyl Dimethicone Crosspolymer, Acrylates/Dimethicone Copolymer, Acrylates/Dimethicone Methacrylate/Ethylhexyl Acrylate Copolymer, Acrylates/Dimethiconol Acrylate Copolymer, Acrylates/Ethylhexyl Acrylate/Dimethicone Methacrylate Copolymer, Acrylates/Octylacrylamide/Diphenyl Amodimethicone Copolymer, Acrylates/Polytrimethylsiloxymethacrylate Copolymer, Acrylates/Propyl Trimethicone Methacrylate Copolymer, Acrylates/Stearyl Acrylate/Dimethicone Methacrylate Copolymer, Acrylates/Tridecyl Acrylate/Triethoxysilylpropyl Methacrylate/Dimethicone Methacrylate Copolymer, Acrylates/Trifluoropropylmethacrylate/Polytrimethyl Siloxymethacrylate Copolymer, Amino Bispropyl Dimethicone, Aminoethylaminopropyl Dimethicone, Aminopropyl Dimethicone, Aminopropyl Phenyl Trimethicone, Aminopropyl Triethoxysilane, Ammonium Dimethicone PEG-7 Sulfate, Amodimethicone, Amodimethicone Hydroxystearate, Amodimethicone/Silsesquioxane Copolymer, Ascorbyl Carboxydecyl Trisiloxane, Ascorbyl Methylsilanol Pectinate, Behenoxy Dimethicone, Behentrimonium Dimethicone PEG-8 Phthalate, Behenyl Dimethicone, Bisamino PEG/PPG-41/3 Aminoethyl PG-Propyl Dimethicone, Bis-Aminopropyl/Ethoxy Aminopropyl Dimethicone, Bis(Butylbenzoate) Diaminotriazine Aminopropyltrisiloxane, Bis-Butyldimethicone Polyglyceryl-3, Bis-Butyloxyamodimethicone/PEG-60 Copolymer, Bis(C13-15 Alkoxy) Hydroxybutamidoamodimethicone, Bis(C13-15 Alkoxy) PG- Amodimethicone, Bis-(C1-8 Alkyl Lauroyl Lysine Decylcarboxamide) Dimethicone, Bis-Cetyl Cetyl Dimethicone, Bis-Cetyl/PEG-8 Cetyl PEG-8 Dimethicone, Bis-Diphenylethyl Disiloxane, Bis-Ethyl Ethyl Methicone, Bis-Gluconamidoethylaminopropyl Dimethicone, Bis-Hydrogen Dimethicone, Bis- Hydroxyethoxypropyl Dimethicone Bis-Hydroxylauryl, Dimethicone/IPDI Copolymer, Bis- Hydroxy/Methoxy Amodimethicone, Bis-Hydroxypropyl Dimethicone Behenate, Bis-Hydroxypropyl Dimethicone/SMDI Copolymer, Bis-Isobutyl PEG-14/Amodimethicone Copolymer, Bis-Isobutyl PEG-15/Amodimethicone Copolymer, Bis-Isobutyl PEG/PPG-20/35/Amodimethicone Copolymer, Bis- Isobutyl PEG/PPG-10/7/Dimethicone Copolymer, Bis-Isobutyl PEG-24/PPG-7/Dimethicone Copolymer, Bis-PEG-1 Dimethicone, Bis-PEG-4 Dimethicone, Bis-PEG-8 Dimethicone, Bis-PEG-12 Dimethicone,Bis-PEG-20 Dimethicone, Bis-PEG-12 Dimethicone Beeswax, Bis-PEG-12 Dimethicone Candelillate, Bis-PEG-15 Dimethicone/IPDI Copolymer, Bis-PEG-15 Methyl Ether Dimethicone, Bis- PEG-18 Methyl Ether Dimethyl Silane, Bis-PEG/PPG-14/14 Dimethicone, Bis-PEG/PPG-15/5 Dimethicone, Bis-PEG/PPG-18/6 Dimethicone, Bis-PEG/PPG-20/20 Dimethicone, Bis-PEG/PPG- 16/16 PEG/PPG-16/16 Dimethicone, Bis-PEG/PPG-20/5 PEG/PPG-20/5 Dimethicone, Bisphenylhexamethicone, Bis-Phenylpropyl Dimethicone, Bispolyethylene Dimethicone, Bis- (Polyglyceryl-3 Oxyphenylpropyl) Dimethicone, Bis-(Polyglyceryl-7 Oxyphenylpropyl) Dimethicone, Bis-PPG-15 Dimethicone/IPDI Copolymer, Bis(PPG-7 Undeceneth-21) Dimethicone, Bis-Stearyl Dimethicone, Bis-Trimethoxysilylethyl Tetramethyldisiloxyethyl Dimethicone, Bis-Vinyldimethicone, Bis-Vinyl Dimethicone/Dimethicone Copolymer, Borage Seed Oil PEG-7 Dimethicone Esters, Butyl Acrylate/C6-14 Perfluoroalkylethyl Acrylate/Mercaptopropyl Dimethicone Copolymer, Butyl Acrylate/Hydroxypropyl Dimethicone Acrylate Copolymer, Butyl Dimethicone Acrylate/Cyclohexylmethacrylate/Ethylhexyl Acrylate Copolymer, Butyldimethicone Methacrylate/Methyl Methacrylate Crosspolymer, t-Butyl Dimethyl Silyl Grape Seed Extract, Butyl Polydimethylsiloxyl Ethylene/Propylene/Vinylnorbornene Copolymer, C6-8 Alkyl C3-6 Alkyl Glucoside Dimethicone, C20-24 Alkyl Dimethicone,C24-28 Alkyl Dimethicone, C26-28 Alkyl Dimethicone, C30-45 Alkyl Dimethicone, C30-60 Alkyl Dimethicone, C32 Alkyl Dimethicone, C30-45 Alkyl Dimethicone/Polycyclohexene Oxide Crosspolymer, C26-28 Alkyldimethylsilyl Polypropylsilsesquioxane, C30-45 Alkyldimethylsilyl Polypropylsilsesquioxane, C20-24 Alkyl Methicone, C24-28 Alkyl Methicone, C26-28 Alkyl Methicone, C30-45 Alkyl Methicone, C20-28 Alkyl Perfluorodecylethoxy Dimethicone, C26-54 Alkyl Tetradecyl Dimethicone, Capryl Dimethicone, Caprylyl Dimethicone Ethoxy Glucoside, Caprylyl Methicone, Caprylyl Trimethicone, Carboxydecyl Trisiloxane, Castor Oil Bis-Hydroxypropyl Dimethicone Esters Cerotyl Dimethicone, Cetearyl Dimethicone Crosspolymer, Cetearyl Dimethicone/Vinyl Dimethicone Crosspolymer, Cetearyl Methicone, Cetrimonium Carboxydecyl PEG-8 Dimethicone, Cetrimonium Dimethicone PEG-7 Phthalate, Cetyl Behenyl Dimethicone, Cetyl Dimethicone, Cetyl Dimethicone/Bis-Vinyldimethicone Crosspolymer, Cetyl Hexacosyl Dimethicone, Cetyloxy Dimethicone, Cetyl PEG-8 Dimethicone, Cetyl PEG/PPG-15/15 Butyl Ether Dimethicone, Cetyl PEG/PPG-7/3 Dimethicone, Cetyl PEG/PPG-10/1 Dimethicone, Cetyl Triethylmonium Dimethicone PEG-8 Phthalate, Cetyl Triethylmonium Dimethicone PEG-8 Succinate, Copper Acetyl Tyrosinate Methylsilanol, Copper PCA Methylsilanol, C4-14 Perfluoroalkylethoxy Dimethicone, Cycloethoxymethicone, Cycloheptasiloxane, Cyclohexasiloxane, Cyclomethicone, Cyclopentasiloxane, Cyclophenylmethicone, Cyclotetrasiloxane,mCyclovinylmethicone, Cystine Bis-PG-Propyl Silanetriol, DEA PG-Propyl PEG/PPG-18/21 Dimethicone, Diisostearoyl Trimethylolpropane Siloxy Silicate, Dilauroyl Trimethylolpropane Siloxy Silicate, Dilinoleamidopropyl Dimethylamine Dimethicone PEG-7 Phosphate, Dimethicone, Dimethicone Crosspolymer, Dimethicone Crosspolymer-3, Dimethicone/Divinyldimethicone/Silsesquioxane Crosspolymer, Dimethicone Ethoxy Glucoside, Dimethicone Hydroxypropyl Trimonium Chloride, Dimethicone/Mercaptopropyl Methicone Copolymer, Dimethicone PEG-15 Acetate Dimethicone PEG-8 Adipate, Dimethicone PEG-7 Avocadoate, Dimethicone PEG-8 Avocadoate, Dimethicone PEG-8 Beeswax, Dimethicone PEG-8 Benzoate, Dimethicone PEG-8 Borageate, Dimethicone PEG-7 Cocoate, Dimethicone/PEG-10 Crosspolymer, Dimethicone/PEG-10/15 Crosspolymer, Dimethicone/PEG-15 Crosspolymer, Dimethicone PEG-7 Isostearate, Dimethicone PEG-8 Isostearate, Dimethicone PEG-7 Lactate, Dimethicone PEG-8 Lanolate, Dimethicone PEG-8 Laurate, Dimethicone PEG-8 Meadowfoamate, Dimethicone PEG-7 Octyldodecyl Citrate, Dimethicone PEG-7 Olivate, Dimethicone PEG-8 Olivate, Dimethicone PEG-7 Phosphate, Dimethicone PEG-8 Phosphate, Dimethicone PEG-10 Phosphate, Dimethicone PEG-7 Phthalate, Dimethicone PEG-8 Phthalate, Dimethicone PEG-8 Polyacrylate, Dimethicone PEG/PPG- 20/23 Benzoate, Dimethicone PEG/PPG-7/4 Phosphate, Dimethicone PEG/PPG-12/4 Phosphate, Dimethicone PEG-7 Succinate, Dimethicone PEG-8 Succinate, Dimethicone PEG-7 Sulfate, Dimethicone PEG-7 Undecylenate, Dimethicone PG-Diethylmonium Chloride, Dimethicone/Phenyl Vinyl Dimethicone Crosspolymer, Dimethicone/Polyglycerin-3 Crosspolymer, Dimethicone/PPG-20 Crosspolymer, Dimethicone Propylethylenediamine Behenate, Dimethicone Propyl PG-Betaine, Dimethicone/Silsesquioxane Copolymer, Dimethicone Silylate, Dimethicone/Vinyl Dimethicone Crosspolymer, Dimethicone/Vinyltrimethylsiloxysilicate Crosspolymer, Dimethiconol, Dimethiconol Arginine, Dimethiconol Beeswax, Dimethiconol Behenate, Dimethiconol Borageate, Dimethiconol Candelillate, Dimethiconol Carnaubate, Dimethiconol Cysteine, Dimethiconol Dhupa Butterate, Dimethiconol Fluoroalcohol Dilinoleic Acid, Dimethiconol Hydroxystearate, Dimethiconol IIIipe Butterate, Dimethiconol/IPDI Copolymer, Dimethiconol Isostearate, Dimethiconol Kokum Butterate, Dimethiconol Lactate, Dimethiconol Meadowfoamate, Dimethiconol Methionine, Dimethiconol/Methylsilanol/Silicate Crosspolymer, Dimethiconol Mohwa Butterate, Dimethiconol Panthenol, Dimethiconol Sal Butterate, Dimethiconol/Silica Crosspolymer, Dimethiconol/Silsesquioxane Copolymer, Dimethiconol Stearate, Dimethiconol/Stearyl, Methicone/Phenyl Trimethicone Copolymer, Dimethoxysilyl Ethylenediaminopropyl Dimethicone, Dimethylaminopropylamido PCA Dimethicone, Dimethyl Oxobenzo Dioxasilane, Dimethylsilanol Hyaluronate, Dioleyl Tocopheryl Methylsilanol, Diphenyl Amodimethicone, Diphenyl Dimethicone, Diphenyl Dimethicone Crosspolymer Diphenyl Dimethicone?/inyl Diphenyl Dimethicone/Silsesquioxane Crosspolymer, Diphenylethyl Benzyloxy Dilsiloxane, Diphenylisopropyl Dimethicone, Diphenylsiloxy Phenyl/Propyl Trimethicone, Diphenylsiloxy Phenyl Trimethicone Disiloxane, Disodium Amodimethicone Disuccinamide, Disodium PEG-12 Dimethicone Sulfosuccinate, Disodium PEG-8 Lauryl Dimethicone Sulfosuccinate, Divinyldimethicone/Dimethicone Copolymer, Divinyldimethicone/Dimethicone Crosspolymer, Drometrizole Trisiloxane, Ethylhexyl Acrylate/VP/Dimethicone Methacrylate Copolymer, Ethyl Methicone, Ethyl Trisiloxane, Fluoro C2-8 Alkyldimethicone, Gluconamidopropyl Aminopropyl Dimethicone, 4-(2-Beta-Glucopyranosiloxy) Propoxy-2-Hydroxybenzophenone, Glyceryl Undecyl Dimethicone, Glycidoxy Dimethicone, Hexadecyl Methicone, Hexyl Dimethicone, Hexyl Methicone, Hexyltrimethoxysilane, Hydrogen Dimethicone, Hydrogen Dimethicone/Octyl Silsesquioxane Copolymer, Hydrolyzed Collagen PG-Propyl Dimethiconol, Hydrolyzed Collagen PG-Propyl Methylsilanediol, Hydrolyzed Collagen PG-Propyl Silanetriol, Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Silk PG-Propyl Methylsilanediol Crosspolymer, Hydrolyzed Soy Protein/Dimethicone PEG-7 Acetate, Hydrolyzed Soy Protein PG-Propyl Methylsilanediol, Hydrolyzed Vegetable Protein PG-Propyl Silanetriol, Hydrolyzed Wheat Protein/Cystine Bis-PG-Propyl Silanetriol Copolymer, Hydrolyzed Wheat Protein/Dimethicone PEG-7 Acetate, Hydrolyzed Wheat Protein/Dimethicone PEG-7 Phosphate Copolymer, Hydrolyzed Wheat Protein PG-Propyl Methylsilanediol, Hydrolyzed Wheat Protein PG-Propyl Silanetriol, Hydroxyethyl Acetomonium PG-Dimethicone, Hydroxypropyldimethicone, Hydroxypropyl Dimethicone Behenate, Hydroxypropyl Dimethicone Isostearate, Hydroxypropyl Dimethicone Stearate, Isobutylmethacrylate/Bis-Hydroxypropyl Dimethicone Acrylate Copolymer, Isobutylmethacrylate/Trifluoroethylmethacrylate/Bis-Hydroxypropyl Dimethicone Acrylate Copolymer, Isopentyl Trimethoxycinnamate Trisiloxane, Isopolyglyceryl-3 Dimethicone, Isopolyglyceryl-3 Dimethiconol, Isopropyl Titanium Triisostearate/Triethoxysilylethyl, Polydimethylsiloxyethyl Dimethicone Crosspolymer, Isostearyl Carboxydecyl PEG-8 Dimethicone, Lactoyl Methylsilanol Elastinate, Lauryl Dimethicone, Lauryl Dimethicone PEG-15 Crosspolymer, Lauryl Dimethicone PEG- 10 Phosphate, Lauryl Dimethicone/Polyglycerin-3 Crosspolymer, Lauryl Methicone, Lauryl PEG-8 Dimethicone, Lauryl PEG-10 Methyl Ether Dimethicone, Lauryl PEG-9 Polydimethylsiloxyethyl Dimethicone, Lauryl PEG/PPG-18/18 Methicone, Lauryl Phenylisopropyl Methicone, Lauryl Phenylpropyl Methicone, Lauryl Polydimethylsiloxyethyl Dimethicone/Bis-Vinyldimethicone Crosspolymer, Lauryl Polyglyceryl-3 Polydimethylsiloxyethyl Dimethicone, Lauryl Trimethicone, Linoleamidopropyl PG-Dimonium Chloride Phosphate Dimethicone, Methacryloyl Propyltrimethoxysilane, Methicone, Methoxy Amodimethicone/Silsesquioxane Copolymer, Methoxycinnamidopropyl Polysilsesquioxane, Methoxycinnamoylpropyl Silsesquioxane Silicate, Methoxy PEG-13 Ethyl Polysilsesquioxane, Methoxy PEG/PPG-7/3 Aminopropyl Dimethicone, Methoxy PEG/PPG-25/4 Dimethicone, Methoxy PEG-10 Propyltrimethoxysilane, Methyleugenyl PEG-8 Dimethicone, Methylpolysiloxane Emulsion, Methylsilanol Acetylmethionate, Methylsilanol Acetyltyrosine, Methylsilanol Ascorbate, Methylsilanol Carboxymethyl Theophylline, Methylsilanol Carboxymethyl Theophylline Alginate, Methylsilanol Elastinate, Methylsilanol Glycyrrhizinate, Methylsilanol Hydroxyproline, Methylsilanol Hydroxyproline Aspartate, Methylsilanol Mannuronate, Methylsilanol PCA, Methylsilanol PEG-7 Glyceryl Cocoate, Methylsilanol/Silicate Crosspolymer, Methylsilanol Spirulinate, Methylsilanol Tri-PEG-8 Glyceryl Cocoate, Methyl Trimethicone, Methyltrimethoxysilane, Myristylamidopropyl Dimethylamine Dimethicone PEG-7 Phosphate, Myristyl Methicone, Myristyl Trisiloxane, Nylon-611/Dimethicone Copolymer, PCA Dimethicone, PEG-7 Amodimethicone, PEG-8 Amodimethicone, PEG-8 Cetyl Dimethicone, PEG-3 Dimethicone, PEG-6 Dimethicone, PEG-7 Dimethicone, PEG-8 Dimethicone, PEG-9 Dimethicone, PEG-10 Dimethicone, PEG-12 Dimethicone, PEG-14 Dimethicone, PEG-17 Dimethicone, PEG-10 Dimethicone Crosspolymer, PEG-12 Dimethicone Crosspolymer, PEG-8 Dimethicone Dimer Dilinoleate, PEG-8 Dimethicone/Dimer Dilinoleic Acid Copolymer, PEG-10 Dimethicone/Vinyl Dimethicone Crosspolymer, PEG-8 Distearmonium Chloride PG-Dimethicone, PEG-10/Lauryl Dimethicone Crosspolymer, PEG- 15/Lauryl Dimethicone Crosspolymer, PEG-15/Lauryl Polydimethylsiloxyethyl Dimethicone Crosspolymer, PEG-8 Methicone, PEG-6 Methicone Acetate, PEG-6 Methyl Ether Dimethicone, PEG- 7 Methyl Ether Dimethicone, PEG-8 Methyl Ether Dimethicone, PEG-9 Methyl Ether Dimethicone, PEG-10 Methyl Ether Dimethicone, PEG-11 Methyl Ether Dimethicone, PEG-32 Methyl Ether Dimethicone, PEG-8 Methyl Ether Triethoxysilane, PEG-10 Nonafluorohexyl Dimethicone Copolymer, PEG-4 PEG-12 Dimethicone, PEG-8 PG-Coco-Glucoside Dimethicone, PEG-9 Polydimethylsiloxyethyl Dimethicone, PEG/PPG-20/22 Butyl Ether Dimethicone, PEG/PPG-22/22 Butyl Ether Dimethicone, PEG/PPG-23/23 Butyl Ether Dimethicone, PEG/PPG-24/18 Butyl Ether Dimethicone, PEG/PPG-27/9 Butyl Ether Dimethicone, PEG/PPG-3/10 Dimethicone, PEG/PPG-4/12 Dimethicone, PEG/PPG-6/4 Dimethicone, PEG/PPG-6/11 Dimethicone, PEG/PPG-8/14 Dimethicone, PEG/PPG-8/26 Dimethicone, PEG/PPG-10/2 Dimethicone, PEG/PPG-12/16 Dimethicone, PEG/PPG- 12/18 Dimethicone, PEG/PPG-14/4 Dimethicone, PEG/PPG-15/5 Dimethicone, PEG/PPG-15/15 Dimethicone, PEG/PPG-16/2 Dimethicone, PEG/PPG-16/8 Dimethicone, PEG/PPG-17/18 Dimethicone, PEG/PPG-18/6 Dimethicone, PEG/PPG-18/12 Dimethicone, PEG/PPG-18/18 Dimethicone, PEG/PPG-19/19 Dimethicone, PEG/PPG-20/6 Dimethicone, PEG/PPG-20/15 Dimethicone, PEG/PPG-20/20 Dimethicone, PEG/PPG-20/23 Dimethicone, PEG/PPG-20/29 Dimethicone, PEG/PPG-22/23 Dimethicone, PEG/PPG-22/24 Dimethicone, PEG/PPG-23/6 Dimethicone, PEG/PPG-25/25 Dimethicone, PEG/PPG-27/27 Dimethicone, PEG/PPG-30/10 Dimethicone, PEG/PPG-25/25 Dimethicone/Acrylates Copolymer, PEG/PPG-20/22 Methyl Ether Dimethicone, PEG/PPG-24/24 Methyl Ether Glycidoxy Dimethicone, PEG/PPG-10/3 Oleyl Ether Dimethicone, PEG/PPG-5/3 Trisiloxane, PEG-4 Trifluoropropyl Dimethicone Copolymer, PEG-8 Trifluoropropyl Dimethicone Copolymer, PEG-10 Trifluoropropyl Dimethicone Copolymer, PEG-8 Trisiloxane, Perfluorocaprylyl riethoxysilylethyl Methicone, Perfluorononyl Dimethicone, Perfluorononyl Dimethicone/Methicone/Amodimethicone Crosspolymer, Perfluorononylethyl Carboxydecyl Behenyl Dimethicone, Perfluorononylethyl Carboxydecyl Hexacosyl Dimethicone, Perfluorononylethyl Carboxydecyl Lauryl/Behenyl Dimethicone, Perfluorononylethyl Carboxydecyl Lauryl Dimethicone, Perfluorononylethyl Carboxydecyl PEG-8 Dimethicone, Perfluorononylethyl Carboxydecyl PEG-10 Dimethicone, Perfluorononylethyl Dimethicone/Methicone Copolymer, Perfluorononylethyl PEG-8 Dimethicone, Perfluorononylethyl Stearyl Dimethicone, Perfluorooctylethyl/Diphenyl Dimethicone Copolymer, Perfluorooctylethyl Triethoxysilane, Perfluorooctylethyl Trimethoxysilane, Perfluorooctylethyl Trisiloxane, Perfluorooctyl Triethoxysilane, PG-Amodimethicone, Phenethyl Dimethicone, Phenethyl Disiloxane, Phenyl Dimethicone, Phenylisopropyl Dimethicone, Phenyl Methicone, Phenyl Methiconol, Phenylpropyldimethylsiloxysilicate, Phenylpropyl Ethyl Methicone, Phenyl Propyl Trimethicone, Phenyl Propyl Trimethicone/Diphenylmethicone, Phenyl Trimethicone, Platinum Divinyldisiloxane, Polyacrylate-6, Polydiethylsiloxane, Polydimethylsiloxyethyl Dimethicone/Bis-Vinyldimethicone Crosspolymer, Polydimethylsiloxyethyl Dimethicone/Methicone Copolymer, Polydimethylsiloxy PEG/PPG-24/19 Butyl Ether Silsesquioxane, Polydimethylsiloxy PPG- 13 Butyl Ether Silsesquioxane, Polyglyceryl-3 Disiloxane Dimethicone, Polyglyceryl-3/Lauryl Polydimethylsiloxyethyl Dimethicone Crosspolymer, Polyglyceryl-3 Polydimethylsiloxyethyl Dimethicone, Poly(Glycol Adipate)/Bis-Hydroxyethoxypropyl Dimethicone Copolymer, Polymethylsilsesquioxane, Polymethylsilsesquioxane/Trimethylsiloxysilicate, Polyphenylsilsesquioxane, Polypropylsilsesquioxane, Polysilicone-1, Polysilicone-2, Polysilicone-3, Polysilicone-4, Polysilicone-5, Polysilicone-6, Polysilicone-7, Polysilicone-8, Polysilicone-9, Polysilicone-10, Polysilicone-11, Polysilicone-12, Polysilicone-13, Polysilicone-14, Polysilicone-15, Polysilicone-16, Polysilicone-17, Polysilicone-18, Polysilicone-19, Polysilicone-20, Polysilicone-21, Polysilicone-18 Cetyl Phosphate, Polysilicone-1 Crosspolymer, Polysilicone-18 Stearate, Polyurethane-10, Potassium Dimethicone PEG-7 Panthenyl Phosphate, Potassium Dimethicone PEG- 7 Phosphate, PPG-12 Butyl Ether Dimethicone, PPG-2 Dimethicone, PPG-12 Dimethicone, PPG-27 Dimethicone, PPG-4 Oleth-10 Dimethicone, Propoxytetramethyl Piperidinyl Dimethicone, Propyl Trimethicone, Quaternium-80, Retinoxytrimethylsilane, Silanediol Salicylate, Silanetriol, Silanetriol Arginate, Silanetriol Glutamate, Silanetriol Lysinate, Silanetriol Melaninate, Silanetriol Trehalose Ether, Silica, Silica Dimethicone Silylate, Silica Dimethyl Silylate, Silica Silylate, Silicon Carbide, Silicone Quaternium-1, Silicone Quaternium-2, Silicone Quaternium-2 Panthenol Succinate, Silicone Quaternium-3, Silicone Quaternium-4, Silicone Quaternium-5, Silicone Quaternium-6, Silicone Quaternium-7, Silicone Quaternium-8, Silicone Quaternium-9, Silicone Quaternium-10, Silicone Quaternium-11, Silicone Quaternium-12, Silicone Quaternium-15, SiliconeQuaternium-16, Silicone Quaternium-16/Glycidoxy Dimethicone Crosspolymer, Silicone Quaternium-17, Silicone Quaternium- 18, Silicone Quaternium-19, Silicone Quaternium-20, Silicone Quaternium-21, Silicone Quaternium- 22, Silicone Quaternium-24, Silicone Quaternium-25, Siloxanetriol Alginate, Siloxanetriol Phytate, Simethicone, Sodium Carboxydecyl PEG-8 Dimethicone, Sodium Dimethicone PEG-7 Acetyl Methyltaurate, Sodium Hyaluronate Dimethylsilanol, Sodium Lactate Methylsilanol, Sodium Mannuronate Methylsilanol, Sodium PCA Methylsilanol, Sodium PG-Propyldimethicone Thiosulfate Copolymer, Sodium PG-Propyl Thiosulfate Dimethicone, Sodium Propoxyhydroxypropyl Thiosulfate Silica, Sorbityl Silanediol, Soy Triethoxysilylpropyldimonium Chloride, Stearalkonium Dimethicone PEG-8 Phthalate, Stearamidopropyl Dimethicone, Steardimonium Hydroxypropyl Panthenyl PEG-7 Dimethicone Phosphate Chloride, Steardimonium Hydroxypropyl PEG-7 Dimethicone Phosphate Chloride, Stearoxy Dimethicone, Stearoxymethicone/Dimethicone Copolymer, Stearoxytrimethylsilane, Stearyl Aminopropyl Methicone, Stearyl Dimethicone, Stearyl/Lauryl Methacrylate Crosspolymer, Stearyl Methicone, Stearyl Triethoxysilanek, Stearyl Trimethicone, Styrene/Acrylates/Dimethicone Acrylate Crosspolymer, Styrene/Acrylates/Dimethicone Copolymer, TEA-Dimethicone PEG-7 Phosphate, Tetrabutoxypropyl Trisiloxane, Tetramethyl Hexaphenyl Tetrasiloxane, Tetramethyl Tetraphenyl Trisiloxane, Tocopheryloxypropyl Trisiloxane, Trideceth-9 PG-Amodimethicone, Triethoxycaprylylsilane, Triethoxysilylethyl Dimethicone/Methicone Copolymer, Triethoxysilylethyl Polydimethylsiloxyethyl Dimethicone, Triethoxysilylethyl Polydimethylsiloxyethyl Hexyl Dimethicone, Triethoxysilylpropylcarbamoyl Ethoxypropyl Butyl Dimethicone, Trifluoromethyl C1-4 Alkyl Dimethicone, Trifluoropropyl Cyclopentasiloxane, Trifluoropropyl Cyclotetrasiloxane, Trifluoropropyl Dimethicone, Trifluoropropyl Dimethicone/PEG-10 Crosspolymer, Trifluoropropyl Dimethicone/Trifluoropropyl Divinyldimethicone Crosspolymer, Trifluoropropyl Dimethicone/Vinyl Trifluoropropyl, Dimethicone/Silsesquioxane Crosspolymer, Trifluoropropyl Dimethiconol, Trifluoropropyldimethyl/trimethylsiloxysilicate, Trifluoropropyl Methicone, Trimethoxycaprylylsilane, Trimethoxysilyl Dimethicone, Trimethyl Pentaphenyl Trisiloxane, Trimethylsiloxyamodimethicone, Trimethylsiloxyphenyl Dimethicone, Trimethylsiloxysilicate, Trimethylsiloxysilicate/Dimethicone Crosspolymer, Trimethylsiloxysilicate/Dimethiconol Crosspolymer, Trimethylsiloxysilylcarbamoyl Pullulan, Trimethylsilyl Hydrolyzed Conchiolin Protein PG-Propyl Methylsilanediol Crosspolymer, Trimethylsilyl Hydrolyzed Silk PG-Propyl Methylsilanediol Crosspolymer, Trimethylsilyl Hydrolyzed Wheat Protein PG-Propyl Methylsilanediol Crosspolymer, Trimethylsilyl Pullulan, Trimethylsilyl Trimethylsiloxy Glycolate, Trimethylsilyl Trimethylsiloxy Lactate, Trimethylsilyl Trimethylsiloxy Salicylate, Triphenyl Trimethicone, Trisiloxane, Tris-Tributoxysiloxymethylsilane, Undecylcrylene Dimethicone, Vinyl Dimethicone, Vinyl Dimethicone/Lauryl Dimethicone Crosspolymer, Vinyl Dimethicone/Methicone Silsesquioxane Crosspolymer, Vinyldimethyl/Trimethylsiloxysilicate Stearyl Dimethicone Crosspolymer, VP/Dimethiconylacrylate/Polycarbamyl/Polyglycol Ester, Zinc Carboxydecyl Trisiloxane, Zinc Dimethicone PEG-8 Succinate, and mixtures thereof.

More preferably the silicones to be contained in the cosmetic or pharmaceutical composition according to the invention are Dimethicone, Cyclomethicone, Cyclopentasiloxane, Cyclotetrasiloxane, Phenyl Trimethicone, and Cyclohexasiloxane.

**Waxes and/or stabilizers:** Besides natural oils used, one or more waxes may also be present in the cosmetic or pharmaceutical composition according to the present invention, more especially natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes and microwaxes; chemically modified waxes (hard waxes) such as, for example, montan ester waxes, sasol waxes, hydrogenated jojoba waxes and synthetic waxes such as, for example, polyalkylene waxes and polyethylene glycol waxes. Metal salts of fatty acids such as, for example, magnesium, aluminium and/or zinc stearate or ricinoleate may be used as stabilizers.

Apart from the abovementioned liquid UV filters, the cosmetic or pharmaceutical composition as defined herein, is advantageously combined with at least one further primary sun protection factor and/or with at least one further secondary sun protection factor, in order to increase the SPF, i.e. to obtain a high SPF and to cover a broad UVA and UVB range.

**Primary sun protection factors:** Primary sun protection factors in the context of the invention are, for example, organic substances (light filters) which are liquid or crystalline at room temperature and which are capable of absorbing ultraviolet radiation and of releasing the energy absorbed in the form of longer-wave radiation, for example heat.

The cosmetic or pharmaceutical composition according to the invention advantageously contains at least one UV-A filter and/or at least one UV-B filter and/or a broadband filter and/or at least one inorganic pigment. Compositions according to the invention preferably contain at least one UV-B filter or a broadband filter, more particularly preferably at least one UV-A filter and at least one UV-B filter. Preferred cosmetic compositions, preferably topical compositions according to the present invention comprise one, two, three or more sun protection factors selected from the group consisting of 4-aminobenzoic acid and derivatives, salicylic acid derivatives, benzophenone derivatives, dibenzoylmethane derivatives, diphenyl acrylates, 3-imidazol-4-yl acrylic acid and esters thereof, benzofuran derivatives, benzylidene malonate derivatives, polymeric UV absorbers containing one or more organosilicon radicals, cinnamic acid derivatives, camphor derivatives, trianilino-s-triazine derivatives, 2-hydroxyphenylbenzotriazole derivatives, phenylbenzimidazole sulfonic acid derivatives and salts thereof, anthranilic acid menthyl esters, benzotriazole derivatives and indole derivatives.

In addition, a combination with active ingredients which penetrate into the skin and protect the skin cells from inside against sunlight-induced damage and reduce the level of cutaneous matrix metalloproteases is advantageously. Preferred respective ingredients are so called arylhydrocarbon receptor antagonists. Preferred is 2-benzylidene-5,6-dimethoxy-3,3-dimethylindan-1-one.

The UV filters cited below which can be used within the context of the present invention are preferred but naturally are not limiting.

UV filters which are preferably used are selected from the group consisting of
- p-aminobenzoic acid
- p-aminobenzoic acid ethyl ester (25 mol) ethoxylated (INCI name: PEG-25 PABA)
- p-dimethylaminobenzoic acid-2-ethylhexyl ester
- p-aminobenzoic acid ethyl ester (2 mol) N-propoxylated
- p-aminobenzoic acid glycerol ester
- salicylic acid homomenthyl ester (homosalates) (Neo Heliopan^{®}HMS)
- salicylic acid-2-ethylhexyl ester (Neo Heliopan^{®}OS)
- triethanolamine salicylate
- 4-isopropyl benzyl salicylate
- anthranilic acid menthyl ester (Neo Heliopan^{®}MA)
- diisopropyl cinnamic acid ethyl ester
- p-methoxycinnamic acid-2-ethylhexyl ester (Neo Heliopan^{®}AV)
- diisopropyl cinnamic acid methyl ester
- p-methoxycinnamic acid isoamyl ester (Neo Heliopan^{®}E 1000)
- p-methoxycinnamic acid diethanolamine salt
- p-methoxycinnamic acid isopropyl ester
- 2-phenylbenzimidazole sulfonic acid and salts (Neo Heliopan^{®}Hydro)
- 3-(4'-trimethylammonium) benzylidene bornan-2-one methyl sulfate
- beta-imidazole-4(5)-acrylic acid (urocanic acid)
- 3-(4'-sulfo)benzylidene bornan-2-one and salts
- 3-(4'-methyl benzylidene)-D,L-camphor (Neo Heliopan^{®}MBC)
- 3-benzylidene-D,L-camphor
- N-[(2 and 4)-[2-(oxoborn-3-ylidene) methyl]benzyl] acrylamide polymer
- 4,4'-[(6-[4-(1,1-dimethyl)aminocarbonyl) phenylamino]-1,3,5-triazine-2,4-diyl)diimino]-bis-(benzoic acid-2-ethylhexyl ester) (Uvasorb^{®}HEB)
- benzylidene malonate polysiloxane (Parsol^{®}SLX)
- glyceryl ethylhexanoate dimethoxycinnamate
- dipropylene glycol salicylate
- tris(2-ethylhexyl)-4,4',4"-(1,3,5-triazine-2,4,6-triyltriimino)tribenzoate (= 2,4,6-trianilino-(p-carbo-2'-ethylhexyl-1'-oxy)-1,3,5-triazine) (Uvinul^{®}T150).

In a preferred variant the cosmetic or pharmaceutical composition according to the present invention comprises a combination with one or more broadband filters which are selected from the group consisting of
- 2-ethylhexyl-2-cyano-3,3-diphenyl acrylate (Neo Heliopan^{®}303)
- ethyl-2-cyano-3,3'-diphenyl acrylate
- 2-hydroxy-4-methoxybenzophenone (Neo Heliopan^{®}BB)
- 2-hydroxy-4-methoxybenzophenone-5-sulfonic acid
- dihydroxy-4-methoxybenzophenone
- 2,4-dihydroxybenzophenone
- tetrahydroxybenzophenone
- 2,2'-dihydroxy-4,4'-dimethoxybenzophenone
- 2-hydroxy-4-n-octoxybenzophenone
- 2-hydroxy-4-methoxy-4'-methyl benzophenone
- sodium hydroxymethoxybenzophenone sulfonate
- disodium-2,2'-dihydroxy-4,4'-dimethoxy-5,5'-disulfobenzophenone
- phenol, 2-(2H-benzotriazol-2-yl)-4-methyl-6-(2-methyl-3(1,3,3,3-tetramethyl-1-(trimethylsilyl)oxy)disiloxyanyl) propyl) (Mexoryl^{®}XL)
- 2,2'-methylene bis-(6-(2H-benzotriazol-2-yl)-4-1,1,3,3-tetramethylbutyl) phenol) (Tinosorb^{®}M)
- 2,4-bis-[4-(2-ethylhexyloxy)-2-hydroxyphenyl]-1,3,5-triazine
- 2,4-bis-[{(4-(2-ethylhexyloxy)-2-hydroxy}phenyl]-6-(4-methoxyphenyl)-1,3,5-triazine (Tinosorb^{®}S)
- 2,4-bis-[{(4-(3-sulfonato)-2-hydroxypropyloxy)-2-hydroxy}phenyl]-6-(4-methoxyphenyl)-1,3,5-triazine sodium salt
- 2,4-bis-[{(3-(2-propyloxy)-2-hydroxypropyloxy)-2-hydroxy}phenyl]-6-(4-methoxyphenyl)-1,3,5-triazine
- 2,4-bis-[{4-(2-ethylhexyloxy)-2-hydroxy}phenyl]-6-[4-(2-methoxyethyl carbonyl) phenylamino]-1,3,5-triazine
- 2,4-bis-[{4-(3-(2-propyloxy)-2-hydroxypropyloxy)-2-hydroxy}phenyl]-6-[4-(2-ethylcarboxyl) phenylamino]-1,3,5-triazine
- 2,4-bis-[{4-(2-ethylhexyloxy)-2-hydroxy }phenyl]-6-(1-methylpyrrol-2-yl)-1,3,5-triazine
- 2,4-bis-[{4-tris-(trimethylsiloxysilylpropyloxy)-2-hydroxy}phenyl]-6-(4-methoxyphenyl)-1,3,5-triazine
- 2,4-bis-[{4-(2"-methylpropenyloxy)-2-hydroxy}phenyl]-6-(4-methoxyphenyl)-1,3,5-triazine
- 2,4-bis-[{4-(1',1',1',3',5',5',5'-heptamethylsiloxy-2"-methylpropyloxy)-2-hydroxy}phenyl]-6-(4-methoxyphenyl)-1,3,5-triazine.

In a preferred variant the cosmetic or pharmaceutical composition according to the present invention comprises a combination with one or more UV-A filters which are selected from the group consisting of
- 4-isopropyl dibenzoyl methane
- terephthalylidene dibornane sulfonic acid and salts (Mexoryl^{®}SX)
- 4-t-butyl-4'-methoxydibenzoyl methane (avobenzone) / (Neo Heliopan^{®}357)
- phenylene bis-benzimidazyl tetrasulfonic acid disodium salt (Neo Heliopan^{®}AP)
- 2,2'-(1,4-phenylene)-bis-(1H-benzimidazole-4,6-disulfonic acid), monosodium salt
- 2-(4-diethylamino-2-hydroxybenzoyl) benzoic acid hexyl ester (Uvinul^{®} A Plus)
- indanylidene compounds.

In a more preferred variant, the cosmetic or pharmaceutical composition according to the present invention comprises a combination with one or more UV filters which are selected from the group consisting of:
- p-aminobenzoic acid
- 3-(4'-trimethylammonium) benzylidene bornan-2-one methyl sulfate
- salicylic acid homomenthyl ester (Neo Heliopan^{®}HMS)
- 2-hydroxy-4-methoxybenzophenone (Neo Heliopan^{®}BB)
- 2-phenylbenzimidazole sulfonic acid (Neo Heliopan^{®}Hydro)
- terephthalylidene dibornane sulfonic acid and salts (Mexoryl^{®}SX)
- 4-tert-butyl-4'-methoxydibenzoyl methane (Neo Heliopan^{®}357)
- 3-(4'-sulfo)benzylidene bornan-2-one and salts
- 2-ethylhexyl-2-cyano-3,3-diphenyl acrylate (Neo Heliopan^{®}303)
- N-[(2 and 4)-[2-(oxoborn-3-ylidene) methyl]benzyl] acrylamide polymer
- p-methoxycinnamic acid-2-ethylhexyl ester (Neo Heliopan^{®}AV)
- p-aminobenzoic acid ethyl ester (25 mol) ethoxylated (INCI name: PEG-25 PABA)
- p-methoxycinnamic acid isoamyl ester (Neo Heliopan^{®}E1000)
- 2,4,6-trianilino-(p-carbo-2'-ethylhexyl-1'-oxy)-1,3,5-triazine (Uvinul^{®}T150)
- phenol, 2-(2H-benzotriazol-2-yl)-4-methyl-6-(2-methyl-3(1,3,3,3-tetramethyl-1-(trimethylsilyl)oxy)disiloxyanyl) propyl) (Mexoryl^{®}XL)
- 4,4'-[(6-[4-(1,1-dimethyl)aminocarbonyl) phenylamino]-1,3,5-triazine-2,4-diyl)diimino]-bis-(benzoic acid-2-ethylhexyl ester) (Uvasorb HEB)
- 3-(4'-methyl benzylidene)-D,L-camphor (Neo Heliopan^{®}MBC)
- 3-benzylidene camphor
- salicylic acid-2-ethylhexyl ester (Neo Heliopan^{®}OS)
- 4-dimethylaminobenzoic acid-2-ethylhexyl ester (Padimate O)
- hydroxy-4-methoxybenzophenone-5-sulfonic acid and Na salt
- 2,2'-methylene bis-(6-(2H-benzotriazol-2-yl)-4-1,1,3,3-tetramethylbutyl) phenol) (Tinosorb^{®}M)
- phenylene bis-benzimidazyl tetrasulfonic acid disodium salt (Neo Heliopan^{®}AP)
- 2,4-bis-[{(4-(2-ethylhexyloxy)-2-hydroxy}phenyl]-6-(4-methoxyphenyl)-1,3,5-triazine (Tinosorb^{®}S)
- benzylidene malonate polysiloxane (Parsol^{®}SLX)
- menthyl anthranilate (Neo Heliopan^{®}MA)
- 2-(4-diethylamino-2-hydroxybenzoyl) benzoic acid hexyl ester (Uvinul^{®} A Plus)
- indanylidene compounds.

In a further preferred variant, the cosmetic or pharmaceutical composition according to the invention contains a total amount of sunscreen agents, i.e. in particular UV filters and/or inorganic pigments (UV filtering pigments) so that the composition according to the invention has a light protection factor of greater than or equal to 2 (preferably greater than or equal to 5). Such compositions according to the invention are particularly suitable for protecting the skin and hair.

**Secondary sun protection factors:** Besides the groups of primary sun protection factors mentioned above, secondary sun protection factors of the antioxidant type may also be advantageously used in the cosmetic or pharmaceutical composition according to the present invention. Secondary sun protection factors of the antioxidant type interrupt the photochemical reaction chain which is initiated when UV rays penetrate into the skin. Typical examples are amino acids (for example glycine, histidine, tyrosine, tryptophane) and derivatives thereof, imidazoles (for example urocanic acid) and derivatives thereof, peptides, such as D,L-carnosine, D-carnosine, L-carnosine and derivatives thereof (for example anserine), carotinoids, carotenes (for example alpha-carotene, beta-carotene, lycopene) and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, alpha-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and hepta-thionine sulfoximine) in very small compatible dosages, also (metal) chelators (for example alpha-hydroxyfatty acids, palmitic acid, phytic acid, lactoferrine), alpha-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, glycosyl rutin, ferulic acid, furfurylidene glucitol, carnosine, butyl hydroxytoluene, butyl hydroxyanisole, nordihydroguaiac resin acid, nordihydroguaiaretic acid, trihydroxybutyrophenone, uric acid and derivatives thereof, mannose and derivatives thereof, superoxide dismutase, titanium dioxide (for example dispersions in ethanol), zinc and derivatives thereof (for example ZnO, ZnSO₄), selenium and derivatives thereof (for example selenium methionine), stilbenes and derivatives thereof (for example stilbene oxide, transstilbene oxide) and derivatives of these active substances suitable for the purposes of the invention (salts, esters, ethers, sugars, nucleotides, nucleosides, peptides and lipids).

Advantageous inorganic secondary light protection pigments are finely dispersed metal oxides and metal salts. The total quantity of inorganic pigments, in particular hydrophobic inorganic micro-pigments in the finished cosmetic preparation according to the present invention is advantageously from 0.1 to 30 % by weight, preferably 0.5 to 10.0 % by weight, in each case based on the total weight of the preparation.

Also preferred are particulate UV filters or inorganic pigments, which can optionally be hydrophobed, can be used, such as the oxides of titanium (TiO₂), zinc (ZnO), iron (Fe₂O₃), zirconium (ZrO₂), silicon (SiO₂), manganese (e.g. MnO), aluminium (Al₂O₃), cerium (e.g. Ce₂O₃), and/or mixtures thereof.

**Actives modulating skin and/or hair pigmentation:** The cosmetic or pharmaceutical composition according to the present invention may include active ingredients for skin and/or hair lightening. Preferred active ingredients for skin and/or hair lightening are selected from the group consisting of kojic acid (5-hydroxy-2-hydroxymethyl-4-pyranone), kojic acid derivatives, preferably kojic acid dipalmitate, arbutin, ascorbic acid, ascorbic acid derivatives, preferably magnesium ascorbyl phosphate, hydroquinone, hydroquinone derivatives, resorcinol, resorcinol derivatives, preferably 4-alkylresorcinols and 4-(1-phenylethyl)1,3-dihydroxybenzene (phenylethyl resorcinol), cyclohexylcarbamates, sulfur-containing molecules, preferably glutathione or cysteine, alpha-hydroxy acids (preferably citric acid, lactic acid, malic acid), salts and esters thereof, N-acetyl tyrosine and derivatives, undecenoyl phenylalanine, gluconic acid, chromone derivatives, preferably aloesin, flavonoids, 1-aminoethyl phosphinic acid, thiourea derivatives, ellagic acid, nicotinamide (niacinamide), zinc salts, preferably zinc chloride or zinc gluconate, thujaplicin and derivatives, triterpenes, preferably maslinic acid, sterols, preferably ergosterol, benzofuranones, preferably senkyunolide, vinyl guiacol, ethyl guiacol, dionic acids, preferably octodecene dionic acid and/or azelaic acid, inhibitors of nitrogen oxide synthesis, preferably L-nitroarginine and derivatives thereof, 2,7-dinitroindazole or thiocitrulline, metal chelators (preferably alpha-hydroxy fatty acids, phytic acid, humic acid, bile acid, bile extracts, EDTA, EGTA and derivatives thereof), retinoids, soy milk and extract, serine protease inhibitors or lipoic acid or other synthetic or natural active ingredients for skin and hair lightening, the latter preferably used in the form of an extract from plants, preferably bearberry extract, rice extract, papaya extract, turmeric extract, mulberry extract, bengkoang extract, nutgrass extract, liquorice root extract or constituents concentrated or isolated therefrom, preferably glabridin or licochalcone A, artocarpus extract, extract of rumex and ramulus species, extracts of pine species (pinus), extracts of vitis species or stilbene derivatives isolated or concentrated therefrom, saxifrage extract, scutelleria extract, grape extract and/or microalgae extract, in particular Tetraselmis suecica Extract.

Preferred skin lighteners are kojic acid and phenylethyl resorcinol as tyrosinase inhibitors, beta- and alpha-arbutin, hydroquinone, nicotinamide, dioic acid, Mg ascorbyl phosphate and vitamin C and its derivatives, mulberry extract, Bengkoang extract, papaya extract, turmeric extract, nutgrass extract, licorice extract (containing glycyrrhizin), alpha-hydroxy-acids, 4-alkylresorcinols, 4-hydroxyanisole. These skin lighteners are preferred due to their very good activity, in particular in combination with sclareolide according to the present invention. In addition, said preferred skin lighteners are readily available.

Advantageous skin and hair tanning active ingredients in this respect are substrates or substrate analogues of tyrosinase such as L-tyrosine, N-acetyl tyrosine, L-DOPA or L-dihydroxyphenylalanine, xanthine alkaloids such as caffeine, theobromine and theophyl-line and derivatives thereof, proopiomelanocortin peptides such as ACTH, alpha-MSH, peptide analogues thereof and other substances which bind to the melanocortin receptor, peptides such as Val-Gly-Val-Ala-Pro-Gly, Lys-lle-Gly-Arg-Lys or Leu-Ile-Gly-Lys, purines, pyrimidines, folic acid, copper salts such as copper gluconate, chloride or pyrrolidonate, 1,3,4-oxadiazole-2-thiols such as 5-pyrazin-2-yl-1,3,4-oxadiazole-2-thiol, curcumin, zinc diglycinate (Zn(Gly)2), manganese(ll) bicarbonate complexes ("pseudocat-alases"), tetrasubstituted cyclohexene derivatives, isoprenoids, melanin derivatives such as Melasyn-100 and MelanZe, diacyl glycerols, aliphatic or cyclic diols, psoralens, prostaglandins and analogues thereof, activators of adenylate cyclase and compounds which activate the transfer of melanosomes to keratinocytes such as serine proteases or agonists of the PAR-2 receptor, extracts of plants and plant parts of the chrysanthemum species, sanguisorba species, walnut extracts, urucum extracts, rhubarb extracts, microalgae extracts, in particular Isochrysis galbana, trehalose, erythru-lose and dihydroxyacetone. Flavonoids which bring about skin and hair tinting or brown-ing (e.g. quercetin, rhamnetin, kaempferol, fisetin, genistein, daidzein, chrysin and api-genin, epicatechin, diosmin and diosmetin, morin, quercitrin, naringenin, hesperidin, phloridzin and phloretin) can also be used.

**Hair growth activators or inhibitors:** Cosmetic or pharmaceutical compositions according to the present invention may also comprise one or more hair growth activators, i.e. agents to stimulate hair growth. Hair growth activators are preferably selected from the group consisting of pyrimidine derivatives such as 2,4-diaminopyrimidine-3-oxide (Aminexil), 2,4-diamino-6-piperidinopyrimidine-3-oxide (Minoxidil) and derivatives thereof, 6-amino-1,2-dihydro-1-hydroxy-2-imino-4-piperidinopyrimidine and its derivatives, xanthine alkaloids such as caffeine, theobromine and theophylline and derivatives thereof, quercetin and derivatives, dihydroquercetin (taxifolin) and derivatives, potassium channel openers, antiandrogenic agents, synthetic or natural 5-reductase inhibitors, nicotinic acid esters such as tocopheryl nicotinate, benzyl nicotinate and C1-C6 alkyl nicotinate, proteins such as for example the tripeptide Lys-Pro-Val, diphencypren, hormones, finasteride, dutasteride, flutamide, bicalutamide, pregnane derivatives, progesterone and its derivatives, cyproterone acetate, spironolactone and other diuretics, calcineurin inhibitors such as FK506 (Tacrolimus, Fujimycin) and its derivatives, Cyclosporin A and derivatives thereof, zinc and zinc salts, polyphenols, procyanidins, proanthocyanidins, phytosterols such as for example beta-sitosterol, biotin, eugenol, (±)-beta-citronellol, panthenol, glycogen for example from mussels, extracts from microorganisms, algae, plants and plant parts of for example the genera dandelion (Leontodon or Taraxacum), Orthosiphon, Vitex, Coffea, Paullinia, Theobroma, Asiasarum, Cucurbita or Styphnolobium, Serenoa repens (saw palmetto), Sophora flavescens, Pygeum africanum, Panicum miliaceum, Cimicifuga racemosa, Glycine max, Eugenia caryophyllata, Cotinus coggygria, Hibiscus rosa-sinensis, Camellia sinensis, Ilex paraguariensis, Isochrysis galbana, licorice, grape, apple, barley or hops or/nd hydrolysates from rice or wheat.

Alternatively, the cosmetic or pharmaceutical composition according to the present invention may include one or more hair growth inhibitors (as described above), i.e. agents to reduce or prevent hair growth. Hair growth inhibitors are preferably selected from the group consisting of activin, activin derivatives or activin agonists, ornithine decarboxylase inhibitors such as alpha-difluoromethylornithine or pentacyclic triterpenes like for example ursolic acid, betulin, betulinic acid, oleanolic acid and derivatives thereof, 5alpha-reductase inhibitors, androgen receptor antagonists, S-adenosylmethionine decarboxylase inhibitors, gamma-glutamyl transpeptidase inhibitors, transglutaminase inhibitors, soybean-derived serine protease inhibitors, extracts from microorganisms, algae, different microalgae or plants and plant parts of for example the families Leguminosae, Solanaceae, Graminae, Asclepiadaceae or Cucurbitaceae, the genera Chondrus, Gloiopeltis, Ceramium, Durvillea, Glycine max, Sanguisorba officinalis, Calendula officinalis, Hamamelis virginiana, Arnica montana, Salix alba, Hypericum perforatum or Gymnema sylvestre.

**Enzyme inhibitors:** The cosmetic or pharmaceutical composition according to the present invention may comprise one or more enzyme inhibitors. Suitable enzyme inhibitors are, for example, esterase inhibitors. These are preferably trialkyl citrates, such as trimethyl citrate, tripropyl citrate, triisopropyl citrate, tributyl citrate and, in particular, triethyl citrate (Hydagen CAT). The substances inhibit enzyme activity, thereby reducing the formation of odour. Other substances which are suitable esterase inhibitors are sterol sulfates or phosphates, such as, for example, lanosterol, cholesterol, campesterol, stigmasterol and sitosterol sulfate or phosphate, dicarboxylic acids and esters thereof, such as, for example, glutaric acid, monoethyl glutarate, diethyl glutarate, adipic acid, monoethyl adipate, diethyl adipate, malonic acid and diethyl malonate, hydroxycarboxylic acids and esters thereof, such as, for example, citric acid, malic acid, tartaric acid or diethyl tartrate, and zinc glycinate.

**Odour absorbers and/or antiperspirant active agents:** The cosmetic or pharmaceutical composition according to the present invention may include one or more odour absorbers and/or antiperspirant active agents (antiperspirants). Suitable odour absorbers are substances which are able to absorb and largely retain odourforming compounds. They lower the partial pressure of the individual components, thus also reducing their rate of diffusion. It is important that perfumes must remain unimpaired in this process. Odour absorbers are not effective against bacteria. They comprise, for example, as main constituent, a complex zinc salt of ricinoleic acid or specific, largely odour-neutral fragrances which are known to the person skilled in the art as "fixatives", such as, for example, extracts of labdanum or styrax or certain abietic acid derivatives. The odour masking agents are fragrances or perfume oils, which, in addition to their function as odour masking agents, give the deodorants their respective fragrance note. Perfume oils which may be mentioned are, for example, mixtures of natural and synthetic fragrances. Natural fragrances are extracts from flowers, stems and leaves, fruits, fruit peels, roots, woods, herbs and grasses, needles and branches, and resins and balsams. Also suitable are animal products, such as, for example, civet and castoreum. Typical synthetic fragrance compounds are products of the ester, ether, aldehyde, ketone, alcohol, and hydrocarbon type. Fragrance compounds of the ester type are, for example, benzyl acetate, p-tert-butylcyclohexyl acetate, linalyl acetate, phenylethyl acetate, linalyl benzoate, benzyl formate, allyl cyclohexylpropionate, styrallyl propionate and benzyl salicylate. The ethers include, for example, benzyl ethyl ether, and the aldehydes include, for example, the linear alkanals having 8 to 18 carbon atoms, citral, citronellal, citronellyloxyacetaldehyde, cyclamen aldehyde, hydroxycitronellal, lilial and bourgeonal, the ketones include, for example, the ionones and methyl cedryl ketone, the alcohols include anethole, citronellol, eugenol, isoeugenol, geraniol, linalool, phenylethyl alcohol and terpineol, and the hydrocarbons include mainly the terpenes and balsams. Preference is, however, given to using mixtures of different fragrances which together produce a pleasing fragrance note. Essential oils of relatively low volatility, which are mostly used as aroma components, are also suitable as perfume oils, e.g. sage oil, camomile oil, oil of cloves, melissa oil, mint oil, cinnamon leaf oil, linden flower oil, juniperberry oil, vetiver oil, olibanum oil, galbanum oil, labdanum oil and lavandin oil. Preference is given to using bergamot oil, dihydromyrcenol, lilial, lyral, citronellol, phenylethyl alcohol, α-hexylcinnamaldehyde, geraniol, benzylacetone, cyclamen aldehyde, linalool, boisambrene forte, ambroxan, indole, hedione, sandelice, lemon oil, mandarin oil, orange oil, allyl amyl glycolate, cyclovertal, lavandin oil, clary sage oil, β-damascone, geranium oil bourbon, cyclohexyl salicylate, Vertofix coeur, iso-E-super, Fixolide NP, evernyl, iraldein gamma, phenylacetic acid, geranyl acetate, benzyl acetate, rose oxide, romilat, irotyl and floramat alone or in mixtures.

Suitable astringent antiperspirant active ingredients are primarily salts of aluminium, zirconium or of zinc. Such suitable antihydrotic active ingredients are, for example, aluminium chloride, aluminium chlorohydrate, aluminium dichlorohydrate, aluminium sesquichlorohydrate and complex compounds thereof, e.g. with 1,2-propylene glycol, aluminium hydroxyallantoinate, aluminium chloride tartrate, aluminium zirconium trichlorohydrate, aluminium zirconium tetrachlorohydrate, aluminium zirconium pentachlorohydrate and complex compounds thereof, e.g. with amino acids, such as glycine.

**Film formers:** The cosmetic or pharmaceutical composition according to the present invention may include one or more film formers. Standard film formers are preferably chitosan, microcrystalline chitosan, quaternized chitosan, polyvinyl pyrrolidone, vinyl pyrrolidone/vinyl acetate copolymers, polymers of the acrylic acid series, quaternary cellulose derivatives, collagen, hyaluronic acid and salts thereof and similar compounds.

**Carriers and hydrotropes:** The cosmetic or pharmaceutical composition according to the present invention may comprise a carrier or a mixture of different carriers. Preferred cosmetics carrier materials are solid or liquid at 25°C and 1013 mbar (including highly viscous substances) as for example glycerol, 1,2-propylene glycol, 1,2-butylene glycol, 1,3-propylene glycol, 1,3-butylene glycol, ethanol, water, and mixtures of two or more of said liquid carrier materials with water. Optionally, these preparations according to the invention may be produced using preservatives or solubilizers. Other preferred liquid carrier substances, which may be a component of a preparation according to the invention are selected from the group consisting of oils such as vegetable oil, neutral oil and mineral oil.

Preferred solid carrier materials, which may be a component of the cosmetic or pharmaceutical composition according to the invention are hydrocolloids, such as starches, degraded starches, chemically or physically modified starches, dextrins, (powdery) maltodextrins (preferably with a dextrose equivalent value of 5 to 25, preferably of 10 - 20), lactose, silicon dioxide, glucose, modified celluloses, gum arabic, ghatti gum, traganth, karaya, carrageenan, pullulan, curdlan, xanthan gum, gellan gum, guar flour, carob bean flour, alginates, agar, pectin, inulin, and mixtures of two or more of these solids, in particular maltodextrins (preferably with a dextrose equivalent value of 15 - 20), lactose, silicon dioxide and/or glucose.

In addition, hydrotropes, for example ethanol, isopropyl alcohol or polyols, may be used to improve flow behaviour. Suitable polyols preferably contain 2 to 15 carbon atoms and at least two hydroxyl groups. The polyols may contain other functional groups, more especially amino groups, or may be modified with nitrogen. Typical examples are
- glycerol;
- alkylene glycols such as, for example, ethylene glycol, diethylene glycol, propylene glycol, butylene glycol, hexylene glycol and polyethylene glycols with an average molecular weight of 100 to 1000 Dalton;
- technical oligoglycerol mixtures with a degree of self-condensation of 1.5 to 10, such as for example technical diglycerol mixtures with a diglycerol content of 40 to 50% by weight;
- methylol compounds such as, in particular, trimethylol ethane, trimethylol propane, trimethylol butane, pentaerythritol and dipentaerythritol;
- lower alkyl glucosides, particularly those containing 1 to 8 carbon atoms in the alkyl group, for example methyl and butyl glucoside;
- sugar alcohols containing 5 to 12 carbon atoms, for example sorbitol or mannitol,
- sugars containing 5 to 12 carbon atoms, for example glucose or sucrose;
- amino sugars, for example glucamine;
- dialcoholamines, such as diethanolamine or 2-aminopropane-1,3-diol.

**Dyes:** The cosmetic or pharmaceutical composition according to the present invention may comprise one or more dyes. Suitable dyes are any of the substances suitable and approved for cosmetic purposes. Examples include cochineal red A (Cl. 16255), patent blue V (Cl. 42051), indigotin (Cl. 73015), chlorophyllin (CI. 75810), quinoline yellow (CI. 47005), titanium dioxide (CI. 77891), indanthrene blue RS (CI. 69800) and madder lake (CI. 58000). Luminol may also be present as a luminescent dye. Advantageous coloured pigments are for example titanium dioxide, mica, iron oxides (e.g. Fe₂O₃ Fe₃O₄, FeO(OH)) and/or tin oxide. Advantageous dyes are for example carmine, Berlin blue, chromium oxide green, ultramarine blue and/or manganese violet.

In addition to the above-described substances, further ingredients commonly used in the cosmetic or pharmaceutical industry, which are suitable or customary in the compositions of the present invention, can be used.

The mixture comprising 1,2-heptanediol and 2,3-heptanediol according to the present invention may be present in the cosmetic or pharmaceutical composition or homecare product in an amount of 0.001 to 15.0 % by weight, based on the total weight of the composition or homecare product. In a preferred variant, the cosmetic or pharmaceutical composition or homecare product comprises the mixture comprising 1,2-heptanediol and 2,3-heptanediol in an amount of 0.01 to 10.0 % by weight, based on the total weight of the composition or homecare product. In a more preferred variant, the mixture comprising 1,2-heptanediol and 2,3-heptanediol is advantageously used in the cosmetic or pharmaceutical composition or homecare product in an amount of at 0.1 to 5.0 % by weight, based on the total weight of the composition or homecare product. In a still more preferred variant, the mixture comprising 1,2-heptanediol and 2,3-heptanediol is advantageously used in the cosmetic or pharmaceutical composition or homecare product in an amount of at 0.3 to 3.0 % by weight, based on the total weight of the composition or homecare product. In a most preferred variant, the mixture comprising 1,2-heptanediol and 2,3-heptanediol is advantageously used in the cosmetic or pharmaceutical composition or homecare product in an amount of 0.5 to 1.0 % by weight, based on the total weight of the composition or homecare product.

For the heptanediol mixture, the above amounts relate to the total content of the 1,2-heptanediol and the 2,3-heptanediol in the mixture, i.e. the amount is the sum of the content of the 1,2-heptanediol and 2,3-heptanediol in the mixture.

The mixture comprising at least one first linear alkanediol and one or more second linear alkanediol according to the present invention may be present in the cosmetic or pharmaceutical composition or homecare product in an amount of 0.001 to 15.0 % by weight, based on the total weight of the composition or homecare product. In a preferred variant, the cosmetic or pharmaceutical composition or homecare product comprises the mixture comprising at least one first linear alkanediol and one or more second linear alkanediol in an amount of 0.01 to 10.0 % by weight, based on the total weight of the composition or homecare product. In a more preferred variant, the mixture comprising at least one first linear alkanediol and one or more second linear alkanediol is advantageously used in the cosmetic or pharmaceutical composition or homecare product in an amount of at 0.1 to 5.0 % by weight, based on the total weight of the composition. In a still more preferred variant, the mixture comprising at least one first linear alkanediol and one or more second linear alkanediol is advantageously used in the cosmetic or pharmaceutical composition or homecare product in an amount of at 0.3 to 3.0 % by weight, based on the total weight of the composition. In a most preferred variant, the the mixture comprising at least one first linear alkanediol and one or more second linear alkanediol is advantageously used in the cosmetic or pharmaceutical composition or homecare product in an amount of at 0.5 to 1.0 % by weight, based on the total weight of the composition or homecare product.

For the alkane mixture, the above amounts relate to the total content of the first linear alkanediol and the second linear alkanediol in the mixture, i.e. the amount is the sum of the content of the first linear alkanediol and the second linear alkanediol in the mixture.

**In** a particular preferred variant according to the present invention, the cosmetic or pharmaceutical composition or the homecare product comprises the 2,3-heptanediol of the mixture comprising 1,2-heptanediol and 2,3-heptanediol in an amount of 0.001 to 15.0 % by weight, preferred in an amount of 0.01 to 10.0 % by weight, more preferred in an amount of 0.1 to 5.0 % by weight, still more preferred in an amount of 0.3 to 3.0 % by weight, and most preferred in an amount of 0.5 to 1.0 % by weight, based on the total weight of the composition or homecare product.

Even more preferred, the cosmetic or pharmaceutical composition or the homecare product comprises the 2,3-heptanediol of the mixture comprising 1,2-heptanediol and 2,3-heptanediol in an amount of 0.001 to 0.5 % weight, preferably in an amount of 0.005 to 0.1 % by weight and most preferably in an amount of 0.01 to 0.075 % by weight, based on the total weight of the composition or homecare product.

**In** a particular preferred variant according to the present invention, the cosmetic or pharmaceutical composition or the homecare product comprises the 2,3-alkanediol of the mixture comprising at least one first linear alkanediol and one or more second linear alkanediol in an amount of 0.001 to 15.0 % by weight, preferably in an amount of 0.01 to 10.0 % by weight, more preferred in an amount of 0.1 to 5.0 % by weight, still more preferred in an amount of 0.3 to 3.0 % by weight, and most preferred in an amount of 0.5 to 1.0 % by weight, based on the total weight of the composition or homecare product.

Even more preferred, the cosmetic or pharmaceutical composition or the homecare product comprises the 2,3-alkanediol of the mixture comprising at least one first linear alkanediol and one or more second linear alkanediol in an amount of 0.001 to 0.5 % weight, preferably in an amount of 0.005 to 0.1 % by weight and most preferably in an amount of 0.01 to 0.0075 % by weight, based on the total weight of the composition or homecare product.

The lipophilic active component is present in the cosmetic or pharmaceutical composition or homecare product according to the present invention, in an amount of 0.001 % to 5.0 by weight, based on the total weight of the composition or homecare product. In a preferred variant, the cosmetic or pharmaceutical composition or homecare product comprises the lipophilic active component in an amount of 0.005 to 4.0 % by weight, based on the total weight of the composition or homecare product. In a particular preferred variant, the lipophilic active component is advantageously used in the cosmetic or pharmaceutical composition or homecare product in an amount of at 0.01 to 3.0 % by weight, based on the total weight of the composition or homecare product.

As is demonstrated in the following examples 1.9 to 1.14, the presence of a mixture of an 1,2-alkanediol and a 2,3-alkanediol considerably improves the solubility of the lipophilic active component as defined herein in a cosmetic or pharmaceutical composition or homecare product compared to the corresponding 1,2-alkanediol or 2,3-alkanediol substance. Surprisingly, the aqueous/glycolic formulations containing a mixture including 1,2-heptanediol and 2,3-heptanediol or a mixture including 1,2-octanediol and 2,3-octanediol showed the lowest turbidity, which indicates highest clarity and best solublity for the lipophilic active component. The value of turbidity unit is by far lower than the value of turbidity unit for the respective single 1,2-alkanediol or 2,3-alkanediol substances. Surprisingly, the solubility of the lipophilic active component was particularly distinguished in an aqueous/glycolic formulation with a high water content. A good solubility of the lipophilic active component can also be observed for emulsions. The combination of a lipophilic active component as defined above with an alkanediol mixture including a 1,2-alkanediol and 2,3-alkanediol surprisingly results in a stable formulation, i.e. in which the lipophilic active component remains solubilized and in which undesired crystallizing-out or precipitation of the lipophilic active component is prevented even when stored.

Due to its better solubility, the availability of the lipophilic active substance or component in the cosmetic or pharmaceutical composition is increased. Additionally, due to its better solubility, the lipophilic active substance can be furnished in a higher concentration in the final formulation or can be furnished in a lower concentration in the final formulation compared to a composition including a lipophilic active component with low solubility and without the additional of an alkanediol.

Due to its better solubility, a uniformly solubilized product, in which the lipophilic active component is uniformly dissolved in the product can be obtained. Thus, the concentration of the lipophilic active component is exactly the same through the whole formulation from the very first splash to the very last ml, untill the bottle is completely empty. Without the use of an alkanediol as specified herein, the distribution of the lipophilic active component would be quite haphazardly in the formulation and would vary from application to application.

The use of a mixture comprising a first linear alkanediol and a second linear alkanediol provides a stable formulation without turbidity or recrystallization or precipitation of the lipophilic active component, which can even maintained during storage.

Without the use of an alkanediol mixture as specified herein, recrystallization or precipitation ot the lipophilic active component as defined herein, would increase, so that storage of such formulations would be problematic or even impossible.

Moreover, the use of a mixture comprising a first linear alkanediol and a second linear alkanediol allows for the use of less additional solubilizing agents.

The cosmetic or pharmaceutical, preferably dermatological, composition according to the present invention is intended for topical applications. The term "topical" is understood to mean external applications on a mammal's skin, which are in particular for the treatment, protection, care and cleansing of the skin, scalp, eyelashes, eyebrows, nails, mucous membranes and hair. The mammal is preferably a human.

For topical application, the cosmetic or pharmaceutical composition is either a rinse off or leave on preparation.

The cosmetic or pharmaceutical, in particular dermatological, composition according to the present invention can be present in different forms of, e.g. in the form of a dispersion, in the form of a liquid surfactant formulation, in the form of a solid surfactant formulation, or in the form of an aqueous, aqueous/alcoholic, in particular aqueous/ethanolic, or an aqueous/glycolic based solution.

In a particular variant, the cosmetic or pharmaceutical composition according to the present invention is a cold formulation, wich can be prepared without a heating step.

The cosmetic or pharmaceutical, preferably dermatological, composition according to the present invention is preferably a dispersion. The term "dispersion" in the context of the present invention means, that the cosmetic or pharmaceutical composition is a disperse two-phase system consisting of colloidal particles (disperse phase) and a medium in which they are suspended (disperse medium). Both phases are not miscible with each other, only with an emulsifier. Such dispersions, for example emulsions, comprise the at least oil component preferably in an amount of ≥ 1 % by weight, more preferably in an amount of ≥ 3 % by weight.

If the cosmetic or pharmaceutical composition according to the present invention is a dispersion, preferably an emulsion, the oil component is present in the cosmetic or pharmaceutical composition in an amount of 0.01 % to 50.0 by weight, based on the total weight of the composition. In a preferred variant, the cosmetic or pharmaceutical composition comprises the oil component in an amount of 1.0 to 40.0 % by weight, based on the total weight of the composition. In a particular preferred variant, the oil component is advantageously used in the cosmetic or pharmaceutical composition in an amount of at 3.0 to 30 % by weight, based on the total weight of the composition.

Preferably, the cosmetic or pharmaceutical composition according to the present invention takes various forms such as an emulsion, in particular a O/W emulsion, a W/O emulsion, a multiple emulsion, a hydrodispersion gel, a balm, a multiple emulsion of the water-in-oil type (W/O/WO) or of the oil-in-water type (O/W/O), **PIT** emulsion, Pickering emulsion, a micro-emulsion, a liquid, a lotion, a suspension, a milk, an ointment, a paste, a gel, a cream based, an oil based or a liposome based formulation.

In a further alternative, the cosmetic or pharmaceutical composition according to the present invention is a liquid surfactant formulation.

Such liquid surfactant formulations include for example shampoo, shower gel, micellar water, liquid soap, cleansing preparations.

If the cosmetic or pharmaceutical composition according to the present invention is a liquid surfactant formulation, the surfactant component is present in the cosmetic or pharmaceutical composition in an amount of 1 to 40 % by weight, preferably in an amount of 3 to 30 % by weight, more preferably in an amount of 5 to 25 % by weight, based on the total weight of the composition.

In a further alternative, the cosmetic or pharmaceutical composition according to the present invention is a solid surfactant formulation.

Such solid surfactant formulations include for example solid shampoos, solid body wash, bar soaps, etc.

Alternatively, the cosmetic or pharmaceutical composition as disclosed herein is an aqueous or aqueous/alcoholic or aqueous/glycolic based solution. The aqueous/alcoholic or aqueous/glycolic based solution comprises an aliphatic alcohol or a glycol in an amount of 0.1 to ≤ 50 % by weight, based on the total weight of the solution. The aliphatic alcohol is preferably selected from the group consisting of ethanol, isopropanol, n-propanol. The glycol is preferably selected from the group consisting of glycerin, propylene glycol, butylene glycol or dipropylene glycol. Preferably, the overall water content in the final composition of such compositions can be ≥ 60 %, more preferably ≥ 70 %, even more preferably ≥ 80 %, and even more preferably ≥ 90 %. New applications such as those including solutions for water wipes have high water content. In a particular variant, the inventive compositions can be used for such wet wipe applications. They may then most preferably contain ≥ 95 % water, or even ≥ 98 % water.

The higher the water content in such a formulation, the lower the solubility of lipophilic active components.

Such aqueous or aqueous/alcoholic or aqueous/glycolic based solutions include for example deo/antiperspirant preparations, after shave, cleansing preparations, anti-acne preparations, or wet wipe solutions.

In one preferred variant, the cosmetic or pharmaceutical composition according to the present invention is in the form of an emulsion as defined herein, advantageously in the form of an oil-in-water (O/W) emulsion comprising an oily phase dispersed in an aqueous phase in the presence of an O/W emulsifier.

In a most preferred variant, the cosmetic or pharmaceutical composition according to the present invention is in the form of an aqueous or aqueous/alcoholic, preferably aqueous/ethanolic, or aqueous/glycolic based solution. Typically, this could be glycerin in water compositions.

Other applications comprise aqueous mouthwashes, with low alcohol/ethanol content. Here, the present compositions are valuable in ensuring good solubility of the active ingredients. **In** particular, for systems that tend to recrystallize over time after solubilization, the alkanediol mixturesaccording to the present invention, have been found to be highly effective in preventing precipitation of actives. For example, o-Cymen-5-ol tends to precipitate.

These cosmetic or pharmaceutical ready-to-use compositions or formulations are prepared according to usual and known methods.

**In** a further aspect, the composition according to the present invention is a preparation for cosmetic and/or non-therapeutic use for personal care, skin protection, skin care, scalp protection, scalp care, hair care, nail care, in particular for the prevention and/or treatment of skin conditions, intolerant or sensitive skin, skin irritation, skin reddening, wheals, pruritus (itching), skin aging, wrinkle formation, loss of skin volume, loss of skin elasticity, pigment spots, pigment abnormalities, skin dryness, flaking, greasiness, hypopigmentation and/or hyperpigmentation of the skin; or for animal care.

Examples of personal care are preferably anti-ageing preparations, skin care emulsions, body oils, body lotions, cleansing lotions, face or body balms, after shave balms, after sun balms, deo emulsions, cationic emulsions, body gels, treatment creams, skin protection ointments, moisturizing gels, face and/or body moisturizers, light protective preparations (sunscreens), micellar water, hair spray, colour protection hair care products, skin lightening product, anti-dark spot preparations, etc.

Alternatively, the composition according to the present invention is a preparation for medical use.

The pharmaceutical, in particular dermatological, composition according to the present invention is a preparation for the prevention and treatment of a condition of the skin or mucosa.

Preferably, the pharmaceutical composition according to the present invention is used in the prevention and/or treatment of dysfunctions of human hair, skin and/or nails, in particular dermatological or keratological diseases, wherein the dermatological or keratological diseases are selected from the group consisting of atopic dermatitis (neurodermitis), psoriasis, acneiform exanthema, sebostasis, xerosis, eczema, hyper seborrhea and hypo seborrhea, dermatitis, rosacea, erythema, wheals, pruritus (itching), inflammation, irritation, fibrosis, *lichen planus, pityriasis rosea, pityriasis versicolor,* autoimmune bullous diseases, urticaria, angioedema, allergic skin reactions, wound healing, tissue regeneration and in the treatment of inflammatory diseases or pain.

In order to be used, the cosmetic or pharmaceutical, in particular dermatological, composition according to the present invention is applied to the skin, hair, scalp and/or nails in an adequate amount in such manner as is customary with cosmetics and dermatological products.

The homecare products according to the present invention are predominantly detergent formulations, usually liquids, powders, sprays, granules or tablets, used to remove dirt, including dust, stains, bad smells and clutter on surfaces or other types of housecleaning or laundry detergent that is added for cleaning laundry or liquid soap. Typical homecare products include all purpose cleaners, dishwashing detergens, hard floor and surface cleaners, glass cleaners, carpet cleaners, oven cleaners, laundry detergents, fabric softeners, laundry scent, scent lotions, car shampoo, rim block gel, all afore mentioned goods also in encapsulated form, air fresheners or furniture polish.

Surprisingly, the use of a mixture comprising 1,2-heptanediol and 2,3-heptanediol or of the mixture comprising at least one first alkanediol and one or more second linear alkanediol as described herein in combination with a lipophilic active according to the present invention improves significantly the solubility of a lipophilic active component as defined herein. The lipophilic active component does not crystallize out or precipitate out and does not lead to turbidity.

Surprisingly, the use of a mixture comprising 1,2-heptanediol and 2,3-heptanediol or of the mixture comprising at least one first alkanediol and one or more second linear alkanediol in combination with a lipophilic active according to the present invention reduces or prevents foam formation upon preparing the cosmetic or pharmaceutical composition or homecare product comprising a solubilizer.

Thus, the present invention relates in a further aspect to the use of a mixture including 1,2-heptanediol and 2,3-heptanediol or of a mixture comprising at least one first linear alkanediol and one or more second linear alkanediol for improving the solubility of a lipophilic active component as defined herein, and, thus, improving the availability of the lipophilic active component in the cosmetic or pharmaceutical composition or homecare product and for reducing or preventing foam formation upon preparing a cosmetic or pharmaceutical composition or homecare product comprising a solubilizer.

Additionally, due to its solubility improving effect, with the use of a mixture comprising 1,2-heptanediol and 2,3-heptaneiol or of a mixture comprising at least one first linear alkanediol and one or more second linear alkanediol in a lipophilic active component containing cosmetic or pharmaceutical composition or honmecare product the amount of solubilizing agent(s) can be reduced. Thus, the potential negative side effects of solubilizing agents like increased foam formation or increased skin permeation can be eliminated or at least minimized.

Thus, the present invention relates further to the use of a mixture comprising 1,2-heptanediol and 2,3-heptanediol or of a mixture comprising at least one first linear alkanediol and one or more second linear alkanediol as a solubilizing agent replacer in a cosmetic or pharmaceutical composition or homecare product.

The present invention shall now be described in detail with reference to the following examples, which are merely illustrative of the present invention, such that the content of the present invention is not limited by or to the following examples.

### Examples

### Example 1: Solubility of lipophilic active components in combination with different 1,2-alkanediols (not in accordance with the present invention)

The following tests were performed to show the influence of different 1,2-alkanediols on the solubility of lipophilic active components according to the present invention:
(b) Bisabolol (log P_{O/W}: 5.63)
(c) Hydrocortisone (log P_{O/W}: 1.62)
(d) Ethylhexylglycerin (log P_{O/W}: 2.22)
(e) o-Cymen-5-ol (log P_{O/W}: 3.52)
(f) Propanediol Caprylate (log P_{O/W}: 2.84)
(g) Retinyl Palmitate (log P_{O/W}: 15.51)
(h) Glyceryl Caprylate (log P_{O/W}: 1.71)
(i) Hydroxyacetophenone (log P_{O/W}: 1.19)

### Example 1.1: Solubility improvement for Bisabolol (not in accordance with the present invention)

The following aqueous/glycolic formulation was used to demonstrate the solubilizing properties of 1,2-pentanediol, 1,2-hexanediol, 1,2-heptanediol and 1,2-octanediol.

**Table 1:**

| | **Raw material** | **INCI** | **D** | **E** | **F** | **G** | **H** |
|---|---|---|---|---|---|---|---|
| B. | Butylene Glycol | Butylene Glycol | 1.0 | 1.0 | 1.0 | 1.0 | 1.0 |
| | Solubilizer #660352 | PEG-40 Hydrogenated Castor Oil, Trideceth-9, Propylene Glycol, Aqua | 0.6 | 0.6 | 0.6 | 0.6 | 0.6 |
| | 1,2-pentanediol | Pentylene Glycol | - | 0.5 | - | - | - |
| | 1,2-hexanediol | 1,2 Hexanediol | - | - | 0.5 | - | - |
| | 1,2-heptanediol | 1,2 Heptanediol | - | - | - | 0.5 | - |
| | 1,2-octanediol | Caprylyl Glycol | - | - | - | - | 0.5 |
| C. | (2R)-6-methyl-2-[(1R)-4-methylcyclohex-3-en-1-yl]hept-5-en-2-ol | Bisabolol | 0.3 | 0.3 | 0.3 | 0.3 | 0.3 |
| D. | Water, dem. | Water (Aqua) | Ad 100 | | | | |
| | Comment | | T++ | T++ | T | T- | T+ |

Production method: A premix B was prepared by mixing solvent and solubilizer with Vortex for 20 seconds. Then diol was added to the premix. The active component was added to the premix and mixed with magnetic stirrer for 30 seconds. Water was added and the resulting mixture was stirred for 5 minutes. Hydrolite 8 was added to the mixture and heated to 40 °C to solve the Caprylyl Glycol.

The visual appearance of the sample solutions was evaluated according to the following scale:

| **Description** | **Coding** |
|---|---|
| Slight turbidity | T- |
| Turbid | T |
| Strong turbidity | T+ |
| Very strong turbidity | T++ |

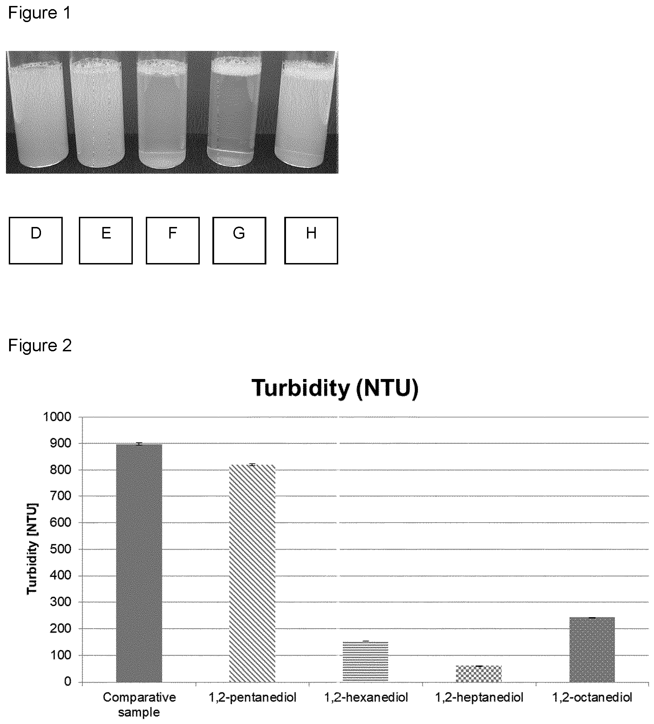

The images of the sample solutions D (comparative), E (containing 1,2-pentanediol), F (containing 1,2-hexanediol), G (containing 1,2-heptanediol) and H (containing 1,2-octanediol) are shown in Figure 1.

Additionally, the turbidity of the sample solutions D to H was determined by measuring the nephelometric turbidity units (NTU) in triple determination. Device: Turb 430 IR ex WTW.

Turbidity is described as the opaqueness of a fluid due to the presence of suspended solids and is measured in terms of nephelometric turbidity units (NTU). NTU signifies that the instrument is measuring scattered light from the sample at a 90-degree angle from the incident light.

**Table 2:**

| **Sample** | **D** | **E** | **F** | **G** | **H** |
|---|---|---|---|---|---|
| | 899 | 824 | 154 | 58.6 | 243 |
| | 901 | 816 | 154 | 58.9 | 241 |
| | 893 | 817 | 154 | 59.0 | 241 |
| **Mean value (NTU)** | **898** | **819** | **154** | **58.3** | **242** |

The results of the nephelometric turbidity units (NTU) of samples D to H are shown in Figure 2.

Surprisingly, the sample containing 1,2-heptanediol (sample G) showed the lowest turbidity unit which indicates highest clarity in comparison to samples E (containing 1,2-pentanediol), F (containing 1,2-hexanediol) and H (containing 1,2-octanediol). Sample D without 1,2-alkanediol serves as a comparative sample.

### Example 1.2: Solubility improvement for Hydrocortisone (not in accordance with the present invention)

The following aqueous/glycolic formulation was used to demonstrate the solubilizing properties of 1,2-pentanediol, 1,2-hexanediol, 1,2-heptanediol and 1,2-octanediol.

**Table 3:**

| | **Raw material** | **INCI** | **A** | **B** | **C** | **D** |
|---|---|---|---|---|---|---|
| B | PG | Propylene Glycol | 2.5 | 2.5 | 2.5 | 2.5 |
| | Solubilizer #660352 | PEG-40 Hydrogenated Castor Oil, Trideceth-9, Propylene Glycol, Aqua | 0.6 | 0.6 | 0.6 | 0.6 |
| | 1,2-pentanediol | Pentylene Glycol | 1.0 | - | - | - |
| | 1,2-hexanediol | 1,2 Hexanediol | - | 1.0 | - | - |
| | 1,2-heptanediol | 1,2 Heptanediol | - | - | 1.0 | - |
| | 1,2-octanediol | Caprylyl Glycol | - | - | - | 1.0 |
| C | (8S,9S,10R,11S,13S,14S,17 R)-11,17-dihydroxy-17-(2-hydroxyacetyl)-10,13-dimethyl-2,6,7,8,9,11,12,14,15,16-decahydro-1H-cyclopenta[a]phenanthren-3-one | Hydrocortisone | 0.2 | 0.2 | 0.2 | 0.2 |
| D | Water dem. | Water (Aqua) | 95.2 | 95.2 | 95.2 | 95.2 |
| | Sum: | | 100 | 100 | 100 | 100 |
| | Comment (after 3 days 23°C) | | S++ | S+ | S | T++ S+ |

Production method: A premix B was prepared by mixing solvent, solubilizer and diol. Hydrocortisone was added. The mixture was heated to 70 °C to solve the active component while stirring with a magnetic stirrer. The mixture was stirred for further 5 minutes at 70 °C. Water was added and the mixture was cooled while stirring (10 min).

The visual appearance of the sample solutions was evaluated according to the following scale:

| **Description** | **Coding** |
|---|---|
| Strong sedimentation | S++ |
| Sedimentation | S+ |
| Slight sedimentation | S |
| Very strong turbidity | T++ |

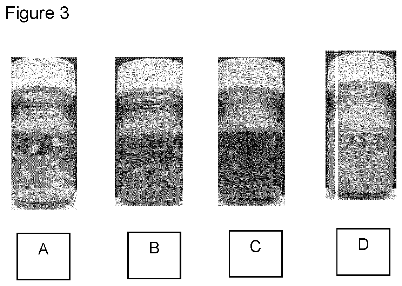

The images of the sample solutions A (containing 1,2-pentanediol), B (containing 1,2-hexanediol), C (containing 1,2-heptanediol) and D (containing 1,2-octanediol) are shown in Figure 3.

Turbidity measurements of samples A to D were no possible due to crystallizations/sedimentation.

Instead, the amount of sedimentation was gravimetric determined. After 3 days storage at 23 °C the samples A to D were filtered and the corresponding amount of solid residue was determined by weighing.

**Table 4:**

| **Sample** | **Hydrocortisone (mg)** |
|---|---|
| A | 61.1 |
| B | 18.5 |
| C | 2.7 |
| D | 3.2 |

Sample C (containing 1,2-heptanediol) showed the lowest amount fo crystallization/sedimentation of 2.7 mg and the highest transparency. Sample A (containing 1,2-pentanediol) and sample B (containing 1,2-hexanediol) showed higher amounts of crystallization/sedimentation of 61.1 mg (sample A) and 18.5 mg (sample B). Sample D (containing 1,2-octanediol) showed less crystallization/sedimentation versus samples A and B but had the strongest turbidity within all samples.

The results lead to the conclusion that 1,2-heptanediol has better solubility improving properties in comparison to 1,2-pentanediol, 1,2-hexanediol and 1,2-octanediol.

### Example 1.3: Solubility improvement for Ethylhexylglycerin (not in accordance with the present invention)

The following aqueous/glycolic formulation was used to demonstrate the solubilizing properties of 1,2-pentanediol, 1,2-hexanediol, 1,2-heptanediol and 1,2-octanediol.

**Table 5:**

| | **Raw material** | **INCI** | **1** | **2** | **3** | **4** | **5** |
|---|---|---|---|---|---|---|---|
| B | DPG | Dipropylene Glycol | 1.5 | 1.5 | 1.5 | 1.5 | 1.5 |
| | Solubilizer #660352 | PEG-40 Hydrogenated Castor Oil, Trideceth-9, | 1.0 | 1.0 | 1.0 | 1.0 | 1.0 |
| | | Propylene Glycol, Aqua | | | | | |
| | 1,2-pentanediol | Pentylene Glycol | - | 0.5 | - | - | - |
| | 1,2-hexanediol | 1,2 Hexanediol | - | - | 0.5 | - | - |
| | 1,2-heptanediol | 1,2 Heptanediol | - | - | - | 0.5 | - |
| | 1,2-octanediol | Caprylyl Glycol | - | - | - | - | 0.5 |
| C | 3-(2-Ethylhexyloxy)-1,2-propanediol | Ethylhexylgylcerin | 0.3 | 0.3 | 0.3 | 0.3 | 0.3 |
| D | Water, dem. | Water (Aqua) | Ad 100 | | | | |
| | Comment | | T | T | T | T- | T |

Production method: A premix B was prepared by mixing solvent and solubilizer with Vortex for 20 seconds. Then diol was added to the premix. The active component was added to the premix and mixed with magnetic stirrer for 30 seconds. Water was added and the resulting mixture was stirred for 5 minutes. Hydrolite 8 was added to the mixture and heated up to 40 °C to solve the Caprylyl Glycol.

The visual appearance of the sample solutions was evaluated according to the following scale:

| **Description** | **Coding** |
|---|---|
| Slight turbidity | T- |
| Turbid | T |
| Strong turbidity | T+ |
| Very strong turbidity | T++ |

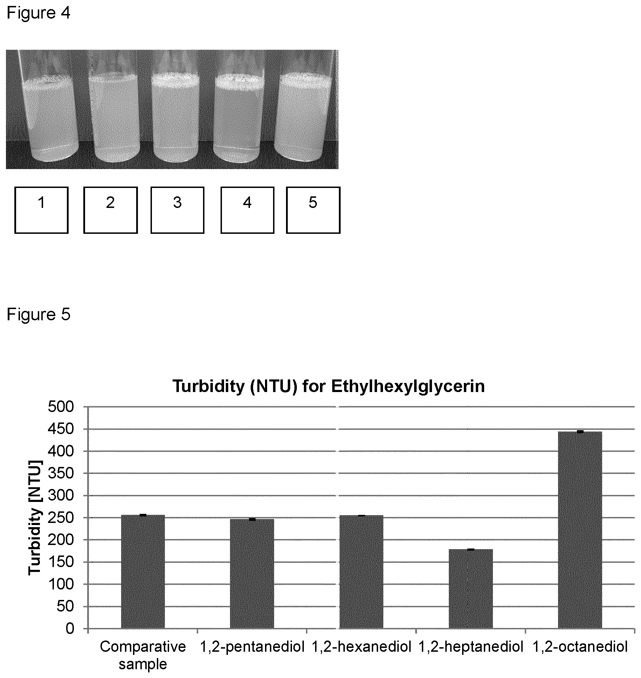

The images of the sample solutions 1 (comparative), 2 (containing 1,2-pentanediol), 3 (containing 1,2-hexanediol), 4 (containing 1,2-heptanediol) and 5 (containing 1,2-octanediol) are shown in Figure 4.

Additionally, the turbidity of the sample solutions 1 to 5 was determined by measuring the nephelometric turbidity units (NTU) in triple determination. Device: Turb 430 IR ex WTW.

**Table 6:**

| **Sample** | **1** | **2** | **3** | **4** | **5** |
|---|---|---|---|---|---|
| | 256 | 245 | 255 | 179 | 442 |
| | 257 | 248 | 255 | 178 | 446 |
| | 255 | 256 | 255 | 178 | 444 |
| **Mean value (NTU)** | **256** | **246** | **255** | **178** | **444** |

The results of the nephelometric turbidity units (NTU) of samples 1 to 5 are shown in Figure 5.

Surprisingly sample 4 (containing 1,2-heptanediol) showed the lowest turbidity unit which indicates highest clarity in comparison to samples 2 (containing 1,2-pentanediol), 3 (containing 1,2-hexanediol) and 5 (containing 1,2-octanediol). Sample 1 without 1,2-alkanediol serves as a comparative sample.

### Example 1.4: Solubility improvement for o-Cymen-5-ol (not in accordance with the present invention)

The following aqueous/glycolic formulation was used to demonstrate the solubilizing properties of 1,2-pentanediol, 1,2-hexanediol, 1,2-heptanediol and 1,2-octanediol.

**Table 7:**

| | **Raw material** | **INCI** | **1** | **2** | **3** | **4** | **5** |
|---|---|---|---|---|---|---|---|
| B. | Butylene Glycol | Butylene Glycol | 0.5 | 0.5 | 0.5 | 0.5 | 0.5 |
| | Solubilizer #660352 | PEG-40 Hydrogenated Castor Oil, Trideceth-9, Propylene Glycol, Aqua | 0.3 | 0.3 | 0.3 | 0.3 | 0.3 |
| | 1,2-pentanediol | Pentylene Glycol | - | 0.5 | - | - | - |
| | 1,2-hexanediol | 1,2 Hexanediol | - | - | 0.5 | - | - |
| | 1,2-heptanediol | 1,2 Heptanediol | - | - | - | 0.5 | - |
| | 1,2-octanediol | Caprylyl Glycol | - | - | - | - | 0.5 |
| C. | 3-methyl-4-propan-2-yl phenol | o-Cymen-5-ol | 0.1 | 0.1 | 0.1 | 0.1 | 0.1 |
| D. | Water, dem. | Water (Aqua) | Ad 100 | | | | |
| | Comment | | T | T | T | T- | T |

Production method: A premix B was prepared by mixing solvent, solubilizer and diol. o-Cymen-5-ol was added. The mixture was heated to 50 °C to solve the active component while stirring with a magnetic stirrer. The mixture was stirred for further 5 minutes at 50 °C. Water was added and the mixture was cooled while stirring (10 min).

The visual appearance of the sample solutions was evaluated according to the following scale:

| **Description** | **Coding** |
|---|---|
| Slight turbidity | T- |
| Turbid | T |
| Strong turbidity | T+ |
| Very strong turbidity | T++ |

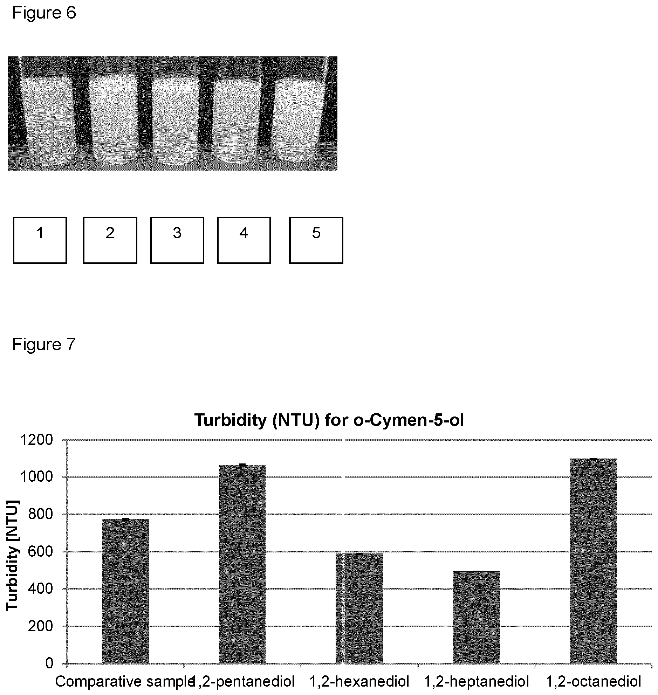

The images of the sample solutions 1 (comparative), 2 (containing 1,2-pentanediol), 3 (containing 1,2-hexanediol), 4 (containing 1,2-heptanediol) and 5 (containing 1,2-octanediol) are shown in Figure 6.

Additionally, the turbidity of the sample solutions 1 to 5 was determined by measuring the nephelometric turbidity units (NTU) in triple determination. Device: Turb 430 IR ex WTW.

**Table 8:**

| **Sample** | **1** | **2** | **3** | **4** | **5** |
|---|---|---|---|---|---|
| | 775 | 1066 | 589 | 496 | >1100 |
| | 770 | 1062 | 587 | 494 | >1100 |
| | 775 | 1065 | 590 | 493 | >1100 |
| **Mean value (NTU)** | **773** | **1064** | **589** | **494** | **>1100** |

The results of the nephelometric turbidity units (NTU) of samples 1 to 5 are shown in Figure 7.

Surprisingly sample 4 (containing 1,2-heptanediol) showed the lowest turbidity unit which indicates highest clarity in comparison to samples 2 (containing 1,2-pentanediol), 3 (containing (1,2-hexanediol) and 5 (containing 1,2-octanediol). Sample 1 without 1,2-alkanediol serves as a comparative sample.

### Example 1.5: Solubility improvement for Propanediol Caprylate (not in accordance with the present invention)

The following aqueous/glycolic formulation was used to demonstrate the solubilizing properties of 1,2-pentanediol, 1,2-hexanediol, 1,2-heptanediol and 1,2-octanediol.

**Table 9:**

| | **Raw material** | **INCI** | **1** | **2** | **3** | **4** | **5** |
|---|---|---|---|---|---|---|---|
| A. | Butylene Glycol | Butylene Glycol | 1.0 | 1.0 | 1.0 | 1.0 | 1.0 |
| | Solubilizer #660352 | PEG-40 Hydrogenated Castor Oil, Trideceth-9, Propylene Glycol, Aqua | 0.6 | 0.6 | 0.6 | 0.6 | 0.6 |
| | 1,2-pentanediol | Pentylene Glycol | - | 0.5 | - | - | - |
| | 1,2-hexanediol | 1,2 Hexanediol | - | - | 0.5 | - | - |
| | 1,2-heptanediol | 1,2 Heptanediol | - | - | - | 0.5 | - |
| | 1,2-octanediol | Caprylyl Glycol | - | - | - | - | 0.5 |
| B. | Propanediol Monocaprylate | Propanediol Caprylate | 0.5 | 0.5 | 0.5 | 0.5 | 0.5 |
| C. | Water, dem. | Water (Aqua) | Ad 100 | | | | |
| | Comment | | T | T+ | T | C | T+ |

Production method: A premix A was prepared by mixing solvent and solubilizer with Vortex for 20 seconds. Then diol was added to the premix. The active component was added to the premix and mixed with magnetic stirrer for 30 seconds. Water was added and the resulting mixture was stirred for 5 minutes. Hydrolite 8 was added to the mixture and heated up to 40 °C to solve the Caprylyl Glycol.

The visual appearance of the sample solutions was evaluated according to the following scale:

| **Description** | **Coding** |
|---|---|
| Slight turbidity | T- |
| Turbid | T |
| Strong turbidity | T+ |
| Very strong turbidity | T++ |

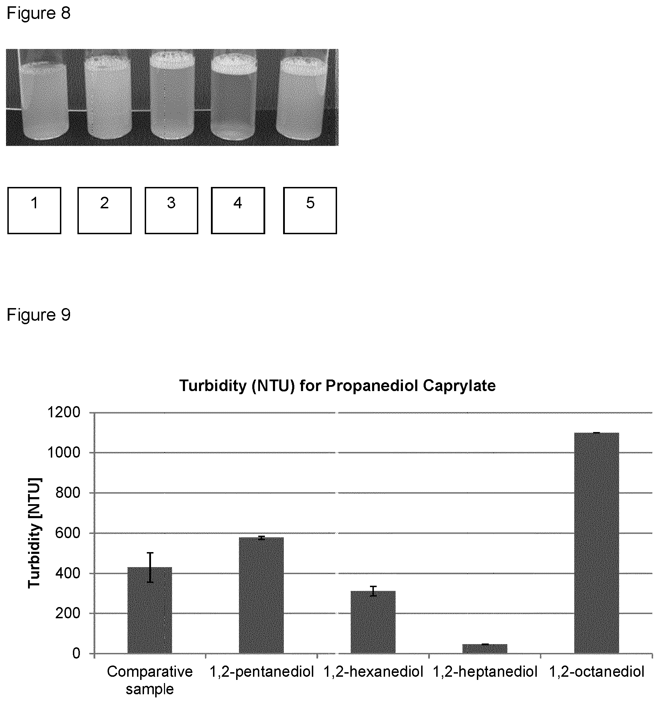

The images of the sample solutions 1 (comparative), 2 (containing 1,2-pentanediol), 3 (containing 1,2-hexanediol), 4 (containing 1,2-heptanediol) and 5 (containing 1,2-octanediol) are shown in Figure 8.

Additionally, the turbidity of the sample solutions 1 to 5 was determined by measuring the nephelometric turbidity units (NTU) in triple determination. Device: Turb 430 IR ex WTW.

**Table 10:**

| **Sample** | **1** | **2** | **3** | **4** | **5** |
|---|---|---|---|---|---|
| | 501 | 586 | 335 | 48.5 | >1100 |
| | 434 | 571 | 314 | 46.4 | >1100 |
| | 354 | 575 | 288 | 46.2 | >1100 |
| **Mean value (NTU)** | **430** | **577** | **312** | **47.0** | **>1100** |

The results of the nephelometric turbidity units (NTU) of samples 1 to 5 are shown in Figure 9.

Surprisingly sample 4 (containing 1,2-heptanediol) showed the lowest turbidity unit which indicates highest clarity in comparison to samples 2 (containing 1,2-pentanediol), 3 (containing (1,2-hexanediol) and 5 (containing 1,2-octanediol). Sample 1 without 1,2-alkanediol serves as a comparative sample.

### Example 1.6: Solubility improvement for Retinyl Palmitate (not in accordance with the present invention)

The following aqueous/glycolic formulation was used to demonstrate the solubilizing properties of 1,2-pentanediol, 1,2-hexanediol, 1,2-heptanediol and 1,2-octanediol.

**Table 11:**

| | Raw **material** | **INCI** | **1** | **2** | **3** | **4** | **5** |
|---|---|---|---|---|---|---|---|
| A. | Butylene Glycol | Butylene Glycol | 2.0 | 2.0 | 2.0 | 2.0 | 2.0 |
| | Solubilizer #660352 | PEG-40 Hydrogenated Castor Oil, Trideceth-9, Propylene Glycol, Aqua | 1.0 | 1.0 | 1.0 | 1.0 | 1.0 |
| | 1,2-pentanediol | Pentylene Glycol | - | 0.5 | - | - | - |
| | 1,2-hexanediol | 1,2 Hexanediol | - | - | 0.5 | - | - |
| | 1,2-heptanediol | 1,2 Heptanediol | - | - | - | 0.5 | - |
| | 1,2-octanediol | Caprylyl Glycol | - | - | - | - | 0.5 |
| B. | [(2E,4E,6E,8E)-3,7-Dimethyl-9-(2,6,6-trimethyl-1-cyclohexenyl)nona-2,4,6,8-tetraenyl] hexadecanoate | Retinyl Palmitate | 0.1 | 0.1 | 0.1 | 0.1 | 0.1 |
| C. | Water, dem. | Water (Aqua) | Ad 100 | | | | |
| | Comment | | T++ | T++ | T++ | T+ | T++ |

Production method: A premix A was prepared by mixing solvent and solubilizer with Vortex for 20 seconds. Then diol was added to the premix. The active component was added to the premix and mixed with magnetic stirrer for 30 seconds. Water was added and the resulting mixture was stirred for 5 minutes. Hydrolite 8 was added to the mixture and heated up to 40 °C to solve the Caprylyl Glycol.

The visual appearance of the sample solutions was evaluated according to the following scale:

| **Description** | **Coding** |
|---|---|
| Slight turbidity | T- |
| Turbid | T |
| Strong turbidity | T+ |
| Very strong turbidity | T++ |

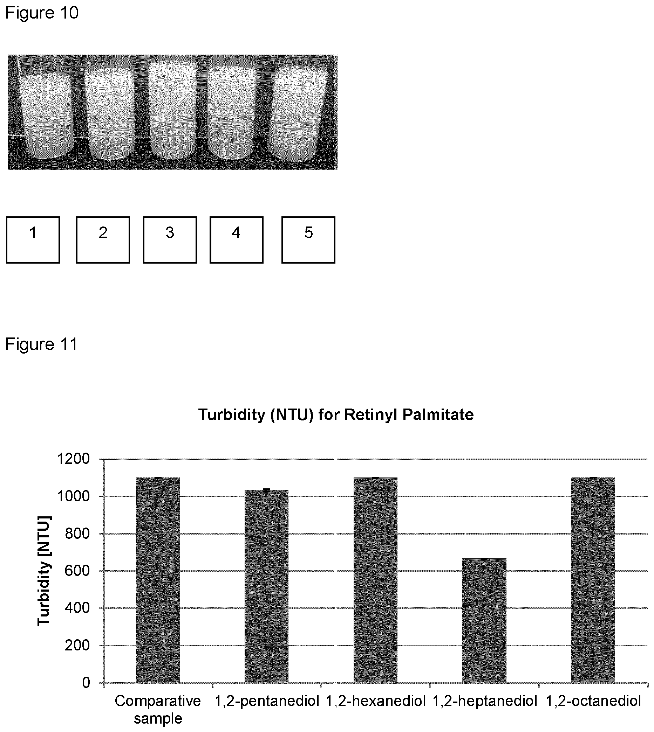

The images of the sample solutions 1 (comparative), 2 (containing 1,2-pentanediol), 3 (containing 1,2-hexanediol), 4 (containing 1,2-heptanediol) and 5 (containing 1,2-octanediol) are shown in Figure10.

Additionally, the turbidity of the sample solutions 1 to 5 was determined by measuring the nephelometric turbidity units (NTU) in triple determination. Device: Turb 430 IR ex WTW.

**Table 12:**

| **Sample** | **1** | **2** | **3** | **4** | **5** |
|---|---|---|---|---|---|
| | >1100 | 1041 | >1100 | 666 | >1100 |
| | >1100 | 1034 | >1100 | 665 | >1100 |
| | >1100 | 1028 | >1100 | 666 | >1100 |
| **Mean value (NTU)** | **>1100** | **1034** | **>1100** | **666** | **>1100** |

The results of the nephelometric turbidity units (NTU) of samples 1 to 5 are shown in Figure 11.

Surprisingly sample 4 (containing 1,2-heptanediol) showed the lowest turbidity unit which indicates highest clarity in comparison to samples 2 (containing 1,2-pentanediol), 3 (containing (1,2-hexanediol) and 5 (containing 1,2-octanediol). Sample 1 without 1,2-alkanediol serves as a comparative sample.

### Example 1.7: Solubility improvement for Glyceryl Caprylate (not in accordance with the present invention)

The following aqueous/glycolic formulation was used to demonstrate the solubilizing properties of 1,2-pentanediol, 1,2-hexanediol, 1,2-heptanediol and 1,2-octanediol.

**Table 13:**

| | **Raw material** | **INCI** | **1** | **2** | **3** | **4** | **5** |
|---|---|---|---|---|---|---|---|
| A. | Butylene Glycol | Butylene Glycol | 1.0 | 1.0 | 1.0 | 1.0 | 1.0 |
| | Solubilizer #660352 | PEG-40 Hydrogenated Castor Oil, Trideceth-9, Propylene Glycol, Aqua | 0.4 | 0.4 | 0.4 | 0.4 | 0.4 |
| | 1,2-pentanediol | Pentylene Glycol | - | 0.5 | - | - | - |
| | 1,2-hexanediol | 1,2 Hexanediol | - | - | 0.5 | - | - |
| | 1,2-heptanediol | 1,2 Heptanediol | - | - | - | 0.5 | - |
| | 1,2-octanediol | Caprylyl Glycol | - | - | - | - | 0.5 |
| B. | 2,3-dihydroxypropyl octanoate | Glyceryl Caprylate | 0.5 | 0.5 | 0.5 | 0.5 | 0.5 |
| C. | Water, dem. | Water (Aqua) | Ad 100 | | | | |
| | Comment | | T | T | T | T- | T |

Production method: A premix A was prepared by mixing solvent, solubilizer and diol. Glyceryl Caprylate was added. The mixture was heated to 50 °C to solve the active component while stirring with a magnetic stirrer. The mixture was stirred for further 5 minutes at 50 °C. Water was added and the mixture was cooled while stirring (10 min).

The visual appearance of the sample solutions was evaluated according to the following scale:

| **Description** | **Coding** |
|---|---|
| Slight turbidity | T- |
| Turbid | T |
| Strong turbidity | T+ |
| Very strong turbidity | T++ |

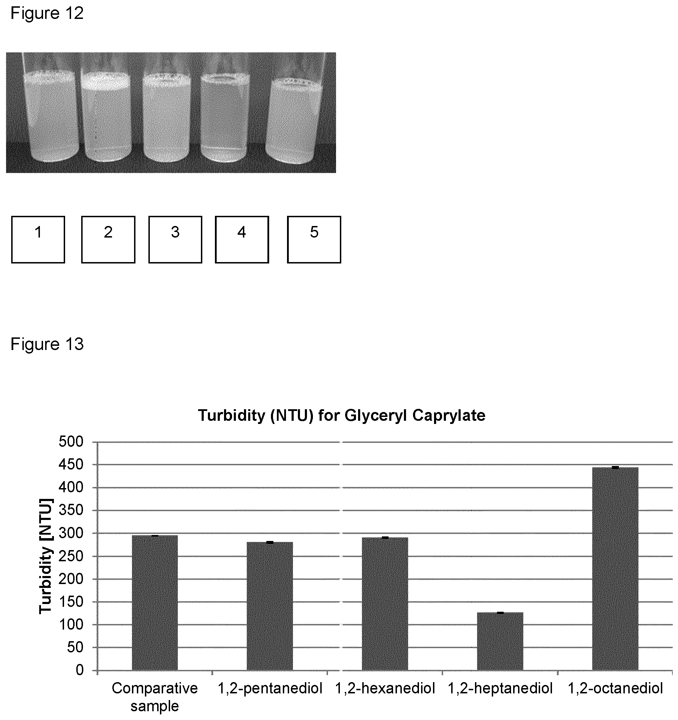

The images of the sample solutions 1 (comparative sample), 2 (containing 1,2-pentanediol), 3 (containing 1,2-hexanediol), 4 (containing 1,2-heptanediol) and 5 (containing 1,2-octanediol) are shown in Figure 12.

Additionally, the turbidity of the sample solutions 1 to 5 was determined by measuring the nephelometric turbidity units (NTU) in triple determination. Device: Turb 430 IR ex WTW.

**Table 14:**

| **Sample** | **1** | **2** | **3** | **4** | **5** |
|---|---|---|---|---|---|
| | 295 | 281 | 291 | 126 | 442 |
| | 295 | 282 | 291 | 127 | 446 |
| | 295 | 279 | 289 | 127 | 444 |
| **Mean value (NTU)** | **295** | **281** | **290** | **127** | **444** |

The results of the nephelometric turbidity units (NTU) of samples 1 to 5 are shown in Figure13.

Surprisingly sample 4 (containing 1,2-heptanediol) showed the lowest turbidity unit which indicates highest clarity in comparison to samples 2 (containing 1,2-pentanediol), 3 (containing (1,2-hexanediol) and 5 (containing 1,2-octanediol). Sample 1 without 1,2-alkanediol serves as a comparative sample.

### Example 1.8: Solubility improvement for Hydroxyacetophenone (not in accordance with the present invention)

The following aqueous formulation was used to demonstrate the solubilizing properties of 1,2-pentanediol, 1,2-hexanediol, 1,2-heptanediol and 1,2-octanediol.

**Table 15:**

| | **Raw material** | **INCI** | **1** | **2** | **3** | **4** | **5** |
|---|---|---|---|---|---|---|---|
| A. | 1,2-pentanediol | Pentylene Glycol | - | 1.0 | - | - | - |
| | 1,2-hexanediol | 1,2 Hexanediol | - | - | 1.0 | - | - |
| | 1,2-heptanediol | 1,2 Heptanediol | - | - | - | 1.0 | - |
| | 1,2-octanediol | Caprylyl Glycol | - | - | - | - | 1.0 |
| B. | 1-(4-hydroxyphenyl)ethanone | Hydroxyacetophenone | 1.0 | 1.0 | 1.0 | 1.0 | 1.0 |
| C. | Water, dem. | Water (Aqua) | Ad 100 | | | | |
| | Comment | | S++ | S++ | S | S | T+ |

Production method: The ingredients were mixed in the given order and stirred for a minimum of 20 minutes to solve the SymSave^{®} H. If necessary, the mixture was heated to 50°C.

The visual appearance of the sample solutions was evaluated according to the following scale:

| **Description** | **Coding** |
|---|---|
| Strong sedimentation | S++ |
| Sedimentation | S+ |
| Slight sedimentation | S |
| Strong turbidity | T+ |

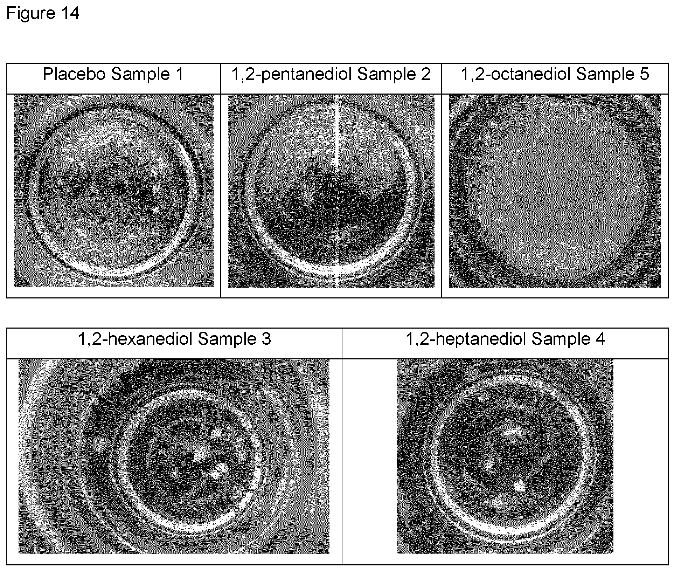

The images of the sample solutions 1 (comparative sample), 2 (containing 1,2-pentanediol), 3 (containing 1,2-hexanediol), 4 (containing 1,2-heptanediol) and 5 (containing 1,2-octanediol) are shown in Figure 14.

Turbidity measurements of samples 1 to 5 were not possible due to crystallizations/sedimentation.

Sample 4 (containing 1,2-heptanediol) showed the lowest amount of crystallization/sedimentation of 2.7 mg and the highest transparency. Sample 1 (Placebo) and sample 2 (containing 1,2-pentanediol) showed higher amounts of crystallization/sedimentation. Sample 3 (containing 1,2-hexanediol) shows high transparency, but higher crystallization (13 crystals) compared to sample 4 (containing 1,2-heptanediol). Sample 5 (containing 1,2-octanediol) shows strong turbidity, so that the amount of crystals could not be evaluated.

The results of the above tests clearly demonstrate that 1,2-heptanediol has better solubility improving properties for lipophilic active components irrespective of their chemical structure in comparison to 1,2-pentanediol, 1,2-hexanediol and 1,2-octanediol.

### Example 1.9: Solubility improvement for Bisabolol

The following aqueous/glycolic formulation was used to demonstrate the solubilizing properties of 1,2-heptanediol, 2,3-heptanediol and a mixture including 1,2-heptanediol/2,3-heptanediol.

**Table 16:**

| | **Raw material** | **INCI** | **A1** | **A2** | **A3** | **A4** |
|---|---|---|---|---|---|---|
| A.. | 1,3 Butandiol | Butylene Glycol | 1.0 | 1.0 | 1.0 | 1.0 |
| | Solubilizer (660352) | PEG-40 Hydrogenated Castor Oil, Trideceth-9, Propylene Glycol, Aqua | 0.6 | 0.6 | 0.6 | 0.6 |
| | 1,2-heptanediol | 1,2-heptanediol | - | 0.5 | - | 0.25 |
| | 2,3-heptanediol | 2,3-heptanediol | - | - | 0.5 | 0.25 |
| B. | Dragosantol 100 | Bisabolol | 0.3 | 0.3 | 0.3 | 0.3 |
| C.. | Water dem. | Water (Aqua) | Add 100 | | | |
| | Comment | | T+ | T+ | T+ | T |

Production method: Phase A was presolved by using a vortex stirrer. Then phase B (active component) was added to phase A and dissolved. Furthermore, phase C was added to the mixture A/B by stirring.

The visual appearance of the sample solutions was evaluated according to the following scale:

| **Description** | **Coding** |
|---|---|
| Slight turbidity | T- |
| Turbid | T |
| Strong turbidity | T+ |
| Very strong turbidity | T++ |

Additionally, the turbidity of the sample solutions A1 to A4 was determined by measuring the nephelometric turbidity units (NTU) in triple determination. Device: Turb 430 IR ex WTW.

**Table 17:**

| **Sample** | **A1** | **A2** | **A3** | **A4** |
|---|---|---|---|---|
| | 413 | 369 | 468 | 263 |
| | 413 | 373 | 464 | 253 |
| | 409 | 370 | 472 | 246 |
| **Mean value (NTU)** | **412** | **371** | **468** | **254** |

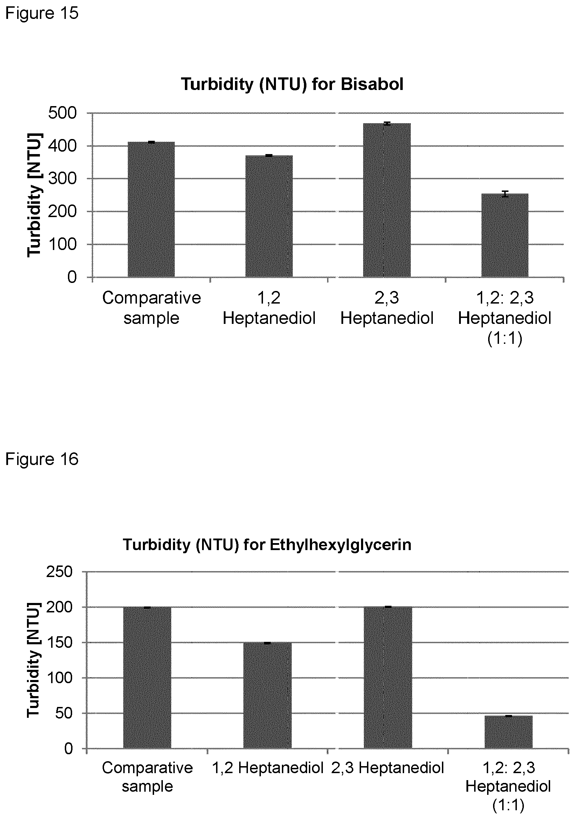

The results of the nephelometric turbidity units (NTU) of samples A1 to A4 are shown in Figure 15.

Sample A4 containing a blend of 1,2-heptanediol and 2,3-heptanediol showed the lowest value of turbidity unit, which indicates highest clarity in comparison to examples A2 and A3 containing 1,2-heptanediol and 2,3-heptanediol alone. Sample A1 without alkanediol serves as a comparative sample and showed the highest NTU, which indicates highest turbidity.

### Example 1.10: Solubility improvement for Ethylhexylglycerin

The following aqueous/glycolic formulation was used to demonstrate the solubilizing properties of 1,2-heptanediol, 2,3-heptanediol and a mixture including 1,2-heptanediol/2,3-heptanediol.

**Table 18:**

| | **Raw material** | **INCI** | **B1** | **B2** | **B3** | **B4** |
|---|---|---|---|---|---|---|
| A. | Dipropylenglycol | Dipropylene Glycol | 5.0 | 5.0 | 5.0 | 5.0 |
| | Solubilizer (660352) | PEG-40 Hydrogenated Castor Oil, Trideceth-9, Propylene Glycol, Aqua | 0.25 | 0.25 | 0.25 | 0.25 |
| | 1,2-heptanediol | 1,2-heptanediol | - | 0.5 | - | 0.25 |
| | 2,3-heptanediol | 2,3-heptanediol | - | - | 0.5 | 0.25 |
| B.. | Hydrolex E | Ethylhexylglycerin | 0.4 | 0.4 | 0.4 | 0.4 |
| C.. | Water dem. | Water (Aqua) | Add 100 | | | |
| | Comment | | T+ | T+ | T+ | T |

Production method: Phase A was presolved by using a vortex stirrer. Then phase B (active component) was added to phase A and dissolved. Next, phase C was added to the mixture A/B by stirring.

The visual appearance of the sample solutions was evaluated according to the following scale:

| **Description** | **Coding** |
|---|---|
| Slight turbidity | T- |
| Turbid | T |
| Strong turbidity | T+ |
| Very strong turbidity | T++ |

Additionally, the turbidity of the sample solutions B1 to B4 was determined by measuring the nephelometric turbidity units (NTU) in triple determination. Device: Turb 430 IR ex WTW.

**Table 19:**

| **Sample** | **B1** | **B2** | **B3** | **B4** |
|---|---|---|---|---|
| | 200 | 150 | 201 | 46.8 |
| | 199 | 149 | 201 | 45.9 |
| | 200 | 149 | 200 | 45.6 |
| **Mean value (NTU)** | **200** | **149** | **201** | **46** |

The results of the nephelometric turbidity units (NTU) of samples A1 to A4 are shown in Figure 16.

Sample B4, containing a mixture of 1,2-heptanediol and 2,3-heptanediol (ratio 1:1) showed the lowest value of turbidity unit which indicates highest clarity in comparison to examples B2 and B3 containing 1,2-heptanediol and 2,3 heptanediol alone. Sample B1 without alkanediol serves as a comparative sample and showed the highest turbidity unit.

### Example 1.11: Solubility improvement for Ethylhexylglycerin

The following aqueous/glycolic formulation was used to demonstrate the solubilizing properties of 1,2-octanediol, 2,3-octanediol and a mixture including 1,2-octanediol/2,3-octanediol.

**Table 20:**

| | **Raw material** | **INCI** | **C1** | **C2** | **C3** | **C4** |
|---|---|---|---|---|---|---|
| A.. | Dipropylenglycol | Dipropylene Glycol | 1.5 | 1.5 | 1.5 | 1.5 |
| | Solubilizer (660352) | PEG-40 Hydrogenated Castor Oil, Trideceth-9, Propylene Glycol, Aqua | 0.4 | 0.4 | 0.4 | 0.4 |
| | 1,2-octanediol | Caprylyl Glycol | - | 0.1 | | 0.05 |
| | 2,3-octanediol | 2,3-octanediol | - | - | 0.1 | 0.05 |
| B. | Hydrolex E | Ethylhexylglycerin | 0.5 | 0.5 | 0.5 | 0.5 |
| C. | Water dem. | Water (Aqua) | Add 100 | | | |
| | Comment | | T | T | T | T- |

Production method: Phase A was presolved by warming up to 60 °C and stirring. Then, phase B was added to phase A and dissolved. Phase C was added to the mixture A/B by stirring.

The visual appearance of the sample solutions was evaluated according to the following scale:

| **Description** | **Coding** |
|---|---|
| Slight turbidity | T- |
| Turbid | T |
| Strong turbidity | T+ |
| Very strong turbidity | T++ |

Additionally, the turbidity of the sample solutions C1 to C4 was determined by measuring the nephelometric turbidity units (NTU) in triple determination. Device: Turb 430 IR ex WTW.

**Table 21:**

| **Sample** | **C1** | **C2** | **C3** | **C4** |
|---|---|---|---|---|
| | 418 | 169 | 272 | 55 |
| | 423 | 180 | 270 | 56 |
| | 420 | 175 | 264 | 53 |
| **Mean value (NTU)** | **420** | **175** | **269** | **55** |

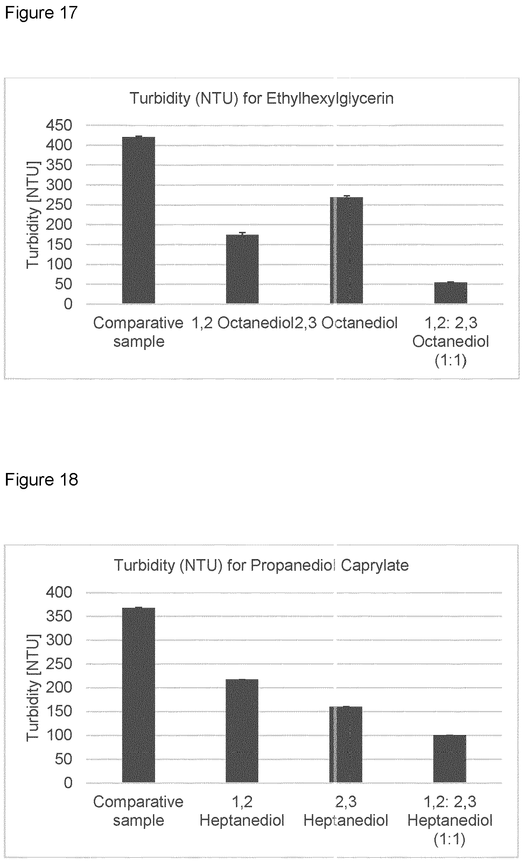

The results of the nephelometric turbidity units (NTU) of samples A1 to A4 are shown in Figure 17.

Sample C4 containing a mixture of 1,2-octanediol and 2,3-octanediol (ratio 1:1) showed the lowest turbidity unit which indicates highest clarity in comparison to examples C2 and C3, containing 1,2-octanediol and 2,3-octanediol alone. Sample C1 without alkanediol (comparative sample) showed the highest turbidity.

### Example 1.12: Solubility improvement for Propandiol Caprylate

The following aqueous/glycolic formulation was used to demonstrate the solubilizing properties of 1,2-heptanediol, 2,3-heptanediol and a mixture including 1,2-heptanediol /2,3-heptanediol.

**Table 22:**

| | **Raw material** | **INCI** | **D1** | **D2** | **D3** | **D4** |
|---|---|---|---|---|---|---|
| A. | 1,3 Butandiol | Butylene Glycol | 1.0 | 1.0 | 1.0 | 1.0 |
| | Solubilizer (660352) | PEG-40 Hydrogenated Castor Oil, Trideceth-9, Propylene Glycol, Aqua | 0.8 | 0.8 | 0.8 | 0.8 |
| | 1,2 Heptanediol | 1,2 Heptanediol | - | 0.5 | - | 0.25 |
| | 2,3 Heptanediol | 2,3- Heptanediol | - | - | 0.5 | 0.25 |
| B.. | Propanediol Monocaprylate | Propanediol caprylate | 0.25 | 0.25 | 0.25 | 0.25 |
| C.. | Water dem. | Water (Aqua) | Add 100 | | | |
| | Comment | | T | T | T | T- |

Production method: Phase A was presolved by stirring. Then, phase B was added to phase A and dissolved. Phase C was added to the mixture A/B by stirring.

The visual appearance of the sample solutions was evaluated according to the following scale:

| **Description** | **Coding** |
|---|---|
| Slight turbidity | T- |
| Turbid | T |
| Strong turbidity | T+ |
| Very strong turbidity | T++ |

Additionally, the turbidity of the sample solutions D1 to D4 was determined by measuring the nephelometric turbidity units (NTU) in triple determination. Device: Turb 430 IR ex WTW.

**Table 23:**

| **Sample** | **D1** | **D2** | **D3** | **D4** |
|---|---|---|---|---|
| | 367 | 217 | 160 | 99.3 |
| | 369 | 217 | 160 | 99.9 |
| | 368 | 217 | 159 | 100.0 |
| **Mean value (NTU)** | **368** | **217** | **160** | **99.7** |

The results of the nephelometric turbidity units (NTU) of samples A1 to A4 are shown in Figure 18.

Sample D4, containing the blend of 1,2-heptanediol and 2,3-heptanediol showed the lowest turbidity unit which indicates highest clarity in comparison to examples D2 and D3 containing 1,2-heptanediol and 2,3- heptanediol alone. Sample D1 (comparative sample) showed the highest turbidity.

### Example 1.13: Solubility improvement for Propandiol Caprylate

The following aqueous/glycolic formulation was used to demonstrate the solubilizing properties of 1,2-octanediol, 2,3-octanediol and a mixture including 1,2-octanediol /2,3-octanediol.

**Table 24:**

| | **Raw material** | **INCI** | **E1** | **E2** | **E3** | **E4** |
|---|---|---|---|---|---|---|
| A. | 1,3 Butandiol | Butylene Glycol | 1.0 | 1.0 | 1.0 | 1.0 |
| | Solubilizer (660352) | PEG-40 Hydrogenated Castor Oil, Trideceth-9, Propylene Glycol, Aqua | 0.8 | 0.8 | 0.8 | 0.8 |
| | 1,2 Octanediol | Capryly Glycol | - | 0.4 | - | 0.2 |
| | 2,3 Octanediol | 2,3 Octanediol | - | - | 0.4 | 0.2 |
| B. | Propanediol Monocaprylate | Propanediol caprylate | 0.5 | 0.5 | 0.5 | 0.5 |
| C.. | Water dem. | Water (Aqua) | Add 100 | | | |
| | Comment | | T | T | T | T- |

Production method: A Phase A was presolved by warming up to 60 °C and stirring. Then, phase B was added to phase A and dissolved. Phase C was added to the mixture A/B by stirring.

The visual appearance of the sample solutions was evaluated according to the following scale:

| **Description** | **Coding** |
|---|---|
| Slight turbidity | T- |
| Turbid | T |
| Strong turbidity | T+ |
| Very strong turbidity | T++ |

Additionally, the turbidity of the sample solutions E1 to E4 was determined by measuring the nephelometric turbidity units (NTU) in triple determination. Device: Turb 430 IR ex WTW.

**Table 25:**

| **Sample** | **D1** | **D2** | **D3** | **D4** |
|---|---|---|---|---|
| | 367 | 217 | 160 | 99.3 |
| | 369 | 217 | 160 | 99.9 |
| | 368 | 217 | 159 | 100.0 |
| **Mean value (NTU)** | **368** | **217** | **160** | **99.7** |

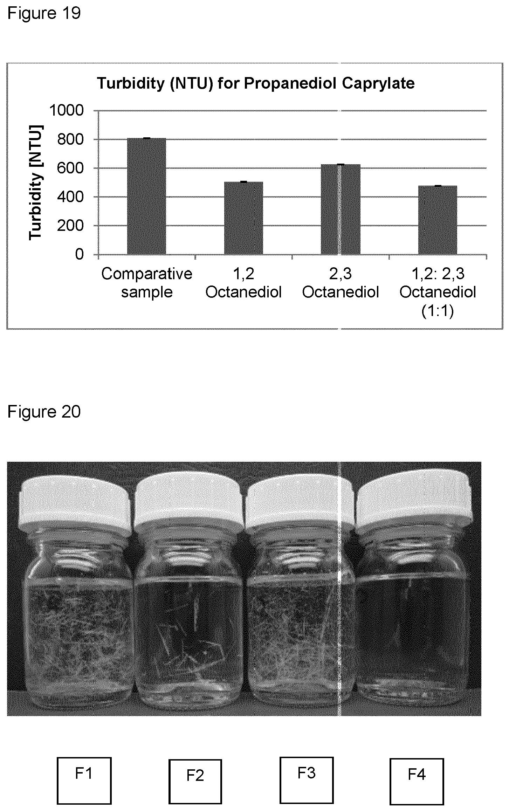

The results of the nephelometric turbidity units (NTU) of samples A1 to A4 are shown in Figure 19.

Sample E4, containing a blend of 1,2-octanediol and 2,3-octanediol showed the lowest turbidity unit which indicates highest clarity in comparison to examples E2 and E3, containing 1,2-octanediol and 2,3-octanediol. Sample E1 without alkanediol serves as a comparative sample and showed the highest turbidity.

### Example 1.14: Solubility improvement for o-Cymen-5-ol

The following aqueous/glycolic formulation was used to demonstrate the solubilizing properties of 1,2-heptanediol, 2,3-heptanediol and a mixture including 1,2-heptanediol /2,3-heptanediol.

**Table 26:**

| | **Raw material** | **INCI** | **F1** | **F2** | **F3** | **F4** |
|---|---|---|---|---|---|---|
| A. | 1,2-heptanediol | 1,2-heptanediol | - | 0.4 | - | 0.2 |
| | 2,3-heptanediol | 2,3-heptanediol | - | - | 0.4 | 0.2 |
| | 3-methyl-4-propan-2-yl phenol | o-Cymen-5-ol | 0.1 | 0.1 | 0.1 | 0.1 |
| B. | Ethanol | Ethanol | 10.00 | 10.00 | 10.00 | 10.00 |
| C. | Water dem. | Water (Aqua) | Ad 100 | | | |
| | 24h 5°C | | S+ | S | S+ | C |

Production method: Phase A was presolved by stirring. Then, phase B was added to phase A and dissolved. Phase C was added to the mixture A/B by stirring.

The visual appearance of the sample solutions was evaluated according to the following scale:

| **Description** | **Coding** |
|---|---|
| Very strong sedimentation | S++ |
| Strong Sedimentation | S+ |
| Sedimentation | S |
| Clear | C |

Samples were evaluated after 24h storage at 5 °C with respect to recrystallization.

The images of the sample solutions F1 (comparative), F2 (containing 1,2-heptanediol), F3 (containing 2,3-heptanediol) and F4 (containing a mixture of 1,2-heptanediol and 2,3-heptanediol) are shown in Figure 20.

Sample F4 comprising a mixture of 1,2-heptanediol and 2,3-heptanediol showed no recrystallization after 24 h. The samples F2 and F3 showed recrystallization/sedimentation after 24 h. Sample F1 without alkanediol serves as a comparative sample and showed the strongest sedimentation.

### Example 2: Solubility of alkanediols in water

For the determination of the maximum water solubility of an alkanediol, water and different concentrations of the respective alkanediol were blended by stirring. The assessment of solubility was carried out at ambient temperature after 24 h storage at 5 °C. The determination of whether the alkanediol has dissolved in water is based entirely on visual observation. An alkanediol compound has dissolved if the mixture is clear and shows no signs of cloudiness or precipitation.

**Table 27:**

| **Tradename/ Chemical name** | **INCI** | **Maximum solubility in water (% by weight)** |
|---|---|---|
| Hydrolite^{®} 5 green | Pentylene Glycol | 50 |
| Hydrolite^{®} 6 | Hexanediol | 50 |
| 1,2-heptanediol | Heptanediol | 2.5 |
| Hydrolite^{®} 8 | Caprylyl Glycol | 0.5 |
| SymDiol^{®} 68 | Hexanediol, Caprylyl Glycol | 1.0 |
| 1,2-nonanediol | Nonanediol | 0.1 |
| SymClariol^{®} | Decylene Glycol | - |
| 1,2-undecanediol | Undecanediol | - |
| 2,3-heptanediol | - | 3.6 |
| 2,3-octanediol | - | 1.5 |
| 2,3-nonanediol | - | 0.3 |
| 2,3-undecanediol | - | - |

### Example 3: Formulation examples

The following formulations according to the present invention were prepared:

**Table 28: Formulations - overview**

| **Category** | **Formulation** |
|---|---|
| Perfume oil | Composition of perfume oil 1; PO1 (amounts in ‰ by weight) |
| | Composition of perfume oil 2; PO2 (amounts in ‰ by weight) |
| | Composition of perfume oil 3; PO3 (amounts in ‰ by weight) |
| | Composition of perfume oil 4; PO4 (amounts in ‰ by weight) |
| Deo | Deodorant Spray |
| | Deodorant Spray with AcH |
| | Deodorant Roll-on |
| | Antiperspirant/deodorant roll on |
| | Deo formulation in form of a roll-on gel |
| | Clear deo anti-perspirant roll-on |
| | Deodorant Aerosol |
| | Deodorant stick |
| | Antiperspirant stick |
| Aroma | Peppermint flavor |
| Oral Care | Protective Mouth Rinse |
| | Refresh your breath Mouth Spray |
| | Mouthwash (Ready to use) without alcohol |
| | Mouthwash concentrate |
| Gel & Spray | Intimate Sensi-Gel |
| | Mild feminie wash |
| | Intimate Rescue Gel |
| | Cooling and Deodorizing Intimate Femine Spray |
| | After Shave Tonic |
| | After Sun Refreshing Gel |
| Hair Care | Fresh Hair shampoo |
| | Scalp soothing with UV-B/UV-A protection, rinse-off |
| | Revitalizing CONDITIONER |
| | Hair Soap SHAMPOO Bar |
| | Conditioner Spray Breath fresh air |
| | Hair Refresher Mist |
| | Virgin Mojito Pre-Shampoo |
| | Sensi SCALP (Green Solid Shampoo) |
| | Shampoo sulfate free |
| | Hair conditioner with UV protection |
| | Hair conditioner, leave on |
| | Anti-itch hair conditioner, leave on |
| | Sprayable hair conditioner with zinc pyrithrione, leave-on |
| | Hair styling gel |
| Emulsion | Aseptic wound cream |
| | Eye contour serum |
| | Intensive Care, Detox& Repair Hand Cream 2-in-1 |
| Mask | Liquid for sheet mask- New skin |
| Wipes | Clear Anti Acne Wipe |
| | PEG-Free Wet Wipe |
| | Baby Soft Wet Wipes |
| | Intimate Wipes |
| | After Shave Wet Wipes |

In the following formulations examples the following five perfume oils PFO1, PFO2, PFO3, PFO4 or PFO5 were each used as fragrance.

**Table 29: Composition of perfume oil 1; PO1 (amounts in ‰ by weight)**

| **Ingredients** | **Amount** |
|---|---|
| ALDEHYDE C14 SO-CALLED | 2 |
| ALLYL AMYL GLYCOLATE 10% DPG | 5 |
| ANISIC ALDEHYDE PURE | 5 |
| APPLE OLIFFAC TYPE | 10 |
| BENZYLACETATE | 50 |
| BERGAMOT IDENTOIL^{®} COLOURLESS | 15 |
| CANTHOXAL | 5 |
| CETALOX 10% IPM | 3 |
| CITRONELLOL 950 | 40 |
| DAMASCENONE TOTAL 1% DPG | 5 |
| DAMASCONE ALPHA 10% DPG | 5 |
| DAMASCONE DELTA 10% DPG | 2 |
| DIMETHYL BENZYL CARBINYL BUTYRATE | 2 |
| DIPROPYLENE GLYCOL | 178 |
| EBANOL | 2 |
| ETHYL DECADIENOATE TRANS CIS-2,4 10% IPM | 2 |
| FLOROSA | 5 |
| FRAMBINON^{®} 10% DPG | 7 |
| GALAXOLIDE 50% IN IPM | 100 |
| GALBEX TYPE BASE | 1 |
| GERANYL ACETATE PURE | 2 |
| HEDIONE | 30 |
| HELIOTROPIN | 10 |
| HEXENYL ACETATE CIS-3 10% DPG | 1 |
| HEXENYL SALICYLATE CIS-3 | 5 |
| HEXYL CINNAMIC ALDEHYDE ALPHA | 70 |
| HEXYL SALICYLATE | 50 |
| HYDROXY CITRONELLAL | 10 |
| ISO E SUPER | 15 |
| ISORALDEINE 70 | 20 |
| LEAFOVERT^{®} | 1 |
| LILIAL | 60 |
| LINALOOL | 60 |
| LINALYL ACETATE | 20 |
| LYRAL | 7 |
| MANZANATE | 2 |
| PHENOXANOL | 7 |
| PHENYLETHYL ALCOHOL | 120 |
| SANDAL MYSORE CORE | 2 |
| SANDRANOL^{®} | 7 |
| STYRALYL ACETATE | 3 |
| TAGETES RCO 10% TEC | 2 |
| TERPINEOL PURE | 20 |
| TETRAHYDROGERANIOL 10% DPG | 5 |
| TONALIDE | 7 |
| VERTOCITRAL 10% DPG | 5 |
| VERTOFIX | 15 |
| **Total:** | **1000** |

**Table 30: Composition of perfume oil 2; PO2 (amounts in ‰ by weight)**

| **Ingredients** | **Amount** |
|---|---|
| Acetophenone, 10% in DPG | 10 |
| n-Undecanal | 5 |
| Aldehyde C14, so-called (peach aldehyde) | 15 |
| Allylamyl glycolate, 10% in DPG | 20 |
| Amyl salicylate | 25 |
| Benzyl acetate | 60 |
| Citronellol | 80 |
| d-Limonene | 50 |
| Decenol trans-9 | 15 |
| Dihydromyrcenol | 50 |
| Dimethylbenzylcarbinyl acetate | 30 |
| Diphenyloxide | 5 |
| Eucalyptol | 10 |
| Geraniol | 40 |
| Nerol | 20 |
| Geranium oil | 15 |
| Hexenol cis-3, 10% in DPG | 5 |
| Hexenyl salicylate cis-3 | 20 |
| Indole, 10% in DPG | 10 |
| Alpha-ionone | 15 |
| Beta-ionone | 5 |
| Lilial^{®} (2-methyl-3-(4-tert-butyl-phenyl)propanal) | 60 |
| Linalool | 40 |
| Methylphenyl acetate | 10 |
| Phenylethyl alcohol | 275 |
| Styrolyl acetate | 20 |
| Terpineol | 30 |
| Tetrahydrolinalool | 50 |
| Cinnamyl alcohol | 10 |
| **Total:** | **1000** |

**Table 31: Composition of perfume oil 3; PO3 (amounts in ‰ by weight)**

| **Ingredients** | **Amount** |
|---|---|
| Benzyl acetate | 60 |
| Citronellyl acetate | 60 |
| Cyclamenaldehyde (2-methyl-3-(4-isopropylphenyl)propanal | 20 |
| Dipropylene glycol (DPG) | 60 |
| Ethyllinalool | 40 |
| Florol (2-isobutyl-4-methyltetrahydro-2*H*-pyran-4-ol) | 30 |
| Globanone^{®} [(E/Z)-8-cyclohexadecen-1-one] | 180 |
| Hedione^{®} (methyldihydrojasmonate) | 140 |
| Hexenyl salicylate, cis-3 | 10 |
| Vertocitral (2,4-dimethyl-3-cyclohexenecarboxaldehyde) | 5 |
| Hydratropaldehyde, 10% in DPG | 5 |
| Isodamascone (1-(2,4,4-trimethyl-2-cyclohexen-1-yl)-2-buten-1-one, 10% in DPG | 5 |
| Isomuscone (cyclohexadecanone) | 40 |
| Jacinthaflor (2-methyl-4-phenyl-1,3-dioxolane) | 10 |
| Cis-jasmone, 10% in DPG | 20 |
| Linalool | 50 |
| Linalyl acetate | 30 |
| Methyl benzoate, 10% in DPG | 25 |
| para-Methyl cresol, 10% in DPG | 10 |
| Nerol | 20 |
| Phenylpropylaldehyde | 5 |
| 2-Phenylethyl alcohol | 82 |
| Tetrahydrogeraniol | 13 |
| 2,2-Dimethyl-3-cyclohexyl-1-propanol | 80 |
| **Total:** | **1000** |

**Table 32: Composition of perfume oil 4; PO4 (amounts in ‰ by weight)**

| **Ingredients** | **Amount** |
|---|---|
| AMBRETTOLIDE (MACRO) | 10 |
| AMBROXIDE 10% in IPM | 10 |
| BENZYL ACETATE | 20 |
| BENZYL SALICYLATE | 15 |
| BERGAMOT OIL. bergapten-free | 60 |
| CALONE^{®} 1951 10% in DPG | 15 |
| COUMARIN | 5 |
| CYCLOGALBANATE^{®} 10% in DPG | 10 |
| ALPHA -DAMASCONE 1% in DPG | 20 |
| DIHYDROMYRCENOL | 10 |
| ETHYL LINALOOL | 75 |
| ETHYL LINALYLACETATE | 50 |
| ETHYL MALTOL 1% in DEP | 10 |
| ETHYLENE BRASSYLATE (MACRO) | 80 |
| FLOROSA | 40 |
| GERANYLACETATE | 10 |
| HEDIONE^{®} HC/30 | 35 |
| HEDIONE^{®} | 210 |
| HELIONAL^{®} | 15 |
| HELVETOLIDE^{®} (ALICYC) | 30 |
| HEXENYLSALICYLATE CIS-3 | 20 |
| ISO E SUPER^{®} | 40 |
| LEAFOVERT^{®} 10% in DEP | 10 |
| LILIAL^{®} | 80 |
| LYRAL^{®} | 20 |
| MANDARIN OIL | 10 |
| STYRALYL ACETATE | 5 |
| SYMROSE^{®} | 15 |
| VANILLIN 10% in DEP | 20 |
| DIPROPYLENE GLYCOL (DPG) | 50 |
| **Total:** | **1000** |

**Table 33: Composition of perfume oil 5; PO5 (amounts in ‰ by weight)**

| **Ingredients** | **Amount** |
|---|---|
| AMAROCITE^{®} | 10 |
| AMBROCENIDE^{®} 10% in DPG | 5 |
| AMBROXIDE | 15 |
| AURELIONE^{®} (7/8-Cyclohexadecenone) (MACRO) | 70 |
| BERGAMOT OIL. bergapten-free | 90 |
| CALONE^{®} 1951 10% in DPG | 20 |
| CARAWAY OIL | 10 |
| CITRAL | 20 |
| COUMARIN | 10 |
| ALPHA-DAMASCONE 1% in DPG | 15 |
| DIHYDROMYRCENOL | 70 |
| ESTRAGON OIL | 10 |
| ETHYL LINALOOL | 100 |
| ETHYL LINALYLACETATE | 90 |
| EUGENOL | 10 |
| EVERNYL^{®} | 5 |
| FRUCTATE^{®} | 5 |
| GERANIUM OIL | 5 |
| HEDIONE^{®} HC/30 | 100 |
| HELIONAL^{®} | 10 |
| INDOLE 10% in DPG | 5 |
| ISO E SUPER^{®} | 100 |
| KEPHALIS^{®} | 5 |
| LAVENDER OIL | 40 |
| CITRUS OIL | 80 |
| LILIAL^{®} | 30 |
| MANDARIN OIL | 20 |
| MUSCENONE (MACRO) | 5 |
| SANDRANOL^{®} | 10 |
| VANILLIN 10% in DPG | 5 |
| DIPROPYLENE GLYCOL | 30 |
| **Total:** | **1000** |

The above perfume oils PO1, PO2, PO3, PO4, or PO5 were incorporated into the formulations presented below.

Cosmetic formulations (compositions) - amounts are indicated as % by weight for all formulations.

**Table 34: Deo/Antiperspirant Deodorant Spray**

| **Inaredients** | **EU INCI** | **Amount** |
|---|---|---|
| Aqua/Water | Aqua | ad 100 |
| Volarest^{™} FL | Acrylates/Beheneth-25 Methacrylate copolymer | 1.2 |
| Dracorin^{®} GOC | Glyceryl oleate citrate; Caprylic/Capric triglyceride | 2.0 |
| SymDeo^{®} Plus | Lauryl alcohol, Phenoxyethanol; 2-Benzylheptanol; Decylene Glycol | 0.5 |
| SymOcide^{®} PS | Phenoxyethanol; Decylene Glycol; 1,2-Hexanediol | 0.8 |
| Dow Corning^{®} 245 Fluid | Cyclomethicone | 5.0 |
| Isopropyl Myristate | Isopropyl myristate | 2.0 |
| Farnesol | Farnesol | 0.25 |
| Ethylhexylglycerin | Ethylhexylglycerin | 0.2 |
| SymDeo^{®} B125 | 2-Methyl 5-Cycloheylpentanol | 0.15 |
| Tego^{®} Cosmo P 813MB | Polyglyceryl-3 Caprylate | 0.25 |
| Perfume oil PO1, PO2, PO3, PO4, or PO5 | Perfume | 0.8 |
| 1,2-heptanediol, 2,3-heptanediol (w/w% 95:5) | 1,2-heptanediol, 2,3-heptanediol | 0.5 |
| 2,3-Nonanediol | 2,3 Nonanediol | 0.3 |
| Sodium Hydroxide, 10% solution | Aqua; Sodium hydroxide | 0.55 |

**Table 35: Deodorant Spray with AcH**

| **Ingredients** | **EU INCI** | **Amount** |
|---|---|---|
| Triethylcitrate | Triethylcitrate | 3.0 |
| Solubilizer^{®} | PEG-40 Hydrogenated Castor Oil, Trideceth-9, Propylene Glycol, Aqua | 1.0 |
| Ethanol 96% | Ethanol | 30.0 |
| 1,2-heptanediol | 1,2-heptanediol | 0.5 |
| 1,2-Octanediol, 2,3-Octanediol (w/w% 95:5) | Caprylyl Glycol, 2,3 Octanediol | 0.2 |
| Ethylhexylglycerin | Ethylhexylglycerin | 0.3 |
| SymDeo^{®} B125 | 2-Methyl 5-Cycloheylpentanol | 0.25 |
| SymClariol^{®} | Decylene Glycol | 0.25 |
| Tego^{®} Cosmo P 813MB | Polyglyceryl-3 Caprylate | 0.5 |
| Locron^{®} L (50%) | Aqua, Aluminum Chlorohydrate | 25.0 |
| Perfume oil PO1, PO2, PO3, PO4, or PO5 | Perfume | 0.5 |
| Water, demin. | Water | ad 100 |

**Table 36: Deodorant Roll-on**

| **Ingredients** | **EU INCI** | **Amount** |
|---|---|---|
| Aqua/ Water | Aqua | ad 100 |
| Hydrolite^{®} CG | Caprylyl Glycol | 0.3 |
| 1,2-heptanediol | 1,2-heptanediol | 0.5 |
| SymSave^{®} H | Hydroxyacetophenone | 0.5 |
| EDTA NA2 | Disodium EDTA | 0.1 |
| Dracorin^{®} GOC | Glyceryl oleate citrate; Caprylic/Capric triglyceride | 4.0 |
| Dragoxat^{®} 89 | Ethylhexyl isononanoate | 5.0 |
| SymMollient^{®} S | Cetearyl Nonanoate | 1.0 |
| Carbopol^{®} Ultrez 20 Polymer | Acrylates/C10-30 Alkyl acrylate crosspolymer | 0.2 |
| Pemulen^{™} TR-2 Polymeric Emulsifier | Acrylates/C10-30 Alkyl acrylate crosspolymer | 0.2 |
| Farnesol | Farnesol | 0.3 |
| SymGuard^{®} CD | Phenylpropanol, o-Cymen-5-ol, Decylene Glycol | 0.3 |
| Perfume oil PO1, PO2, PO3, PO4, or PO5 | Perfume | 1.0 |
| Sodium Hydroxide, 50% solution | Aqua; Sodium hydroxide | 0.2 |

**Table 37: Antiperspirant/deodorant roll-on**

| **Ingredients** | **INCI** | **Amount** |
|---|---|---|
| SymLite^{®}G8 | Glyceryl caprylate | 0.15 |
| Dragosantol^{®} 100 | Bisabolol | 0.1 |
| 1,2-Heptanediol, 2,3-heptanediol (w/w% 95:5) | 1,2-Heptanediol, 2,3-heptanediol | 0.6 |
| Ethanol 96 % | Ethanol | 30.0 |
| Farnesol | Farnesol | 0.5 |
| Perfume oil PO1, PO2, PO3, PO4, or PO5 | Perfume | 1.5 |
| Frescolat^{®}ML cryst. | Menthyl Lactate | 0.2 |
| Irgasan^{®} DP 300 | Triclosan | 0.3 |
| Natrosol^{™} 250 HHR | Hydroxyethyl-cellulose | 0.3 |
| Solubilizer | PEG-40 Hydrogenated Castor Oil, Trideceth-9, Water (Aqua) | 2.0 |
| SymDeo^{®} B125 | 2-Methyl 5-Cyclohexylpentanol | 0.5 |
| Water (demineralized) | Water (Aqua) | ad 100 |
| Zirkonal L 450 | Aluminium Zirconium Pentachlorohydrate (40 % aqueous solution) | 37.0 |

**Table 38: Deodorant formulation in the form of a roll-on gel**

| **Ingredients** | **INCI** | **Amount** |
|---|---|---|
| 1,3-butylene glycol | 1,3-butylene glycol | 2.0 |
| Cremophor^{®} CO 40 Surfactant | PEG-40-hydrogenated castor oil | 2.0 |
| Hydroxyethylcellulose | Hydroxyethylcellulose | 0.5 |
| Perfume oil PO1, PO2, PO3, PO4, or PO5 | Perfume | 0.3 |
| 1,3-propanediol | 1,3-prpanediol | 0.5 |
| SymGuard^{®} CD | 3-Phenylpropanol, o-cymen-3-ol, Decylene glycol | 0.4 |
| Ethylhexyl glycerin | Ethylhexyl glycerin | 0.1 |
| 1,2-Heptanediol, 2,3-heptanediol (w/w% 95:5) | 1,2-heptanediol, 2,3-heptanediol | 0.5 |
| SymLite^{®} G8 | Glyceryl Caprylate | 0.15 |
| Water | Aqua | ad 100 |

**Table 39: Clear deo anti-perspirant roll-on**

| **Ingredients** | **INCI** | **Amount** |
|---|---|---|
| Methocel^{™} E4M Premium | Hydroxypropyl Methylcellulose | 0.5 |
| Water | Water (Aqua) | ad 100 |
| Neo-PCL Water Soluble N | Trideceth-9, PEG-5 Ethylhexanoate, Water (Aqua) | 1.0 |
| Solubilizer | PEG-40 Hydrogenated Castor Oil, Trideceth-9, Propylene Glycol, Water (Aqua) | 3.0 |
| Deolite | Dimethyl Phenylpropanol, Pentylene Glycol | 0.5 |
| Locron^{®} LW | Aluminium Chlorohydrate | 25.0 |
| Aloe Vera Gel Concentrate 10/1 | Aloe Barbadensis Leaf Juice | 1.0 |
| 1,2-Propylene Glycol 99 P GC | Propylene Glycol | 4.0 |
| Ethanol 96 % | Alcohol Denat. | 30.0 |
| Perfume oil PO1, PO2, PO3, PO4, or PO5 | Perfume | 1.0 |
| 1,2-Heptanediol | 1,2-heptanediol | 0.95 |
| 2,3-Heptanediol | 2,3-Heptanediol | 0.05 |

**Table 40: Deodorant Aerosol (not in accordance with the present invention)**

| **Ingredients** | **EU INCI** | **Amount** |
|---|---|---|
| Belsil^{®} DM 0.65 | Disiloxane | ad 100 |
| Isoadipate | Diisopropyl adipate | 5.0 |
| Tegosoft^{®} TN | C12-15 Alkyl benzoate | 10.0 |
| Vitamin E Acetate | Tocopheryl acetate | 0.5 |
| Farnesol | Farnesol | 0.3 |
| Ethylhexylglycerin | Ethylhexylglycerin | 0.2 |
| 2,3-Heptanediol | 2,3-heptanediol | 0.3 |
| SymDeo^{®} MPP | Dimethyl Phenylbutanol | 0.2 |
| Perfume oil PO1, PO2, PO3, PO4, or PO5 | Perfume | 0.6 |

**Table 41: Deodorant stick (not in accordance with the present invention)**

| **Ingredients** | **INCI** | **Amount** |
|---|---|---|
| Sodium stearate | Sodium stearate | 8.0 |
| PPG-3 Myristyl ether | PPG-3 Myristyl ether | 70.0 |
| 1,2-propylene glycol | 1,2-propylene glycol | 10.0 |
| 1,1-dimethyl-3-phenylpropanol | 1,1-dimethyl-3-phenylpropanol | 0.2 |
| 2-butyloctanoic acid | 2-butyloctanoic acid | 0.2 |
| Perfume oil PO1, PO2, PO3, PO4, or PO5 | Perfumee | 0.6 |
| Water | Aqua | ad 100 |
| SymDeo^{®} Plus | Lauryl Alcohol, Phenoxyethanol, 2-Benzylheptanol, Decylene Glycol | 0.5 |
| SymLite^{®} G8 | Glyceryl Caprylate | 0.15 |
| 1,2-Heptanediol | 1,2-Heptanediol | 0.50 |

**Table 42: Antiperspirant stick**

| **Ingredients** | **INCI** | **Amount** |
|---|---|---|
| PCL Liquid^{®} 100 | Cetearyl ethylhexanonate | ad 100 |
| Silicone Fluid 345 | Cyclomethicone | 10.0 |
| CRODACOL^{™} C90 | Cetyl Alcohol | 8.0 |
| SYNCROWAX^{™} HGLC | C18-36 Triglyceride | 8.0 |
| CRODAMOL^{™} PTC | Pentaerythritol Tetracaprylate/Caprate | 5.0 |
| SYNCROWAX^{™} HRC | Tribehenin | 4.0 |
| Brij^{™} O5 | Oleth-5 | 1.0 |
| Titanium Dioxide | Titanium Dioxide | 1.0 |
| Rezal 36GP | Aluminium Tetrachlorohydrex GLY | 20.0 |
| Dry Flo C | Aluminium Starch Octenyl Succinate | 22.5 |
| Preservative | Phenoxyethanol | 0.8 |
| Perfume oil PO1, PO2, PO3, PO4, or PO5 | Perfume | 0.6 |
| 1,2-Hexanediol, 2,3-Hexanediol (w/w% 95:5) | 1,2 Hexanediol, 2,3 Hexanediol | 0.7 |
| 2,3 Octanediol | 2,3 Octanediol | 0.05 |
| SymLite^{®} G8 | Glyceryl caprylate | 0.15 |

The aroma composition (A1) described below was incorporated into the oral care formulations presented below.

**Table 43: Aroma composition (A1); Peppermint flavour (amounts in ‰ by weight)**

| **Ingredients** | **Amount** |
|---|---|
| Isobutyraldehyde | 0.5 |
| 3-Octanol | 0.5 |
| Dimethyl sulphide | 0.5 |
| Trans-2-Hexanal | 1.0 |
| Cis-3-Hexenol | 1.0 |
| 4-Terpineol, natural | 1.0 |
| Isopulegol | 1.0 |
| Piperitone, natural, from eucalyptus | 2.0 |
| Linalool | 3.0 |
| 8-Ocimenyl acetate 10% in triacetin | 5.0 |
| Isoamyl alcohol | 10.0 |
| Isovaleraldehyde | 10.0 |
| Alpha-Pinene, natural | 25.0 |
| Beta-Pinene, natural | 25.0 |
| Neomenthol. Racemic | 40.0 |
| Eucalyptiol (1.8-cineol).natural | 50.0 |
| L-Menthyl acetate of the formula D | 70.0 |
| L-Menthone | 220.0 |
| D-Isomenthone | 50.0 |
| L-Menthol | 483.5 |
| Nonenolide | 1.0 |
| **Total:** | **1000** |

**Table 44: Protective Mouth Rinse (not in accordance with the present invention)**

| **Ingredients** | **EU INCI** | **Amount** |
|---|---|---|
| Cremophor CO 40 Surfactant | PEG-40 Hydrogenated Castor oil | 3.0 |
| 1,2-Heptanediol | 1,2-heptanediol | 1.0 |
| Flavour | Aroma 1 (A1) | 0.5 |
| SymGuard^{®} CD | Phenylpropanol, o-Cymen-5-ol, Decylene Glycol | 0.3 |
| Glycerin | Glycerin | 5.0 |
| Propylene Glycol | Propylene Glycol | 10.0 |
| SymOcide^{®} PH | Phenoxyethanol, Hydroxyacetophenone, Caprylyl Glycol, Aqua | 0.5 |
| Sorbitol Liq EP 70% | Sorbitol | 10.0 |
| Aqua/ Water | Aqua | ad 100 |
| Sodium Saccharin | Sodium Sachharin | 0.1 |
| Colour I | | 1.2 |
| Colour II | | 0.2 |

**Table 45: Refresh your breath-Mouth spray**

| **Ingredients** | **EU INCI** | **Amount** |
|---|---|---|
| Aqua | Aqua | ad 100 |
| Sorbitol Liq. EP 70% | Sorbitol | 1.0 |
| Sodium Saccharin | Sodium Saccharin | 0.1 |
| Cremophor^{®} CO455 | PEG_40 Hydrogenated castor oil, Propylene Glycol | 5.0 |
| Aroma 1 (A1) | Aroma 1 (A1) | 0.8 |
| Frescolat^{®} ML Cryst | Mentyl lactate | 2.0 |
| 1,2-heptanediol. 2,3-heptanediol (w/w% 95:5) | 1,2-heptanediol, 2,3-heptanediol | 1.0 |
| Propylene Glycol | Propylene Glycol | 10.0 |
| SymOcide^{®} C | o-Cymen-5-ol | 0.1 |
| SymSave^{®} H | Hydroxyacetophenone | 0.25 |
| SymDiol^{®} 68 | 1,2-Hexanediol, Caprylyl Glycol | 0.5 |
| Extrapone^{®} Peppermint | Aqua, Propylene Glycol, Mentha piperita leaf extract | 2.0 |
| Colour | | 0.194 |

**Table 46: Mouthwash (Ready to use) without alcohol**

| **Ingredients** | **EU INCI** | **Amount** |
|---|---|---|
| Cremophor^{®} CO40 | PEG_40 Hydrogenated castor oil | 2.5 |
| SymGuard^{®} CD | Phenylpropanol, o-Cymen-5-ol, Decylene Glycol | 0.5 |
| Propylene Glycol | Propylene Glycol | 5.0 |
| Aroma 1 (A1) | Aroma 1 (A1) | 0.2 |
| Aqua | Aqua | Ad 100 |
| Sorbitol Liq. EP 70% | Sorbitol | 10.0 |
| 1,2-heptanediol, 2,3-heptanediol (w/w% 95:5) | 1,2-heptanediol, 2,3-heptanediol | 0.5 |
| 2,3-Octanediol | 2,3-Octanediol | 0.025 |
| Sodium Saccharin | Sodium Saccharin | 0.07 |
| Sodium Fluorid | Sodium Fluorid | 0.18 |
| SymSave^{®} H | Hydroxyacetophenone | 0.3 |

**Table 47: Mouthwash concentrate**

| **Ingredients** | **EU INCI** | **Amount** |
|---|---|---|
| Ethanol | Ethanol | ad 100 |
| Aroma 1 (A1) | Aroma 1 (A1) | 0.8 |
| 1,2-heptanediol | 1,2-heptanediol | 0.75 |
| 2,3-heptanediol | 2,3-heptanediol | 0.05 |
| Menthol | Menthol | 0.1 |
| Cremophor CO 40 Surfactant | PEG-40 Hydrogenated Castor oil | 3.0 |
| Disodium Azacycloheptane Diphosphonate | Disodium Azacycloheptane Diphosphonate | 0.5 |
| Cetylpyridinium chloride | Cetylpyridinium chloride | 0.1 |
| Zinc-Chloride | Zinc-Chloride | 0.1 |
| Disodium Phosphate | Disodium Phosphate | 0.15 |
| Trisodium Phosphate | Trisodium Phosphate | 0.05 |
| Sodium Fluoride | Sodium Fluoride | 0.18 |
| Aqua | Aqua | 45 |

**Table 48: Intimate Sensi-Gel (not in accordance with the present invention)**

| **Ingredients** | **EU INCI** | **Amount** |
|---|---|---|
| Aqua/Water | Aqua | ad 100 |
| Sodium Benzoate | Sodium benzoate | 0.3 |
| Keltrol^{®} CG-T | Xanthan Gum | 0.55 |
| Glycerin | Glycerin | 4.0 |
| Tego^{®} Betain F50 | Cocamidopropyl betaine | 14.0 |
| Solubilizer^{®} | PEG-40 Hydrogenated castor oil, Trideceth-9, Propylene Glycol, Aqua | 3.0 |
| 1,2-heptanediol | 1,2-heptanediol | 0.5 |
| Hydrolite^{®} 6 | 1,2 Hexanediol | 1.0 |
| SymGuard^{®} CD | Phenylpropanol, o-Cymen-5-ol, Decylene Glycol | 0.3 |
| SymRelief^{®} 100 | Bisabolol, Zingiber officinale root extract | 0.1 |
| Perfume oil PO1, PO2, PO3, PO4, or PO5 | Perfume | 0.2 |
| Frescolat^{®} X-cool | Menthyl ethylamido oxalate | 0.4 |
| Plantacare^{®} 2000 UP | Decyl Glucoside | 2.0 |
| Lactic acid 90% Nat. | Lactic Acid, Aqua | 0.3 |

**Table 49: Mild feminine wash**

| **Ingredients** | **EU INCI** | **Amount** |
|---|---|---|
| Aqua/Water | Aqua | ad 100 |
| SymSave^{®} H | Hydroxyacetophenone | 0.5 |
| Keltrol^{®} CG-T | Xanthan Gum | 0.5 |
| Glycerin | Glycerin | 2.0 |
| Propylene Glycol | Propylene Glycol | 3.0 |
| 1,2-Heptanediol | 1,2-Heptanediol | 0.5 |
| 2,3-Heptanediol | 2,3-Heptanediol | 0.03 |
| 2,3-Octanediol | 2,3-Octanediol | 0.01 |
| Tego^{®} Betain F50 | Cocamidopropyl betaine | 14.0 |
| SymGuard^{®} CD | Phenylpropanol, o-Cymen-5-ol, Decylene Glycol | 0.3 |
| Plantacare^{®} 2000 UP | Decyl Glucoside | 2.0 |
| Extrapone^{®} Witch Hazel GW | Glycerin, Aqua, Hamamelis Virginiana Bark/-Leaf/Twig Extract | 1.0 |
| Solubilizer | PEG-40 Hydrogenated castor oil, Trideceth-9, Propylene Glycol, Aqua | 1.5 |
| Perfume oil PO1, PO2, PO3, PO4, or PO5 | Perfume | 0.2 |
| SymCalmin^{®} | Pentylene Glycol, Butylene Blycol, Hydroxyphenyl propamidobenzoic acid | 1.0 |
| Lactic acid 90% Nat. | Lactic Acid, Aqua | 0.32 |

**Table 50: Intimate Rescue Gel**

| **Ingredients** | **EU INCI** | **Amount** |
|---|---|---|
| Aqua/Water | Aqua | ad 100 |
| SymSave^{®} H | Hydroxyacetophenone | 0.3 |
| Keltrol^{®} CG-T | Xanthan Gum | 0.55 |
| Glycerin | Glycerin | 4.0 |
| Tego^{®} Betain F50 | Cocamidopropyl betaine | 14.0 |
| Sodium Hydroxide 10% solution | Aqua, Sodium hydroxide | 1.0 |
| Solubilizer | PEG-40 Hydrogenated castor oil, Trideceth-9, Propylene Glycol, Aqua | 3.0 |
| Hydrolite^{®} 6 | 1,2-Hexanediol | 1.0 |
| 1,2-octanedioll / 2,3-octanediol blend (w/w% 95:5) | 1,2-Octanediol, 2,3-Octanediol | 0.6 |
| SymGuard^{®} CD | Phenylpropanol, o-Cymen-5-ol, Decylene Glycol | 0.3 |
| SymCalmin^{®} | Pentylene Glycol, Butylene Blycol, Hydroxyphenyl propamidobenzoic acid | 1.0 |
| Perfume oil PO1, PO2, PO3, PO4, or PO5 | Perfume | 0.2 |
| Frescolat^{®} MGA Plus | Menthone Glycerin Acetal, Menthol | 0.5 |
| Plantacare^{®} 2000 UP | Decyl Glucoside | 2.0 |

**Table 51: Cooling and Deodorizing Intimate Feminine Spray (not in accordance with the present invention)**

| **Ingredients** | **EU INCI** | **Amount** |
|---|---|---|
| Water | Aqua | ad 100 |
| EDTA | Disodium EDTA | 0.1 |
| SymSave^{®} H | Hydroxyacetophenone | 0.5 |
| Ethylhexylglycerin | Ethylhexylglycerin | 0.1 |
| Hydrolite^{®} 5 green | Pentylene Glycol | 5.0 |
| Hydrolite^{®} 6 | 1,2-Hexaendiol | 1.0 |
| SymDeo^{®} MPP | Dimethyl Phenylbutanol | 0.3 |
| SymDeo^{®} B125 | 2-Methyl 5-Cycloheylpentanol | 0.3 |
| Solubilizer | PEG-40 Hydrogenated castor oil, Trideceth-9, Propylene Glycol, Aqua | 1.0 |
| Frescolat^{®} ML Cryst | Menthyl Lactate | 0.2 |
| Perfume oil PO1, PO2, PO3, PO4, or PO5 | Perfume | 0.1 |
| SymMollient^{®} W/S | Trideceth-9, PEG-5 Isononanoate, Aqua | 1.0 |
| 1,2-heptanediol | 1,2-heptanediol | 0.7 |
| SymGuard^{®} CD | Phenylpropanol, o-Cymen-5-ol, Decylene Glycol | 0.3 |

**Table 52: After Shave Tonic**

| **Ingredients** | **INCI** | **Amount** |
|---|---|---|
| SymSol^{®} PF-3 | Water (Aqua), Pentylene Glycol, Sodium Lauryl Sulfoacetate, Sodium Oleoyl Sarcosinate, Sodium Chloride, Disodium Sulfoacetate, SodiumOleate, Sodium Sulfate | 3.0 |
| SymSitive^{®} 1609 | Pentylene Glycol, 4-t-Butylcyclohexanol | 1.0 |
| Frescolat^{®} ML | Menthyl Lactate | 0.3 |
| Glycerol 99,5 P. | Glycerol | 5.0 |
| Water | Water (Aqua) | ad 100 |
| Extrapone^{®} Glacier Water GW | Glycerol, Water (Aqua) | 1.0 |
| SymCalmin^{®} | Butylene Glycol, Pentylene Glycol, Hydroxyphenyl Propamidobenzoic Acid | 0.5 |
| Dragosine^{®} | Carnosine | 0.1 |
| Hydrolite^{®} 5 green | Pentylene Glycol | 5.0 |
| Ethanol 96 % | Alcohol Denat. | 5.0 |
| Colour Pigment | Colour Pigment | 0.05 |
| Perfume oil PO1, PO2, PO3, PO4, or PO5 | Perfume | 0.15 |
| 1,2-heptanediol, 2,3-heptanediol (w/w% 95:5) | 1,2-heptanediol, 2,3-heptanediol | 1.0 |
| 2,3 Octanediol | 2,3 Octanediol | 0.05 |

**Table 53: After Sun Refreshing Gel**

| **Ingredients** | **EU INCI** | **Amount** |
|---|---|---|
| Aqua/Water | Aqua | Ad 100 |
| Hydrolite^{®} 5 green | Pentylene Glycol | 2.5 |
| SymSave^{®} H | Hydroxyacetophenone | 0.5 |
| Panthenol 75W | Panthenol | 0.5 |
| 1,3 Butylene Glycol | Butylene Glycol | 3.0 |
| Glycerin 99,5% | Glycerin | 2.0 |
| DragoCalm^{®} | Aqua, Glycerin, Avena sativa kernel extract | 1.5 |
| SymGlucan^{®} | Aqua, Glycerin, 1,2-Hexanediol, Caprylyl Glycol, Beta-Glucan | 5.0 |
| Dragosine^{®} | Carnosine | 0.1 |
| Carbopol^{®} Ultrez 21 Polymer | Acrylates/C10-30 Alkyl Acrylate Crosspolymer | 0.4 |
| Solubilizer | PEG-40 Hydrogenated castor oil, Trideceth-9, Propylene Glycol, Aqua | 1.5 |
| SymMollient^{®} W/S | Trideceth-9, PEG-5 Isononanoate, Aqua | 1.0 |
| SymBright^{®} 2036 | Sclareolide | 0.1 |
| SymSitive^{®} 1609 | Pentylene Glycol, 4-t-Butylcyclohexanol | 1.0 |
| SymUrban^{®} | Benzylidene Dimethoxydimethylidanone | 0.15 |
| 1,2-heptanediol, 2,3-heptanediol (w/w% 95:5) | 1,2-heptanediol, 2,3-heptanediol | 1.0 |
| 1,2-nonanediol | 1,2-nonanediol | |
| Frescolat^{®} ML Cryst | Menthyl Lactate | 0.3 |
| Perfume oil PO1, PO2, PO3, PO4, or PO5 | Perfume | 0.05 |
| Sodium Hydroxide 10% solution | Aqua, Sodium hydroxide | 0.8 |
| Colour | | 0.4 |
| Extrapone^{®} Cucumber CL | Aqua, Propylene Glycol, cucumis sativus (Cucumber) Juice | 1.0 |
| SymFinity^{®} 1298 | Echinacea Purpurea Extract | 0.06 |

**Table 54: Fresh Hair shampoo**

| **Ingredients** | **EU INCI** | **Amount** |
|---|---|---|
| Aqua | Aqua | Ad 100 |
| EDTA B Powder | Disodium EDTA | 0.1 |
| Carbopol^{®} Aqua SF-1 Polymer | Acrylates copolymer | 11.0 |
| Texapon^{®} NSO UP | Sodium Laureth sulfate | 35.0 |
| Dehyton^{®} K | Cocamidopropyl betaine | 8.0 |
| Plantacare^{®} 2000 UP | Decyl Glucoside | 4.0 |
| Perfume oil PO1, PO2, PO3, PO4, or PO5 | Pearfum | 1.0 |
| SymSave^{®} H | Hydroxyacetophenone | 0.5 |
| Hydrolite^{®} 6 | 1,2-Hexanediol | 1.0 |
| 1,2-heptanediol, 2,3-heptanediol (w/w% 95:5) | 1,2-heptanediol, 2,3-heptanediol | 0,5 |
| 1,2-heptanediol | 1,2-heptanediol | 0,5 |
| SymClariol^{®} | Decylene Glycol | 0.5 |
| SymLite^{®} G8 | Glyceryl Caprylate | 0.2 |
| Frescolat^{®} ML cryst | Menthyl Lactate | 1.0 |
| SymCalmin^{®} | Pentylene Glycol, Butylene Glycol, Hydroxyphenyl propamidobenzoic acid | 0.2 |
| Crinipan^{®} AD | Climbazole | 0.2 |
| Propylene Glycol | Propylene Glycol | 2.0 |
| SymHair^{®} Shield | Pentylene Glycol, Aqua, Glycerin, Triticum vulgare bran extract, 1,2-Hexanediol, Capryly glycol | 1.0 |
| Sodium Hydroxide 10% solution | Aqua, Sodium hydroxide | 2.2 |
| Actipone^{®} Alpha-Pulp | Aqua, Butylene Glycol, Malic acid, Actinidia chinensis fruit extract, citrus aurantium dulcis juice, citrus paradise juice,pyrus malus juice | 0.2 |

**Table 55: Scalp soothing hair conditioner with UV-B/UV-A protection, rinse off**

| **Ingredients** | **INCI** | **Amount** |
|---|---|---|
| SymLite^{®} G8 | Glyceryl caprylate | 0.2 |
| Crinipan^{®} PMC green | Propanediol caprylate | 0.5 |
| 1,2-heptanediol | 1,2-heptanediol | 0.5 |
| 2,3-octanediol | 2,3-octanediol | 0,1 |
| Abil 350 | Dimethicone | 0.1 |
| Dehyquart A CA | Cetrimonium Chloride | 0.5 |
| Dehyquart SP | Quaternium-52 | 4.0 |
| Dracorin^{®} CE | Glyceryl Stearate Citrate | 1.0 |
| EDETA BD | Disodium EDTA | 0.1 |
| Extrapone^{®} Green Tea GW | Glycerin, Water (Aqua), Camellia Sinensis Leaf Extract | 0.7 |
| Perfume oil PO1, PO2, PO3, PO4, or PO5 | Perfume | 0.5 |
| Lara Care^{®} A-200 | Galactoarabinan | 0.5 |
| Neutral Oil | Caprylic/Capric Triglyceride | 1.0 |
| PCL Liquid^{®} 100 | Cetearyl Ethylhexoate | 0.3 |
| PCL Solid | Stearyl Heptanoate, Stearyl Caprylate | 3.0 |
| SymOcide^{®}PS | Phenoxyethanol, Decylene Glycol, 1,2-Hexanediol | 1.0 |
| Water (demineralized) | Water (Aqua) | ad 100 |

**Table 56: Revitalizing Conditioner**

| **Ingredients** | **EU INCI** | **Amount** |
|---|---|---|
| Aqua | Aqua | Ad 100 |
| EDETA BD | Disodium EDTA | 0.05 |
| Glycerin 99.5% | Glycerin | 1.00 |
| Lactic Acid | Lactic acid | 0.85 |
| Genamin^{®} KDMP | Behentrimonium chloride | 2.50 |
| SymSave^{®} H | Hydroxyacetophenone | 0.50 |
| Hydrolite^{®} CG | Caprylyl Glycol | 0.50 |
| 1,2-heptanediol, 2,3-heptanediol (w/w% 95:5) | 1,2-heptanediol, 2,3-heptanediol | 0.50 |
| Tego^{®} Amid S18 | Stearamidopropyl Dimethylamine | 3.00 |
| VARISOFT^{®} EQ65 | Distearoylethyl Dimonium chloride (and) ceterayl alcohol | 4.00 |
| SymMollient^{®} S green | Ceteraryl Nonanoate | 2.00 |
| Cetiol^{®} SB 45 | Butyrospermum parkii butter | 3.00 |
| Lanette^{®}16 | Cetyl alcohol | 4.75 |
| Lanette^{®} 18 | Stearyl alcohol | 4.25 |
| Beeswax | Beeswax | 0.80 |
| SymOleo^{®} Vita7 | Glycine soja oil, Gossypium herbaceum seed oil, mangifera idica seed butter, olea europaea fruitoil, persea gratissima oil, prununs amygdalus dulcis (sweet almond) oil, Theobroma cacao seed butter | 3.00 |
| Eumulgin^{®} B2 | Ceteareth-20 | 1.00 |
| Perfume oil PO1, PO2, PO3, PO4, or PO5 | Perfume | 1.00 |
| SymHair^{®} Restore | Glycerin, Triticum vulgare protein, Aqua | 1.00 |
| Lactic acid | Lactic acid | 0.50 |

**Table 57: Hair Soap Shampoo Bar**

| **Ingredients** | **EU INCI** | **Amount** |
|---|---|---|
| Talco | Talc | ad 100 |
| Texapon^{®} ZACD | Sodium Lauryl Sulfate | 10.00 |
| SymOleo^{®} Vita7 | Glycine soja oil, Gossypium herbaceum seed oil, mangifera idica seed butter, olea europaea fruitoil, persea gratissima oil, prununs amygdalus dulcis (sweet almond) oil, Theobroma cacao seed butter | 2.00 |
| Cetiol^{®} SB 45 | Butyrospermum parkii butter | 3.00 |
| Mackamide^{®} CMA | Cocamide Mea | 4.00 |
| SymSol^{®} PF-3 | Aqua, Pentylene Glycol, Sodium Lauryl sulfoacetate, sodium oleoyl sarcosinate, Sodium Chloride, Sodium Oleate | 5.00 |
| Food Color Beta Carotene E160A OilSoluble | Helianthus annuus (Sunflower) seed oil, Beta-Carotene (C140800) | 0.45 |
| 1,2-octanediol, 2,3-octanediol (w/w% 95:5) | 1,2-octanediol, 2,3-octanediol | 1.0 |
| Dragosantol^{®} 100 | Bisabolol | 0.2 |
| SymOcide^{®} PH | Phenoxyethanol, Hydroxyacetophenone, Caprylyl Glycol, Aqua | 1.00 |
| SymHair^{®} Restore | Glycerin, Triticum vulgare protein, Aqua | 1.00 |
| Perfume oil PO1, PO2, PO3, PO4, or PO5 | Perfume | 1.50 |

**Table 58: Conditioner Spray Breath fresh air (not in accordance with the present invention)**

| **Ingredients** | **EU INCI** | **Amount** |
|---|---|---|
| Aqua/Water | Aqua | ad 100 |
| Edeta^{®} BD | Disodium EDTA | 0.10 |
| Glycerin 99.5% | Glycerin | 1.00 |
| SymSave^{®} H | Hydroxyacetophenone | 0.50 |
| Ucare^{™} Polymer JR-400 | Polyquaternium-10 | 0.30 |
| VARISOFT^{®}300 | Cetrimonium Chloride | 3.50 |
| Tocopherol | Tocopherol | 0.10 |
| SymHair^{®} Shield | Pentylene Glycol, Aqua, Glycerin, Triticum vulgare bran extract, 1,2-Hexanediol, Caprylyl Glycol | 1.00 |
| Cremophor^{®} CO 40 Surfactant | PEG-40 Hydrogenated castor oil | 1.00 |
| Perfume oil PO1, PO2, PO3, PO4, or PO5 | Perfume | 0.70 |
| 1,2-heptanediol | 1,2-heptanediol | 0.50 |
| SymHair^{®} Force 1631 | Pentylene Glycol, Isochrysis Galbana extract | 1.00 |
| Citric Acid 50% Sol. | Aqua, Citric acid | 0.10 |

**Table 59: Hair Refresher Mist**

| **Ingredients** | **EU INCI** | **Amount** |
|---|---|---|
| SymDeo^{®} Plus | Lauryl alcohol, Phenoxyethanol, 2-Benzyleptanol, Decylene Glycol | 0.5 |
| Perfume oil PO1, PO2, PO3, PO4, or PO5 | Perfume | 0.1 |
| Frescolat^{®} ML nat | Mentyl Lactate | 0.3 |
| Crinipan^{®} PMC green | Propanediol Caprylate | 0.3 |
| Solubilizer | PEG-40 Hydrogenated Castor oil, Trideceth-9, Propylene glycol, Aqua | 5.0 |
| SymCalmin^{®} | Pentylene Glycol, Butylene Glycol, Hydroxyphenyl Propamidobenzoic acid | 0.2 |
| SymControl^{®} Scalp | Water (Aqua), Glycerin, Mannitol, Tetraselmis Suecica Extract | 2.0 |
| 1,2-octanediol, 2,3-octanediol (w/w% 98:2) | 1,2-octanediol, 2,3-octanediol | 0,3 |
| 1,2-heptanediol, 2,3-heptanediol (w/w% 95:5) | 1,2-heptanediol, 2,3-heptanediol | 0.6 |
| Propylene Glycol | Propylene Glycol | 3.0 |
| Butylene Glycol | Butylene Glycol | 3.0 |
| Aqua | Aqua | ad 100 |
| Edeta^{®} BD | Disodium EDTA | 0.1 |
| Sodium Benzoate | Sodium benzoate | 0.4 |
| Color | Color | 0.5 |
| Actipone^{®} Rosemary GW | Glycerin, Aqua, Rosmarinus officinalis leaf extract | 0.5 |

**Table 60: Virgin Mojito Pre-Shampoo**

| **Ingredients** | **EU INCI** | **Amount** |
|---|---|---|
| Aqua/Water | Aqua | ad 100 |
| Pemulen^{™} TR-2 Polymeric Emulsifier | Acrylates/C10-30 Alkyl Acrylate Crosspolymer | 0.2 |
| Cosmedia^{®} SP | Sodium Polyacrylate | 0.4 |
| Glycerin | Glycerin | 2.0 |
| SymSave^{®} H | Hydroxyacetophenone | 0.5 |
| Hydrolite^{®} 5 green | Pentylene Glycol | 3.0 |
| Solubilizer | PEG-40 Hydrogenated castor oil, Trideceth-9, Propylene Glycol, Aqua | 3.0 |
| Oxynex^{®} ST Liquid | Diethylhexyl Syringylidenemalonate, Caprylic/capric triglyeride | 0.1 |
| Frescolat^{®} Plus | Menthol, Menthyl Lactate | 1.0 |
| Perfume oil PO1, PO2, PO3, PO4, or PO5 | Perfume | 0.5 |
| 1,2-octanediol, 2,3-octanediol (w/w% 98:2) | 1,2-octanediol, 2,3-octanediol | 0.5 |
| 1,2-heptanediol, 2,3-heptanediol (w/w% 99:1) | 1,2-heptanediol, 2,3-heptanediol | 0.5 |
| SymReboot^{™} L19 | Maltodextrin, Lactobacillus ferment | 0.5 |
| Extrapone^{®} Lime | Propylene Glycol, Aqua, Citrus aurantifolia juice | 1.0 |
| Color I: FD&C Yellow no.5 -0.01 % solution | Aqua, C119140, Sodium Chloride, Sodium Sulfate | 0.8 |
| Color II: FD&C Blue no.1 -0.01 % solution | Aqua, C142090, Sodium Chloride, Sodium Sulfate | 0.4 |
| Sodium Hydroxide 10% solution | Aqua, Sodium Hydroxide | 0.2 |

**Table 61: Sensi-SCALP (Green Solid Shampoo)**

| **Ingredients** | **EU INCI** | **Amount** |
|---|---|---|
| AMISOFT^{®} MS-11 | Sodium Myristoyl Glutamate | 30.0 |
| ELFAN^{®}AT 84 | Sodium Cocoyl Isethionate | 15.0 |
| ImerCare^{®} 02K-S | Kaolin | 18.8 |
| Hydrolite^{®} 5 green | Pentylene Glycol | 1.5 |
| 1,2-octanediol, 2,3-octanediol (w/w% 95:5) | 1,2-octanediol, 2,3-octanediol | 0.3 |
| 1,2-heptanediol, 2,3-heptanediol (w/w% 99:1) | 1,2-heptanediol, 2,3-heptanediol | 0.5 |
| Dragosantol^{®} 100 | Bisabolol | 0.1 |
| Potassium Sorbate | Potassium Sorbate | 0.3 |
| Aqua/Water | Aqua | Ad 100 |
| SymDecanox^{™} HA | Caprylic/Capric Triglyceride, Hydroxymethoxyphenyl decanone | 2.0 |
| Cetiol^{®} SB45 | Butyrospermum parkii butter | 13.0 |
| Extrapone^{®} Rooibus GW | Aqua, Glycerin, Aspalathus Linearis leaf extract | 1.0 |
| SymReboot^{™} L19 | Maltodextrin, Lactobacillus Ferment | 0.5 |
| SymOleo^{®} Vita7 | Glycine soja oil, Gossypium herbaceum seed oil, mangifera idica seed butter, olea europaea fruitoil, persea gratissima oil, prununs amygdalus dulcis (sweet almond) oil, Theobroma cacao seed butter | 1.0 |
| Perfume oil PO1, PO2, PO3, PO4, or PO5 | Perfume | 1.2 |

**Table 62: Shampoo Sulfate-Free**

| **Ingredients** | **EU INCI** | **Amount** |
|---|---|---|
| Aqua/Water | Aqua | Ad 100 |
| Akypo^{®} Foam RL 40 | Sodium Laureth-5 Carboxylate | 15.00 |
| Plantacare^{®}2000 UP | Decyl Glucoside | 5.00 |
| EDTA NA2 | Disodium EDTA | 0.10 |
| SymOcide^{®} PH | Phenoxyethanol, Hydroxyacetophenone, Caprylyl Glycol, Aqua | 1.45 |
| Aqua/Water | Aqua | 10.00 |
| Novethix^{™} L-10 Polymer | Acrylates/Beheneth-25 Methacrylate Copolymer | 7.00 |
| Dehyton^{®} PK 45 | Cocamidopropyl Betaine | 10.00 |
| 1,2-octanediol, 2,3-octanediol (w/w% 98:2) | 1,2-octanediol, 2,3-octanediol | 0.1 |
| 1,2-heptanediol, 2,3-heptanediol (w/w% 95:5) | 1,2-heptanediol, 2,3-heptanediol | 0.1 |
| SymMollient^{®} W/S | Trideceth-9, PEG-5 Isononanoate, Aqua | 0.50 |
| SymCalmin^{®} | Pentylene Glycol, Butylene Glycol, Hydroxyphenyl Propamidobenzoic acid | 1.00 |
| Antil^{®} 141 Liquid | Propylene Glycol, PEG-55 Proplyene Glycol Oleate | 2.00 |
| Acusol^{™} OP 301 | Styrene/Acrylates Copolymer | 1.00 |
| SymHair^{®} Restore | Glycerin, Triticum Vulgare Protein, Aqua | 1.00 |
| Perfume oil PO1, PO2, PO3, PO4, or PO5 | Perfume | 1.00 |

**Table 63: Hair conditioner with UV protection**

| **Ingredients** | **INCI** | **Amount** |
|---|---|---|
| Renex^{™} PEG 6000 | PEG-150 | 2.5 |
| Hair Conditioner Base | Cetyl alcohol, behentrimonium chloride, Triticum Vulgare (Wheat) bran extract, linoleic acid | 3.0 |
| PCL-Solid | Stearyl heptanoate, stearyl caprylate | 0.5 |
| Dowsil^{™} 5200 | Laurylmethicone copolyol | 0.5 |
| Natrosol^{™} 250 HR | Hydroxyethylcellulose | 0.5 |
| Benzophenone-4 | Benzophenone-4 | 1.0 |
| Neo Heliopan^{®} AP | Disodiumphenyldibenz-im idazole tetrasulphonate | 1.0 |
| Amino methyl propanol | Amino methyl propanol | 2.0 |
| Xiameter^{®} 949 MEM-0949 Emulsion | Amodimethicone, cetrimonium chloride, trideceth-12 | 2.0 |
| Perfume oil PO1, PO2, PO3, PO4, or PO5 | Perfume | 0.8 |
| Hydrolite^{®}6 | 1,2-Hexanediol | 0.5 |
| 1,2-heptanediol, 2,3-heptanediol (w/w% 95:5) | 1,2-Heptanediol, 2,3-heptanediol | 0.5 |
| Water | Water (Aqua) | ad 100 |

**Table 64: Hair conditioner, leave on**

| **Ingredients** | **INCI** | **Amount** |
|---|---|---|
| 2,3-heptanediol | 2,3-heptanediol | 0.1 |
| Dehyquart^{®} A CA | Cetrimonium Chloride | 0.2 |
| Dehyquart^{®} SP | Quaternium-52 | 2.0 |
| Dracorin^{®} CE | Glyceryl Stearate Citrate | 1.0 |
| Drago-Calm^{®} | Water, Glycerin, Avena Sativa (Oat) Kernel Extract | 2.0 |
| Dragosantol^{®} 100 | Bisabolol | 0.15 |
| Farnesol^{®} | Farnesol | 0.1 |
| Perfume oil PO1, PO2, PO3, PO4, or PO5 | Perfume | 0.5 |
| Lara Care^{®} A-200 | Galactoarabinan | 0.1 |
| Ucare^{™} Polymer JR-400 | Polyquaternium-10 | 0.1 |
| Propylene Glycol | Propylene Glycol | 0.8 |
| SymMollient^{®} WS | Trideceth-9, PEG-5 Isononanoate, Water | 1.0 |
| SymSol^{®} PF3 | Water, Pentylene Glycol, Sodium Lauryl Sulfoacetate, Sodium Oleoyl Sarcosinate, Sodium Chloride, Disodium Sulfoacetate, Sodium Oleate, Sodium Sulfate | 1.5 |
| SymTriol^{®} | Caprylyl Glycol, 1,2-Hexanediol, Methylbenzyl Alcohol | 1.0 |
| Water (demineralized) | Water (Aqua) | ad 100 |

**Table 65: Anti-itch hair conditioner, leave on**

| **Ingredients** | **INCI** | **Amount** |
|---|---|---|
| SymLite^{®} G8 | Glyceryl caprylate | 0.15 |
| 1,2-heptanediol, 2,3-heptanediol (w/w% 95:5) | 1,2-heptanediol, 2,3-heptanediol | 0.8 |
| Dragosantol^{®} 100 | Bisabolol | 0.1 |
| Dehyquart^{®} A CA | Cetrimonium Chloride | 0.5 |
| Dehyquart^{®} SP | Quaternium-52 | 4.0 |
| Dracorin^{®} CE | Glyceryl Stearate Citrate | 1.0 |
| Drago-Oat-Active | Water (Aqua), Butylene Glycol, Avena Sativa (Oat) Kernel Extract | 2.0 |
| Perfume oil PO1, PO2, PO3, PO4, or PO5 | Perfume | 0.1 |
| Lara Care^{®} A-200 | Galactoarabinan | 1.5 |
| Plant oil Triglyceride | Caprylic/Capric Triglyceride | 1.0 |
| PCL Liquid^{®} 100 | Cetearyl Ethylhexoate | 0.3 |
| Ucare^{™} Polymer JR 400 | Polyquaternium-10 | 0.1 |
| Propylene Glycol | Propylene Glycol | 0.8 |
| SymGlucan^{®} | Aqua, Glycerin, 1,2-Hexanediol, Caprylyl Glycol, Beta-Glucan | 5 |
| SymMollient^{®} W/S | Trideceth-9, PEG-5 Isononanoate, Water (Aqua) | 2.0 |
| SymCalmin^{®} | Butylene Glycol, Pentylene Glycol, Hydroxyphenyl Propamidobenzoic Acid | 2.0 |
| SymOcide^{®} PH | Phenoxyethanol, Hydroxyacetophenone, Caprylyl Glycol, Aqua | 1.2 |
| SymSol^{®}PF3 | Water, Pentylene Glycol, Sodium Lauryl Sulfoacetate, Sodium Oleoyl Sarcosinate, Sodium Chloride, Disodium Sulfoacetate, Sodium Oleate, Sodium Sulfate | 1.5 |
| Water, demineralized | Water (Aqua) | ad 100 |

**Table 66: Sprayable hair conditioner with zinc pyrithrione, leave-on**

| **Ingredients** | **INCI** | **Amount** |
|---|---|---|
| Monomuls^{®} 60-35 C | Hydrogenated Palm Glycerides | 1.7 |
| Cetiol^{®} OE | Dicaprylyl Ether | 7.2 |
| Abil^{®} 100 | Dimethicone | 3.6 |
| Dehyquart^{®} F 75 | Distearoylethyl Hydroxyethylmonium, Methosulfate, Cetearyl Alcohol | 4.0 |
| Eumulgin^{®} B1 | Ceteareth-12 | 3.5 |
| Cetiol^{®} S | Diethylhexylcyclohe xane | 7.2 |
| D-Panthenol | Panthenol | 0.1 |
| Glycerol 99,5 % | Glycerol | 1.5 |
| Water | Water (Aqua) | ad 100 |
| Actipone^{®} Rosemary | Water (Aqua), Propylene, Glycol, Rosmarinus Officinalis, (Rosemary) Leaf Extract | 0.1 |
| Frescolat^{®} ML Cryst. | Menthyl Lactate | 0.5 |
| Dragosantol^{®}100 | Bisabolol | 0.1 |
| Crinipan^{®} PMC Green | Propanediol Caprylate | 0.1 |
| Perfume oil PO1, PO2, PO3, PO4, or PO5 | Perfume | 0.4 |
| 2-Phenoxyethyl alcohol | Phenoxyethanol | 0.4 |
| SymSave H | Hydroxyacetophenone | 0.3 |
| SymDiol 68 | 1,2-Hexanediol, Caprylyl glycol | 0.3 |
| 1,2-octanediol, 2,3-octanediol (w/w% 90:10) | 1,2-octanediol, 2,3-octanediol | 0.2 |
| 1,2-heptanediol, 2,3-heptanediol (w/w% 90:10) | 1,2-heptanediol, 2,3-heptanediol | 0.2 |

**Table 67: Hair styling gel (not in accordance with the present invention)**

| **Ingredients** | **Amount** |
|---|---|
| Water | ad 100 |
| PVM/MA Decadiene Crosspolymer | 0.6 |
| PVP | 3.0 |
| Isocetyl Stearate | 4.0 |
| Ethylhexyl Methoxycinnamate | 0.5 |
| Aminomethyl Propanol | 0.4 |
| Perfume oil PO1, PO2, PO3, PO4, or PO5 (Perfume) | 0.6 |
| SymDiol^{®} 68T (1,2-Hexanediol, 1,2-Octanediol, Tropolone) | 0.4 |
| Phenoxyethanol | 0.2 |
| Ethylhexylglycerin | 0.1 |
| 1,2-octanediol, 2,3-octanediol (w/w% 98:2) | 0.5 |

**Table 68: Aseptic wound cream**

| **Ingredients** | **Amount** |
|---|---|
| Sorbitan Isostearate, Hydrogenated Castor Oil, Ceresin, Beeswax (Cera Alba) | 6.0 |
| Petrolatum | 21.0 |
| Cera Alba | 5.0 |
| Cetearyl Alcohol | 7.0 |
| Prunus Dulcis | 7.0 |
| Lanolin | 5.0 |
| Paraffinum Liquidum | 12.0 |
| Perfume oil PO1, PO2, PO3, PO4, or PO5 | 0.3 |
| Water (Aqua) | ad 100 |
| Panthenol | 7.0 |
| Magnesium Sulfate | 0.7 |
| Pentylene Glycol | 1.0 |
| Tocopheryl Acetate | 1.0 |
| Octenidine dihydrochloride | 0.1 |
| Hydrocortisone | 0.1 |
| Phenoxyethanol | 0.5 |
| 1,2-octanediol, 2,3-octanediol (w/w% 95:5) | 0.1 |
| 1,2-heptanediol, 2,3-heptanediol (w/w% 95:5) | 0.5 |

**Table 69: Eye contour serum (not in accordance with the present invention)**

| **Ingredients** | **EU INCI** | **Amount** |
|---|---|---|
| Dracorin^{®} GOC | Glyceryl Oleate Citrate, Caprylic/Capric Triglyceride | 1.50 |
| Dracorin^{®} CE | Glyceryl Stearate Citrate | 3.00 |
| Dragoxat^{®} 89 | Ethylhexyl Isononanoate | 3.00 |
| PCL-Liquid^{®}100 | Ceterayl Ethylhexanonate | 2.00 |
| SymMollient^{®} S | Ceterayl Nonanoate | 2.00 |
| SymRelief^{®} 100 | Bisabolol, Zingiber officinale root extract | 0.10 |
| SymDetox^{™} 1711 | 3,3,5 Trimethylcyclohexyl succinate dimethylamide | 0.50 |
| Tocopheryl Acetate | Tocopheryl acetate | 0.25 |
| Aqua/Water | Aqua | ad 100 |
| Keltrol^{®} CG-F | Xanthan Gum | 0.20 |
| Carbopol^{®} Ultrez 10 Polymer | Carbomer | 0.30 |
| SymLift^{®} | Aqua, Trehalose, Glycerin, Pentylene Glycol, Beta-Glucan, Hordeum vulgare seed extract, sodium hyluronate, 1,2-Hexanediol, Caprylyl Glycol | 5.00 |
| SymSave^{®} H | Hydroxyacetophenone | 0.50 |
| EDTA NA2 | Disodium EDTA | 0.10 |
| Sodium Hydroxide 10% Solution | Aqua, Sodium Hydroxide | 1.80 |
| Hydrolite^{®} 6 | Hexanediol | 1.00 |
| 1,2-heptanediol | 1,2-heptanediol | 1.00 |
| Glycerin | Glycerin | 4.00 |
| Caffeine | Caffeine | 0.10 |
| Retinyl Palmitate | Retinyl Palmitate | 0.10 |
| Perfume oil PO1, PO2, PO3, PO4, or PO5 | Perfume | 0.50 |

**Table 70: Intensive Care, Detox& Repair Hand Cream 2-in-1**

| **Ingredients** | **EU INCI** | **Amount** |
|---|---|---|
| Emulsiophos^{®} | Potassium cetyl phosphate, hydrogenated palm glycerides | 1.50 |
| Lanette^{®} O | Cetearyl alcohol | 1.50 |
| Beeswax | Beeswax | 1.00 |
| PCL-Liquid^{®}100 | Cetearyl Ethylhexanoate | 3.00 |
| Softisan^{®}100 | Hydrogenated coco-glycerides | 5.00 |
| PCL-Solid | Stearyl Heptanoate, Stearyl Caprylate | 5.00 |
| Caprylic/Capric Triglyceride | Caprylic/ Capric Triglyceride | 1.50 |
| Tegosoft^{®} MM | Myristyl myristate | 2.50 |
| SymDetox^{®}1711 | 3,3,5-Trimethylcyclohexyl succinate dimethylamide | 0.50 |
| SymRepair^{®} 100 | Hexyldecanol, Bisabolol, Cethylhydroxyproline palmitamide, stearic acid, Brassica campestris (Rapeseed) sterols | 1.00 |
| SymDecanox^{™} HA | Caprylic/Capric Triglyceride, Hydroxymethoxyphenyl decanone | 1.00 |
| Cosmedia^{®} SP | Sodium Polyacrylate | 0.60 |
| Aristoflex^{®} AVC | Ammonium AcryloyoldimethyltaurateNP Copolymer | 0.60 |
| Aqua/Water | Aqua | ad 100 |
| Edeta^{®} BD | Disodium Edta | 0.10 |
| SymSave^{®} H | Hydroxyacetophenone | 0.50 |
| Hydroviton^{®} PLUS 2290 | Aqua, Pentylene Glycol, Glycerin, Fructose, Urea, Citric acid, Sodium hydroxide, Maltose, Sodium PCA, Sodium chloride, Sodium Lactate, Trehalose, Allantoin, Sodium Hyaluronate, Glucose | 2.00 |
| SymEssence^{®} Camomile CC | Chamomilla recutita (Matricaria) Flower/Leaf/Stem water | 1.00 |
| Colour | | 0.80 |
| Tinogard^{®} Q | Tris(tetramethylhydroxypiperidino )citrate | 0.05 |
| Perfume oil PO1, PO2, PO3, PO4, or PO5 | Perfume | 0.40 |
| Sensocel^{®}10 | Cellulose | 2.00 |
| Dipropylene glycol | Dipropylene Glycol | 0.80 |
| Retinyl Palmitate | Retinyl Palmitate | 0.10 |
| 1,2-heptanediol | 1,2-heptanediol | 0.95 |
| 2,3-Heptanediol | 2,3-Heptanediol | 0.05 |
| SymOcide^{®}C | o-Cymen-5-ol | 0.10 |

**Table 71: Liquid for sheet mask - New Skin**

| **Ingredients** | **EU INCI** | **Amount** |
|---|---|---|
| EDTA NA2 | Disodium EDTA | 0.1 |
| Aqua/Water | Aqua | ad 100 |
| SymSave^{®} H | Hydroxyacetophenone | 0.5 |
| Glycerin | Glycerin | 5.0 |
| Sepimax^{™}ZEN | Polyacrylate crosspolymer-6 | 0.4 |
| Hydrolite^{®}5 green | Pentylene Glycol | 3.0 |
| Dragosine^{®} | Carnosine | 0.2 |
| SymGlucan^{®} | Aqua, Glycerin, 1,2-Hexanediol, Caprylyl Glycol, Beta-Glucan | 4.0 |
| Extrapone^{®} Glacier Water GW | Glycerin, Aqua | 1.0 |
| Perfume oil PO1, PO2, PO3, PO4, or PO5 | Perfume | 0.15 |
| 1,2-heptanediol, 2,3-heptanediol (w/w% 95:5) | 1,2-heptanediol, 2,3-heptanediol | 0.7 |
| Dragosantoi^{®}100 | Bisabolol | 0.05 |
| Solubilizer | PEG-40 Hydrogenated castor oil, Trideceth-9, Aqua | 0.10 |
| Citric acid 50% sol | Aqua, Citric acid | 0.12 |

**Table 72: Clear Anti Acne Wipe**

| **Ingredients** | **INCI** | **Amount** |
|---|---|---|
| Farnesol | Farnesol | 0.2 |
| Methylisothiazolinone | Methylisothiazolinone | 0.1 |
| Isopropanol | Isopropanol | 45.0 |
| Water, distilled | Water (Aqua) | ad 100 |
| Camomile CL | Chamomilla Recutita (Matricaria) Flower Water, Butylene Glycol, Pentylene Glycol | 1.0 |
| Witch Hazel-Distillate | Hamamelis Virginiana (Witch Hazel) Water, Water (Aqua), Alcohol, Hamamelis Virginiana (Witch Hazel) Extract | 1.0 |
| Citric Acid 10 % aqueous solution | Citric Acid | 0.03 |
| Perfume oil PO1; PO2; PO3; PO4 or PO5 | Perfume | 0.1 |
| Hydroxyethoxyphenyl Butanone | Hydroxyethoxyphenyl Butanone | 0.4 |
| 1,2-heptanediol / 2,3-heptanediol blend (95:5w/w%) | 1,2-heptanediol, 2,3-heptanediol | 0.5 |
| 2,3-heptanediol | 2,3-heptanediol | 0.5 |

**Table 73: PEG-free Wet Wipe**

| **Ingredients** | **INCI** | **Amount** |
|---|---|---|
| Teposoft^{®} PC 41 | Polyglyceryl-4 Caprate | 2.0 |
| Tegosoft^{®} M | Isopropyl Myristate | 0.2 |
| Perfume oil PO1; PO2; PO3; PO4 or PO5 | Perfume | 0.1 |
| Propylengycol | Propylene Glycol | 3.0 |
| Water, distilled | Water (Aqua) | ad 100 |
| Cetrimonium Bromide, 40% | Cetrimonium Bromide | 0.25 |
| Hydroxyethoxyphenyl Butanol | Hydroxyethoxyphenyl Butanol | 0.6 |
| 1,2-heptanediol/2,3-heptanediol blend (95:5 w/w%) | 1,2-heptanediol | 3.0 |
| | 2,3-heptanediol | |

**Table 74: Baby Soft Wet Wipes**

| **Ingredients** | **INCI** | **Amount** |
|---|---|---|
| Mineral Oil | Paraffinum Liquidum | 4.0 |
| Isopropylpalmitat | Isopropyl Palmitate | 3.0 |
| Eumulgin B2 | Ceteareth-20 | 2.5 |
| Lanette O | Cetearyl Alcohol | 2.5 |
| Dragosantol | Bisabolol | 0.1 |
| Olive Oil | Olea Europaea (Olive) Fruit Oil | 0.5 |
| Isopropylmyristat | Isopropyl Myristate | 0.5 |
| Dow Corning 345 Fluid | Cyclomethicone | 0.2 |
| Super Hartolan | Lanolin Alcohol | 0.2 |
| Tagat L2 | PEG-20 Glyceryl Laurate | 2.5 |
| Water, distilled | Water (Aqua) | 80.0 |
| Glycerin 99,5% | Glycerin | 3.0 |
| Citric Acid Sol, aqueous solution 10% | Citric Acid | 0.1 |
| Perfume oil PO1; PO2; PO3; PO4 or PO5 | Perfume | 0.1 |
| Sensiva SC 50 | Ethylhexylglycerin | 0.3 |
| 1,2-heptanediol | 1,2-heptanediol | 1.0 |
| 1,2-octanediol/2,3-octanediol (95:5 w/w %) | 1,2-octanediol/2,3-octanediol (95:5 w/w %) | 1.0 |

**Table 75: Intimate Wipes**

| **Ingredients** | **INCI** | **Amount** |
|---|---|---|
| Dracorin^{®} GOC | Glyceryl Oleate Citrate, Caprylic/Capric Triglyceride | 2.2 |
| PCL-Liquid^{®} 100 | Cetearyl Ethylhexanoate | 3.0 |
| SymCalmin^{®} | Pentylene Glycol, Butylene Glycol, Hydroxyphenyl Propamidobenzoic Acid | 1.0 |
| Frescolat^{®} X-Cool | Menthyl Ethylamido Oxalate | 0.5 |
| Perfume oil PO1; PO2; PO3; PO4 or PO5 | Perfume | 0.2 |
| Water, distilled | Water (Aqua) | ad 100 |
| Pemulen^{™} TR-2 Polymeric Emulsifier | Acrylates/C10-30 Alkyl Acrylate Crosspolymer | 0.2 |
| SymOcide^{®} PH | Phenoxyethanol, Hydroxyacetophenone, Caprylyl Glycol, Aqua | 1.3 |
| Potassium Sorbate | Potassium Sorbate | 0.1 |
| Extrapone^{®} Camomille GW | Glycerin,Aqua, Chamomilla, Recutita Flower Extract | 0.5 |
| Sodium Hydroxide 10% solution | Aqua, Sodium Hydroxide | 0.45 |
| PCA ethyl cocoyl arginate | PCA ethyl cocoyl arginate | 0.1 |
| 1,2-heptanediol | 1,2-heptanediol | 0.4 |
| 1,2-octanediol / 2,3-octanediol blend (98:2 w/w%) | 1,2-octanediol, 2,3 octanediol | 0.5 |

**Table 76: After-Shave Wipes**

| **Ingredients** | **INCI** | **Amount** |
|---|---|---|
| Acrylates/C10-30 Alkyl Acrylate Crosspolymer | Acrylates/C10-30 Alkyl Acrylate Crosspolymer | 0.1 |
| Water, distilled | Water (Aqua) | ad 100 |
| Sodium Hydroxide 10% solution | Aqua, Sodium Hydroxide | 0.13 |
| Glycerin | Glycerin | 4.0 |
| DOWSIL^{™} 2501 Cosmetic Wax | Bis-PEG-18 Methyl Ether Dimethyl Silane | 2.0 |
| Aloe Vera | Aloe Vera | 0.6 |
| Propylene Glycol | Propylene Glycol | 0.3 |
| Witch Hazel Extract | Hamamelis Virginiana (Witch Hazel) Extract | 0.7 |
| C12-15 Alkyl Benzoate | C12-15 Alkyl Benzoate | 2.7 |
| Polysorbate-20 | Polysorbate-20 | 0.5 |
| DOWSIL^{™} 9040 Silicone Elastomer Blend | Cyclopentasiloxane (and) Dimethicone Crosspolymer | 3.5 |
| XIAMETER^{™} PMX-1184 Silicone Fluid | Dimethicone (and) Trisiloxane | 5.8 |
| Perfume oil PO1; PO2; PO3; PO4 or PO5 | Perfume | 0.3 |
| DOWSIL^{™} 9509 Silicone Elastomer Suspension | Dimethicone / Vinyl Dimethicone Crosspolymer (and) C12-14 Pareth-12 | 2.0 |
| Phytic Acid | Phytic Acid | 0.1 |
| Salicylic Acid | Salicylic Acid | 0.1 |
| 1,2-heptanediol/ 2,3-heptanediol blend (96:4 w/w%) | 1,2-heptanediol, 2,3-heptanediol | 0.5 |
| 1,2-octanediol/2,3-octanediol blend (98:2 w/w%) | 1,2-octanediol, 2,3 octanediol | 0.5 |

**Table 77: Air freshener (A/F spray water based)**

| **No** | | **Ingredients** | **Amount** |
|---|---|---|---|
| 1 | | Fragrance | 4 |
| 2 | | Ethanol | 8 |
| 3 | | PEG-40 Hydrogenated Castor Oil | 5 |
| 4 | | 1,2-heptanediol /2,3-heptanediol blend (ratio 99:1) | 1 |
| 5 | | Aqua | add to 100 |
| 6 | | Ethylhexylglycerin | 0.2 |

### Colorless liquid, pH neutral

**Table 78: All purpose cleaner (not in accordance with the present invention)**

| **No** | **Ingredients** | **Amount** |
|---|---|---|
| 1 | Fettalkoholethoxylat | 4 |
| 2 | Sulfonic acid, C 13-17-sec-Alkyl-, Sodium salt | 6.2 |
| 3 | 2-octyl-2H-isothiazol-3-one | 0.1 |
| 4 | Fragrance | 3.5 |
| 5 | 1,2-heptanediol | 1 |
| 6 | Aqua | add to 100 |
| 7 | Farnesol | 0.1 |

### Colorless liquid, pH 7

**Table 79: All purpose cleaner**

| **No** | **Ingredients** | **Amount** |
|---|---|---|
| 1 | Fettalkoholethoxylat | 4 |
| 2 | Sulfonic acid, C 13-17-sec-Alkyl-, Sodium salt | 6.2 |
| 3 | 2-octyl-2H-isothiazol-3-one | 0.1 |
| 4 | Fragrance | 3.5 |
| 5 | 2,3-octanediol | 0.1 |
| 6 | 1,2-heptanediol | 0.9 |
| 7 | Aqua | add to 100 |
| 8 | Hydroxyacetophenone | 0.2 |

### Colorless liquid, pH 7

**Table 80: APC alkaline (not in accordance with the present invention)**

| **No** | **Ingredients** | **Amount** |
|---|---|---|
| 1 | Coco-Glycoside | 1 |
| 2 | Benzalkonium Chloride | 0.2 |
| 3 | Sodiumbicarbonat | 0.1 |
| 4 | Tri-sodiumcitrate-dihydrate | 0.1 |
| 5 | Methylglycinediacetic acid | 0.1 |
| 6 | 1,2-Benzisothiazol-3(2H)-on | 0.1 |
| 7 | Fragrance | 0.1 |
| 8 | 1,2-heptanediol | 0.5 |
| 9 | Aqua | add to 100 |
| 10 | o-cymen-5-ol | 0.1 |

### Colorless liquid, pH 9

**Table 81: Cleaner, liquid, citric acid (not in accordance with the present invention)**

| **No** | **Ingredients** | **Amount** |
|---|---|---|
| 1 | Sulfonic acid, C 13-17-sec-Alkyl-, Sodium salt | 1 |
| 2 | Citric Acid | 5 |
| 3 | Xanthan Gum | 0.5 |
| 4 | 1,2-heptanediol | 0.5 |
| 5 | Fragrance | 0.3 |
| 6 | Aqua | add 100 |
| 7 | Farnesol | 0.1 |

Liquid of low viscosity, pH 2

**Table 82: Cleaner, liquid, citric acid**

| **No** | **Ingredient** | **Amount** |
|---|---|---|
| 1 | Sulfonic acid, C 13-17-sec-Alkyl-, Sodium salt | 1 |
| 2 | Citric Acid | 5 |
| 3 | Xanthan Gum | 0.5 |
| 4 | 1,2-heptanediol/2,3 heptanediol 90 : 10 | 0.5 |
| 5 | Fragrance | 0.3 |
| 6 | Aqua | add 100 |
| 7 | Hydroxyacetophenone | 0.2 |

### Liquid of low viscosity, pH 2

**Table 83: Detergent liquid light duty**

| **No** | **Ingredients** | **Amount** |
|---|---|---|
| 1 | Cocos fatty acid | 0.85 |
| 2 | potassium hydroxide | 0.45 |
| 3 | 2-octyl-2H-isothiazol-3-one | 0.2 |
| 4 | Sulfonic acid, C 13-17-sec-Alkyl-, Sodium salt | 20 |
| 5 | Sodium Laureth Sulfate | 3 |
| 6 | Trideceth-9 | 5 |
| 7 | Sodium Chloride | 1.3 |
| 8 | 2,3-octanediol | 0.1 |
| 9 | 1,2-heptanediol | 0.9 |
| 10 | Fragrance | 0.5 |
| 11 | Aqua | add to 100 |
| 12 | o-Cymen-5-ol | 0.1 |

### Liquid, pH 7.3

**Table 84: Detergent liquid light duty**

| **No** | **Ingredients** | **Amount** |
|---|---|---|
| 1 | Cocos fatty acid | 0.85 |
| 2 | potassium hydroxide | 0.45 |
| 3 | 2-octyl-2H-isothiazol-3-one | 0.2 |
| 4 | Sulfonic acid, C 13-17-sec-Alkyl-, Sodium salt | 20 |
| 5 | Sodium Laureth Sulfate | 3 |
| 6 | Trideceth-9 | 5 |
| 7 | Sodium Chloride | 1.3 |
| 8 | 2,3-heptanediol | 0.1 |
| 9 | 1,2-heptanediol | 0.9 |
| 10 | Fragrance | 0.5 |
| 11 | Aqua | add to 100 |
| 12 | Farnesol | 0.1 |

### Liquid, pH 7.3

**Table 85: Dishwash liquid manual**

| **No** | **Ingredients** | **Amount** |
|---|---|---|
| 1 | Sodium Laureth Sulfate | add 100 |
| 2 | Ethanol | 2.9 |
| 3 | lauramine oxide | 7.7 |
| 4 | propylene glycol | 1.9 |
| 5 | Phenoxyethanol | 0.1 |
| 6 | Benzisothiazolinone, Methylisothiazolinone, Laurylamine Dipropylenediamine | 0.1 |
| 7 | 1,2-heptanediol /2,3-heptanediol blend (ratio 98:2) | 0.5 |
| 8 | sodium chloride | 3.85 |
| 9 | Fragrance | 0.3 |
| 10 | Bisabolol | 0.1 |

### Colorless liquid, pH 8.5

**Table 86: Fabric softener (not in accordance with the present invention)**

| **No** | **Ingredients** | **Amount** |
|---|---|---|
| 1 | Di-(Talg Carboxyethyl) Hydroxyethyl methylammonium-methosulfat | 5.5 |
| 2 | Simethicone | 0.3 |
| 3 | 2-octyl-2H-isothiazol-3-one | 0.1 |
| 4 | 1,2-heptanediol | 0.5 |
| 5 | Fragrance | 0.2 |
| 6 | Aqua | add 100 |
| 7 | Hydroxymethoxyphenyldecanone | 0.05 |

### Liquid, pH 3

**Table 87: Fabric softener concentrate, encapsulated (not in accordance with the present invention)**

| **No** | **Ingredients** | **Amount** |
|---|---|---|
| 1 | Di-(Talg Carboxyethyl) Hydroxyethyl methylammonium-methosulfat | 16.6 |
| 2 | 2-octyl-2H-isothiazol-3-one | 0.1 |
| 3 | Simethicone | 0.3 |
| 4 | magnesium chloride | 0.8 |
| 5 | encapsulated perfume oil | 0.3 |
| 6 | crosslinked cationic polymer | 0.15 |
| 7 | 1,2-heptanediol | 0.5 |
| 8 | Fragrance | 0.6 |
| 9 | Aqua | add 100 |
| 10 | o-Cymen-5-ol | 0.1 |

### White liquid, pH 2.5

**Table 88: Fabric softener concentrate, encapsulated**

| **No** | **Ingredients** | **Amount** |
|---|---|---|
| 1 | Di-(Talg Carboxyethyl) Hydroxyethyl methylammonium-methosulfat | 16.6 |
| 2 | 2-octyl-2H-isothiazol-3-one | 0.1 |
| 3 | Simethicone | 0.3 |
| 4 | magnesium chloride | 0.8 |
| 5 | encapsulated perfume oil | 0.3 |
| 6 | crosslinked cationic polymer | 0.15 |
| 7 | 2,3-heptanediol | 0.05 |
| 8 | 1,2-heptanediol | 0.5 |
| 9 | Fragrance | 0.6 |
| 10 | Aqua | add 100 |
| 11 | o-Cymen-5-ol | 0.1 |

### White liquid, pH 2.5

**Table 89: Hand soap, liquid (not in accordance with the present invention)**

| **No** | **Ingredients** | **Amount** |
|---|---|---|
| 1 | Sodium Laureth Sulfate | 18 |
| 2 | Cocoamidopropylbetaine | 6 |
| 3 | Cocamide DEA | 3 |
| 4 | citric acid | 0.5 |
| 5 | sodium chloride | 1.9 |
| 6 | Glycerol | 2 |
| 7 | Fragrance | 0.2 |
| 8 | 1,2-heptanediol | 0.25 |
| 9 | Aqua | add 100 |
| 10 | Farnesol | 0.2 |

### Slightly colored liquid, pH 6

**Table 90: Rim block gel (not in accordance with the present invention)**

| **No** | **ingredients** | **Amount** |
|---|---|---|
| 1 | Sodium Laureth Sulfate | 11 |
| 2 | Trideceth-9 | 2 |
| 3 | Xanthan gum | 2 |
| 4 | Hydroxyethylcellulose | 0.5 |
| 5 | Citric Acid | 0.4 |
| 6 | Ethanol | 6 |
| 7 | Fragrance | 5 |
| 8 | 1,2-heptanediol | 0.5 |
| 9 | Aqua | add 100 |
| 10 | o-Cymen-5-ol | 0.05 |

### Colorless paste/gel, pH = 4.5

**Table 91: Scent Lotion with capsules (not in accordance with the present invention)**

| **No** | **ingredients** | **Amount** |
|---|---|---|
| 1 | microcrystalline cellulose/cellulose gum | 1 |
| 2 | PEG-40 hydrogenated Castor oil/ Trideceth-9 | 0.5 |
| 3 | encapsulated perfume oil | 1 |
| 4 | 2-octyl-2H-isothiazol-3-one | 0.1 |
| 5 | Fragrance | 5 |
| 6 | 1,2-heptanediol | 1 |
| 7 | Aqua | add 100 |
| 8 | Farnesol | 0.3 |

### White emulsion, pH neutral

**Table 92: Cleaner, liquid, lactic acid (not in accordance with the present invention)**

| **No** | **ingredients** | **Amount** |
|---|---|---|
| 1 | Sodium Laureth Sulfate | 1.8 |
| 2 | Trideceth-9 | 2.1 |
| 3 | Xanthan gum | 0.35 |
| 4 | Lactic Acid | 2,8 |
| 5 | 1,2-heptanediol | 1 |
| 6 | Fragrance | 0.3 |
| 7 | Aqua | add 100 |
| 8 | o-Cymen-5-ol | 0.1 |

### Colorless liquid, pH 2,0

**Table 93: Cleaner, liquid, citric acid (not in accordance with the present invention)**

| **No** | **ingredients** | **Amount** |
|---|---|---|
| 1 | Sulfonic acid, C 13-17-sec-Alkyl-, Sodium salt | 1 |
| 2 | Xanthan gum | 0-3 |
| 3 | citric acid | 5 |
| 4 | 1,2-heptanediol | 0.25 |
| 5 | Fragrance | 0.3 |
| 6 | Aqua | add 100 |
| 7 | o-Cymen-5-ol | 0.1 |

Liquid, pH 2

## Claims

1. A cosmetic or pharmaceutical composition or homecare product, comprising or consisting of
(a) a mixture comprising at least one first linear alkanediol having a carbon chain of 5 to 14 carbon atoms and one or more second linear alkanediol having a carbon chain of 5 to 14 carbon atoms which is different to the first linear alkanediol;
(b) at least one lipophilic active componenthaving a log P_{O/W} value of ≥ 0 to 25.00; and
(c) optionally at least one active substance for a cosmetic or pharmaceutical composition for a homecare product and/or additive, which is/are different to the lipophilic active component (b);
wherein the mixture of at least one first linear alkanediol and one or more second linear alkanediol is selected from the group consisting of
a mixture comprising 1,2-pentanediol and 2,3-pentanediol;
a mixture comprising 1,2-hexanediol and 2,3-hexanediol;
a mixture comprising 1,2-heptanediol and 2,3-heptanediol;
a mixture comprising 1,2-octanediol and 2,3-octanediol;
a mixture comprising 1,2-nonanediol and 2,3-nonanediol;
a mixture comprising 1,2-decanediol and 2,3-decanediol;
a mixture comprisjng 1,2-undecanediol and 2,3-undecanediol;
a mixture comprising 1,2-dodecanediol and 2,3-dodecanediol; and
a mixture comprising 1,2-tridecanediol and 2,3-tridecanediol;
or
wherein the mixture comprising at least one first linear alkanediol and one or more second linear alkanediol is selected from the group consisting of a mixture comprising 1,2-hexanediol and 2,3-octanediol;
a mixture comprising 1,2-octanediol and 2,3-hexanediol; and
a mixture comprising 1,2-octanediol and 2,3-heptanediol;
or
wherein the mixture comprising at least one first linear alkanediol and one or more second linear alkanediol is selected from the group consisting of a mixture comprising 1,2-pentanediol and 2,3-hexanediol;
a mixture comprising 1,2-pentanediol and 2,3-heptanediol;
a mixture comprising 1,2-pentanediol and 2,3-octanediol;
a mixture comprising 1,2-pentanediol and 1,2-nonanediol; and
a mixture comprising1,2-pentanediol and 2,3-nonanediol;
or
wherein the mixture comprising at least one first linear alkanediol and one or more second linear alkanediol is selected from the group consisting of a mixture comprising 1,2-heptanediol and 1,2-octanediol;
a mixture comprising 1,2-heptanediol and 2,3-octanediol;
a mixture comprising 1,2-heptanediol and 1,2-nonanediol; and
a mixture comprising 1,2-heptanediol and 2,3-nonanediol.

2. Cosmetic or pharmaceutical composition or homecare product according to claim 1, wherein the mixture comprising at least one first linear alkanediol and one or more second linear alkanediol comprises the first linear alkanediol and the second alkanediol in a ratio in a range of 50 : 50 to 99.9 : 0.1, preferably in a ratio in a range of 75 : 25 to 99 : 1, more preferred in a ratio in a range of 80 : 20 to 98 : 2, still more preferred in a ratio of 90 : 10 to 95 : 5.

3. Cosmetic or pharmaceutical composition or homecare product according to claim 1 or claim 2,
wherein the at least one lipophilic active component is a lipophilic cosmetically active component selected from the group consisting of Tocopherol, Bisabolol, Ethylhexylglycerin, o-Cymen-5-ol, Propanediol Caprylate, Glyceryl Caprylate, Retinyl Palmitate, Retinol, Farnesol, Glyceryl Laurate, Polyglyceryl-2 caprylate, Hydroxyacetophenone, Caffeine, Undecylenoyl Phenylalanine, Phenylethyl Resorcinol, Hydroxymethoxyphenyl decanone, Zingiber Officinale (Ginger) Root Extract, Myristoyl Nonapeptide-3, Palmitoyl Tripeptide-1, Palmitoyl Tetrapeptide-7, Palmitoyl Pentapeptide-4, Hydroxypinacolone Retinoate, Isochrysis Galbana Extract, Sclareolide, Vanillyl Butylether, Hydroxyphenyl Propamidobenzoic Acid, 4-t-Butylcyclohexanol, Ubiquinone-10, Climbazole and mixtures of two or more of the aforesaid active components; and/or wherein the at least one lipophilic acitve component is a lipophilic pharmaceutically active component selected from the group consisting of Erythromycin, Betamethasone, Ibuprofen, Ketoprofen, Diclofenac, Metronidazole, Imiquimod, Celecoxib, Indomethacin, Caffeine, Minoxidil, Hydrocortisone, and mixtures of two or more of the aforesaid lipophilic pharmaceutically active components.

4. Cosmetic or pharmaceutical composition or homecare product according to any one of claims 1 to 3, further comprising at least one solubilizing agent, wherein the solubilizing agent is selected from the group consisting of PEG-40 Hydrogenated Castor Oil, Polysorbate-20, 60 and 80, PEG-8, PEG-7 Glyceryl Cocoate, Decyl Glucoside, Ceteareth-20, Cetearyl Glucoside, PEG-60 Hydrogenated Castor Oil, Steareth-2, Steareth-20, PPG-26-buteth-26, PPG-5-Laureth-5, Steareth-21, Trideceth-9, PEG-32, Glycereth-26, Polyglyceryl-3 Distearate, Methyl Gluceth-20, PEG-12 Laurate, Polyglyceryl-4 Caprylate, Oleth-20, Oleth-2, Polyglyceryl-4 Caprate, PPG-1-PEG-9 Lauryl Glycol Ether, Polyglyceryl-2 Caprate, Isoceteth-20, PPG-13-decyltetradeceth-24, or mixtures of two or more of the aforementioned solubilizing agents; and/or at least one hydrophilic solvent which is different to the compound (a), wherein the hydrophilic solvent is selected from the group consisting of Glycerin, Propylene Glycol, Butylene Glycol, Isopropyl Alcohol, Propanediol, Dipropylene Glycol, Propylene Carbonate, Ethanol, Hexylene Glycol, Glycerin Carbonate, Methylpropanediol, Butylene Carbonate, Dimethyl Isosorbide, 1,2-pentanediol, 1,2-hexanediol, 1,2-heptanediol, 2,3-pentanediol, 2,3-hexanediol, 2,3-heptanediol, 2,3-octanediol, 2,3-nonanediol, 2,3-decanediol, or mixtures of two or more of the aforementioned hydrophilic solvents.

5. Cosmetic or pharmaceutical composition or homecare product according to any one of claims 1 to 4, wherein the at least one active substance for a cosmetic or pharmaceutical composition or homecareproduct and/or additive, which is/are different to the lipophilic active component (b), is/are selected from the group consisting of agents against ageing of the skin, anti-microbial agent(s), antioxidants, chelating agents, emulsifiers, surfactants, green and synthetic polymers, skin-cooling agents, rheology additives, oils, fragrances or perfume oils, and mixtures of two or more of the aforementioned substances, wherein the anti-microbial agent is selected from the group consisting of 4-hydroxybenzoic acid and its salts and esters, N-(4-chlorophenyl)-N'-(3,4-dichlorophenyl)urea, 2,4,4'-trichloro-2'-hydroxy-diphenyl ether (triclosan), 4-chloro-3,5-dimethylphenol, 2,2'-methylenebis(6-bromo-4-chlorophenol), 3-methyl-4-(1-methylethyl)phenol, 2-benzyl-4-chloro-phenol, 3-(4-chlorophenoxy)-1,2-propanediol, 3-iodo-2-propynyl butylcarbamate, chlorhexidine, 3,4,4'-trichlorocarbanilide (TTC), antibacterial fragrances, thymol, thyme oil, eugenol, oil of cloves, menthol, mint oil, farnesol, phenoxyethanol, glycerol monocaprate, glycerol monocaprylate, glycerol monolaurate (GML), diglycerol monocaprate (DMC), salicylic acid N-alkylamides, n-octylsalicylamide and n-decylsalicylamide.

6. Cosmetic or pharmaceutical composition or homecare product according to any one of claims 1 to 5, comprising the mixture comprising at least one first linear alkanediol and one or more second linear alkanediol in an amount of 0.001 to 15.0 % by weight, preferably in an amount of 0.01 to 10.0 % by weight, more preferably in an amount of 0.1 to 5.0 % by weight, even more preferably in an amount of 0.3 to 3.0 % by weight, and most preferably in an amount 0f 0.5 to 1.0 % by weight, based on the total weight of the composition or homecare product; and/or
comprising the 2,3-alkanediol of the mixture comprising at least one first linear alkanediol and one or more second linear alkanediol in an amount of 0.001 to 15.0 % by weight, preferably in an amount of 0.01 to 10.0 % by weight, more preferably in an amount of 0.1 to 5.0 % by weight, even more preferably in an amount of 0.3 to 3.0 % by weight, and most preferably in an amount of 0.5 to 1.0 % by weight, based on the total weight of the composition or homecare product; preferably in an amount of 0.001 to 0.5 % by weight, more preferably in an amount of 0.005 to 0.1 % by weight, and most preferably in an amount of 0.01 to 0.075 % by weight, based on the total weight of the composition or homecare product; and/or
comprising the at least one lipophilic active component in an amount of 0.001 to 5.0 % by weight, preferably in an amount of 0.005 to 4.0 % by weight, and most preferably in an amount of 0.01 to 3.0 % by weight, based on the total weight of the composition.

7. Cosmetic or pharmaceutical composition or homecare product according to any one of claims 1 to 6
- in the form of a dispersion comprising an oil component in an amount of ≥ 1% by weight, in particular in an amount of ≥ 3 % by weight, preferably in the form of an emulsion, in particular an oil-inwater (O/W) emulsion, a water-in-oil (W/O) emulsion, a multiple emulsion, a hydrodispersion gel, a balm, a multiple emulsion of the water-in-oil type (W/O/W) or of the oil-in-water type (O/W/O), a PIT emulsion, a Pickering emulsion, a micro-emulsion, a liquid, a lotion, as suspension, a milk, an ointment, a paste, a gel, a cream based formulation, an oil based formulation or a liposome-based formulation; or
- in the form of a liquid surfactant formulation; or
- in the form of a solid surfactant formulation; or
- in the form of an aqueous or aqueous/alcoholic, particularly aqueous/ethanolic, or aqueous/glycolic based solution, comprising the alcohol or glycol in an amount of 0.1 % to ≤ 50 % by weight, based on the total weight of the solution;
in particular, wherein the cosmetic or pharmaceutical composition is a cold formulation preparation.

8. Cosmetic non-therapeutic use of the cosmetic composition according to any one of claims 1 to 7, for personal care, in skin protection, skin care, scalp protection, scalp care, hair care, nail care, or in the prevention and/or treatment of skin conditions, skin aging, wrinkle formation, loss of skin volume, loss of skin elasticity, pigment spots, skin dryness, flaking, greasiness, for moisturizing the skin, or for animal care.

9. Pharmaceutical composition according to any one of claims 1 to 7 for use as a medicament; and preferably for use in the prevention and/or treatment of intolerant or sensitive skin, skin irritation, skin reddening, wheals, pigment abnormalities, hypopigmentation and/or hyperpigmentation of the skin, dysfunctions of human hair, skin and/or nails, in particular dermatological or keratological diseases, wherein the dermatological or keratological diseases are selected from the group consisting of atopic dermatitis (neurodermitis), psoriasis, acneiform exanthma, sebostasis, xerosis, eczema, hyper seborrhea and hypo seborrhea, dermatitis, rosacea, erythema, wheals, *pruritus,* inflammation, irritation, fibrosis, *lichen planus, pityriasis rosea, pityriasis versicolor,* autoimmune bullous diseases, urticaria, angioedema, allergic skin reactions, wound healing, tissue regeneration and in the treatment of inflammatory or pain.

10. Use of a mixture comprising at least one first linear alkanediol and one or more second linear alkanediol,
- for increasing the solubility of a lipophilic active component, preferably a lipophilic active component as defined in claim 3, in a cosmetic or pharmaceutical composition or homecare product, compared to a cosmetic or pharmaceutical composition or homecare product including the lipophilic active component without the use of an alkanediol, and/or
- for increasing the concentration of a lipophilic active component, preferably a lipophilic active component as defined in claim 3, in a cosmetic or pharmaceutical composition or homecare product, compared to a cosmetic or pharmaceutical composition or homecare product including the lipophilic active component without the use of an alkanediol, and/or
- for reducing or preventing the foam formation of a cosmetic or pharmaceutical composition or homecare product comprising a solubilizing agent, compared to a cosmetic or pharmaceutical composition or homecare product including the the solubilizing agent without the use of an alkanediol;
or
use of a mixture comprising at least one first linear alkanediol and one or more second linear alkanediol as solubilizing agent replacer in a cosmetic or pharmaceutical composition or homecare product;
wherein the mixture of at least one first linear alkanediol with one or more second linear alkanediol is selected from the group consisting of
a mixture comprising 1,2-pentanediol and 2,3-pentanediol;
a mixture comprising 1,2-hexanediol and 2,3-hexanediol;
a mixture comprising 1,2-heptanediol and 2,3-heptanediol;
a mixture comprising 1,2-octanediol and 2,3-octanediol;
a mixture comprising 1,2-nonanediol and 2,3-nonanediol;
a mixture comprising 1,2-decanediol and 2,3-decanediol;
a mixture comprisjng 1,2-undecanediol and 2,3-undecanediol;
a mixture comprising 1,2-dodecanediol and 2,3-dodecanediol; and
a mixture comprising 1,2-tridecanediol and 2,3-tridecanediol;
or
wherein the mixture comprising at least one first linear alkanediol and one or more second linear alkanediol is selected from the group consisting of a mixture comprising 1,2-hexanediol and 2,3-octanediol;
a mixture comprising 1,2-octanediol and 2,3-hexanediol; and
a mixture comprising 1,2-octanediol and 2,3-heptanediol;
or
wherein the mixture comprising at least one first linear alkanediol and one or more second linear alkanediol is selected from the group consisting of a mixture comprising 1,2-pentanediol and 2,3-hexanediol;
a mixture comprising 1,2-pentanediol and 2,3-heptanediol;
a mixture comprising 1,2-pentanediol and 2,3-octanediol;
a mixture comprising 1,2-pentanediol and 1,2-nonanediol; and
a mixture comprising1,2-pentanediol and 2,3-nonanediol;
or
wherein the mixture comprising at least one first linear alkanediol and one or more second linear alkanediol is selected from the group consisting of a mixture comprising 1,2-heptanediol and 1,2-octanediol;
a mixture comprising 1,2-heptanediol and 2,3-octanediol;
a mixture comprising 1,2-heptanediol and 1,2-nonanediol; and
a mixture comprising 1,2-heptanediol and 2,3-nonanediol.

## Patentansprüche

1. Kosmetische oder pharmazeutische Zusammensetzung oder Heimpflegeprodukt, das aufweist oder besteht aus:
(a) einem Gemisch, das mindestens ein erstes lineares Alkandiol mit einer Kohlenstoffkette von 5 bis 14 Kohlenstoffatomen und ein oder mehrere zweite lineare Alkandiole mit einer Kohlenstoffkette von 5 bis 14 Kohlenstoffatomen aufweist, die sich vom dem ersten linearen Alkandiol unterscheidet;
(b) mindestens eine lipophile aktive Komponente mit einem log Po/w-Wert von ≥ 0 bis 25,00; und
(c) optional mindestens einen Wirkstoff für eine kosmetische oder pharmazeutische Zusammensetzung für ein Heimpflegeprodukt und/oder einen Zusatzstoff, der sich von der lipophilen aktiven Komponente (b) unterscheidet;
wobei die Mischung aus mindestens einem ersten linearen Alkandiol und einem oder mehreren zweiten linearen Alkandiolen aus der Gruppe ausgewählt ist, die besteht aus:
einem Gemisch, das 1,2-Pentandiol und 2,3-Pentandiol aufweist;
einem Gemisch, das 1,2-Hexandiol und 2,3-Hexandiol aufweist;
einem Gemisch, das 1,2-Heptandiol und 2,3-Heptandiol aufweist;
einem Gemisch, das 1,2-Octandiol und 2,3-Octandiol aufweist;
einem Gemisch, das 1,2-Nonandiol und 2,3-Nonandiol aufweist;
einem Gemisch, das 1,2-Decandiol und 2,3-Decandiol aufweist;
einem Gemisch, das 1,2-Undecandiol und 2,3-Undecandiol aufweist;
einem Gemisch, das 1,2-Dodecandiol und 2,3-Dodecandiol aufweist; und
einem Gemisch, das 1,2-Tridecandiol und 2,3-Tridecandiol aufweist;
oder
wobei das Gemisch, das mindestens ein erstes lineares Alkandiol und ein oder mehrere zweite lineare Alkandiole aufweist, die aus der Gruppe ausgewählt sind, die besteht aus:
einem Gemisch, das 1,2-Hexandiol und 2,3-Octandiol aufweist;
einem Gemisch, das 1,2-Octandiol und 2,3-Hexandiol aufweist; und
einem Gemisch, das 1,2-Octandiol und 2,3-Heptandiol aufweist;
oder
wobei das Gemisch, das mindestens ein erstes lineares Alkandiol und ein oder mehrere zweite lineare Alkandiole aufweist, die aus der Gruppe ausgewählt sind, die besteht aus:
einem Gemisch, das 1,2-Pentandiol und 2,3-Hexandiol aufweist;
einem Gemisch, das 1,2-Pentandiol und 2,3-Heptandiol aufweist;
einem Gemisch, das 1,2-Pentandiol und 2,3-Octandiol aufweist;
einem Gemisch, das 1,2-Pentandiol und 1,2-Nonandiol aufweist; und
einem Gemisch, das 1,2-Pentandiol und 2,3-Nonandiol aufweist;
oder
wobei das Gemisch, das mindestens ein erstes lineares Alkandiol und ein oder mehrere zweite lineare Alkandiole aufweist, aus der Gruppe ausgewählt sind, die besteht aus
einem Gemisch, das 1,2-Heptandiol und 1,2-Octandiol aufweist;
einem Gemisch, das 1,2-Heptandiol und 2,3-Octandiol aufweist;
einem Gemisch, das 1,2-Heptandiol und 1,2-Nonandiol aufweist; und
einem Gemisch, das 1,2-Heptandiol und 2,3-Nonandiol aufweist.

2. Kosmetische oder pharmazeutische Zusammensetzung oder Heimpflegeprodukt nach Anspruch 1, wobei das Gemisch, das mindestens ein erstes lineares Alkandiol und ein oder mehrere zweite lineare Alkandiole aufweist, das erste lineare Alkandiol und das zweite Alkandiol in einem Verhältnis in einem Bereich von 50 : 50 bis 99,9 : 0,1, vorzugsweise in einem Verhältnis in einem Bereich von 75 : 25 bis 99 : 1, besonders bevorzugt in einem Verhältnis in einem Bereich von 80 : 20 bis 98 : 2, ganz besonders bevorzugt in einem Verhältnis von 90 : 10 bis 95 : 5 aufweist.

3. Kosmetische oder pharmazeutische Zusammensetzung oder Heimpflegeprodukt nach Anspruch 1 oder Anspruch 2,
wobei die mindestens eine lipophile aktive Komponente eine lipophile kosmetisch aktive Komponente ist, die aus der Gruppe ist, die aus Tocopherol, Bisabolol, Ethylhexylglycerin, o-Cymen-5-ol, Propandiol-Caprylat, Glyceryl-Caprylat, Retinyl-Palmitat, Retinol, Farnesol, Glyceryl-Laurat, Polyglyceryl-2-Caprylat, Hydroxyacetophenon, Koffein, Undecylenoyl-Phenylalanin, Phenylethyl-Resorcinol, Hydroxymethoxyphenyl-Decanon, Zingiber Officinale (Ingwer)-Wurzelextrakt, Myristoyl Nonapeptid-3, Palmitoyl Tripeptid-1, Palmitoyl Tetrapeptid-7, Palmitoyl Pentapeptid-4, Hydroxypinacolon Retinoat, Isochrysis-Galbana-Extrakt, Sclareolid, Vanillylbutylether, Hydroxyphenylpropamidobenzoesäure, 4-t-Butylcyclohexanol, Ubiquinon-10, Climbazol und Mischungen aus zwei oder mehreren der vorgenannten aktiven Komponenten besteht; und/oder wobei die mindestens eine lipophile aktive Komponente eine lipophile pharmazeutische aktive Komponente ist, die aus der Gruppe ausgewählt ist, die aus Erythromycin, Betamethason, Ibuprofen, Ketoprofen, Diclofenac, Metronidazol, Imiquimod, Celecoxib, Indomethacin, Koffein, Minoxidil, Hydrocortison und Gemischen aus zwei oder mehreren der vorgenannten lipophilen pharmazeutischen aktiven Komponenten besteht.

4. Kosmetische oder pharmazeutische Zusammensetzung oder Heimpflegeprodukt nach einem der Ansprüche 1 bis 3, das ferner mindestens ein Lösungshilfsmittel aufweist, wobei das Lösungshilfsmittel aus der Gruppe ausgewählt ist, die aus PEG-40 Hydrogenated Castor Oil, Polysorbate-20, 60 und 80, PEG-8, PEG-7 Glyceryl Cocoate, Decyl Glucoside, Ceteareth-20, Cetearyl Glucoside, PEG-60 Hydrogenated Castor Oil, Steareth-2, Steareth-20, PPG-26-Buteth-26, PPG-5-Laureth-5, Steareth-21, Trideceth-9, PEG-32, Glycereth-26, Polyglyceryl-3 Distearate, Methyl Gluceth-20, PEG-12 Laurate, Polyglyceryl-4 Caprylate, Oleth-20, Oleth-2, Polyglyceryl-4 Caprate, PPG-1-PEG-9 Laurylglykolether, Polyglyceryl-2 Caprate, Isoceteth-20, PPG-13-Decyltetradeceth-24 oder Mischungen aus zwei oder mehreren der vorgenannten Lösungshilfsmittel besteht; und/oder mindestens ein hydrophiles Lösungsmittel, das von der Verbindung (a) unterscheidet, wobei das hydrophile Lösungsmittel aus der Gruppe ausgewählt ist, die aus Glycerin, Propylenglycol,
Butylenglycol, Isopropylalkohol, Propandiol, Dipropylenglycol, Propylencarbonat, Ethanol, Hexylenglycol, Glycerincarbonat, Methylpropandiol, Butylencarbonat, Dimethylisosorbid, 1,2-Pentandiol, 1,2-Hexandiol, 1,2-Heptandiol, 2,3-Pentandiol, 2,3-Hexandiol, 2,3-Heptandiol, 2,3-Octandiol, 2,3-Nonandiol, 2,3-Decandiol oder Mischungen aus zwei oder mehreren der vorgenannten hydrophilen Lösungsmittel besteht.

5. Kosmetische oder pharmazeutische Zusammensetzung oder Heimpflegeprodukt nach einem der Ansprüche 1 bis 4, wobei der mindestens eine Wirkstoff für eine kosmetische oder pharmazeutische Zusammensetzung oder ein Heimpflegeprodukt und/oder ein Zusatzstoff, der sich von der lipophilen Wirkkomponente (b) unterscheidet, aus der Gruppe ausgewählt ist, die aus Mitteln gegen die Hautalterung, antimikrobiellen Mitteln, Antioxidantien, Chelatbildnern, Emulgatoren, Tensiden, grünen und synthetischen Polymeren, Hautkühlungsmitteln, Rheologieadditiven, Ölen, Duftstoffen oder Parfümölen und Mischungen aus zwei oder mehreren der vorgenannten Stoffe besteht, wobei das antimikrobielle Mittel aus der Gruppe ausgewählt ist, die aus 4-Hydroxybenzoesäure und ihrer Salze und Ester, N-(4-Chlorphenyl)-N'-(3,4-dichlorphenyl)harnstoff, 2,4,4'-Trichlor-2'-hydroxy-diphenylether (Triclosan), 4-Chlor-3,5-dimethyl-phenol, 2,2'-Methylenbis(6-brom-4-chlorphenol), 3-Methyl-4-(1 -methylethyl)phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakteriellen Duftstoffen, Thymol, Thymianöl, Eugenol, Nelkenöl, Menthol, Minzöl, Farnesol, Phenoxyethanol, Glycerinmonocaprat, Glycerinmonocaprylat, Glycerinmonolaurat (GML), Diglycerinmonocaprat (DMC), Salicylsäure-N-alkylamide, n-Octylsalicylamid und n-Decylsalicylamid besteht.

6. Kosmetische oder pharmazeutische Zusammensetzung oder Heimpflegeprodukt nach einem der Ansprüche 1 bis 5, das das Gemisch aufweist, das mindestens ein erstes lineares Alkandiol und ein oder mehrere zweite lineare Alkandiole in einer Menge von 0,001 bis 15,0 Gew.-%, vorzugsweise in einer Menge von 0.01 bis 10,0 Gew.-%, vorzugsweise in einer Menge von 0,1 bis 5,0 Gew.-%, noch bevorzugter in einer Menge von 0,3 bis 3,0 Gew.-% und am meisten bevorzugt in einer Menge von 0,5 bis 1,0 Gew.-%, bezogen auf das Gesamtgewicht der Zusammensetzung oder des Heimpflegeprodukts aufweist; und/oder
das das 2,3-Alkandiol der Mischung, die mindestens ein erstes lineares Alkandiol und ein oder mehrere zweite lineare Alkandiole in einer Menge von 0,001 bis 15,0 Gew.-%, vorzugsweise in einer Menge von 0,01 bis 10,0 Gew.-%, noch bevorzugter in einer Menge von 0,1 bis 5,0 Gew.-%, noch bevorzugter in einer Menge von 0,3 bis 3,0 Gew.-% und am meisten bevorzugt in einer Menge von 0.5 bis 1,0 Gew.-% bezogen auf das Gesamtgewicht der Zusammensetzung oder des Heimpflegeprodukts aufweist; vorzugsweise in einer Menge von 0,001 bis 0,5 Gew.-%, noch bevorzugter in einer Menge von 0,005 bis 0,1 Gew.-% und am meisten bevorzugt in einer Menge von 0,01 bis 0,075 Gew.-%, bezogen auf das Gesamtgewicht der Zusammensetzung oder des Heimpflegeprodukts; und/oder
das die mindestens einen lipophile aktive Komponente in einer Menge von 0,001 bis 5,0 Gew.-%, vorzugsweise in einer Menge von 0,005 bis 4,0 Gew.-% und besonders bevorzugt in einer Menge von 0,01 bis 3,0 Gew.-%, bezogen auf das Gesamtgewicht der Zusammensetzung, aufweist.

7. Kosmetische oder pharmazeutische Zusammensetzung oder Heimpflegeprodukt nach einem der Ansprüche 1 bis 6
- in Form einer Dispersion, die eine Ölkomponente in einer Menge von ≥ 1 Gew.-%, insbesondere in einer Menge von ≥ 3 Gew.-%, vorzugsweise in Form einer Emulsion, insbesondere einer Öl-in-Wasser-Emulsion (O/W), einer Wasser-in-Öl-Emulsion (W/O), einer Mehrfachemulsion, eines Hydrodispersionsgels, eines Balsams, einer Mehrfachemulsion vom Typ Wasser-in-Öl (W/O/W) oder vom Typ Öl-in-Wasser (O/W/O), eine PIT-Emulsion, eine Pickering-Emulsion, eine Mikroemulsion, einer Flüssigkeit, einer Lotion, einer Suspension, einer Milch, einer Salbe, einer Paste, eines Gels, einer Formulierung auf Cremebasis, einer Formulierung auf Ölbasis oder einer Formulierung auf Liposomenbasis; oder in Form einer flüssigen Tensidformulierung; oder
- in Form einer festen Tensidformulierung; oder
- in Form einer wässrigen oder wässrig/alkoholischen, insbesondere wässrig/ethanolischen, oder wässrig/glykolischen Lösung, die den Alkohol oder das Glykol in einer Menge von 0,1 bis ≤ 50 Gew.-%, bezogen auf das Gesamtgewicht der Lösung, aufweist;
wobei es sich bei der kosmetischen oder pharmazeutischen Zusammensetzung insbesondere um eine Kaltformulierungszubereitung handelt.

8. Kosmetische nicht-therapeutische Verwendung der kosmetischen Zusammensetzung nach einem der Ansprüche 1 bis 7 zur Körperpflege, zum Hautschutz, zur Hautpflege, zum Schutz der Kopfhaut, zur Kopfhautpflege, zur Haarpflege, zur Nagelpflege oder zur Vorbeugung und/oder Behandlung von Hautleiden, Hautalterung, Faltenbildung, Volumenverlust der Haut, Elastizitätsverlust der Haut , Pigmentflecken, Hauttrockenheit, Schuppenbildung, Fettigkeit, zur Befeuchtung der Haut oder zur Tierpflege.

9. Pharmazeutische Zusammensetzung nach einem der Ansprüche 1 bis 7 zur Verwendung als Arzneimittel; und vorzugsweise zur Vorbeugung und/oder Behandlung von intoleranter oder empfindlicher Haut, Hautreizungen, Hautrötungen, Quaddeln, Pigmentstörungen, Hypopigmentierung und/oder Hyperpigmentierung der Haut, Funktionsstörungen der menschlichen Haare, Haut und/oder Nägel, insbesondere dermatologischen oder keratologischen Erkrankungen, wobei die dermatologischen oder keratologischen Erkrankungen ausgewählt sind aus der Gruppe bestehend aus atopischer Dermatitis (Neurodermitis) Psoriasis, akneiformes Exanthma, Sebostase, Xerose, Ekzem, Hyperseborrhoe und Hyposeborrhoe, Dermatitis, Rosacea, Erythem, Quaddeln, Pruritus, Entzündung, Reizung, Fibrose, Lichen planus, Pityriasis rosea, Pityriasis versicolor, autoimmune bullöse Erkrankungen, Urtikaria, Angioödem, allergische Hautreaktionen, Wundheilung, Geweberegeneration und bei der Behandlung von Entzündungen oder Schmerzen.

10. Verwendung einer Mischung, die mindestens ein erstes lineares Alkandiol und ein oder mehrere zweite lineare Alkandiole aufweist,
- zur Erhöhung der Löslichkeit eines lipophilen Wirkstoffs, vorzugsweise eines lipophilen Wirkstoffs nach Anspruch 3, in einer kosmetischen oder pharmazeutischen Zusammensetzung oder einem Heimpflegeprodukt im Vergleich zu einer kosmetischen oder pharmazeutischen Zusammensetzung oder einem Heimpflegeprodukt, die bzw. das den lipophilen Wirkstoff ohne die Verwendung eines Alkandiols aufweist, und/oder
- zur Erhöhung der Konzentration eines lipophilen Wirkstoffs, vorzugsweise eines lipophilen Wirkstoffs nach Anspruch 3, in einer kosmetischen oder pharmazeutischen Zusammensetzung oder einem Körperpflegemittel im Vergleich zu einer kosmetischen oder pharmazeutischen Zusammensetzung oder einem Heimpflegeprodukt, die bzw. das den lipophilen Wirkstoff ohne die Verwendung eines Alkandiols aufweist, und/oder
- zur Verringerung oder Verhinderung der Schaumbildung einer kosmetischen oder pharmazeutischen Zusammensetzung oder eines Heimpflegeprodukts, die/das ein Lösungshilfsmittel aufweist, im Vergleich zu einer kosmetischen oder pharmazeutischen Zusammensetzung oder einem Heimpflegeprodukt, die/das das Lösungshilfsmittel ohne die Verwendung eines Alkandiols aufweist;
oder
Verwendung einer Mischung, die mindestens ein erstes lineares Alkandiol und ein oder mehrere zweite lineare Alkandiole aufweist, als Lösungshilfsmittel-Austauschstoff in einer kosmetischen oder pharmazeutischen Zusammensetzung oder einem Heimpflegeprodukt;
wobei die Mischung aus mindestens einem ersten linearen Alkandiol mit einem oder mehreren zweiten linearen Alkandiolen aus der Gruppe ausgewählt ist, die besteht aus
einem Gemisch, das 1,2-Pentandiol und 2,3-Pentandiol aufweist;
einem Gemisch, das 1,2-Hexandiol und 2,3-Hexandiol aufweist;
einem Gemisch, das 1,2-Heptandiol und 2,3-Heptandiol aufweist;
einem Gemisch, das 1,2-Octandiol und 2,3-Octandiol aufweist;
einem Gemisch, das 1,2-Nonandiol und 2,3-Nonandiol aufweist;
einem Gemisch, das 1,2-Decandiol und 2,3-Decandiol aufweist;
einem Gemisch, das 1,2-Undecandiol und 2,3-Undecandiol enthält;
einem Gemisch, das 1,2-Dodecandiol und 2,3-Dodecandiol aufweist; und
einem Gemisch, das 1,2-Tridecandiol und 2,3-Tridecandiol aufweist;
oder
wobei das Gemisch, das mindestens ein erstes lineares Alkandiol und ein oder mehrere zweite lineare Alkandiole aufweist, aus der Gruppe ausgewählt ist, die besteht aus
einem Gemisch, das 1,2-Hexandiol und 2,3-Octandiol aufweist;
einem Gemisch, das 1,2-Octandiol und 2,3-Hexandiol aufweist; und
einem Gemisch, das 1,2-Octandiol und 2,3-Heptandiol aufweist;
oder
wobei das Gemisch, das mindestens ein erstes lineares Alkandiol und ein oder mehrere zweite lineare Alkandiole aufweist, aus der Gruppe ausgewählt ist, die besteht aus
einem Gemisch, das 1,2-Pentandiol und 2,3-Hexandiol aufweist;
einem Gemisch, das 1,2-Pentandiol und 2,3-Heptandiol aufweist;
einem Gemisch, das 1,2-Pentandiol und 2,3-Octandiol aufweist;
einem Gemisch, das 1,2-Pentandiol und 1,2-Nonandiol aufweist; und
einem Gemisch, das 1,2-Pentandiol und 2,3-Nonandiol aufweist;
oder
wobei das Gemisch, das mindestens ein erstes lineares Alkandiol und ein oder mehrere zweite lineare Alkandiole aufweist, aus der Gruppe ausgewählt ist, die besteht aus
einem Gemisch, das 1,2-Heptandiol und 1,2-Octandiol aufweist;
einem Gemisch, das 1,2-Heptandiol und 2,3-Octandiol aufweist;
einem Gemisch, das 1,2-Heptandiol und 1,2-Nonandiol aufweist; und
einem Gemisch, das 1,2-Heptandiol und 2,3-Nonandiol aufweist.

## Revendications

1. Composition cosmétique ou pharmaceutique ou produit d'entretien, comprenant, ou consistant en
(a) un mélange comprenant au moins un premier alcanediol linéaire ayant une chaîne carbonée de 5 à 14 atomes de carbone, et un ou plusieurs deuxièmes alcanediols linéaires ayant une chaîne carbonée de 5 à 14 atomes de carbone, différente de celle du premier alcanediol linéaire ;
(b) au moins un composant lipophile actif ayant une valeur log Po/w ≥ 0 à 25,00 ; et
(c) facultativement au moins une substance active pour une composition cosmétique ou pharmaceutique pour un produit d'entretien et/ou un additif, différent(s) du composant lipophile actif (b) ;
où le mélange d'au moins un premier alcanediol linéaire et d'un ou de plusieurs deuxièmes alcanediols linéaires est sélectionné dans le groupe comprenant un mélange comprenant du 1,2-pentanediol et du 2,3-pentanediol ;
un mélange comprenant du 1,2-hexanediol et du 2,3-hexanediol ;
un mélange comprenant du 1,2-heptanediol et du 2,3-heptanediol ;
un mélange comprenant du 1,2-octanediol et du 2,3-octanediol ;
un mélange comprenant du 1,2-nonanediol et du 2,3-nonanediol ;
un mélange comprenant du 1,2-décanediol et du 2,3-décanediol ;
un mélange comprenant du 1,2-undécanediol et du 2,3-undécanediol ;
un mélange comprenant du 1,2-dodécanediol et du 2,3-dodécanediol ; et
un mélange comprenant du 1,2-tridécanediol et du 2,3-tridécanediol ;
ou
où le mélange comprenant au moins un premier alcanediol linéaire et un ou plusieurs deuxièmes alcanediols linéaires est sélectionné dans le groupe comprenant
un mélange comprenant du 1,2-hexanediol et du 2,3-octanediol ;
un mélange comprenant du 1,2-octanediol et du 2,3-hexanediol ; et
un mélange comprenant du 1,2-octanediol et du 2,3-heptanediol ;
ou
où le mélange comprenant au moins un premier alcanediol linéaire et un ou plusieurs deuxièmes alcanediols linéaires est sélectionné dans le groupe comprenant
un mélange comprenant du 1,2-pentanediol et du 2,3-hexanediol ;
un mélange comprenant du 1,2-pentanediol et du 2,3-heptanediol ;
un mélange comprenant du 1,2-pentanediol et du 2,3-octanediol ;
un mélange comprenant du 1,2-pentanediol et du 1,2-nonanediol ; et
un mélange comprenant du 1,2-pentanediol et du 2,3-nonanediol ;
ou
où le mélange comprenant au moins un premier alcanediol linéaire et un ou plusieurs deuxièmes alcanediols linéaires est sélectionné dans le groupe comprenant
un mélange comprenant du 1,2-heptanediol et du 1,2-octanediol ;
un mélange comprenant du 1,2-heptanediol et du 2,3-octanediol ;
un mélange comprenant du 1,2-heptanediol et du 1,2-nonanediol ; et
un mélange comprenant du 1,2-heptanediol et du 2,3-nonanediol.

2. Composition cosmétique ou pharmaceutique ou produit d'entretien selon la revendication 1, où le mélange comprenant au moins un premier alcanediol linéaire et un ou plusieurs deuxièmes alcanediols linéaires comprend le premier alcanediol linéaire et le deuxième alcanediol dans un rapport compris entre 50:50 et 99,9:0,1, avantageusement dans un rapport compris entre 75:25 et 99:1, de préférence dans un rapport compris entre 80:20 et 98:2, et tout particulièrement dans un rapport compris entre 90:10 et 95:5.

3. Composition cosmétique ou pharmaceutique ou produit d'entretien selon la revendication 1 ou la revendication 2,
où ledit au moins un composant lipophile actif est un composant lipophile cosmétiquement actif sélectionné dans le groupe comprenant tocophérol, bisabolol, éthylhexylglycérine, o-cymène-5-ol, caprylate de propanediol, caprylate de glycéryle, palmitate de rétinyle, rétinol, farnésol, laurate de glycéryle, caprylate de polyglycéryle-2, hydroxyacétophénone, caféine, undécylénoyl phénylalanine, phényléthyl résorcinol, hydroxyméthoxyphényl décanone, extrait de racine de Zingiber Officinale (gingembre), myristoyl nonapeptide-3, palmitoyl tripeptide-1, palmitoyl tétrapeptide-7, palmitoyl pentapeptide-4, hydroxypinacolone rétinoate, , extrait d'Isochrysis Galbana, sclaréolide, vanillyl butyléther, acide hydroxyphényl propamidobenzoïque, 4-t-butylcyclohexanol, ubiquinone-10, climbazole et mélanges de deux ou de plusieurs des composants actifs susmentionnés ; et/ou où ledit au moins un composant lipophile actif est un composant lipophile pharmaceutiquement actif sélectionné dans le groupe comprenant érythromycine, bétaméthasone, ibuprofène, kétoprofène, diclofénac, métronidazole, imiquimod, célécoxib, indométhacine, caféine, minoxidil, hydrocortisone et des mélanges de deux ou plusieurs des composants lipophiles pharmaceutiquement actifs susmentionnés.

4. Composition cosmétique ou pharmaceutique ou produit d'entretien selon l'une des revendications 1 à 3, comprenant en outre au moins un agent solubilisant, où ledit agent solubilisant est sélectionné dans le groupe comprenant huile de ricin hydrogénée PEG-40, polysorbate-20, 60 et 80, PEG-8, PEG-7 cocoate de glycéryle, décylglucoside, ceteareth-20, cétéaryl glucoside, PEG-60 huile de ricin hydrogénée, stéareth-2, stéareth-20, PPG-26-buteth-26, PPG-5-laureth-5, stéareth-21, tridéceth-9, PEG-32, glycéreth-26, distéarate de polyglycéryle-3, méthyl gluceth-20, laurate de PEG-12, caprylate de polyglycéryle-4, oléth-20, oléth-2, caprate de polyglycéryle-4, PPG-1-PEG-9 éther de laurylglycol, caprate de polyglycéryle-2, isocéteth-20, PPG-13-décyltétradéceth-24, ou des mélanges de deux ou plusieurs des agents solubilisants susmentionnés ; et/ou au moins un solvant hydrophile différent du composé (a), ledit solvant hydrophile étant sélectionné dans le groupe comprenant glycérine, propylène glycol, butylène glycol, alcool isopropylique, propanediol, dipropylène glycol, carbonate de propylène, éthanol, hexylène glycol, carbonate de glycérine, méthylpropanediol, carbonate de butylène, diméthyl isosorbide, 1,2-pentanediol, 1,2-hexanediol, 1,2-heptanediol, 2,3-pentanediol, 2,3-hexanediol, 2,3-heptanediol, 2,3-octanediol, 2,3-nonanediol, 2,3-decanediol, ou des mélanges de deux ou de plusieurs des solvants hydrophiles susmentionnés.

5. Composition cosmétique ou pharmaceutique ou produit d'entretien selon l'une des revendications 1 à 4, où ladite au moins une substance active pour une composition cosmétique ou pharmaceutique pour un produit d'entretien et/ou un additif, différent(s) du composant lipophile actif (b), sont sélectionnés dans le groupe comprenant agents antivieillissement de la peau, agents antimicrobiens, antioxydants, agents chélateurs, émulsifiants, tensioactifs, polymères verts et synthétiques, agents rafraîchissants pour la peau, additifs rhéologiques, huiles, parfums ou huiles parfumées, et mélanges de deux ou de plusieurs des substances susmentionnées, où l'agent antimicrobien est sélectionné dans le groupe comprenant acide 4-hydroxybenzoïque et ses sels et esters, N-(4-chlorophényl)-N'-(3,4-dichlorophényl)urée, 2,4,4'-trichloro-2'-hydroxy-diphényléther (triclosan), 4-chloro-3,5-diméthylphénol, 2,2'-méthylènebis(6-bromo-4-chlorophénol), 3-méthyl-4-(1-méthyléthyl)phénol, 2-benzyl-4-chlorophénol, 3-(4-chlorophénoxy)-1,2-propanediol, 3-iodo-2-propynyl butylcarbamate, chlorhexidine, 3,4,4'-trichlorocarbanilide (TTC), parfums antibactériens, thymol, huile de thym, eugénol, huile de clou de girofle, menthol, huile de menthe, farnésol, phénoxyéthanol, monocaprate de glycérol, monocaprylate de glycérol, monolaurate de glycérol (GML), monocaprate de diglycérol (DMC), N-alkylamides d'acide salicylique, n-octylsalicylamide et n-décylsalicylamide.

6. Composition cosmétique ou pharmaceutique ou produit d'entretien selon l'une des revendications 1 à 5, comprenant le mélange comprenant au moins un premier alcanediol linéaire et un ou plusieurs deuxièmes alcanediols linéaires dans une teneur de 0,001 à 15,0 % en poids, avantageusement dans une teneur de 0,01 à 10,0 % en poids, de préférence dans une teneur de 0,1 à 5,0 % en poids, plus particulièrement dans une teneur de 0,3 à 3,0 % en poids, et tout particulièrement dans une teneur de 0,5 à 1,0 % en poids, par rapport au poids total de la composition ou du produit d'entretien ; et/ou comprenant le 2,3-alcanediol du mélange comprenant au moins un premier alcanediol linéaire et un ou plusieurs deuxièmes alcanediols linéaires dans une teneur de 0,001 à 15,0 % en poids, avantageusement dans une teneur de 0,01 à 10,0 % en poids, de préférence dans une teneur de 0,1 à 5,0 % en poids, plus particulièrement dans une teneur de 0,3 à 3,0 % en poids, et tout particulièrement dans une teneur de 0,5 à 1,0 % en poids, par rapport au poids total de la composition ou du produit d'entretien ; avantageusement dans une teneur de 0,001 à 0,5 % en poids, de préférence dans une teneur de 0,005 à 0,1 % en poids, et tout particulièrement dans une teneur de 0,01 à 0,075 % en poids, par rapport au poids total de la composition ou du produit d'entretien ; et/ou
comprenant au moins un composant lipophile actif dans une teneur de 0,001 à 5,0 % en poids, avantageusement dans une teneur de 0,005 à 4,0 % en poids, et de préférence dans une teneur de 0,01 à 3,0 % en poids, par rapport au poids total de la composition.

7. Composition cosmétique ou pharmaceutique ou produit d'entretien selon l'une des revendications 1 à 6
- sous la forme d'une dispersion comprenant un composant huileux dans une teneur ≥ 1 % en poids, en particulier dans une teneur ≥ 3 % en poids, de préférence sous la forme d'une émulsion, en particulier une émulsion huile dans eau (O/W), une émulsion eau dans huile (W/O), une émulsion multiple, un gel hydrodispersé, un baume, une émulsion multiple de type eau dans huile (W/O/W) ou de type huile dans eau (O/W/O), une émulsion PIT, une émulsion Pickering, une micro-émulsion, un liquide, une lotion, une suspension, un lait, une pommade, une pâte, un gel, une formulation à base de crème, une formulation à base d'huile ou une formulation à base de liposomes ; ou
- sous la forme d'une formulation tensioactive liquide ; ou
- sous la forme d'une formulation tensioactive solide ; ou
- sous la forme d'une solution aqueuse ou aqueuse/alcoolique, en particulier aqueuse/éthanolique, ou aqueuse/glycolique, comprenant l'alcool ou le glycol dans une teneur de 0,1 % à < 50 % en poids, par rapport au poids total de la solution ;
en particulier, où la composition cosmétique ou pharmaceutique est une préparation à froid.

8. Utilisation cosmétique non thérapeutique de la composition cosmétique selon l'une des revendications 1 à 7, pour l'hygiène personnelle, la protection de la peau, les soins de la peau, la protection du cuir chevelu, les soins du cuir chevelu, les soins des cheveux, les soins des ongles, ou la prévention et/ou le traitement des affections cutanées, du vieillissement cutané, de la formation de rides, de la perte de volume cutané, de la perte d'élasticité de la peau, des taches pigmentaires, de la sécheresse cutanée, de la desquamation, de la peau grasse, pour l'hydratation de la peau, ou pour les soins aux animaux.

9. Composition pharmaceutique selon l'une des revendications 1 à 7, destinée à être utilisée comme médicament ; et de préférence destinée à être utilisée dans la prévention et/ou le traitement de l'intolérance ou de la sensibilité cutanée, de l'irritation cutanée, des rougeurs cutanées, des papules, des anomalies pigmentaires, de l'hypopigmentation et/ou de l'hyperpigmentation de la peau, des affections capillaires, cutanées et/ou des ongles des personnes, en particulier des maladies dermatologiques ou kératologiques, lesdites maladies dermatologiques ou kératologiques étant sélectionnées dans le groupe comprenant dermatite atopique (neurodermatite), psoriasis, exanthème acnéiforme, sébostase, xérose, eczéma, hyperséborrhée et hyposéborrhée, dermatite, rosacée, érythème, papules, prurit, inflammation, irritation, fibrose, lichen plan, pityriasis rosé, pityriasis versicolor, maladies bulleuses auto-immunes, urticaire, angio-œdème, réactions cutanées allergiques, cicatrisation des plaies, régénération tissulaire et dans le traitement des inflammations ou de la douleur.

10. Utilisation d'un mélange comprenant au moins un premier alcanediol linéaire et un ou plusieurs deuxièmes alcanediols linéaires,
- pour augmenter la solubilité d'un composant lipophile actif, de préférence un composant lipophile actif tel que défini en revendication 3, dans une composition cosmétique ou pharmaceutique ou un produit d'entretien, par rapport à une composition cosmétique ou pharmaceutique ou un produit d'entretien comprenant le composant lipophile actif sans utilisation d'un alcanediol, et/ou
- pour augmenter la concentration d'un composant lipophile actif, de préférence un composant lipophile actif tel que défini en revendication 3, dans une composition cosmétique ou pharmaceutique ou un produit d'entretien, par rapport à une composition cosmétique ou pharmaceutique ou un produit d'entretien comprenant le composant lipophile actif sans utilisation d'un alcanediol, et/ou
- pour réduire ou empêcher la formation de mousse d'une composition cosmétique ou pharmaceutique ou d'un produit d'entretien comprenant un agent solubilisant, par rapport à une composition cosmétique ou pharmaceutique ou un produit d'entretien comprenant l'agent solubilisant sans utilisation d'un alcanediol ;
ou
utilisation d'un mélange comprenant au moins un premier alcanediol linéaire et un ou plusieurs deuxièmes alcanediols linéaires comme substitut d'agent solubilisant dans une composition cosmétique ou pharmaceutique ou un produit d'entretien ;
où le mélange d'au moins un premier alcanediol linéaire avec un ou plusieurs deuxièmes alcanediols linéaires est sélectionné dans le groupe comprenant un mélange comprenant du 1,2-pentanediol et du 2,3-pentanediol ;
un mélange comprenant du 1,2-hexanediol et du 2,3-hexanediol ;
un mélange comprenant du 1,2-heptanediol et du 2,3-heptanediol ;
un mélange comprenant du 1,2-octanediol et du 2,3-octanediol ;
un mélange comprenant du 1,2-nonanediol et du 2,3-nonanediol ;
un mélange comprenant du 1,2-décanediol et du 2,3-décanediol ;
un mélange comprenant du 1,2-undécanediol et du 2,3-undécanediol ;
un mélange comprenant du 1,2-dodécanediol et du 2,3-dodécanediol ; et
un mélange comprenant du 1,2-tridécanediol et du 2,3-tridécanediol ;
ou
où le mélange comprenant au moins un premier alcanediol linéaire et un ou plusieurs deuxièmes alcanediols linéaires est sélectionné dans le groupe comprenant
un mélange comprenant du 1,2-hexanediol et du 2,3-octanediol ;
un mélange comprenant du 1,2-octanediol et du 2,3-hexanediol ; et
un mélange comprenant du 1,2-octanediol et du 2,3-heptanediol ;
ou
où le mélange comprenant au moins un premier alcanediol linéaire et un ou plusieurs deuxièmes alcanediols linéaires est sélectionné dans le groupe comprenant
un mélange comprenant du 1,2-pentanediol et du 2,3-hexanediol ;
un mélange comprenant du 1,2-pentanediol et du 2,3-heptanediol ;
un mélange comprenant du 1,2-pentanediol et du 2,3-octanediol ;
un mélange comprenant du 1,2-pentanediol et du 1,2-nonanediol ; et
un mélange comprenant du 1,2-pentanediol et du 2,3-nonanediol ;
ou
où le mélange comprenant au moins un premier alcanediol linéaire et un ou plusieurs deuxièmes alcanediols linéaires est sélectionné dans le groupe comprenant
un mélange comprenant du 1,2-heptanediol et du 1,2-octanediol ;
un mélange comprenant du 1,2-heptanediol et du 2,3-octanediol ;
un mélange comprenant du 1,2-heptanediol et du 1,2-nonanediol ; et
un mélange comprenant du 1,2-heptanediol et du 2,3-nonanediol.
